# EUROPÄISCHE PATENTSCHRIFT

(11) **EP 1 551 830 B1**
(45) Veröffentlichungstag und Bekanntmachung des Hinweises auf die Patenterteilung: **08.04.2009**
(21) Anmeldenummer: 03757806.9
(22) Anmeldetag: 09.09.2003
(51) Int. Cl.: C07D 413/06, A61K 31/404, A61P 43/00, C07D 403/06, C07D 401/06, C07D 209/34, C07D 405/06, C07D 403/14, C07D 405/14, C07D 401/14

(54) **HETEROCYCLISCH SUBSTITUIERTE INDOLINONE UND DEREN VERWENDUNG ALS REZEPTOR-TYROSINKINASEN INHIBITOREN**
HETEROCYCLICALLY SUBSTITUTED INDOLINONES AND THEIR USE AS RECEPTOR TYROSINE KINASE INHIBATORS
INDOLINONES A SUBTITUTION HETEROCYCLIQUE, ET LEUR UTILISATION EN TANT QU'INHIBITEURS DES RECEPTEURS DE TYROSINE KINASE

(30) Priorität: 12.09.2002 DE 10242350; 14.11.2002 DE 10252969
(43) Veröffentlichungstag der Anmeldung: 13.07.2005
(73) Patentinhaber: Boehringer Ingelheim Pharma GmbH & Co. KG, 55216 Ingelheim am Rhein (DE)
(72) Erfinder: KLEY, Jörg, 88441 Mittelbiberach (DE); HECKEL, Armin, 88400 Biberach (DE); HILBERG, Frank, A-1050 Wien (AT); ROTH, Gerald, Jürgen, 88400 Biberach (DE); LEHMANN-LINTZ, Thorsten, 88416 Ochsenhausen (DE); LOTZ, Ralf, R., H., 88433 Schemmerhofen (DE); TONTSCH-GRUNT, Ulrike, A-2500 Baden (AT); VAN MEEL, Jacobus, C., A., A-2340 Moedling (AT)
(86) Internationale Anmeldenummer: PCT/EP2003/009978
(87) Internationale Veröffentlichungsnummer: WO 2004/026829

(56) Entgegenhaltungen:
- WO-A-01/16130
- WO-A-01/27080
- WO-A-01/27081

## Beschreibung

Die vorliegende Erfindung betrifft neue heterocyclisch substituierte lndolinone der allgemeinen Formel 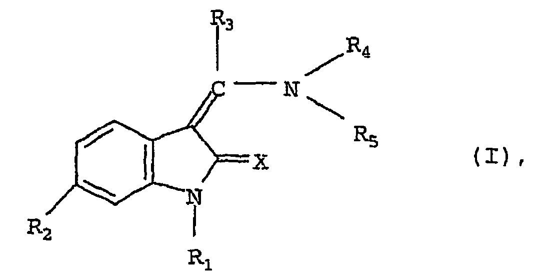 in denen R₁ bis R₅ und X wie in Anspruch 1 definiert sind, deren Tautomere, deren Diastereomere, deren Enantiomere, deren Gemische, deren Prodrugs und deren Salze, insbesondere deren physiologisch verträgliche Salze, welche wertvolle Eigenschaften aufweisen.

Die obigen Verbindungen der allgemeinen Formel I weisen wertvolle pharmakologische Eigenschaften auf, insbesondere eine inhibierende Wirkung auf verschiedene Kinasen, vor allem auf Rezeptor-Tyrosinkinasen wie VEGFR1, VEGFR2, VEGFR3, PDGFRα, PDGFRβ, FGFR1, FGFR3, EGFR, HER2, IGF1R und HGFR, sowie auf Komplexe von CDK's (Cyclin Dependent Kinases) wie CDK1, CDK2, CDK3, CDK4, CDK5, CDK6, CDK7, CDK8 und CDK9 mit ihren spezifischen Cyclinen (A, B1, B2, C, D1, D2, D3, E, F, G1, G2, H, I und K) und auf virales Cyclin (siehe L. Mengtao in J. Virology 71(3), 1984-1991 (1997)), sowie auf die Proliferation kultivierter humaner Zellen, insbesondere die von Endothelzellen, z.B. bei der Angiogenese, aber auch auf die Proliferation anderer Zellen, insbesondere von Tumorzellen.

Gegenstand der vorliegenden Erfindung sind somit die obigen Verbindungen der allgemeinen Formel I, welche wertvolle pharmakologische Eigenschaften aufweisen, die die pharmakologisch wirksamen Verbindungen enthaltenden Arzneimittel, deren Verwendung und Verfahren zu ihrer Herstellung.

In der obigen allgemeinen Formel I bedeuten
X ein Sauerstoffatom,
R₁ und R₅ jeweils ein Wasserstoffatom,
R₂ ein Fluor-, Brom- oder Chloratom,
R₃ eine 3,4-Methylendioxy-1-phenyl-, 3,4-Ethylendioxy-1-phenyl-, Chinoxalin-6-yl-, Benzimidazol-5-yl-, 2-Methylbenzimidazol-5-yl- oder 1-Methyl-benzimidazol-5-yl-gruppe, und
R₄ eine in 4-Position durch die Gruppe R₆ substituierte Phenylgruppe, die zusätzlich in 3-Position durch ein Fluor- oder Chloratom oder eine Methoxygruppe substituiert sein kann, wobei
- R₆: eine 1-(C₁₋₂-Alkyl)-imidazol-2-yl-gruppe,
eine 3,5-Dimethyl-pyrazol-1-yl-gruppe,
eine Pyrrolid-2-on-1-ylgruppe,
oder eine durch die Gruppe R₇ substituierte Methylgruppe bedeutet, wobei
R₇ eine Methylamino-, Ethylamino-, Isobutylamino-, Di-(C₁₋₂-alkyl)-amino-, N-(2-Hydroxyethyl)-methylamino-oder N-(2-Methoxyethyl)-methylaminogruppe,
eine Pyrrolidino-, 3-Hydroxypyrrolidino-, 2-Hydroxymethyl-pyrrolidino-, 4-Hydroxypiperidino-, Morpholino-, Piperazin-1-yl- oder 1-Methyl-piperazin-4-yl-gruppe, oder eine 1,2,4-Triazol-1-yl-, 1,2,3-Triazol-1-yl- oder 1,2,3-Triazol-2-yl-gruppe bedeutet, oder
- R₆: eine N-Acetyl-methylamino- oder N-Methoxyacetyl-methylaminogruppe,
eine Gruppe der Formel

-CO-R₈,

in der R₈ eine in 4-Stellung gegebenenfalls durch eine Methylgruppe substituierte Piperazino- oder Perhydro-1,4-diazepinogruppe bedeutet,
eine 4-Methyl-piperazin-1-yl-carbonyl-methyl-gruppe,
eine Gruppe der Formel

-CO-NR₉R₁₀,

in der
R₉ eine Methyl-, Cyanomethyl- oder Ethylgruppe, und
R₁₀ eine Methyl-, 1-Methylpiperidin-4-yl-, 2-Methylamino-ethyl-, 2-Dimethylamino-ethyl- oder 3-Dimethylamino-propylgruppe bedeuten,
eine Gruppe der Formel

-N(R₁₅)-CO-(CH₂)ₛ-NMe₂,

in der
s eine der Zahlen 1 oder 2, und
R₁₅ eine Methyl- oder Ethylgruppe oder, sofern n die Zahl 2 darstellt, auch eine 3-Pyridylgruppe bedeuten,
eine Gruppe der Formel

-N(R₁₅')-CO-(CH₂)ₛ-H,

in der
s eine der Zahlen 1 oder 2 und
R₁₅' eine 2-(Dimethylamino)-ethyl- oder 3-(Dimethylamino)-propylgruppe bedeuten,
eine Gruppe der Formel

-N(Me)-CO-(CH₂)ₛ-R₁₆',

in der
s eine der Zahlen 1 oder 2, und
R₁₆' eine Dimethylaminogruppe, oder, sofern s die Zahl 1 darstellt, auch eine 4-(C₁₋₂-Alkyl)-piperazin-1-yl-gruppe bedeuten,
eine Gruppe der Formel

-N(R₁₇)-SO₂-R₁₈,

in der a) R₁₇ eine Dimethylaminoethylgruppe und R₁₈ eine Methyl-, Ethyl- oder Propylgruppe bedeutet, oder
in der b) R₁₇ und R₁₈ jeweils eine Methylgruppe bedeuten,
eine Gruppe der Formel

-SO₂-N(R₂₀)-(CH₂)ᵤ-NMe₂,

in der
R₂₀ ein Wasserstoffatom oder eine Methylgruppe, und
u eine der Zahlen 2 oder 3 bedeuten,
eine Gruppe der Formel

-SO₂-R₂₆ ,

in der
R₂₆ eine Methylgruppe oder eine 2-Di-(C₁₋₂-alkyl)-amino-ethylgruppe bedeutet, oder
eine 2-Di-(C₁₋₂-alkyl)-amino-ethoxy-gruppe bedeutet,
wobei die in den vorstehend genannten Resten enthaltenen Dialkylaminogruppen zwei gleiche oder zwei unterschiedliche Alkylgruppen enthalten können,
deren Tautomere, deren Diastereomere, deren Enantiomere, deren Gemische und deren Salze.

Bevorzugte Verbindungen der allgemeinen Formel I sind:
(a) 3-(*Z*)-{1-[4-(Dimethylaminomethyl)-phenylamino]-1-(3,4-methylendioxyphenyl)-methylen}-6-chlor-2-indolinon
(b) 3-(*Z*)-{1-(4-[*N*-Methyl-*N*-(4-methylpiperazin-1-yl-methylcarbonyl)-amino]-phenylamino)-1-(chinoxalin-6-yl)-methylen}-6-chlor-2-indolinon
(c) 3-(*Z*)-{1-[4-(*N*-Ethyl-*N*-methyl-aminomethyl)-phenylamino]-1-{3,4-methylendioxyphenyl)-methylen}-6-chlor-2-indolinon
(d) 3-(*Z*)-{1-[4-(N-Methyl-N-{2-(dimethylamino)-ethyl-carbonyl}-amino)-phenylamino]-1-(3,4-ethylendioxyphenyl)-methylen}-6-fluor-2-indolinon
(e) 3-(*Z*)-{1-[4-(1,2,4-Triazol-1-yl-methyl)-phenylamino]-1-(1-methylbenzimidazol-5-yl)-methylen}-6-fluor-2-indolinon
(f) 3-(*Z*)-{1-[4-(Dimethylaminomethyl)-phenylamino]-1-(1-methyl-benzimidazol-5-yl)-methylen}-6-chlor-2-indolinon
(g) 3-(*Z*)-{1-[4-(Dimethylaminomethyl)-phenylamino]-1-(3,4-ethylendioxyphenyl)-methylen}-6-chlor-2-indolinon
(h) 3-(*Z*)-{1-(4-[*N*-Methansulfonyl-*N*-(2-dimethylaminoethyl)-amino]-phenylamino)-1-(3,4-ethylendioxyphenyl)-methylen}-6-chlor-2-indolinon
(k) 3-(*Z*)-{1-(4-[*N*-Acetyl-*N*-(2-dimethylaminoethyl)-amino]-phenylamino)-1-(3,4-ethylend ioxyphenyl)-methylen}-6-fluor-2-indolinon
(l) 3-(*Z*)-{1-[4-(Ethylaminomethyl)-phenylamino]-1-(3,4-ethylendioxyphenyl)-methylen}-6-fluor-2-indolinon
(m) 3-(*Z*)-{1-(4-[*N*-Acetyl-*N*-(3-dimethylaminopropyl)-amino]-phenylamino)-1-(3,4-ethylendioxyphenyl)-methylen}-6-chlor-2-indolinon
(n) 3-(*Z*)-{1-(4-[*N*-Propionyl-*N*-(3-dimethylaminopropyl)-amino]-phenylamino)-1-(3,4-ethylendioxyphenyl)-methylen}-6-chlor-2-indolinon
(o) 3-(*Z*)-{1-(4-[*N*-Propionyl-*N*-(2-dimethylaminoethyl)-amino]-phenylamino)-1-(3,4-ethylendioxyphenyl)-methylen}-6-chlor-2-indolinon
(p) 3-(*Z*)-{1-(4-[*N*-Acetyl-*N*-(2-dimethylaminoethyl)-amino]-phenylamino)-1-(3,4-ethylendioxyphenyl)-methylen}-6-chlor-2-indolinon
(q) 3-(*Z*)-{1-(4-[4-Methylpiperazin-1-yl-methyl]-phenylamino)-1-(3,4-methylendioxyphenyl)-methylen}-6-chlor-2-indolinon
(y) 3-(*Z*)-{1-[4-(Dimethylaminocarbonyl)-phenylamino]-1-(1-methylbenzimidazol-5-yl)-methylen}-6-fluor-2-indolinon
(z) 3-(*Z*)-{1-(4-[*N*-Propionyl-*N*-(3-dimethylaminopropyl)-amino]-phenylamino)-1-(3,4-ethylendioxyphenyl)-methylen}-6-fluor-2-indolinon
(aa) 3-(*Z*)-{1-(4-[*N*-Propionyl-*N*-(2-dimethylaminoethyl)-amino]-phenylamino)-1-(3,4-ethylendioxyphenyl)-methylen}-6-fluor-2-indolinon
(ab) 3-(*Z*)-{1-(4-[*N*-Methansulfonyl-*N*-(2-dimethylaminoethyl)-amino]-phenylamino)-1-(3,4-ethylendioxyphenyl)-methylen}-6-fluor-2-indolinon
(az) 3-(*Z*)-{1-(4-[*N*-Methyl-*N*-(4-methylpiperazin-1-yl-methylcarbonyl)-amino]-phenylamino)-1-(3,4-methylendioxyphenyl)-methylen}-6-chlor-2-indolinon
(be) 3-(*Z*)-{1-(4-[*N*-Methyl-*N*-(4-methylpiperazin-1-yl-methylcarbonyl)-amino]-phenylamino)-1-(3,4-methylendioxyphenyl)-methylen}-6-fluor-2-indolinon und
(bf) 3-(*Z*)-{1-(4-[*N*-Methyl-*N*-(4-methylpiperazin-1-yl-methylcarbonyl)-amino]-phenylamino)-1-(3,4-methylendioxyphenyl)-methylen}-6-brom-2-indolinon,
deren Tautomere, deren Diastereomere, deren Enantiomere, deren Gemische, und deren Salze.

Die vorliegende Erfindung betrifft auch physiologisch verträgliche Salze der Verbindungen gemäß den Ansprüchen 1 oder 2.

Die vorliegende Erfindung betrifft auch einen Arzneimittel, enthaltend eine Verbindung nach einem der Ansprüche 1 oder 2 oder ein Salz gemäß Anspruch 3 neben gegebenenfalls einem oder mehreren inerten Trägerstoffen und/oder Verdünnungsmitteln.

Die vorliegende Erfindung betrifft auch die Verwendung einer Verbindung nach einem der Ansprüche 1 oder 2 oder ein Salz gemäß Anspruch 3 zur Herstellung eines Arzneimittels, welches zur Behandlung von exzessiven oder anomalen Zellproliferationen geeignet ist.

Die vorliegende Erfindung betrifft auch ein Verfahren zur Herstellung eines Arzneimittels gemäß Anspruch 4 **dadurch gekennzeichnet, daß** auf nichtchemischem Wege eine Verbindung nach einem der Ansprüche 1 oder 2 oder ein Salz gemäß Anspruch 3 in einen oder mehrere inerte Trägerstoffe und/oder Verdünnungsmittel eingearbeitet wird.

Die vorliegende Erfindung betrifft auch ein Verfahren zur Herstellung der Verbindungen gemäß Anspruch 1 **dadurch gekennzeichnet, daß**
a. Eine Verbindung der allgemeinen Formel 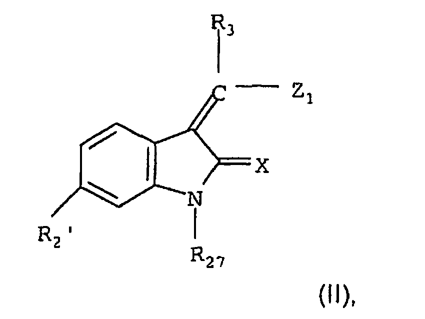 in der
   X und R₃ wie in Anspruch 1 erwähnt definiert sind,
   R₂' die für R₂ in Anspruch 1 erwähnten Bedeutungen besitzt,
   R₂₇ ein Wasserstoffatom oder eine Schutzgruppe für das Stickstoffatom der Lactamgruppe, wobei einer der Reste R₂' und R₂₇ auch eine gegebenenenfalls über einen Spacer gebildete Bindung an eine Festphase darstellen kann und der andere der Reste R₂' und R₂₇ die vorstehend erwähnten Bedeutungen besitzt, und Z₁ ein Halogenatom, eine Hydroxy-, Alkoxy- oder Aryl-alkoxygruppe bedeuten,
   mit einem Amin der allgemeinen Formel 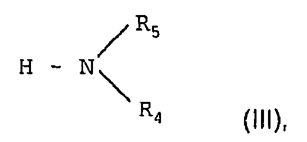 in der
   R₄ und R₅ wie in Anspruch 1 erwähnt definiert sind, umgesetzt wird, und erforderlichenfalls anschließend eine verwendete Schutzgruppe für das Stickstoffatom der Lactamgruppe oder eine so erhaltene Verbindung von einer Festphase abgespalten wird, oder
b. Zur Herstellung einer Verbindung der allgemeinen Formel 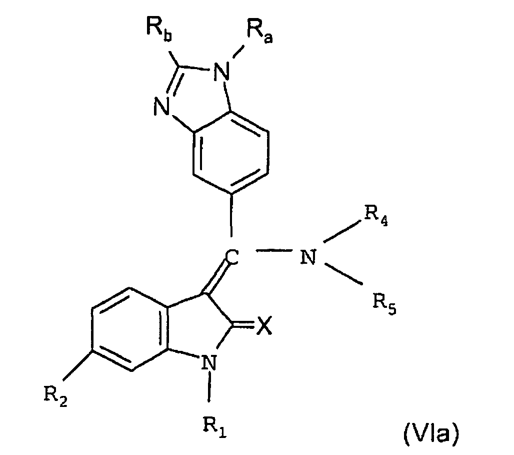 oder 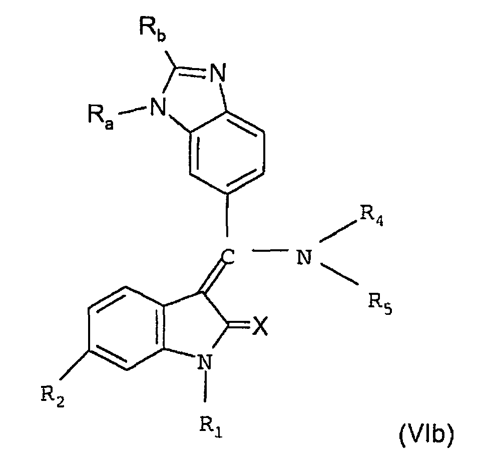 in der
   X, R₁, R₂, R₄ und R₅ wie in Anspruch 1 erwähnt definiert sind und
   Rₐ und R_{b} jeweils unabhängig voneinander ein Wasserstofftom, eine C₁₋₃-Alkylgruppe oder eine Carboxy-C₁₋₃-alkylgruppe sein können:
   eine Verbindung der allgemeinen Formel 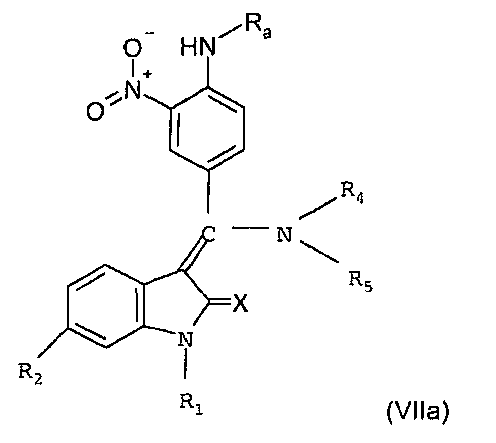
   beziehungsweise 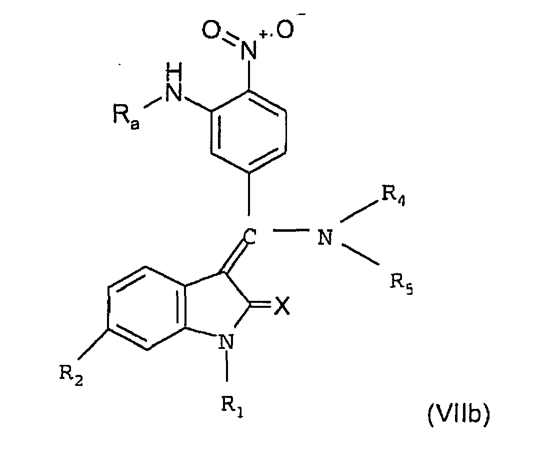
   in der
   X, R₁, R₂, R₄ und R₅ wie in Anspruch 1 erwähnt definiert sind und
   Rₐ ein Wasserstoffatom, eine C₁₋₃-Alkylgruppe oder eine gegebenenfalls geschützte Carboxy-C₁₋₃-alkylgruppe sein kann, reduziert wird, oder
c. Zur Herstellung einer Verbindung der allgemeinen Formel I, in der R₄ eine durch die Gruppe R₆ in 3- oder 4-Position substituierte Phenylgruppe, die zusätzlich wie in Anspruch 1 beschrieben substituiert sein kann, darstellt, und
R₈ eine durch R₇ substituierte C₁₋₃-Alkylgruppe bedeutet, wobei
R₇ eine Heteroarylgruppe, welche über einen Iminostickstoff gebunden ist,
eine Hydroxy- oder C₁₋₃-Alkoxygruppe,
eine Amino-, C₁₋₇-Alkylamino-, Di-(C₁₋₇-alkyl)-amino-, N-(C₁₋₇-Alkyl)-allylamino-, Phenylamino-, N-Phenyl-C₁₋₃-alkyl-amino-, Phenyl-C₁₋₃-alkylamino-, N-(C₁₋₃-Alkyl)-phenyl-C₁₋₃-alkylamino- oder Di-(phenyl-C₁₋₃-alkyl)-aminogruppe,
eine Allylaminogruppe, in der ein oder zwei vinylische Wasserstoffatome jeweils durch eine Methylgruppe ersetzt sein können,
eine ω-Hydroxy-C₂₋₃-alkyl-amino-, N-(C₁₋₃-Alkyl)-ω-hydroxy-C₂₋₃-alkyl-amino-, Di-(ω-hydroxy-C₂₋₃-alkyl)-amino-, N-(C₁₋₃-Alkyl)-[ω-(C₁₋₃-alkoxy)-C₂₋₃-alkyl]-amino-, Di-(ω-(C₁₋₃-alkoxy)-C₂₋₃-alkyl)-amino- oder N-(Dioxolan-2-yl)-C₁₋₃-alkyl-aminogruppe,
eine C₁₋₃-Alkyl-carbonylamino-C₂₋₃-alkyl-amino- oder C₁₋₃-Alkyl-carbonyl-amino-C₂₋₃-alkyl-N-(C₁₋₃-alkyl)-aminogruppe,
eine Pyridylaminogruppe,
eine C₁₋₃-Alkylsulfonylamino-, N-(C₁₋₃-Alkyl)-C₁₋₃-alkylsulfonylamino-, C₁₋₃-Alkylsulfonylamino-C₂₋₃-alkyl-amino- oder C₁₋₃-Alkylsulfonyl-amino-C₂₋₃-alkyl-N-(C₁₋₃-alkyl)-aminogruppe,
eine Hydroxycarbonyl-C₁₋₃-alkylamino- oder N-(C₁₋₃-Alkyl)-hydroxycarbonyl-C₁₋₃-alkyl-aminogruppe,
eine Guanidinogruppe, in der ein oder zwei Wasserstoffatome jeweils durch eine C₁₋₃-Alkylgruppe ersetzt sein können,
eine 2-Pyrrolidon-1-yl-gruppe, in der die der Carbonylgruppe benachbarte Methylengruppe durch ein Sauerstoffatom oder durch eine -NH- oder -N(C₁₋₃-Alkyl)- Gruppe ersetzt sein kann,
eine Gruppe der Formel

-N(R₁₁)-CO-(CH₂)ₚ-R₁₂ ,

in der
R₁₁ ein Wasserstoffatom oder eine Allyl-, C₁₋₃-Alkyl-, C₁₋₃-Alkyl-amino-C₂₋₃-alkyl- oder Di-(C₁₋₃-alkyl)-amino-C₂₋₃-alkylgruppe,
p eine der Zahlen 0, 1, 2 oder 3 und
R₁₂ eine Amino-, C₁₋₄-Alkylamino-, Allylamino-, Di-allyl-amino-, Di-(C₁₋₄-alkyl)-amino-, Phenylamino-, N-(C₁₋₄-Alkyl)-phenylamino-, Benzylamino-, N-(C₁₋₄-Alkyl)-benzylamino-, C₁₋₄-Alkoxy- oder 2,5-Dihydropyrrol-1-yl-gruppe oder
eine 4- bis 7-gliedrige Cycloalkyleniminogruppe,
wobei jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Allyl)-, -N(Phenyl)-, -N(C₁₋₃-Alkyl-carbonyl)- oder -N(Benzoyl)- Gruppe ersetzt sein kann,
oder, sofern n eine der Zahlen 1, 2 oder 3 darstellt, auch ein Wasserstoffatom bedeuten,
eine Gruppe der Formel

-N(R₁₃)-(CH₂)_{q}-(CO)ᵣ-R₁₄ ,

in der
R₁₃ ein Wasserstoffatom oder eine C₁₋₃-Alkyl-, Allyl-, C₁₋₃-Alkylcarbonyl-, Arylcarbonyl-, Pyridylcarbonyl-, Phenyl-C₁₋₃-alkyl-carbonyl-, C₁₋₃-Alkylsulfonyl-, Arylsulfonyl- oder Phenyl-C₁₋₃-alkylsulfonylgruppe,
q eine der Zahlen 1, 2, 3 oder 4,
r die Zahl 1 oder, sofern q eine der Zahlen 2, 3 oder 4 ist, auch die Zahl 0 und
R₁₄ eine Hydroxy-, Amino-, C₁₋₄-Alkylamino-, Di-(C₁₋₄-alkyl)-amino-, Phenylamino-, N-(C₁₋₄-Alkyl)-phenylamino-, Benzylamino-, N-(C₁₋₄-Alkyl)-benzylamino-, C₁₋₄-Alkoxy- oder C₁₋₃-Alkoxy-C₁₋₃-alkoxygruppe,
eine in 1-Position gegebenenfalls durch eine C₁₋₃-Alkylgruppe substituierte Di-(C₁₋₄-alkyl)-amino-C₁₋₃-alkylaminogruppe oder
eine 4- bis 7-gliedrige Cycloalkyleniminogruppe,
wobei der Cycloalkylenteil mit einem Phenylring kondensiert sein kann oder
jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Phenyl)-, -N(C₁₋₃-Alkyl-carbonyl)- oder -N(Benzoyl)- Gruppe ersetzt sein kann, bedeuten,
eine C₄₋₇-Cycloalkylamino-, C₄₋₇-Cycloalkyl-C₁₋₃-alkylamino- oder C₄₋₇-Cycloalkenylaminogruppe, in der die Position 1 des Rings nicht an der Doppelbindung beteiligt ist und wobei die vorstehend genannten Gruppen jeweils zusätzlich am Aminstickstoffatom durch eine C₅₋₇-Cycloalkyl-, C₂₋₄-Alkenyl- oder C₁₋₄-Alkylgruppe substituiert sein können,
eine 2,5-Dihydro-pyrrol-1-yl-gruppe oder
eine 4- bis 7-gliedrige Cycloalkyleniminogruppe, in der
der Cycloalkylenteil mit einer Phenylgruppe oder mit einer gegebenenfalls durch ein Fluor-, Chlor-, Brom- oder lodatom, durch eine Nitro-, C₁₋₃-Alkyl-, C₁₋₃-Alkoxy- oder Aminogruppe substituierten Oxazolo-, Imidazolo-, Thiazolo-, Pyridino-, Pyrazino- oder Pyrimidinogruppe kondensiert sein kann oder/und
ein oder zwei Wasserstoffatome jeweils durch eine C₁₋₃-Alkyl-, C₃₋₇-Cycloalkyl-, Hydroxy-, C₁₋₃-Alkoxy-, Hydroxy-C₁₋₃-alkyl- oder C₁₋₃-Alkoxy-C₁₋₃-alkyl- oder Phenylgruppe ersetzt sein können oder/und
die Methylengruppe in Position 3 einer 5-gliedrigen Cycloalkyleniminogruppe durch eine Hydroxy-, Hydroxy-C₁₋₃-alkyl-, C₁₋₃-Alkoxy- oder C₁₋₃-Alkoxy-C₁₋₃-alkylgruppe substituiert sein kann,
jeweils die Methylengruppe in Position 3 oder 4 einer 6- oder 7-gliedrigen Cycloalkyleniminogruppe durch eine Hydroxy-, Hydroxy-C₁₋₃-alkyl-, C₁₋₃-Alkoxy-, C₁₋₃-Alkoxy-C₁₋₃-alkyl-, Carboxy-, C₁₋₄-Alkoxycarbonyl-, Aminocarbonyl-, C₁₋₃-Alkylaminocarbonyl-, Di-(C₁₋₃-alkyl)-aminocarbonyl-, Phenyl-C₁₋₃-alkylamino- oder N-(C₁₋₃-Alkyl)-phenyl-C₁₋₃-alkylaminogruppe substituiert oder
die Methylengruppe in 4-Position einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Allyl)-, -N(Phenyl)-, -N(Phenyl-C₁₋₃-alkyl)-, -N(C₁₋₃-Alkyl-carbonyl)-, -N(C₁₋₄-Hydroxy-carbonyl)-, -N(C₁₋₄-Alkoxy-carbonyl)-, - N(Benzoyl)- oder -N(Phenyl-C₁₋₃-alkyl-carbonyl)- Gruppe ersetzt sein kann,
wobei eine mit einem Imino-Stickstoffatom der Cycloalkyleniminogruppe verknüpfte Methylengruppe durch eine Carbonyl- oder Sulfonylgruppe ersetzt sein kann oder in einer 5- bis 7-gliedrigen monocyclischen oder mit einer Phenylgruppe kondensierten Cycloalkyleniminogruppe beide mit dem Imino-Stickstoffatom verknüpften Methylengruppen jeweils durch eine Carbonylgruppe ersetzt sein können,
bedeutet:
eine Verbindung der allgemeinen Formel 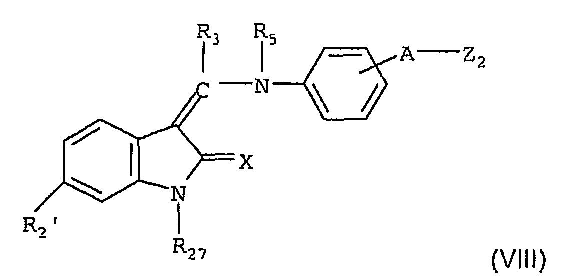 in der
R₃, R₅ und X wie in Anspruch 1 erwähnt definiert sind,
R₂' die für R₂ in Anspruch 1 erwähnten Bedeutungen besitzt,
R₂₇ ein Wasserstoffatom oder eine Schutzgruppe für das Stickstoffatom der Lactamgruppe, wobei einer der Reste R₂' und R₂₇ auch eine gegebenenenfalls über einen Spacer gebildete Bindung an eine Festphase darstellen kann und der andere der Reste R₂' und R₂₇ die vorstehend erwähnten Bedeutungen besitzt,
A eine C₁₋₃-Alkylgruppe und
Z₂ eine Austrittsgruppe darstellt, mit einem Amin der allgemeinen Formel

H-R_{7'} (IX),

in der
R_{7'} die vorstehend für R₇ genannten Bedeutungen besitzt, umgesetzt wird und erforderlichenfalls anschließend eine verwendete Schutzgruppe für das Stickstoffatom der Lactamgruppe oder eine so erhaltene Verbindung von einer Festphase abgespalten wird, und/oder
gewünschtenfalls anschließend eine so erhaltene Verbindung der allgemeinen Formel I, die eine Alkoxycarbonylgruppe enthält, mittels Hydrolyse in eine entsprechende Carboxyverbindung übergeführt wird oder
eine so erhaltene Verbindung der allgemeinen Formel I, die eine Amino- oder Alkylaminogruppe enthält, mittels reduktiver Alkylierung in eine entsprechende Alkylamino- oder Dialkylaminoverbindung übergeführt wird oder
eine so erhaltene Verbindung der allgemeinen Formel I, die eine Amino- oder Alkylaminogruppe enthält, mittels Acylierung oder Sulfonierung in eine entsprechende Acyl- oder Sulfonylverbindung übergeführt wird oder
eine so erhaltene Verbindung der allgemeinen Formel I, die eine Carboxygruppe enthält, mittels Veresterung oder Amidierung in eine entsprechende Ester- oder Aminocarbonylverbindung übergeführt wird oder
eine so erhaltene Verbindung der allgemeinen Formel I, die eine Cycloalkyleniminogruppe enthält, in der eine Methylengruppe durch ein Schwefelatom ersetzt ist, mittels Oxidation in eine entsprechende Sulfinyl- oder Sulfonylverbindung übergeführt wird, oder
eine so erhaltene Verbindung der allgemeinen Formel I, die eine Nitrogruppe enthält, mittels Reduktion in eine entsprechende Aminoverbindung übergeführt wird oder
eine so erhaltene Verbindung der allgemeinen Formel I, in der R₄ eine durch eine Amino-, Alkylamino-, Aminoalkyl- oder N-Alkyl-aminogruppe substituierte Phenylgruppe darstellt, mittels Umsetzung mit einem entsprechenden Cyanat, Isocyanat oder Carbamoylhalogenid in eine entsprechende Harnstoffverbindung der allgemeinen Formel I übergeführt wird oder
eine so erhaltene Verbindung der allgemeinen Formel I, in der R₄ eine durch eine Amino-, Alkylamino-, Aminoalkyl- oder N-Alkyl-aminogruppe substituierte Phenylgruppe darstellt, mittels Umsetzung mit einer entsprechenden die Amidinogruppe übertragenden Verbindung oder durch Umsetzung mit einem entsprechenden Nitril in eine entsprechende Guanidinoverbindung der allgemeinen Formel I übergeführt wird oder
erforderlichenfalls ein während den Umsetzungen zum Schutze von reaktiven Gruppen verwendeter Schutzrest abgespalten wird oder
gewünschtenfalls anschließend eine so erhaltene Verbindung der allgemeinen Formel I in ihre Stereoisomere aufgetrennt wird oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.
Offenbart werden Verbindungen der allgemeinen Formel I in denen
X ein Sauerstoff- oder Schwefelatom,
R₁ ein Wasserstoffatom oder einen Prodrugrest wie eine C₁₋₄-Alkoxy-carbonyl- oder C₂₋₄-Alkanoylgruppe,
R₂ ein Wasserstoff-, Fluor-, Chlor- oder Bromatom,
eine Cyano- oder Nitrogruppe,
eine Carboxygruppe, eine lineare oder verzweigte C₁₋₆-Alkoxy-carbonylgruppe, eine C₃₋₆-Cycloalkoxy-carbonyl- oder eine Aryloxycarbonylgruppe,
eine gegebenenfalls durch eine oder zwei Methylgruppen substituierte Allyloxycarbonylgruppe,
eine lineare oder verzweigte C₁₋₄-Alkoxy-carbonylgruppe, die im Alkylteil terminal durch eine Phenyl-, Heteroaryl-, Carboxy-, C₁₋₃-Alkoxy-carbonyl-, Aminocarbonyl-, C₁₋₃-Alkylamino-carbonyl- oder Di-(C₁₋₃-alkyl)-aminocarbonylgruppe substituiert ist,
eine lineare oder verzweigte C₂₋₆-Alkoxy-carbonylgruppe, die im Alkylteil terminal durch ein Chloratom oder eine Hydroxy-, C₁₋₃-Alkoxy-, Amino-, C₁₋₃-Alkylamino-oder Di-(C₁₋₃-alkyl)-aminogruppe substituiert ist, oder
eine Aminocarbonyl-, C₁₋₄-Alkyl-aminocarbonyl- oder eine Di-(C₁₋₄-alkyl)-amino-carbonylgruppe, wobei die Alkylgruppen, sofern sie mehr als ein Kohlenstoffatom besitzen, terminal durch eine Hydroxy-, C₁₋₃-Alkoxy- oder Di-(C₁₋₃-alkyl)-aminogruppe substituiert sein können,
R₃ eine fünf- oder sechsgliedrige Heteroarylgruppe, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-, Carboxy-C₁₋₃-alkyl- oder Phenyl-C₁₋₃-alkylgruppe substituierte Iminogruppe, ein Sauerstoff- oder Schwefelatom oder
eine gegebenenfalls durch eine C₁₋₃-Alkyl-, Carboxy-C₁₋₃-alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe, ein Sauerstoffatom oder ein Schwefelatom und zwei Stickstoffatome enthält,
und außerdem an die vorstehend erwähnten monocyclischen heterocyclischen Gruppen über zwei benachbarte Kohlenstoffatome ein Phenylring ankondensiert sein kann, das Wasserstoffatom einer Methingruppe durch eine C₁₋₃-Alkyl-, Carboxy-C₁₋₃-alkyl-, Amino-, C₁₋₃-Alkylamino-, Di-(C₁₋₃-alkyl)-amino-, Phenyl-C₁₋₃-alkyl-amino- oder Di-(phenyl-C₁₋₃-alkyl)-aminogruppe ersetzt sein kann und die Bindung über ein Kohlenstoffatom des heterocyclischen Teils erfolgt,
ein 5- bis 6-gliedriger cyclischer Oximether, der über das dem Stickstoffatom benachbarte Kohlenstoffatom mit der Methylidengruppe verknüpft ist,
eine Imidazo[1,2-a]pyridin-6-yl- oder Imidazo[1,2-a]pyridin-7-yl-Gruppe oder eine bicyclische Gruppe bestehend aus
einem Phenylring, der mit der Methylidengruppe verknüpft ist, und
einer -O-CH₂-CH₂-, -O-CH₂-O-, -O-CF₂-O-, -O-CH₂-CH₂-O-, -O-CH=CH-O-, -S-CH=N-, -NH-CH=N-, -N=C(C₁₋₃-Alkyl)-NH-, -N=C(Carboxy-C₁₋₃-alkyl)-NH-, -N(C₁₋₃-Alkyl)-CH=N-, -N(Carboxy-C₁₋₃-alkyl)-CH=N-, -N(C₁₋₃-Alkyl)-C(C₁₋₃-Alkyl)=N-, -N=CH-CH=N-, -N=CH-N=CH-, -N=CH-N=C(C₁₋₃-Alkyl)-, -N=CH-N=C(Carboxy-C₁₋₃-alkyl)-, -N=CH-CH=CH-, -N=CH-CH=C(C₁₋₃-Alkyl)-, -N=CH-CH=C(Carboxy-C₁₋₃-alkyl)-, -N=N-NH-, -N=N-N(C₁₋₃-Alkyl)-, -N=N-N(Carboxy-C₁₋₃-alkyl)-, -CH=CH-NH-, -CH=CH-N(C₁₋₃-Alkyl)-, -CH=CH-N(Carboxy-C₁₋₃-alkyl)-, -N=CH-C(O)-N(C₁₋₃-Alkyl)-, -O-CH₂-C(O)-N(C₁₋₃-Alkyl)-, -CH=N-N=CH-, -O-C(O)-CH₂-N(C₁₋₃-Alkyl)-, -O-CH₂-C(O)-NH-, -O-CH₂-CH₂-N(C₁₋₃-Alkyl)-, -O-C(O)-N(C₁₋₃-Alkyl)-, -O-C(O)-NH-, -CO-NH-CO- oder -CO-N(C₁₋₃-Alkyl)-CO-Brücke, die jeweils mit zwei benachbarten Kohlenstoffatomen des Phenylrings verknüpft ist,
wobei das Wasserstoffatom einer ggf. in R₃ enthaltenen Carboxygruppe durch einen Prodrug-Rest ersetzt sein kann,
R₄ eine C₃₋₇-Cycloalkylgruppe,
wobei die Methylengruppe in Position 4 einer 6- oder 7-gliedrigen Cycloalkylgruppe durch eine Amino-, C₁₋₃-Alkylamino- oder Di-(C₁₋₃-alkyl)-aminogruppe substituiert oder durch eine -NH- oder -N(C₁₋₃-Alkyl)- Gruppe ersetzt sein kann,
oder eine durch die Gruppe R₆ in 3- oder 4-Position substituierte Phenylgruppe, die zusätzlich durch Fluor-, Chlor-, Brom- oder lodatome, durch C₁₋₅-Alkyl-, Trifluormethyl-, Hydroxy-, C₁₋₃-Alkoxy-, Carboxy-, C₁₋₃-Alkoxycarbonyl-, Amino-, Acetylamino-, C₁₋₃-Alkyl-sulfonylamino-, Aminocarbonyl-, C₁₋₃-Alkyl-aminocarbonyl-, Di-(C₁₋₃-alkyl)-aminocarbonyl-, Aminosulfonyl-, C₁₋₃-Alkyl-aminosulfonyl-, Di-(C₁₋₃-alkyl)-aminosulfonyl-, Nitro- oder Cyanogruppen mono- oder disubstituiert sein kann, wobei die Substituenten gleich oder verschieden sein können und wobei
R₆ ein Wasserstoff-, Fluor-, Chlor-, Brom- oder lodatom,
eine Cyano-, Nitro-, Amino-, C₁₋₅-Alkyl-, C₃₋₇-Cycloalkyl-, Trifluormethyl-, Phenyl-oder Heteroarylgruppe,
eine gegebenenfalls durch eine C₁₋₃-Alkylgruppe substituierte Tetrazolylgruppe,
eine 2-Pyrrolidon-1-yl-gruppe, in der die der Carbonylgruppe benachbarte Methylengruppe durch ein Sauerstoffatom oder eine -NH- oder -N(C₁₋₃-Alkyl)-Gruppe ersetzt sein kann,
die Gruppe der Formel 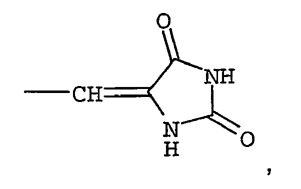 in der die an ein Stickstoffatom gebundenen Wasserstoffatome unabhängig voneinander jeweils durch eine C₁₋₃-Alkylgruppe ersetzt sein können,
eine Gruppe der Formel

-(CH₂)ₙ-CO-R₈

in der
R₈ eine Hydroxy- oder C₁₋₄-Alkoxygruppe,
eine 5- bis 7-gliedrige Cycloalkyleniminogruppe,
wobei die Methylengruppe in Position 3 oder 4 einer 5-, 6- oder 7-gliedrigen Cycloalkyleniminogruppe durch eine Amino-, C₁₋₃-Alkylamino- oder Di-(C₁₋₃-alkyl)-aminogruppe substituiert sein kann
oder die Methylengruppe in Position 4 einer 6- oder 7-gliedrigen Cycloalkyleniminogruppe durch ein Sauerstoffatom, ein Schwefelatom, eine Sulfinyl- oder Sulfonylgruppe, eine -NH-, -N(Allyl)-oder -N(C₁₋₃-Alkyl)- Gruppe ersetzt sein kann
und wobei in den genannten cyclischen Gruppen ein oder zwei Wasserstoffatome durch eine C₁₋₃-Alkylgruppe ersetzt sein können,
eine 2,5-Dihydropyrrol-1-yl-gruppe oder
eine C₃₋₇-Cycloalkyl-gruppe,
wobei die Methylengruppe in Position 3 oder 4 des 5-, 6- oder 7-gliedrigen Cycloalkylteils durch eine Amino-, C₁₋₃-Alkylamino- oder Di-(C₁₋₃-alkyl)-aminogruppe substituiert oder
die Methylengruppe in Position 4 des 6- oder 7-gliedrigen Cycloalkylteils durch eine -NH-, -N(Allyl)- oder -N(C₁₋₃-Alkyl)- Gruppe ersetzt sein kann,
und n eine der Zahlen 0, 1 oder 2 bedeuten,
eine Gruppe der Formel

-(CH₂)ₒ-CO-NR₉R₁₀ ,

in der
R₉ ein Wasserstoffatom,
eine Allylgruppe,
eine gegebenenfalls durch eine Cyano-, Carboxy-, Phenyl- oder Pyridylgruppe substituierte C₁₋₄-Alkylgruppe oder
eine terminal durch eine Hydroxy- oder C₁₋₃-Alkoxygruppe substituierte C₂₋₄-Alkylgruppe,
R₁₀ ein Wasserstoffatom,
eine C₁₋₃-Alkylgruppe,
eine terminal durch eine Hydroxy-, C₁₋₃-Alkoxy-, C₁₋₃-Alkylamino- oder Di-(C₁₋₃-alkyl)-aminogruppe substituierte C₂₋₃-Alkylgruppe oder
eine 3- bis 7-gliedrige Cycloalkylgruppe,
in der eine Methylengruppe durch ein Sauerstoffatom oder eine -NH-oder -N(C₁₋₃-Alkyl)- Gruppe ersetzt sein kann und unabhängig davon eine Methylengruppe durch eine Carbonylgruppe ersetzt sein kann,
und o eine der Zahlen 0, 1 oder 2 bedeuten,
eine durch die Gruppe R₇ substituierte C₁₋₃-Alkylgruppe, wobei
R₇ eine C₃₋₇-Cycloalkylgruppe,
wobei eine der Methylengruppen durch eine Amino-, C₁₋₃-Alkylamino- oder Di-(C₁₋₃-alkyl)-aminogruppe substituiert oder
die Methylengruppe in Position 3 oder 4 einer 5-, 6- oder 7-gliedrigen Cycloalkylgruppe durch eine -NH-, -N(Allyl)- oder -N(C₁₋₃-Alkyl)- Gruppe ersetzt sein kann oder
in einer 5- bis 7-gliedrigen Cycloalkylgruppe eine -(CH₂)₂-Gruppe durch eine -CO-NH-Gruppe ersetzt sein kann, eine -(CH₂)₃-Gruppe durch eine -NH-CO-NH- oder -CO-NH-CO-Gruppe ersetzt sein kann oder eine -(CH₂)₄-Gruppe durch eine -NH-CO-NH-CO-Gruppe ersetzt sein kann, wobei jeweils ein an ein Stickstoffatom gebundenes Wasserstoffatom durch eine C₁₋₃-Alkylgruppe ersetzt sein kann,
eine Aryl- oder Heteroarylgruppe,
eine Triazolylgruppe,
eine Hydroxy- oder C₁₋₃-Alkoxygruppe,
eine Amino-, C₁₋₇-Alkylamino-, Di-(C₁₋₇-alkyl)-amino-, N-(C₁₋₇-Alkyl)-allylamino-, Phenylamino-, N-Phenyl-C₁₋₃-alkyl-amino-, Phenyl-C₁₋₃-alkylamino-, N-(C₁₋₃-Alkyl)-phenyl-C₁₋₃-alkylamino- oder Di-(phenyl-C₁₋₃-alkyl)-aminogruppe,
eine Allylaminogruppe, in der ein oder zwei vinylische Wasserstoffatome jeweils durch eine Methylgruppe ersetzt sein können,
eine ω-Hydroxy-C₂₋₃-alkyl-amino-, N-(C₁₋₃-Alkyl)-ω-hydroxy-C₂₋₃-alkyl-amino-, Di-(ω-hydroxy-C₂₋₃-alkyl)-amino-, ω-(C₁₋₃-Alkoxy)-C₂₋₃-alkyl-amino-N-(C₁₋₃-Alkyl)-[ω-(C₁₋₃-alkoxy)-C₂₋₃-alkyl]-amino-, Di-[ω-(C₁₋₃-alkoxy)-C₂₋₃-alkyl]-amino- oder N-(Dioxolan-2-yl)-C₁₋₃-alkyl-aminogruppe,
eine C₁₋₃-Alkylcarbonylamino-C₂₋₃-alkyl-amino- oder C₁₋₃-Alkylcarbonylamino-C₂₋₃-alkyl-N-(C₁₋₃-alkyl)-aminogruppe,
eine Pyridylaminogruppe,
eine C₁₋₃-Alkylsulfonylamino-, N-(C₁₋₃-Alkyl)-C₁₋₃-alkylsulfonylamino-, C₁₋₃-Alkyl-sulfonylamino-C₂₋₃-alkyl-amino- oder C₁₋₃-Alkylsulfonylamino-C₂₋₃-alkyl-N-(C₁₋₃-alkyl)-aminogruppe,
eine Hydroxycarbonyl-C₁₋₃-alkylamino- oder N-(C₁₋₃-Alkyl)-hydroxycarbonyl-C₁₋₃-alkyl-aminogruppe,
eine Guanidinogruppe, in der ein oder zwei Wasserstoffatome jeweils durch eine C₁₋₃-Alkylgruppe ersetzt sein können,
eine 2-Pyrrolidon-1-yl-gruppe, in der die der Carbonylgruppe benachbarte Methylengruppe durch ein Sauerstoffatom oder durch eine -NH- oder -N(C₁₋₃-Alkyl)- Gruppe ersetzt sein kann,
eine Gruppe der Formel

-N(R₁₁)-CO-(CH₂)ₚ-R₁₂ ,

in der
R₁₁ ein Wasserstoffatom oder eine Allyl-, C₁₋₃-Alkyl-, C₁₋₃-Alkyl-amino-C₂₋₃-alkyl- oder Di-(C₁₋₃-alkyl)-amino-C₂₋₃-alkylgruppe,
p eine der Zahlen 0, 1, 2 oder 3 und
R₁₂ eine Amino-, C₁₋₄-Alkylamino-, Allylamino-, Di-allyl-amino-, Di-(C₁₋₄-alkyl)-amino-, Phenylamino-, N-(C₁₋₄-Alkyl)-phenylamino-, Benzylamino-, N-(C₁₋₄-Alkyl)-benzylamino-, C₁₋₄-Alkoxy- oder 2,5-Dihydropyrrol-1-yl-gruppe oder
eine 4- bis 7-gliedrige Cycloalkyleniminogruppe,
wobei jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Allyl)-, -N(Phenyl)-, -N(C₁₋₃-Alkyl-carbonyl)- oder -N(Benzoyl)- Gruppe ersetzt sein kann,
oder, sofern n eine der Zahlen 1, 2 oder 3 darstellt, auch ein Wasserstoffatom bedeuten,
eine Gruppe der Formel

-N(R₁₃)-(CH₂)_{q}-(CO)ᵣ-R₁₄ ,

in der
R₁₃ ein Wasserstoffatom oder eine C₁₋₃-Alkyl-, Allyl-, C₁₋₃-Alkyl-carbonyl-, Arylcarbonyl-, Pyridylcarbonyl-, Phenyl-C₁₋₃-alkyl-carbonyl-, C₁₋₃-Alkylsulfonyl-, Arylsulfonyl- oder Phenyl-C₁₋₃-alkylsulfonylgruppe,
q eine der Zahlen 1, 2, 3 oder 4,
r die Zahl 1 oder, sofern q eine der Zahlen 2, 3 oder 4 ist, auch die Zahl 0 und
R₁₄ eine Hydroxy-, Amino-, C₁₋₄-Alkylamino-, Di-(C₁₋₄-alkyl)-amino-, Phenylamino-, N-(C₁₋₄-Alkyl)-phenylamino-, Benzylamino-, N-(C₁₋₄-Alkyl)-benzylamino-, C₁₋₄-Alkoxy- oder C₁₋₃-Alkoxy-C₁₋₃-alkoxygruppe,
eine in 1-Position gegebenenfalls durch eine C₁₋₃-Alkylgruppe substituierte Di-(C₁₋₄-alkyl)-amino-C₁₋₃-alkylaminogruppe oder
eine 4- bis 7-gliedrige Cycloalkyleniminogruppe,
wobei der Cycloalkylenteil mit einem Phenylring kondensiert sein kann oder
jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Phenyl)-, -N(C₁₋₃-Alkyl-carbonyl)- oder -N(Benzoyl)- Gruppe ersetzt sein kann, bedeuten,
eine C₄₋₇-Cycloalkylamino-, C₄₋₇-Cycloalkyl-C₁₋₃-alkylamino- oder C₄-Cyclo-alkenylaminogruppe, in der die Position 1 des Rings nicht an der Doppelbindung beteiligt ist und wobei die vorstehend genannten Gruppen jeweils zusätzlich am Aminstickstoffatom durch eine C₅₋₇-Cycloalkyl-, C₂₋₄-Alkenyl-oder C₁₋₄-Alkylgruppe substituiert sein können,
eine 2,5-Dihydro-pyrrol-1-yl-gruppe oder
eine 4- bis 7-gliedrige Cycloalkyleniminogruppe, in der
der Cycloalkylenteil mit einer Phenylgruppe oder mit einer gegebenenfalls durch ein Fluor-, Chlor-, Brom- oder lodatom, durch eine Nitro-, C₁₋₃-Alkyl-, C₁₋₃-Alkoxy- oder Aminogruppe substituierten Oxazolo-, Imidazolo-, Thiazolo-, Pyridino-, Pyrazino- oder Pyrimidinogruppe kondensiert sein kann oder/und
ein oder zwei Wasserstoffatome jeweils durch eine C₁₋₃-Alkyl-, C₃₋₇Cycloalkyl-, Hydroxy-, C₁₋₃-Alkoxy-, Hydroxy-C₁₋₃-alkyl-, C₁₋₃-Alkoxy-C₁₋₃-alkyl-oder Phenylgruppe ersetzt sein können oder/und
die Methylengruppe in Position 3 einer 5-gliedrigen Cycloalkyleniminogruppe durch eine Hydroxy-, Hydroxy-C₁₋₃-alkyl-, C₁₋₃-Alkoxy- oder C₁₋₃-Alkoxy-C₁₋₃-alkylgruppe substituiert sein kann,
jeweils die Methylengruppe in Position 3 oder 4 einer 6- oder 7-gliedrigen Cycloalkyleniminogruppe durch eine Hydroxy-, Hydroxy-C₁₋₃-alkyl-, C₁₋₃-Alkoxy-, C₁₋₃-Alkoxy-C₁₋₃-alkyl-, Carboxy-, C₁₋₄-Alkoxycarbonyl-, Aminocarbonyl-, C₁₋₃-Alkylaminocarbonyl-, Di-(C₁₋₃-alkyl)-aminocarbonyl-, Di-(C₁₋₃-alkyl)-amino-, Phenyl-C₁₋₃-alkylamino- oder N-(C₁₋₃-Alkyl)-phenyl-C₁₋₃-alkylaminogruppe substituiert oder
die Methylengruppe in 4-Position einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Allyl)-, -N(Phenyl)-, -N(Phenyl-C₁₋₃-alkyl)-, -N(C₁₋₃-Alkyl-carbonyl)-, N(C₁₋₄-Hydroxy-carbonyl)-, -N(C₁₋₄-Alkoxy-carbonyl)-, -N(Benzoyl)- oder -N(Phenyl-C₁₋₃-alkylcarbonyl)- Gruppe ersetzt sein kann,
wobei eine mit einem Imino-Stickstoffatom der Cycloalkyleniminogruppe verknüpfte Methylengruppe durch eine Carbonyl- oder Sulfonylgruppe ersetzt sein kann oder in einer 5- bis 7-gliedrigen monocyclischen oder mit einer Phenylgruppe kondensierten Cycloalkyleniminogruppe beide mit dem Imino-Stickstoffatom verknüpften Methylengruppen jeweils durch eine Carbonylgruppe ersetzt sein können,
bedeutet,
oder R₆ eine Gruppe der Formel

-N(R₁₅)-CO-(CH₂)ₛ-R₁₆ ,

in der
R₁₅ ein Wasserstoffatom, eine Allyl-, C₁₋₆-Alkyl-, C₃₋₇-Cycloalkyl- oder Pyridinylgruppe,
eine terminal durch eine Phenyl-, Heteroaryl-, Trifluormethyl-, Aminocarbonyl-, C₁₋₄-Alkylamino-carbonyl-, Di-(C₁₋₄-alkyl)-amino-carbonyl-, C₁₋₃-Alkyl-carbonyl-, C₁₋₃-Alkyl-aminosulfonyl- oder Di-(C₁₋₃-alkyl)-aminosulfonylgruppe substituierte C₁₋₃-Alkylgruppe oder
eine terminal durch eine Hydroxy-, C₁₋₃-Alkoxy-, Amino-, C₁₋₃-Alkylamino-, Allylamino-, Di-(C₁₋₃-alkyl)-amino-, C₁₋₃-Alkyl-sulfonylamino- oder N-(C₁₋₃-Alkyl)-C₁₋₃-alkyl-sulfonylaminogruppe substituierte C₂₋₃-Alkylgruppe und
s eine der Zahlen 0, 1, 2 oder 3 darstellen und
R₁₆ die Bedeutungen der vorstehend erwähnten Gruppe R₇ annimmt oder
eine Carboxygruppe bedeutet oder,
sofern s eine der Zahlen 1, 2 oder 3 darstellt, auch ein Wasserstoffatom bedeutet,
eine Gruppe der Formel

-N(R₁₇)-SO₂-R₁₈ ,

in der
R₁₇ ein Wasserstoffatom,
eine Allyl-, C₁₋₄-Alkyl- oder Cyanomethylgruppe oder
eine terminal durch eine Cyano-, Amino-, C₁₋₃-Alkylamino-, Di-(C₁₋₃-alkyl)-amino-, Trifluormethyl-carbonyl-amino- oder N-(C₁₋₃-Alkyl)-trifluormethyl-carbonyl-aminogruppe substituierte C₂₋₄-Alkylgruppe und
R₁₈ eine C₁₋₄-Alkyl-, Phenyl- oder Pyridylgruppe bedeuten,
eine durch eine Di-(C₁₋₃-alkyl)-amino-C₁₋₃-alkyl-carbonyl- oder Di-(C₁₋₃-alkyl)-amino-C₁₋₃-alkyl-sulfonylgruppe und eine Di-(C₁₋₃-alkyl)-aminocarbonyl-C₁₋₃-alkylgruppe substituierte Aminogruppe,
oder eine Gruppe der Formel

-A-(CH₂)ₜ-R₁₉ ,

in der
A ein Sauerstoff- oder Schwefelatom oder eine Sulfinyl- oder Sulfonylgruppe,
R₁₉ ein Wasserstoffatom, eine Hydroxy-, C₁₋₃-Alkoxy-, Aryl-, Heteroaryl-, Amino-, C₁₋₄-Alkylamino- oder Di-(C₁₋₃-alkyl)-aminogruppe
oder eine 4- bis 7-gliedrige Cycloalkyleniminogruppe,
wobei die Methylengruppe in Position 3 einer 5-gliedrigen Cycloalkyleniminogruppe durch eine Hydroxy-, Hydroxy-C₁₋₃-alkyl-, C₁₋₃-Alkoxy-C₁₋₃-alkylgruppe substituiert sein kann,
jeweils die Methylengruppe in Position 3 oder 4 einer 6- oder 7-gliedrigen Cycloalkyleniminogruppe durch eine Hydroxy-, Hydroxy-C₁₋₃-alkyl-, C₁₋₃-Alkoxy-, C₁₋₃-Alkoxy-C₁₋₃-alkyl-, Carboxy-, C₁₋₄-Alkoxycarbonyl-, Aminocarbonyl-, C₁₋₃-Alkylaminocarbonyl- oder Di-(C₁₋₃-alkyl)-amino-carbonylgruppe substituiert oder
durch ein Sauerstoff- oder Schwefelatom, durch eine Sulfinyl-, Sulfonyl-, -NH- oder -N(C₁₋₃-Alkyl-)- Gruppe ersetzt sein kann, und
t eine der Zahlen 2 oder 3 oder,
sofern R₁₉ ein Wasserstoffatom, eine Aryl- oder Heteroarylgruppe darstellt, auch die Zahl 1 oder,
sofern A eine Sulfonylgruppe darstellt, auch die Zahl 0 bedeuten,
oder eine Gruppe der Formel

-SO₂-N(R₂₀)-(CH₂)ᵤ-R₂₁ ,

in der
R₂₀ ein Wasserstoffatom, eine Allyl- oder C₁₋₃-Alkylgruppe,
R₂₁ ein Wasserstoffatom, eine Hydroxy-, C₁₋₃-Alkoxy-, Amino-, C₁₋₃-Alkylamino- oder eine Di-(C₁₋₃-alkyl)-aminogruppe und
u eine der Zahlen 2, 3 oder 4 oder,
sofern R₂₁ ein Wasserstoffatom ist, auch die Zahl 1 bedeuten,
wobei alle in den unter R₆ genannten Resten enthaltenen einfach gebundenen oder ankondensierten Phenylgruppen durch Fluor-, Chlor-, Brom- oder lodatome, durch C₁₋₅-Alkyl-, Trifluormethyl-, Hydroxy-, C₁₋₃-Alkoxy-, Carboxy-, C₁₋₃-Alkoxy-carbonyl-, Aminocarbonyl-, C₁₋₃-Alkylamino-carbonyl-, Di-(C₁₋₃-alkyl)-amino-carbonyl-, Aminosulfonyl-, C₁₋₃-Alkyl-aminosulfonyl-, Di-(C₁₋₃-alkyl)-aminosulfonyl-, C₁₋₃-Alkyl-sulfonylamino-, Nitro- oder Cyanogruppen mono- oder disubstituiert sein können, wobei die Substituenten gleich oder verschieden sein können, oder zwei benachbarte Wasserstoffatome der Phenylgruppen durch eine Methylendioxygruppe ersetzt sein können,
oder R₄ eine Gruppe der Formel 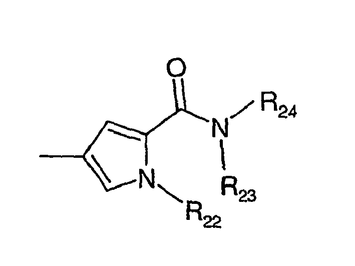 in der
R₂₂ eine C₁₋₃-Alkylgruppe,
R₂₃ ein Wasserstoffatom,
eine Allylgruppe,
eine gegebenenfalls durch eine Cyano-, Carboxy-, Phenyl- oder Pyridylgruppe substituierte C₁₋₄-Alkylgruppe oder
eine terminal durch eine Hydroxy- oder C₁₋₃-Alkoxygruppe substituierte C₂₋₄-Alkylgruppe und
R₂₄ ein Wasserstoffatom,
eine C₁₋₃-Alkylgruppe,
eine terminal durch eine Hydroxy-, C₁₋₃-Alkoxy-, C₁₋₃-Alkylamino- oder Di-(C₁₋₃-alkyl)-aminogruppe substituierte C₂₋₃-Alkylgruppe ,
oder eine 3-7-gliedrige Cycloalkylgruppe,
wobei eine Methylengruppe durch ein Sauerstoffataom oder durch eine -NH- oder -N(C₁₋₃-Alkyl)- Gruppe ersetzt sein kann und unabhängig davon eine Methylengruppe durch eine Carbonylgruppe ersetzt sein kann, bedeuten
oder R₂₃ und R₂₄ zusammen mit dem Stickstoffatom, mit dem sie verknüpft sind, bilden
eine 2,5-Dihydro-pyrrol-1-yl-gruppe oder
eine 5- bis 7-gliedrige Cycloalkyleniminogruppe,
wobei die Methylengruppe in Position 4 einer 6- oder 7-gliedrigen Cycloalkyleniminogruppe durch eine Amino-, C₁₋₃-Alkylamino- oder Di-(C₁₋₃-alkyl)-aminogruppe substituiert oder durch ein Sauerstoffatom, ein Schwefelatom, eine Sulfinyl- oder Sulfonylgruppe oder eine -NH- oder -N(C₁₋₃-Alkyl)- Gruppe ersetzt sein kann
und wobei ein oder zwei Wasserstoffatome in der 5- bis 7-gliedrigen Cycloalkyleniminogruppe durch eine C₁₋₃-Alkylgruppe ersetzt sein können,
und
R₅ ein Wasserstoffatom oder eine C₁₋₃-Alkylgruppe,
wobei unter dem Ausdruck eine Arylgruppe eine gegebenenfalls durch ein Fluor-, Chlor-, Brom- oder lodatom, durch eine Cyano-, Trifluormethyl-, Nitro-, Carboxy-, Aminocarbonyl-, C₁₋₃-Alkyl- oder C₁₋₃-Alkoxygruppe mono- oder disubstituierte Phenyl- oder Naphthylgruppe und
unter dem Ausdruck eine Heteroarylgruppe, soweit nicht anders angegeben, eine im Kohlenstoffgerüst gegebenenfalls durch eine oder zwei C₁₋₃-Alkylgruppen substituierte monocyclische 5- oder 6-gliedrige Heteroarylgruppe, wobei
die 6-gliedrige Heteroarylgruppe ein, zwei oder drei Stickstoffatome und
die 5-gliedrige Heteroarylgruppe eine gegebenenfalls durch eine C₁₋₃-Alkyl-oder Phenyl-C₁₋₃-alkylgruppe substituierte Iminogruppe, ein Sauerstoff- oder Schwefelatom oder
eine gegebenenfalls durch eine C₁₋₃-Alkyl- oder Phenyl-C₁₋₃-alkylgruppe substituierte Iminogruppe oder ein Sauerstoff- oder Schwefelatom und zusätzlich ein Stickstoffatom oder
eine gegebenenfalls durch eine C₁₋₃-Alkyl- oder Phenyl-C₁₋₃-alkylgruppe substituierte Iminogruppe und zwei Stickstoffatome enthält,
und außerdem an die vorstehend erwähnten monocyclischen heterocyclischen Gruppen über zwei benachbarte Kohlenstoffatome ein Phenylring ankondensiert sein kann, das Wasserstoffatom einer oder zweier Methingruppen durch eine C₁₋₃-Alkyl-, Amino-, C₁₋₃-Alkyl-amino- oder Di-(C₁₋₃-alkyl)-aminogruppe ersetzt sein kann und die Bindung über ein Stickstoffatom oder über ein Kohlenstoffatom des heterocyclischen Teils oder eines ankondensierten Phenylrings erfolgt,
zu verstehen ist,
die Wasserstoffatome in den vorstehend genannten Alkyl- und Alkoxygruppen oder in den in vorstehend definierten Gruppen der Formel I enthaltenen Alkylteilen teilweise oder ganz durch Fluoratome ersetzt sein können,
die in den vorstehend definierten Gruppen vorhandenen gesättigten Alkyl- und - Alkoxyteile, die mehr als zwei Kohlenstoffatome enthalten, auch deren verzweigte Isomere, wie beispielsweise die Isopropyl-, tert.Butyl-, Isobutylgruppe, einschließen, sofern nichts anderes erwähnt wurde, und
wobei zusätzlich das Wasserstoffatom einer vorhandenen Carboxygruppe oder ein an ein Stickstoffatom gebundenes Wasserstoffatom, beispielsweise einer Amino-, Alkylamino- oder Iminogruppe oder eines gesättigten N-Heterocyclus wie der Piperidinylgruppe, jeweils durch einen in-vivo abspaltbaren Rest ersetzt sein kann.

Unter einem von einer Imino- oder Aminogruppe in-vivo abspaltbaren Rest ist beispielsweise eine Hydroxygruppe, eine Acylgruppe wie die Benzoyl- oder Pyridinoylgruppe oder eine C₁₋₁₆-Alkanoylgruppe wie die Formyl-, Acetyl-, Propionyl-, Butanoyl-, Pentanoyl- oder Hexanoylgruppe, eine Allyloxycarbonylgruppe, eine C₁₋₁₆-Alkoxycarbonylgruppe wie die Methoxycarbonyl-, Ethoxycarbonyl-, Propoxy-carbonyl-, Isopropoxycarbonyl-, Butoxycarbonyl-, tert.Butoxycarbonyl-, Pentoxycarbonyl-, Hexyloxycarbonyl-, Octyloxycarbonyl-, Nonyloxycarbonyl-, Decyloxycarbonyl-, Undecyloxycarbonyl-, Dodecyloxycarbonyl- oder Hexadecyloxycarbonylgruppe, eine Phenyl-C₁₋₆-alkoxycarbonylgruppe wie die Benzyloxycarbonyl-, Phenylethoxycarbonyl- oder Phenylpropoxycarbonylgruppe, eine C₁₋₃-Alkylsulfonyl-C₂₋₄-alkoxycarbonyl-, C₁₋₃-Alkoxy-C₂₋₄-alkoxy-C₂₋₄-alkoxycarbonyl- oder RₑCO-O-(R_{f}CR_{g})-O-CO-Gruppe, in der
Rₑ eine C₁₋₈-Alkyl-, C₅₋₇-Cycloalkyl-, Phenyl- oder Phenyl-C₁₋₃-alkylgruppe,
R_{f} ein Wasserstoffatom, eine C₁₋₃-Alkyl-, C₅₋₇-Cycloalkyl- oder Phenylgruppe und
R₉ ein Wasserstoffatom oder eine C₁₋₃-Alkylgruppe darstellen,
wobei zusätzlich für eine Aminogruppe die Phthalimidogruppe in Betracht kommt, zu verstehen, wobei die vorstehend erwähnten Esterreste ebenfalls als in-vivo in eine Carboxygruppe überführbare Gruppe verwendet werden können.

Als bevorzugte Prodrug-Reste, die das Wasserstoffatom einer Carboxygruppe ersetzen können, kommen eine C₁₋₆-Alkylgruppe wie die Methyl-, Ethyl-, n-Propyl-, isopropyl-, n-Butyl-, n-Pentyl-, n-Hexyl- oder Cyclohexylgruppe oder Phenyl-C₁₋₃-alkylgruppe wie die Benzylgruppe in Betracht.

Offenbart werden auch Verbindungen der allgemeinen Formel I, in denen X ein Sauerstoffatom,
R₁ ein Wasserstoffatom oder einen Prodrugrest wie eine C₁₋₄-Alkoxy-carbonyl- oder C₂₋₄-Alkanoylgruppe,
R₂ ein Wasserstoff-, Fluor-, Chlor- oder Bromatom,
eine Cyano- oder Nitrogruppe,
eine Carboxygruppe, eine lineare oder verzweigte C₁₋₄-Alkoxy-carbonylgruppe oder eine C₃₋₄-Cycloalkoxy-carbonylgruppe,
eine gegebenenfalls durch eine oder zwei Methylgruppen substitutierte Allyloxycarbonylgruppe,
eine lineare oder verzweigte C₂₋₃-Alkoxy-carbonylgruppe, die im Alkylteil terminal durch eine Hydroxy- oder C₁₋₃-Alkoxygruppe substituiert ist, oder
eine Aminocarbonyl-, C₁₋₄-Alkyl-aminocarbonyl- oder eine Di-(C₁₋₃-alkyl)-amino-carbonylgruppe, wobei die Alkylgruppen, sofern sie mehr als ein Kohlenstoffatom besitzen, terminal durch eine C₁₋₃-Alkoxygruppe substituiert sein können,
R₃ eine 2-Pyrrolyl-, 3-Pyrrolyl-, 1-(C₁₋₃-Alkyl)-3-pyrrolyl-,1-(Carboxy-C₁₋₃-alkyl)-3-pyrrolyl-, 2-Furyl-, 3-Furyl-, 2-Thienyl-, 3-Thienyl-, 2-(Carboxy-C₁₋₃-alkyl)-thien-5-yl-, 2-(Carboxy-C₁₋₃-alkyl)-thien-4-yl-, 3-(Carboxy-C₁₋₃-alkyl)-thien-5-yl-, 4-Imidazol-, 1-(C₁₋₃-Alkyl)-5-imidazolyl-, 1-(C₁₋₃-Alkyl)-4-imidazolyl-, 1-Benzyl-5-imidazolyl-, 5-(C₁₋₃-Alkyl)-isoxazol-3-yl-, 3-Pyridyl-, 4-Pyridyl-, 2-(Carboxy-C₁₋₃-alkyl)-pyridin-5-yl-, 3-(Carboxy-C₁₋₃-alkyl)-pyridin-5-yl-, 2-(Carboxy-C₁₋₃-alkyl)-pyridin-4-yl-, 2-Pyrazinyl-, 4-Pyridazinyl-Gruppe oder
eine Pyrazol-3-yl-Gruppe,
in welcher unabhängig voneinander die 1- und/oder 5-Position jeweils durch eine C₁₋₃-Alkyl-oder Carboxy-C₁₋₃-alkylgruppe substituiert sein kann,
ein 5- bis 6-gliedriger cyclischer Oximether, der über das dem Stickstoffatom benachbarte Kohlenstoffatom mit der Methylidengruppe verknüpft ist,
eine Imidazo[1,2-a]pyridin-6-yl- oder Imidazo[1,2-a]pyridin-7-yl-Gruppe
oder eine bicyclische Gruppe bestehend aus
einem Phenylring, der mit der Methylidengruppe verknüpft ist, und
einer -O-CH₂-CH₂-, -O-CH₂-O-, -O-CF₂-O-, -O-CH₂-CH₂-O-, -O-CH=CH-O-, -S-CH=N-,
-NH-CH=N-, -N=C(C₁₋₃-Alkyl)-NH-, -N=C(Carboxy-C₁₋₃-alkyl)-NH-, -N(C₁₋₃-Alkyl)-CH=N-, -N(Carboxy-C₁₋₃-alkyl)-CH=N-, -N(C₁₋₃-Alkyl)-C(C₁₋₃-Alkyl)=N-, -N=CH-CH=N-, -N=CH-N=CH-, -N=CH-N=C(C₁₋₃-Alkyl)-, -N=CH-CH=CH-, -N=CH-CH=C(C₁₋₃-Alkyl)-, -CH=N-N=CH-, -CH=CH-NH-, -CH=CH-N(C₁₋₃-Alkyl)-, -N=N-NH-, -N=N-N(C₁₋₃-Alkyl)-, -O-CH₂-C(O)-N(C₁₋₃-Alkyl)-, -O-C(O)-CH₂-N(C₁₋₃-Alkyl)-, -O-C(O)-N(C₁₋₃-Alkyl)-, -O-C(O)-NH-, -O-CH₂-CH₂-N(C₁₋₃-Alkyl)-, oder -CO-N(C₁₋₃-Alkyl)-CO-Brücke, die jeweils mit zwei benachbarten Kohlenstoffatomen des Phenylrings verknüpft ist,
wobei das Wasserstoffatom einer ggf. in R₃ enthaltenen Carboxygruppe durch einen Prodrug-Rest ersetzt sein kann,
R₄ eine durch die Gruppe R₆ in 3- oder 4-Position substituierte Phenylgruppe, die zusätzlich in einer noch verbleibenden 3-, 4- oder 5-Position durch ein Fluor-, Chlor-, Brom- oder lodatom oder durch eine C₁₋₃-Alkyl-, Trifluormethyl-, Hydroxy-, C₁₋₃-Alkoxy-, Amino-, Nitro- oder Cyanogruppe substituiert sein kann, wobei
R₆ ein Wasserstoff-, Fluor-, Chlor-, Brom- oder Iodatom,
eine gegebenenfalls durch eine C₁₋₃-Alkylgruppe substituierte Tetrazolylgruppe,
eine am Iminostickstoff und/oder an einem Kohlenstoffatom durch eine C₁₋₃-Alkylgruppe substituierte Imidazolylgruppe,
eine am Iminostickstoff und/oder an einem oder zwei Kohlenstoffatomen jeweils unabhängig voneinander durch eine C₁₋₃-Alkylgruppe substituierte Pyrazolylgruppe,
eine 2-Pyrrolidon-1-yl-gruppe, in der die der Carbonylgruppe benachbarte Methylengruppe durch ein Sauerstoffatom oder eine -NH- oder -N(C₁₋₃-Alkyl)-Gruppe ersetzt sein kann,
eine Gruppe der Formel

-(CH₂)ₙ-CO-R₈ ,

in der
R₈ eine Hydroxygruppe,
eine 2,5-Dihydropyrrol-1-yl-gruppe oder
eine 5- bis 7-gliedrige Cycloalkyleniminogruppe,
wobei die Methylengruppe in Position 3 oder 4 einer 5-, 6- oder 7-gliedrigen Cycloalkyleniminogruppe durch eine Amino-, C₁₋₃-Alkylamino- oder Di-(C₁₋₃-alkyl)-aminogruppe substituiert sein kann
oder die Methylengruppe in Position 4 einer 6- oder 7-gliedrigen Cycloalkyleniminogruppe durch ein Sauerstoffatom, ein Schwefelatom, eine Sulfinyl- oder Sulfonylgruppe, eine -NH- oder -N(C₁₋₃-Alkyl)- Gruppe ersetzt sein kann
und wobei in den genannten cyclischen Gruppen ein oder zwei Wasserstoffatome durch eine C₁₋₃-Alkylgruppe ersetzt sein können,
und n eine der Zahlen 0 oder 1 bedeuten,
eine Gruppe der Formel

-(CH₂)ₒ-CO-NR₉R₁₀ ,

in der
R₉ ein Wasserstoffatom,
eine Allylgruppe,
eine gegebenenfalls durch eine Cyano- oder Carboxygruppe substituierte C₁₋₄-Alkylgruppe oder
eine terminal durch eine Hydroxy- oder C₁₋₃-Alkoxygruppe substituierte C₂₋₄-Alkylgruppe,
R₁₀ ein Wasserstoffatom,
eine C₁₋₃-Alkylgruppe,
eine terminal durch eine Hydroxy-, C₁₋₃-Alkoxy-, C₁₋₃-Alkylamino- oder Di-(C₁₋₃-alkyl)-aminogruppe substituierte C₂₋₃-Alkylgruppe oder
eine 3- bis 7-gliedrige Cycloalkylgruppe,
in der eine Methylengruppe durch ein Sauerstoffatom oder eine -NH-oder -N(C₁₋₃-Alkyl)- Gruppe ersetzt sein kann,
und o eine der Zahlen 0 oder 1 bedeuten,
eine durch die Gruppe R₇ substituierte C₁₋₂-Alkylgruppe, wobei
R₇ eine C₃₋₇-Cycloalkylgruppe,
wobei die Methylengruppe in Position 4 einer 6- oder 7-gliedrigen Cycloalkylgruppe durch eine -NH- oder -N(C₁₋₃-Alkyl)- Gruppe ersetzt sein kann oder
eine gegebenenfalls durch eine C₁₋₃-Alkylgruppe substituierte Pyridyl- oder Imidazolylgruppe,
eine Triazolylgruppe,
eine Hydroxy- oder C₁₋₃-Alkoxygruppe,
eine Amino-, C₁₋₄-Alkylamino-, Di-(C₁₋₃-alkyl)-amino-, N-(C₁₋₃-Alkyl)-allylamino-, Phenyl-C₁₋₂-alkylamino- oder N-(C₁₋₃-Alkyl)-phenyl-C₁₋₂-alkylaminogruppe,
eine Allylaminogruppe, in der ein oder zwei vinylische Wasserstoffatome jeweils durch eine Methylgruppe ersetzt sein können,
eine ω-Hydroxy-C₂₋₃-alkyl-amino-, N-(C₁₋₃-Alkyl)-ω-hydroxy-C₂₋₃-alkyl-amino-, Di-(ω-hydroxy-C₂₋₃-alkyl)-amino-, ω-(C₁₋₃-Alkoxy)-C₂₋₃-alkyl-amino-N-(C₁₋₃-Alkyl)-[ω-(C₁₋₃-alkoxy)-C₂₋₃-alkyl]-amino- oder Di-[ω-(C₁₋₃-alkoxy)-C₂₋₃-alkyl]-aminogruppe,
eine Pyridylaminogruppe,
eine N-(C₁₋₃-Alkyl)-C₁₋₃-alkylsulfonylaminogruppe,
eine Hydroxycarbonyl-C₁₋₃-alkylamino- oder N-(C₁₋₃-Alkyl)-hydroxycarbonyl-C₁₋₃-alkyl-aminogruppe,
eine 2-Pyrrolidon-1-yl-gruppe, in der die der Carbonylgruppe benachbarte Methylengruppe durch ein Sauerstoffatom oder eine -NH- oder -N(C₁₋₃-Alkyl)-Gruppe ersetzt sein kann,
eine Gruppe der Formel

-N(R₁₁)-CO-(CH₂)ₚ-R₁₂ ,

in der
R₁₁ ein Wasserstoffatom oder eine Allyl-, C₁₋₃-Alkylgruppe, C₁₋₃-Alkylamino-C₂₋₃-alkyl- oder Di-(C₁₋₃-alkyl)-amino-C₂₋₃-alkylgruppe,
p eine der Zahlen 0, 1 oder 2 und
R₁₂ eine Amino-, C₁₋₃-Alkylamino-, Allylamino-, Di-(C₁₋₂-alkyl)-amino-, C₁₋₃-Alkoxy- oder 2,5-Dihydro-pyrrol-1-yl-gruppe oder
eine 4- bis 7-gliedrige Cycloalkyleniminogruppe,
wobei jeweils die Methylengruppe in Position 4 einer 6- oder 7-gliedrigen Cycloalkyleniminogruppe durch ein Sauerstoff- oder Schwefelatom, durch eine -NH-, -N(Allyl)- oder -N(C₁₋₃-Alkyl)- Gruppe ersetzt sein kann,
oder, sofern n eine der Zahlen 1 oder 2 darstellt, auch ein Wasserstoffatom bedeuten,
eine Gruppe der Formel

-N(R₁₃)-(CH₂)_{q}-(CO)ᵣ-R₁₄,

in der
R₁₃ ein Wasserstoffatom oder eine C₁₋₃-Alkyl- oder Pyridylcarbonylgruppe,
q eine der Zahlen 1 oder 2,
r die Zahl 1 oder, sofern q die Zahl 2 ist, auch die Zahl 0 und
R₁₄ eine Hydroxy-, Amino-, C₁₋₃-Alkylamino-, Di-(C₁₋₃-alkyl)-amino-, C₁₋₃-Alkoxygruppe oder
eine 4- bis 7-gliedrige Cycloalkyleniminogruppe,
wobei jeweils die Methylengruppe in Position 4 einer 6- oder 7-gliedrigen Cycloalkyleniminogruppe durch ein Sauerstoff- oder Schwefelatom, durch eine -NH- oder -N(C₁₋₃-Alkyl)- Gruppe ersetzt sein kann, bedeuten,
eine C₄₋₇-Cycloalkylamino-, C₃₋₅-Cycloalkyl-C₁₋₂-alkylamino- oder C₄₋₇-Cyclo-alkenylaminogruppe, in der die Position 1 des Rings nicht an der Doppelbindung beteiligt ist und wobei die vorstehend genannten Gruppen jeweils zusätzlich am Aminstickstoffatom durch eine C₂₋₄-Alkenyl- oder C₁₋₃-Alkylgruppe substituiert sein können,
eine 2,5-Dihydro-pyrrol-1-yl-gruppe oder
eine 4- bis 7-gliedrige Cycloalkyleniminogruppe, in der
ein oder zwei Wasserstoffatome jeweils durch eine C₁₋₃-Alkyl-, Hydroxy-, C₁₋₃-Alkoxy-, Hydroxy-C₁₋₃-alkyl- oder C₁₋₃-Alkoxy-C₁₋₃-alkylgruppe ersetzt sein können oder/und
jeweils die Methylengruppe in Position 3 oder 4 einer 6- oder 7-gliedrigen Cycloalkyleniminogruppe durch eine Carboxy-, Aminocarbonyl-, C₁₋₃-Alkylaminocarbonyl- oder Di-(C₁₋₃-alkyl)-aminocarbonylgruppe substituiert oder
die Methylengruppe in 4-Position einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl-)-, -N(Allyl)- oder -N(C₁₋₃-Alkyl-carbonyl)- Gruppe ersetzt sein kann,
wobei eine mit einem Imino-Stickstoffatom der Cycloalkyleniminogruppe verknüpfte Methylengruppe durch eine Carbonyl- oder Sulfonylgruppe ersetzt sein kann,
bedeutet,
oder R₆ eine Gruppe der Formel

-N(R₁₅)-CO-(CH₂)ₛ-R₁₆,

in der
R₁₅ ein Wasserstoffatom, eine Allyl-, C₁₋₄-Alkyl-, C₃₋₅-Cycloalkyl- oder Pyridinylgruppe,
eine terminal durch eine Pyridyl-, Trifluormethyl- oder Di-(C₁₋₂-alkyl)-amino-carbonylgruppe substituierte C₁₋₃-Alkylgruppe oder
eine terminal durch eine Hydroxy-, C₁₋₃-Alkoxy-, Amino-, C₁₋₃-Alkylamino- oder Di-(C₁₋₃-alkyl)-aminogruppe substituierte C₂₋₃-Alkylgruppe und
s eine der Zahlen 0, 1, 2 oder 3 darstellen und
R₁₆ eine Hydroxy-, C₁₋₃-Alkoxy-, Carboxy-, Amino-, C₁₋₃-Alkylamino-, Di-(C₁₋₃-alkyl)-amino-, 2,5-Dihydropyrrol-1-yl- oder Pyridinylgruppe oder eine 5- bis 7-gliedrige Cycloalkyleniminogruppe bedeutet,
wobei die Methylengruppe in Position 3 einer 5-gliedrigen Cycloalkyleniminogruppe durch eine Hydroxy-, Hydroxy-C₁₋₃-alkyl- oder C₁₋₃-Alkoxy-C₁₋₃-alkylgruppe substituiert sein kann,
jeweils die Methylengruppe in Position 3 oder 4 einer 6- oder 7-gliedrigen Cycloalkyleniminogruppe durch eine Di-(C₁₋₃-alkyl)-amino-, Hydroxy-, Hydroxy-C₁₋₃-alkyl-, C₁₋₃-Alkoxy- oder C₁₋₃-Alkoxy-C₁₋₃-alkylgruppe substituiert oder
die Methylengruppe in 4-Position einer 6- oder 7-gliedrigen Cycloalkyleniminogruppe durch ein Sauerstoff- oder Schwefelatom, durch eine -NH-oder -N(C₁₋₃-Alkyl-)- Gruppe ersetzt sein kann,
oder, sofern s eine der Zahlen 1, 2 oder 3 darstellt, auch ein Wasserstoffatom bedeutet,
eine Gruppe der Formel

-N(R₁₇)-SO₂-R₁₈,

in der
R₁₇ ein Wasserstoffatom,
eine C₁₋₃-Alkyl- oder Cyanomethylgruppe oder
eine terminal durch eine Cyano-, Amino-, C₁₋₃-Alkylamino- oder Di-(C₁₋₃-alkyl)-aminogruppe substituierte C₂₋₃-Alkylgruppe und
R₁₈ eine C₁₋₄-Alkyl- oder Pyridylgruppe bedeuten,
oder eine Gruppe der Formel

-A-(CH₂)ₜ-R₁₉ ,

in der
A ein Sauerstoff-oder Schwefelatom oder eine Sulfinyl- oder Sulfonylgruppe,
R₁₉ ein Wasserstoffatom oder eine Hydroxy-, C₁₋₃-Alkoxy-, Amino-, C₁₋₄-Alkylamino- oder Di-(C₁₋₃-alkyl)-aminogruppe
oder eine 4- bis 7-gliedrige Cycloalkyleniminogruppe,
wobei jeweils die Methylengruppe in Position 3 oder 4 einer 6- oder 7-gliedrigen durch ein Sauerstoff- oder Schwefelatom, durch eine -NH- oder -N(C₁₋₃-Alkyl-)- Gruppe ersetzt sein kann,
und t eine der Zahlen 2 oder 3
oder, sofern R₁₉ ein Wasserstoffatom ist, auch die Zahl 1 bedeuten
oder eine Gruppe der Formel

-SO₂-N(R₂₀)-(CH₂)ᵤ-R₂₁ ,

in der
R₂₀ ein Wasserstoffatom oder eine Allyl- oder C₁₋₃-Alkylgruppe,
R₂₁ ein Wasserstoffatom, eine Hydroxy-, C₁₋₃-Alkoxy-, Amino-, C₁₋₃-Alkylamino- oder eine Di-(C₁-₃-alkyl)-aminogruppe und
u eine der Zahlen 2, 3 oder 4
oder, sofern R₂₁ ein Wasserstoffatom ist, auch die Zahl 1 bedeuten, oder R₄ eine Gruppe der Formel 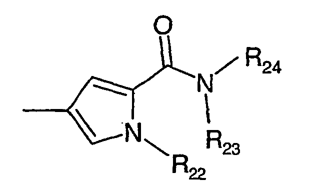 in der
R₂₂ eine Methylgruppe,
R₂₃ ein Wasserstoffatom oder eine Allyl- oder C₁₋₃-Alkylgruppe und
R₂₄ ein Wasserstoffatom, eine C₁₋₃-Alkylgruppe oder
eine terminal durch eine Hydroxy-, C₁₋₃-Alkoxy-, C₁₋₃-Alkylaminogruppe oder durch eine Di-(C₁₋₃-alkyl)-aminogruppe substituierte C₂₋₃-Alkylgruppe bedeuten,
oder R₂₃ und R₂₄ zusammen mit dem Stickstoffatom, mit dem sie verknüpft sind, bilden
eine 2,5-Dihydro-pyrrol-1-yl-gruppe oder
eine 5- bis 7-gliedrige Cycloalkyleniminogruppe,
wobei die Methylengruppe in Position 4 einer 6- oder 7-gliedrigen Cycloalkylenlminogruppe durch eine Amino-, C₁₋₃-Alkylamino- oder Di-(C₁₋₃-alkyl)-aminogruppe substituiert oder durch ein Sauerstoffatom, eine -NH- oder -N(C₁₋₃-Alkyl)-Gruppe ersetzt sein kann,
und
R₅ ein Wasserstoffatom bedeuten,
wobei die Wasserstoffatome in den vorstehend genannten Alkyl- und Alkoxygruppen oder in den in vorstehend definierten Gruppen der Formel I enthaltenen Alkylteilen teilweise oder ganz durch Fluoratome ersetzt sein können,
die in den vorstehend definierten Gruppen vorhandenen gesättigten Alkyl- und Alkoxyteile, die mehr als 2 Kohlenstoffatome enthalten, auch deren verzweigte Isomere, wie beispielsweise die Isopropyl-, tert.Butyl-, Isobutylgruppe, einschließen, sofern nichts anderes erwähnt wurde.

Offenbart werden auch Verbindungen der allgemeinen Formel I, in denen
X ein Sauerstoffatom,
R₁ und R₅ jeweils ein Wasserstoffatom,
R₂ ein Wasserstoff-, Fluor-, Chlor- oder Bromatom,
eine Cyanogruppe oder
eine Carboxy- C₁₋₂-Alkoxycarbonyl-, Allyloxycarbonyl-, C₁₋₃-Alkylaminocarbonyl-oder Di-(C₁₋₂-alkyl)-aminocarbonylgruppe
R₃ eine 2-Pyrrolyl-, 2-Furyl-, 3-Furyl-, 2-Thienyl-, 3-Thienyl-, 2-(Carboxy-C₁₋₃-alkyl)-thien-5-yl-, 2-(Carboxy-C₁₋₃-alkyl)-thien-4-yl-, 3-(Carboxy-C₁₋₃-alkyl)-thien-5-yl-, 4-Imidazolyl-, 5-(C₁₋₃-Alkyl)-pyrazol-3-yl-, 5-(C₁₋₃-Alkyl)-isoxazol-3-yl-, 3-Pyridyl-, 4-Pyridyl-, 2-Pyrazinyl-, 4-Pyridazinyl-, Benzimidazol-5-yl-, 1-(C₁₋₃-Alkyl)-benzimidazol-5-yl-, 2-(C₁₋₃-Alkyl)-benzimidazol-5-yl-, 2,3-Dihydro-benzofuran-5-yl-, 2,3-Dihydro-benzofuran-6-yl-, 3,4-Methylendioxy-1-phenyl-, 3,4-Ethylendioxy-1-phenyl-, 3,4-(Difluormethylendioxy)-1-phenyl-, 2-(C₁₋₃-Alkyl)-isoindol-1,3-dion-5-yl-, Chinoxalin-6-yl- oder 1-(C₁₋₃-Alkyl)-benzotriazol-5-yl-Gruppe,
R₄ eine durch die Gruppe R₆ in 3- oder 4-Position substituierte Phenylgruppe, die in der noch verbleibenden 3- bzw. 4-Position zusätzlich durch ein Fluor- oder Chloratom oder durch eine (C₁₋₃)-Alkoxy- oder Cyanogruppe substituiert sein kann, wobei
R₆ eine 1-(C₁₋₃-Alkyl)-imidazol-2-yl-gruppe,
eine 5-(C₁₋₃-Alkyl)-pyrazol-1-yl-gruppe, die zusätzlich in 3-Position durch eine C₁₋₃-Alkylgruppe substituiert sein kann,
eine Pyrrolid-2-on-1-yl-gruppe,
eine terminal durch die Gruppe R₇ substituierte C₁₋₂-Alkylgruppe, wobei
R₇ eine Amino-, Allylamino-, C₁₋₄-Alkylamino- oder Di-(C₁₋₃-alkyl)-aminogruppe,
eine ω-Hydroxy-C₂₋₃-alkyl-amino-, N-(C₁₋₃-Alkyl)-ω-hydroxy-C₂₋₃-alkyl-amino-, ω-(C₁-₃-Alkoxy)-C₂₋₃-alkyl-amino- oder N-(C₁₋₃-Alkyl)-[ω-(C₁₋₃-alkoxy)-C₂₋₃-alkyl]-amino-gruppe,
eine Pyridylaminogruppe,
eine 5- bis 7-gliedrige Cycloalkyleniminogruppe, in der
ein Kohlenstoffatom mit einer Hydroxy- oder Hydroxymethylgruppe substituiert sein kann, wobei die Substitution durch eine Hydroxylgruppe an einem dem Stickstoffatom benachbarten Kohlenstoffatom ausgenommen ist,
eine 6- bis 7-gliedrige Cycloalkyleniminogruppe, in der die Methylengruppe in 4-Position durch ein Sauerstoffatom oder eine -NH-, -N-(Allyl)- oder-N(C₁₋₃-Alkyl)- Gruppe ersetzt sein kann, oder
eine über das Stickstoffatom in 1-Position oder 2-Position gebundene Triazolylgruppe,
oder R₆ eine Gruppe der Formel

-(CH₂)ₙ-CO-R₈

in der
R₈ eine Pyrrolidino-, 2,5-Dihydro-pyrrol-1-yl-, Piperidino-, Morpholino-, Thiomorpholino- oder eine in 4-Stellung gegebenenfalls durch eine C₁₋₃-Alkylgruppe substituierte Piperazino- oder Perhydro-1,4-diazepinogruppe
und n eine der Zahlen 0 oder 1 bedeuten,
eine Gruppe der Formel

-CO-NR₉R₁₀

in der
R₉ ein Wasserstoffatom, eine Allylgruppe oder eine gegebenenfalls terminal durch eine Cyanogruppe substituierte C₁₋₃-Alkylgruppe und
R₁₀ ein Wasserstoffatom,
eine C₁₋₃-Alkylgruppe,
eine terminal durch eine C₁₋₃-Alkylamino- oder Di-(C₁₋₃-alkyl)-aminogruppe substituierte C₂₋₃-Alkylgruppe oder
eine 3- bis 7-gliedrige-Cycloalkylgruppe, in der eine Methylengruppe durch eine -NH- oder -N(C₁₋₃-Alkyl)- Gruppe ersetzt sein kann, bedeuten
eine Gruppe der Formel

-N(R₁₅)-CO-(CH₂)ₛ-R₁₆,

in der
R₁₅ ein Wasserstoffatom, eine Allyl-, C₁₋₃-Alkyl-, Pyridinyl-, ω-[(C₁₋₃-Alkyl)-amino]-C₂₋₃-alkyl- oder ω-[Di-(C₁₋₃-alkyl)-amino]-C₂₋₃-alkylgruppe,
s eine der Zahlen 0, 1 oder 2 und
R₁₆ eine C₁₋₃-Alkoxy-, Amino-, C₁₋₃-Alkylamino-, Di-(C₁₋₃-alkyl)-amino- oder Pyridinylgruppe,
eine Pyrrolidino-, 2,5-Dihydropyrrol-1-yl-, Piperidino-, Morpholino- oder Thiomorpholinogruppe oder
eine in 4-Stellung gegebenenfalls durch eine C₁₋₃-Alkylgruppe substituierte Piperazino- oder Perhydro-1,4-diazepinogruppe
oder, sofern s die Zahl 1 oder 2 darstellt, auch ein Wasserstoffatom bedeuten, eine Gruppe der Formel

-N(R₁₇)-SO₂-R₁₈ ,

in der
R₁₇ ein Wasserstoffatom,
eine C₁₋₃-Alkylgruppe oder
eine terminal durch eine Amino-, C₁₋₃-Alkyl- amino- oder Di-(C₁₋₃-alkyl)-aminogruppe substituierte C₂₋₃-Alkylgruppe und
R₁₈ eine C₁₋₃-Alkylgruppe bedeuten,
eine Gruppe der Formel

-SO₂-(CH₂)ₜ-R₁₉,

in der
t eine der Zahlen 1, 2 oder 3 und
R₁₉ ein Wasserstoffatom oder, sofern n eine der Zahlen 2 oder 3 darstellt, auch eine Di-(C₁₋₃-alkyl)-aminogruppe bedeuten,
oder eine Gruppe der Formel

-O-(CH₂)ₜ-R₁₉ ,

in der
t eine der Zahlen 1, 2 oder 3 und
R₁₉ ein Wasserstoffatom oder, sofern n eine der Zahlen 2 oder 3 darstellt, auch eine Di-(C₁₋₃-alkyl)-aminogruppe bedeuten,
oder eine Gruppe der Formel

-SO₂-NR₂₀R₂₅ ,

in der
R₂₀ ein Wasserstoffatom oder eine Allyl- oder C₁₋₃-Alkylgruppe und
R₂₅ eine C₁₋₃-Alkylgruppe oder
eine durch eine Amino-, C₁₋₃-Alkylamino- oder Di-(C₁₋₃-alkyl)-aminogruppe substituierte C₂₋₃-Alkylgruppe bedeuten,
bedeuten,
wobei die in den vorstehend genannten Resten enthaltenen Dialkylaminogruppen zwei gleiche oder zwei unterschiedliche Alkylgruppen enthalten können und
die in den vorstehend genannten Resten enthaltenen gesättigten Alkyl- und Alkoxyteile, die mehr als 2 Kohlenstoffatome enthalten, linear oder verzweigt sein können, sofern nichts anderes erwähnt wurde,
deren Tautomere, deren Diastereomere, deren Enantiomere, deren Gemische und deren Salze.

Offenbart under auch Verbindungen der allgemeinen Formel I, in denen
X ein Sauerstoffatom,
R₁ und R₅ jeweils ein Wasserstoffatom,
R₂ ein Wasserstoff-, Fluor- oder Chloratom oder eine Methoxycarbonyl-, Ethoxycarbonyl-, Dimethylaminocarbonyl-, N-Ethyl-N-methyl-aminocarbonyl- oder Diethylaminocarbonylgruppe,
R₃ eine 3,4-Methylendioxy-1-phenyl-, 3,4-Ethylendioxy-1-phenyl-, Chinoxalin-6-yl-, Benzimidazol-5-yl-, 2-Methylbenzimidazol-5-yl- oder 1-Methyl-benzimidazol-5-yl-gruppe und
R₄ eine in 4-Position durch die Gruppe R₆ substituierte Phenylgruppe, die zusätzlich in 3-Position durch ein Fluor- oder Chloratom oder eine Methoxygruppe substituiert sein kann, wobei
R₆ eine 1-(C₁₋₂-Alkyl)-imidazol-2-yl-gruppe,
eine 3,5-Dimethyl-pyrazol-1-yl-gruppe,
eine Pyrrolid-2-on-1-ylgruppe,
eine durch die Gruppe R₇ substituierte Methylgruppe, wobei
R₇ eine Methylamino-, Ethylamino-, Isobutylamino-, Di-(C₁₋₂-alkyl)-amino-, N-(2-Hydroxyethyl)-methylamino- oder N-(2-Methoxyethyl)-methylaminogruppe,
eine Pyrrolidino-, 3-Hydroxypyrrolidino-, 2-Hydroxymethyl-pyrrolidino-, 4-Hydroxypiperidino-, Morpholino-, Piperazin-1-yl- oder 1-Methyl-piperazin-4-yl-gruppe oder
eine 1,2,4-Triazol-1-yl-, 1,2,3-Triazol-1-yl- oder 1,2,3-Triazol-2-yl-gruppe,
oder R₆ eine N-Acetyl-methylamino- oder N-Methoxyacetyl-methylaminogruppe,
eine Gruppe der Formel

-CO-R₈ ,

in der
R₈ eine in 4-Stellung gegebenenfalls durch eine Methylgruppe substituierte Piperazino- oder Perhydro-1,4-diazepinogruppe bedeutet,
eine 4-Methyl-piperazin-1-yl-carbonyl-methyl-gruppe,
eine Gruppe der Formel

-CO-NR₉R₁₀

in der
R₉ eine Methyl-, Cyanomethyl- oder Ethylgruppe und
R₁₀ eine Methyl-, 1-Methylpiperidin-4-yl-, 2-Methylamino-ethyl-, 2-Dimethylamino-ethyl- oder 3-Dimethylamino-propylgruppe bedeuten
eine Gruppe der Formel

-N(R₁₅)-CO-(CH₂)ₛ-NMe₂,

in der
s eine der Zahlen 1 oder 2 und
R₁₅ eine Methyl- oder Ethylgruppe oder, sofern n die Zahl 2 darstellt, auch eine 3-Pyridylgruppe bedeuten,
eine Gruppe der Formel

-N(R₁₅')-CO-(CH₂)ₛ-H,

in der
s eine der Zahlen 1 oder 2 und
R₁₅' eine 2-(Dimethylamino)-ethyl- oder 3-(Dimethylamino)-propylgruppe bedeuten,
oder eine Gruppe der Formel

-N(Me)-CO-(CH₂)ₛ-R₁₆' ,

in der
s eine der Zahlen 1 oder 2 und
R₁₆' eine Dimethylaminogruppe, oder, sofern s die Zahl 1 darstellt, auch eine 4-(C₁₋₂-Alkyl)-piperazin-1-yl-gruppe bedeuten,
eine Gruppe der Formel

-N(R₁₇)-SO₂-R₁₈',

in der a) R₁₇ eine Dimethylaminoethylgruppe und R₁₈ eine Methyl-, Ethyl- oder Propylgruppe bedeutet oder
in der b) R₁₇ und R₁₈ jeweils eine Methylgruppe bedeuten,
eine Gruppe der Formel

-SO₂-N(R₂₀)-(CH₂)ᵤ-NMe₂ ,

in der
R₂₀ ein Wasserstoffatom oder eine Methylgruppe und
u eine der Zahlen 2 oder 3 bedeuten,
eine Gruppe der Formel

-SO₂-R₂₆ ,

in der
R₂₆ eine Methylgruppe oder eine 2-Di-(C₁₋₂-alkyl)-amino-ethylgruppe bedeutet,
oder eine 2-Di-(C₁₋₂-alkyl)-amino-ethoxy-gruppe bedeuten,
wobei die in den vorstehend genannten Resten enthaltenen Dialkylaminogruppen zwei gleiche oder zwei unterschiedliche Alkylgruppen enthalten können,
deren Tautomere, deren Diastereomere, deren Enantiomere, deren Gemische und deren Salze.

Offenbart werden auch folgende Verbindungen
(a) 3-(*Z*)-{1-[4-(Dimethylaminomethyl)-phenylamino]-1-(3,4-methylendioxyphenyl)-methylen}-6-chlor-2-indolinon
(b) 3-(*Z*)-{1-(4-[*N*-Methyl-*N*-(4-methylpiperazin-1-yl-methylcarbonyl)-amino]-phenylamino)-1-(chinoxalin-6-yl)-methylen}-6-chlor-2-indolinon
(c) 3-(*Z*)-{1-[4-(*N-*Ethyl-*N-*methyl-aminomethyl)-phenylamino]-1-(3,4-methylendioxyphenyl)-methylen}-6-chlor-2-indolinon
(d) 3-(*Z*)-{1-[4-(N-Methyl-N-{2-(dimethylamino)-ethyl-carbonyl}-amino)-phenylamino]-1-(3,4-ethylendioxyphenyl)-methylen}-6-fluor-2-indolinon
(e) 3-(*Z*)-{1-[4-(1,2,4-Triazol-1-yl-methyl)-phenylamino]-1-(1-methyl-benzimidazol-5-yl )-methylen}-6-fluor-2-indolinon
(f) 3-(*Z*)-{1-[4-(Dimethylaminomethyl)-phenylamino]-1-(1-methyl-benzimidazol-5-yl)-methylen}-6-chlor-2-indolinon
(g) 3-(*Z*)-{1-[4-(Dimethylaminomethyl)-phenylamino]-1-(3,4-ethylendioxyphenyl)-methylen}-6-chlor-2-indolinon
(h) 3-(*Z*)-{1-(4-[*N*-Methansulfonyl-*N*-(2-dimethylaminoethyl)-amino]-phenylamino)-1-(3,4-ethylendioxyphenyl)-methylen}-6-chlor-2-indolinon
(i) 3-(*Z*)-{1-[4-(Dimethylaminomethyl)-phenylamino]-1-(3,4-ethylendioxyphenyl)-methylen}-6-methoxycarbonyl-2-indolinon
(j) 3-(*Z*)-{1-(4-[*N*-Methyl-*N*-(4-methylpiperazin-1-yl-methylcarbonyl)-amino]-phenylamino)-1-(3,4-ethylendioxyphenyl)-methylen}-6-methoxycarbonyl-2-indolinon
(k) 3-(*Z*)-{1-(4-[*N*-Acetyl-*N*-(2-dimethylaminoethyl)-amino]-phenylamino)-1-(3,4-ethylendioxyphenyl)-methylen}-6-fluor-2-indolinon
(l) 3-(*Z*)-{1-[4-(Ethylaminomethyl)-phenylamino]-1-(3,4-ethylendioxyphenyl)-methylen}-6-fluor-2-indolinon
(m) 3-(*Z*)-{1-(4-[*N*-Acetyl-*N*-(3-dimethylaminopropyl)-amino]-phenylamino)-1-(3,4-ethylendioxyphenyl)-methylen}-6-chlor-2-indolinon
(n) 3-(*Z*)-{1-(4-[*N*-Propionyl-*N*-(3-dimethylaminopropyl)-amino]-phenylamino)-1-(3,4-ethylendioxyphenyl)-methylen}-6-chlor-2-indolinon
(o) 3-(*Z*)-{1-(4-[*N-*Propionyl-*N-*(2-dimethylaminoethyl)-amino]-phenylamino)-1-(3,4-ethylendioxyphenyl)-methylen}-6-chlor-2-indolinon
(p) 3-(*Z*)-{1-(4-[*N*-Acetyl-*N*-(2-dimethylaminoethyl)-amino]-phenylamino)-1-(3,4-ethylendioxyphenyl)-methylen}-6-chlor-2-indolinon
(q) 3-(*Z*)-{1-(4-[4-Methylpiperazin-1-yl-methyl]-phenylamino)-1-(3,4-methylendioxyphenyl)-methylen}-6-chlor-2-indolinon
(r) 3-(*Z*)-{1-(4-[*N*-Methansulfonyl-*N*-(2-dimethylaminoethyl)-amino]-phenylamino)-1-(3,4-ethylendioxyphenyl)-methylen}-6-methoxycarbonyl-2-indolinon
(s) 3-(*Z*)-{1-(4-[Pyrrolidin-1-yl-methyl]-phenylamino)-1-(3,4-ethylendioxyphenyl)-methylen}-6-methoxycarbonyl-2-indolinon
(t) 3-(*Z*)-{1-(4-[*N*-Methyl-*N*-(dimethylaminomethylcarbonyl)-amino]-phenylamino)-1-(3,4-ethylendioxyphenyl)-methylen}-6-methoxycarbonyl-2-indolinon
(u) 3-(*Z*)-{1-(4-[Ethylamino-methyl]-phenylamino)-1-(3,4-ethylendioxyphenyl)-methylen}-6-methoxycarbonyl-2-indolinon
(v) 3-(*Z*)-{1-(4-[4-Methylpiperazin-1-yl-methyl]-phenylamino)-1-(3,4-ethylendioxyphenyl)-methylen}-6-methoxycarbonyl-2-indolinon
(w) 3-(*Z*)-{1-(4-[Dimethylamino-methyl]-phenylamino)-1-(3,4-methylendioxyphenyl)-methylen}-6-methoxycarbonyl-2-indolinon
(x) 3-(*Z*)-{1-(4-[Diethylamino-methyl]-phenylamino)-1-(3,4-methylendioxyphenyl)-methylen}-6-methoxycarbonyl-2-indolinon
(y) 3-(*Z*)-{1-[4-(Dimethylaminocarbonyl)-phenylamino]-1-(1-methyl-benzimidazol-5-yl)-methylen}-6-fluor-2-indolinon
(z) 3-(*Z*)-{1-(4-[*N*-Propionyl-*N*-(3-dimethylaminopropyl)-amino]-phenylamino)-1-(3,4-ethylendioxyphenyl)-methylen}-6-fluor-2-indolinon
(aa) 3-(*Z*)-{1-(4-[*N*-Propionyl-*N*-(2-dimethylaminoethyl)-amino]-phenylamino)-1-(3,4-ethylendioxyphenyl)-methylen}-6-fluor-2-indolinon
(ab) 3-(*Z*)-{1-(4-[*N*-Methansulfonyl-*N*-(2-dimethylaminoethyl)-amino]-phenylamino)-1-(3,4-ethylendioxyphenyl)-methylen}-6-fluor-2-indolinon
(ac) 3-(*Z*)-{1-(4-[*N*-Acetyl-*N*-(2-dimethylaminoethyl)-amino]-phenylamino)-1-(3,4-ethylendioxyphenyl)-methylen}-6-methoxycarbonyl-2-indolinon
(ad) 3-(*Z*)-{1-(4-[N-Methyl-N-(2-dimethylaminoethyl)aminocarbonyl]-phenylamino)-1-(3,4-ethylendioxyphenyl)-methylen}-6-methoxycarbonyl-2-indolinon
(ae) 3-(*Z*)-{1-(4-[N-Methyl-N-(3-dimethylaminopropyl)aminocarbonyl]-phenylamino)-1-(3,4-ethylendioxyphenyl)-methylen}-6-methoxycarbonyl-2-indolinon
(af) 3-(*Z*)-{1-(4-[N-Methyl-N-(2-dimethylaminoethyl)aminocarbonyl]-phenylamino)-1-(3,4-methylendioxyphenyl)-methylen}-6-methoxycarbonyl-2-indolinon
(az) 3-(*Z*)-{1-(4-[*N*-Methyl-*N*-(4-methylpiperazin-1-yl-methylcarbonyl)-amino]-phenylamino)-1-(3,4-methylendioxyphenyl)-methylen}-6-chlor-2-indolinon
(be) 3-(*Z*)-{1-(4-[*N*-Methyl-*N*-(4-methylpiperazin-1-yl-methylcarbonyl)-amino]-phenylamino)-1-(3,4-methylendioxyphenyl)-methylen}-6-fluor-2-indolinon und
(bf) 3-(*Z*)-{1-(4-[*N*-Methyl-*N*-(4-methylpiperazin-1-yl-methylcarbonyl)-amino]-phenylamino)-1-(3,4-methylendioxyphenyl)-methylen}-6-brom-2-indolinon,
   deren Tautomere und deren Salze.

Nach folgenden im Prinzip literaturbekannten Verfahren können Verbindungen der Formel I hersgestellt werden:
a. Umsetzung einer Verbindung der allgemeinen Formel 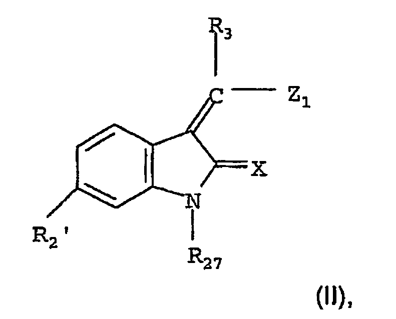 in der
   X und R₃ wie eingangs erwähnt definiert sind,
   R₂' die für R₂ eingangs erwähnten Bedeutungen besitzt,
   R₂₇ ein Wasserstoffatom oder eine Schutzgruppe für das Stickstoffatom der Lactamgruppe, wobei einer der Reste R₂' und R₂₇ auch eine gegebenenenfalls über einen Spacer gebildete Bindung an eine Festphase darstellen kann und der andere der Reste R₂' und R₂₇ die vorstehend erwähnten Bedeutungen besitzt, und Z₁ ein Halogenatom, eine Hydroxy-, Alkoxy- oder Aryl-alkoxygruppe, z.B. ein Chlor- oder Bromatom, eine Methoxy-, Ethoxy- oder Benzyloxygruppe, bedeuten,
   mit einem Amin der allgemeinen Formel 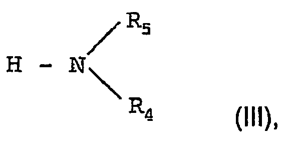 in der
   R₄ und R₅ wie eingangs erwähnt definiert sind,
   und erforderlichenfalls anschließende Abspaltung einer verwendeten Schutzgruppe für das Stickstoffatom der Lactamgruppe oder von einer Festphase.
   Als Schutzgruppe für das Stickstoffatom der Lactamgruppe kommt beispielsweise eine Acetyl-, Benzoyl-, Ethoxycarbonyl-, tert.Butyloxycarbonyl- oder Benzyloxycarbonylgruppe und
   als Festphase ein Harz wie ein 4-(2',4'-Dimethoxyphenylaminomethyl)-phenoxyharz, wobei die Bindung zweckmäßigerweise über die Aminogruppe erfolgt, oder ein p-Benzyloxybenzylalkoholharz, wobei die Bindung zweckmäßigerweise über ein Zwischenglied wie ein 2,5-Dimethoxy-4-hydroxy-benzylderivat erfolgt, in Betracht.
   Die Umsetzung wird zweckmäßigerweise in einem Lösungsmittel wie Dimethylformamid, Toluol, Acetonitril, Tetrahydrofuran, Dioxan, Methanol, Ethanol, 2-Propanol, Dimethylsulfoxid, Methylenchlorid oder deren Gemischen gegebenenfalls in Gegenwart einer inerten Base wie Triethylamin, N-Ethyl-diisopropylamin oder Natriumhydrogencarbonat bei Temperaturen zwischen 20 und 175°C durchgeführt, wobei eine verwendete Schutzgruppe infolge Umamidierung gleichzeitig abgespalten werden kann.
   Bedeutet Z₁ in einer Verbindung der allgemeinen Formel II ein Halogenatom, dann wird die Umsetzung vorzugsweise in Gegenwart einer inerten Base bei Temperaturen zwischen 20 und 120°C, durchgeführt.
   Bedeutet Z₁ in einer Verbindung der allgemeinen Formel II eine Hydroxy-, Alkoxy-oder Arylalkoxygruppe, dann wird die Umsetzung vorzugsweise bei Temperaturen zwischen 20 und 200°C, durchgeführt.
   Die gegebenenfalls erforderliche anschließende Abspaltung einer verwendeten Schutzgruppe wird zweckmäßigerweise entweder hydrolytisch in einem wäßrigen oder alkoholischen Lösungsmittel, z.B. in Methanol/Wasser, Ethanol/Wasser, Isopropanol/Wasser, Tetrahydrofuran/Wasser, Dioxan/Wasser, Dimethylformamid/Wasser, Methanol oder Ethanol in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C,
   oder vorteilhafterweise durch Umamidierung mit einer organischen Base wie Ammoniak, Butylamin, Dimethylamin oder Piperidin in einem Lösungsmittel wie Methanol, Ethanol, Dimethylformamid und deren Gemischen oder in einem Überschuß des eingesetzten Amins bei Temperaturen zwischen 0 und 100°C, vorzugsweise bei Temperaturen zwischen 10 und 50°C, durchgeführt.
   Die Abspaltung von einer verwendeten Festphase erfolgt vorzugsweise mittels Trifluoressigsäure und Wasser bei Temperaturen zwischen 0 und 35°C, vorzugsweise bei Raumtemperatur.
b. Zur Herstellung einer Verbindung der allgemeinen Formel I, in der
   R₂ eine lineare oder verzweigte C₁₋₆-Alkoxy-carbonylgruppe, eine C₃₋₆-Cycloalkoxy-carbonyl- oder eine Aryloxycarbonylgruppe,
   eine gegebenenfalls durch eine oder zwei Methylgruppen substituierte Allyloxycarbonylgruppe,
   eine lineare oder verzweigte C₁₋₄-Alkoxy-carbonylgruppe, die im Alkylteil terminal durch eine Phenyl-, Heteroaryl-, Carboxy-, C₁₋₃-Alkoxy-carbonyl-, Aminocarbonyl-, C₁₋₃-Alkylamino-carbonyl- oder Di-(C₁₋₃-alkyl)-aminocarbonylgruppe substituiert ist,
   eine lineare oder verzweigte C₂₋₆-Alkoxy-carbonylgruppe, die im Alkylteil terminal durch ein Chloratom oder eine Hydroxy-, C₁₋₃-Alkoxy-, Amino-, C₁₋₃-Alkylamino-oder Di-(C₁₋₃-alkyl)-aminogruppe substituiert ist, oder
   eine Aminocarbonyl-, C₁₋₄-Alkyl-aminocarbonyl- oder eine Di-(C₁₋₄-alkyl)-amino-carbonylgruppe, wobei die Alkylgruppen, sofern sie mehr als ein Kohlenstoffatom besitzen, terminal durch eine Hydroxy-, C₁₋₃-Alkoxy- oder Di-(C₁₋₃-alkyl)-aminogruppe substituiert sein können, bedeutet:
   Umsetzung einer Verbindung der allgemeinen Formel 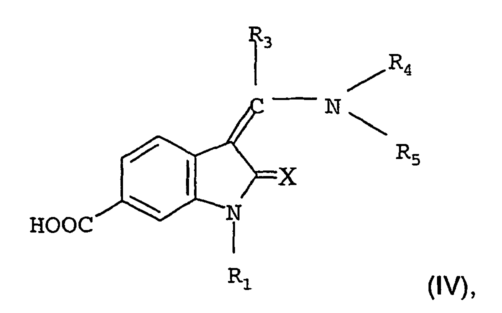 in der
   X, R₁ und R₃ bis R₅ wie eingangs erwähnt definiert sind, oder deren reaktionsfähigen Derivaten mit einer Verbindung der allgemeinen Formel

   H-R₂₈ (V),

   in der
   R₂₈ ein linearer oder verzweigter C₁₋₆-Alkanol, ein C₃₋₆-Cycloalkanol oder ein aromatischer Alkohol,
   ein gegebenenfalls durch eine oder zwei Methylgruppen substituierter Allyl-alkohol,
   ein linearer oder verzweigter C₁₋₄-Alkanol, der im Alkylteil terminal durch eine Phenyl-, Heteroaryl-, Carboxy-, C₁₋₃-Alkoxy-carbonyl-, Aminocarbonyl-, C₁₋₃-Alkylamino-carbonyl- oder Di-(C₁₋₃-alkyl)-aminocarbonylgruppe substituiert ist,
   eine linearer oder verzweigter C₂₋₆-Alkanol, der im Alkylteil terminal durch ein Chloratom oder eine Hydroxy-, C₁₋₃-Alkoxy-, Amino-, C₁₋₃-Alkylamino- oder Di-(C₁₋₃-alkyl)-aminogruppe substituiert ist, oder
   eine Amino-, C₁₋₄-Alkyl-amino- oder eine Di-(C₁₋₄-alkyl)-aminogruppe, wobei die Alkylgruppen, sofern sie mehr als ein Kohlenstoffatom besitzen, terminal durch eine Hydroxy-, C₁₋₃-Alkoxy- oder Di-(C₁₋₃-alkyl)-aminogruppe substituiert sein können, bedeutet.
   Die Veresterung oder Amidierung wird vorzugsweise in einem Lösungsmittel wie Methylenchlorid, Diethylether, Tetrahydrofuran, Toluol, Dioxan, Acetonitril, Dimethylsulfoxid oder Dimethylformamid gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt. Hierbei wird die Umsetzung mit einer entsprechenden Säure vorzugsweise in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenyl-phosphin/Tetrachlorkohlenstoff, und gegebenenfalls unter Zusatz einer Base wie Pyridin, 4-Dimethylaminopyridin, N-Methyl-morpholin oder Triethylamin zweckmäßigerweise bei Temperaturen zwischen 0 und 150°C, vorzugsweise bei Temperaturen zwischen 0 und 100°C, und die Acylierung mit einer entsprechenden reaktionsfähigen Verbindung wie deren Anhydrid, Ester, Imidazolide oder Halogenide gegebenenfalls in Gegenwart einer tertiären organischen Base wie Triethylamin, N-Ethyl-diisopropylamin oder N-Methyl-morpholin bei Temperaturen zwischen 0 und 150°C, vorzugsweise bei Temperaturen zwischen 50 und 100°C, durchgeführt.
c. Zur Herstellung einer Verbindung der allgemeinen Formel 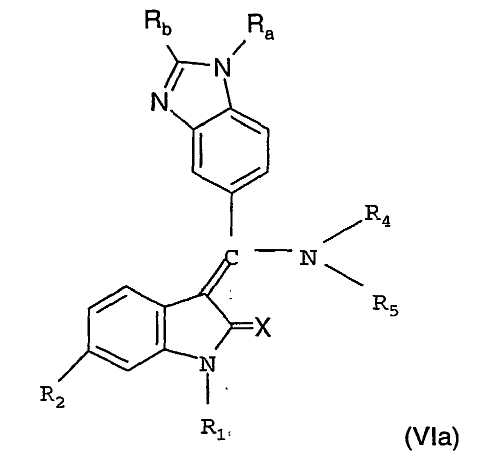 oder 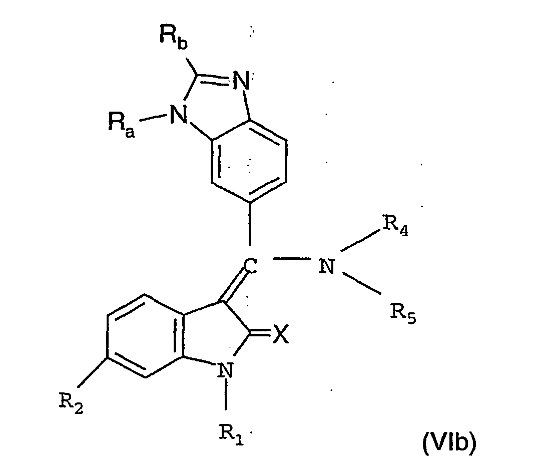 in der
   X, R₁, R₂, R₄ und R₅ wie eingangs erwähnt definiert sind und
   Rₐ und R_{b} jeweils unabhängig voneinander ein Wasserstofftom, eine C₁₋₃-Alkylgruppe oder eine Carboxy-C₁₋₃-alkylgruppe sein können:
   Reduktion einer Verbindung der allgemeinen Formel 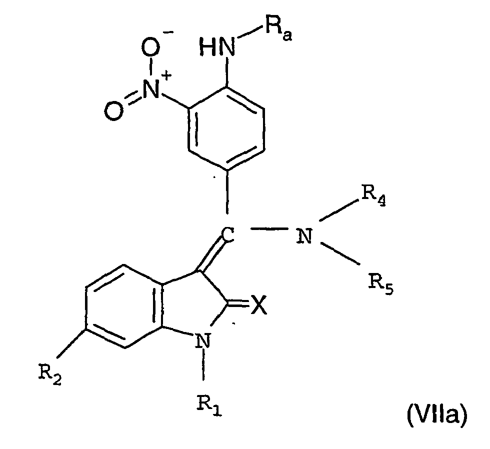 beziehungsweise 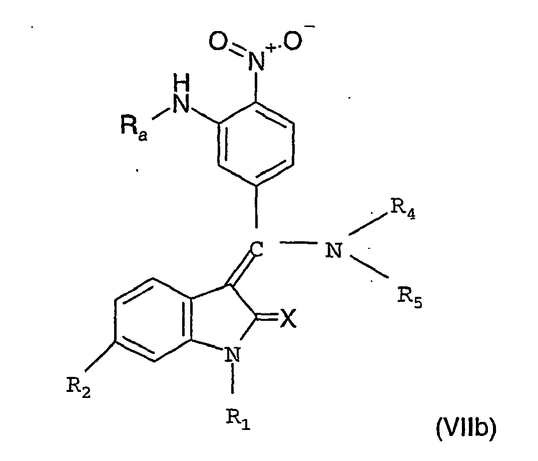 in der
   X, R₁, R₂, R₄ und R₅ wie eingangs erwähnt definiert sind und
   Rₐ ein Wasserstoffatom, eine C₁₋₃-Alkylgruppe oder eine gegebenenfalls geschützte Carboxy-C₁₋₃-alkylgruppe sein kann,
   mit Wasserstoff (1-10 bar) oder einem Reagens zur Transferhydrierung, wie beispielsweise Cyclohexen, 1,3-Cyclohexadien oder Ammoniumformiat in Anwesenheit eines Hydrierkatalysators wie beispielsweise Raney-Nickel oder Palladium auf Aktivkohle bei Temperaturen von 0°C bis 150 °C, bevorzugt bei 10°C bis zur Siedetemperatur des Lösungsmittels bzw. -gemisches,
   entweder
   c1. in Ameisensäure, gegebenenfalls unter Verwendung eines Kosolvens, wobei man Verbindungen der allgemeinen Formel VIa bzw. Vlb erhält, in denen R_{b} ein Wasserstoffatom und
      Rₐ ein Wasserstoffatom, eine C₁₋₃-Alkylgruppe oder eine gegebenenfalls geschützte Carboxy-C₁₋₃-alkylgruppe ist.
      oder
   c2. in einem Lösungsmittel wie beispielsweise Essigsäure, Propionsäure, Methanol, Ethanol oder Mischungen derselben untereinander oder mit anderen Lösungmitteln wie beispielweise Essigester, THF oder Dioxan,
      wobei man Verbindungen der allgemeinen Formel I erhält, in denen R₃ ein an einer der Aminogruppen mit Rₐ substituierter 3,4-Diaminophenylrest ist,
      welche weiter umgesetzt werden
      in R_{b}-COOH oder, falls R_{b} eine Carboxyalkylgruppe ist, auch einem geschützten Derivat wie einem entsprechenden Halbester, als Solvens und Reaktant oder gegebenenfalls - insbesondere, wenn R_{b}-COOH bei der gewählten Reaktionstemperatur ein Reststoff ist - unter Zusatz eines Lösungsmittels wie beispielsweise Methanol, Ethanol, 2-Propanol, Essigsäure, Propionsäure, THF, Dioxan, Dichlormethan oder Essigester bei Temperaturen von 10 °C -150 °C, bevorzugt bei 20 °C bis zum Siedepunkt des Lösungsmittels bzw. -gemisches zu Verbindungen der allgemeinen Formel VIa bzw. VIb in denen
      Rₐ und R_{b} unabhängig voneinander ein Wasserstoffatom, eine C₁₋₃-Alkylgruppe oder eine gegebenenfalls geschützte Carboxy-C₁₋₃-alkylgruppe sein können.
d. Zur Herstellung einer Verbindung der allgemeinen Formel I, in der R₄ eine durch die Gruppe R₆ in 3- oder 4-Position substituierte Phenylgruppe, die zusätzlich wie oben beschrieben substituiert sein kann, darstellt, und R₆ eine durch R₇ substituierte C₁₋₃-Alkylgruppe bedeutet, wobei
   R₇ eine Heteroarylgruppe, welche über einen Iminostickstoff gebunden ist,
   eine Hydroxy- oder C₁₋₃-Alkoxygruppe,
   eine Amino-, C₁₋₇-Alkylamino-, Di-(C₁₋₇-alkyl)-amino-, N-(C₁₋₇-Alkyl)-allylamino-, Phenylamino-, N-Phenyl-C₁₋₃-alkyl-amino-, Phenyl-C₁₋₃-alkylamino-, N-(C₁₋₃-Alkyl)-phenyl-C₁₋₃-alkylamino- oder Di-(phenyl-C₁₋₃-alkyl)-aminogruppe,
   eine Allylaminogruppe, in der ein oder zwei vinylische Wasserstoffatome jeweils durch eine Methylgruppe ersetzt sein können,
   eine ω-Hydroxy-C₂₋₃-alkyl-amino-, N-(C₁₋₃-Alkyl)-ω-hydroxy-C₂₋₃-alkyl-amino-, Di-(ω-hydroxy-C₂₋₃-alkyl)-amino-, N-(C₁₋₃-Alkyl)-[ω-(C₁₋₃-alkoxy)-C₂₋₃-alkyl]-amino-, Di-(ω-(C₁₋₃-alkoxy)-C₂₋₃-alkyl)-amino- oder N-(Dioxolan-2-yl)-C₁₋₃-alkyl-aminogruppe,
   eine C₁₋₃-Alkyl-carbonylamino-C₂₋₃-alkyl-amino- oder C₁₋₃-Alkyl-carbonylamino-C₂₋₃-alkyl-N-(C₁₋₃-alkyl)-aminogruppe,
   eine Pyridylaminogruppe,
   eine C₁₋₃-Alkylsulfonylamino-, N-(C₁₋₃-Alkyl)-C₁₋₃-alkylsulfonylamino-, C₁₋₃-Alkyl-sulfonylamino-C₂₋₃-alkyl-amino- oder C₁₋₃-Alkylsulfonylamino-C₂₋₃-alkyl-N-(C₁₋₃-alkyl)-aminogruppe,
   eine Hydroxycarbonyl-C₁₋₃-alkylamino- oder N-(C₁₋₃-Alkyl)-hydroxycarbonyl-C₁₋₃-alkyl-aminogruppe,
   eine Guanidinogruppe, in der ein oder zwei Wasserstoffatome jeweils durch eine C₁₋₃-Alkylgruppe ersetzt sein können,
   eine 2-Pyrrolidon-1-yl-gruppe, in der die der Carbonylgruppe benachbarte Methylengruppe durch ein Sauerstoffatom oder durch eine -NH- oder -N(C₁₋₃-Alkyl)- Gruppe ersetzt sein kann,
   eine Gruppe der Formel

   -N(R₁₁)-CO-(CH₂)ₚ-R₁₂,
in der
R₁₁ ein Wasserstoffatom oder eine Allyl-, C₁₋₃-Alkyl-, C₁₋₃-Alkyl-amino-C₂₋₃-alkyl- oder Di-(C₁₋₃-alkyl)-amino-C₂₋₃-alkylgruppe,
p eine der Zahlen 0, 1, 2 oder 3 und
R₁₂ eine Amino-, C₁₋₄-Alkylamino-, Allylamino-, Di-allyl-amino-, Di-(C₁₋₄-alkyl)-amino-, Phenylamino-, N-(C₁₋₄-Alkyl)-phenylamino-, Benzylamino-, N-(C₁₋₄-Alkyl)-benzylamino-, C₁₋₄-Alkoxy- oder 2,5-Dihydropyrrol-1-yl-gruppe oder
eine 4- bis 7-gliedrige Cycloalkyleniminogruppe,
wobei jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Allyl)-, -N(Phenyl)-, -N(C₁₋₃-Alkyl-carbonyl)- oder -N(Benzoyl)- Gruppe ersetzt sein kann,
oder, sofern n eine der Zahlen 1, 2 oder 3 darstellt, auch ein Wasserstoffatom bedeuten,
eine Gruppe der Formel

-N(R₁₃)-(CH₂)_{q}-(CO)ᵣ-R₁₄,

in der
R₁₃ ein Wasserstoffatom oder eine C₁₋₃-Alkyl-, Allyl-, C₁₋₃-Alkyl-carbonyl-, Arylcarbonyl-, Pyridylcarbonyl-, Phenyl-C₁₋₃-alkyl-carbonyl-, C₁₋₃-Alkylsulfonyl-, Arylsulfonyl- oder Phenyl-C₁₋₃-alkylsulfonylgruppe,
q eine der Zahlen 1, 2, 3 oder 4,
r die Zahl 1 oder, sofern q eine der Zahlen 2, 3 oder 4 ist, auch die Zahl 0 und
R₁₄ eine Hydroxy-, Amino-, C₁₋₄-Alkylamino-, Di-(C₁₋₄-alkyl)-amino-, Phenylamino-, N-(C₁₋₄-Alkyl)-phenylamino-, Benzylamino-, N-(C₁₋₄-Alkyl)-benzylamino-, C₁₋₄-Alkoxy- oder C₁₋₃-Alkoxy-C₁₋₃-alkoxygruppe,
eine in 1-Position gegebenenfalls durch eine C₁₋₃-Alkylgruppe substituierte Di-(C₁₋₄-alkyl)-amino-C₁₋₃-alkylaminogruppe oder
eine 4- bis 7-gliedrige Cycloalkyleniminogruppe,
wobei der Cycloalkylenteil mit einem Phenylring kondensiert sein kann oder
jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Phenyl)-, -N(C₁₋₃-Alkyl-carbonyl)- oder -N(Benzoyl)- Gruppe ersetzt sein kann, bedeuten,
eine C₄₋₇-Cycloalkylamino-, C₄₋₇Cycloalkyl-C₁₋₃-alkylamino- oder C₄₋₇-Cyclo-alkenylaminogruppe, in der die Position 1 des Rings nicht an der Doppelbindung beteiligt ist und wobei die vorstehend genannten Gruppen jeweils zusätzlich am Aminstickstoffatom durch eine C₅₋₇Cycloalkyl-, C₂₋₄-Alkenyl-oder C₁₋₄-Alkylgruppe substituiert sein können,
eine 2,5-Dihydro-pyrrol-1-yl-gruppe oder
eine 4- bis 7-gliedrige Cycloalkyleniminogruppe, in der
der Cycloalkylenteil mit einer Phenylgruppe oder mit einer gegebenenfalls durch ein Fluor-, Chlor-, Brom- oder lodatom, durch eine Nitro-, C₁₋₃-Alkyl-, C₁₋₃-Alkoxy- oder Aminogruppe substituierten Oxazolo-, Imidazolo-, Thiazolo-, Pyridino-, Pyrazino- oder Pyrimidinogruppe kondensiert sein kann oder/und
ein oder zwei Wasserstoffatome jeweils durch eine C₁₋₃-Alkyl-, C₃₋₇-Cycloalkyl-, Hydroxy-, C₁₋₃-Alkoxy-, Hydroxy-C₁₋₃-alkyl- oder C₁₋₃-Alkoxy-C₁₋₃-alkyl- oder Phenylgruppe ersetzt sein können oder/und
die Methylengruppe in Position 3 einer 5-gliedrigen Cycloalkyleniminogruppe durch eine Hydroxy-, Hydroxy-C₁₋₃-alkyl-, C₁₋₃-Alkoxy- oder C₁₋₃-Alkoxy-C₁₋₃-alkylgruppe substituiert sein kann,
jeweils die Methylengruppe in Position 3 oder 4 einer 6- oder 7-gliedrigen Cycloalkyleniminogruppe durch eine Hydroxy-, Hydroxy-C₁₋₃-alkyl-, C₁₋₃-Alkoxy-, C₁₋₃-Alkoxy-C₁₋₃-alkyl-, Carboxy-, C₁₋₄-Alkoxycarbonyl-, Aminocarbonyl-, C₁₋₃-Alkylaminocarbonyl-, Di-(C₁₋₃-alkyl)-aminocarbonyl-, Phenyl-C₁₋₃-alkylamino- oder N-(C₁₋₃-Alkyl)-phenyl-C₁₋₃-alkylaminogruppe substituiert oder
die Methylengruppe in 4-Position einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Allyl)-, -N(Phenyl)-, -N(Phenyl-C₁₋₃-alkyl)-, -N(C₁₋₃-Alkyl-carbonyl)-, -N(C₁₋₄-Hydroxy-carbonyl)-, -N(C₁₋₄-Alkoxy-carbonyl)-, -N(Benzoyl)- oder -N(Phenyl-C₁₋₃-alkylcarbonyl)- Gruppe ersetzt sein kann,
wobei eine mit einem Imino-Stickstoffatom der Cycloalkyleniminogruppe verknüpfte Methylengruppe durch eine Carbonyl- oder Sulfonylgruppe ersetzt sein kann oder in einer 5- bis 7-gliedrigen monocyclischen oder mit einer Phenylgruppe kondensierten Cycloalkyleniminogruppe beide mit dem Imino-Stickstoffatom verknüpften Methylengruppen jeweils durch eine Carbonylgruppe ersetzt sein können,
bedeutet:
Umsetzung einer Verbindung der allgemeinen Formel 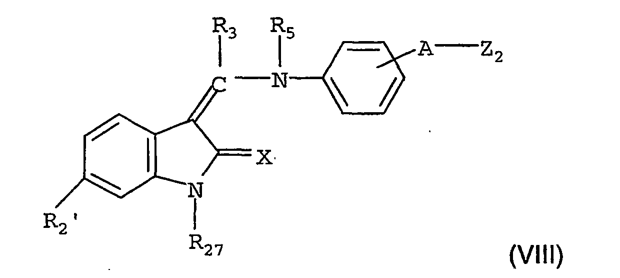 in der
R₃, R₅ und X wie eingangs erwähnt definiert sind,
R₂' die für R₂ eingangs erwähnten Bedeutungen besitzt,
R₂₇ ein Wasserstoffatom oder eine Schutzgruppe für das Stickstoffatom der Lactamgruppe, wobei einer der Reste R₂' und R₂₇ auch eine gegebenenenfalls über einen Spacer gebildete Bindung an eine Festphase darstellen kann und der andere der Reste R₂' und R₂₇ die vorstehend erwähnten Bedeutungen besitzt,
A eine C₁₋₃-Alkylgruppe und
Z₂ eine Austrittsgruppe, beispielsweise eine Alkyl- oder Arylsulfonyloxygruppe wie die Methylsulfonyloxy-, Ethylsulfonyloxy-, p-Toluolsulfonyloxy-, oder Trifluormethansulfonyloxygruppe darstellt, mit einer Verbindung der allgemeinen Formel

H-R_{7'} (IX),

in der
R_{7'} die vorstehend für R₇ genannten Bedeutungen besitzt, und erforderlichenfalls anschließende Abspaltung einer verwendeten Schutzgruppe für das Stickstoffatom
der Lactamgruppe oder von einer Festphase.

Die Umsetzung wird zweckmäßigerweise in einem Lösungsmittel wie Methylenchlorid, Tetrahydrofuran, 1,4-Dioxan, Toluol, Acetonitril, Dimethylsulfoxid, Dimethylformamid, Dimethylacetamid, N-Methylpyrrolidon oder deren Gemischen, gegebenenfalls unter Zusatz von Wasser als Cosolvens oder/und unter Zusatz einer inerten Hilfsbase, beispielsweise Natriumhydrogencarbonat, Pyridin, 2,4,6-Trimethylpyridin, Chinolin, Triethylamin, N-Ethyldiisopropylamin, N-Ethyl-dicyclohexylamin, 1,4-Diazabicyclo[2,2,2]octan oder 1,8-Diazabicyclo[5,4,0]undec-7-en, bei Temperaturen zwischen -50°C und +100°C, vorzugsweise zwischen -10°C und +50°C, durchgeführt, wobei eine verwendete Schutzgruppe infolge Umamidierung gleichzeitig abgespalten werden kann.

Die gegebenenfalls erforderliche Abspaltung einer verwendeten Schutzgruppe für das Stickstoffatom der Lactamgruppe oder von einer Festphase erfolgt wie vorstehend unter Verfahren (a) beschrieben.

Erhält man eine Verbindung der allgemeinen Formel I, die eine Alkoxycarbonylgruppe enthält, so kann diese mittels Hydrolyse in eine entsprechende Carboxyverbindung übergeführt werden, oder
eine Verbindung der allgemeinen Formel I, die eine Amino- oder Alkylaminogruppe enthält, so kann diese mittels reduktiver Alkylierung in eine entsprechende Alkylamino- oder Dialkylaminoverbindung übergeführt werden, oder
eine Verbindung der allgemeinen Formel I, die eine Amino- oder Alkylaminogruppe enthält, so kann diese mittels Acylierung oder Sulfonierung in eine entsprechende Acyl- oder Sulfonylverbindung übergeführt werden, oder
eine Verbindung der allgemeinen Formel I, die eine Carboxygruppe enthält, so kann diese mittels Veresterung oder Amidierung in eine entsprechende Ester- oder Aminocarbonylverbindung übergeführt werden, oder
eine Verbindung der allgemeinen Formel I, die eine Cycloalkyleniminogruppe enthält, in der eine Methylengruppe durch ein Schwefelatom ersetzt ist, so kann diese mittels Oxidation in eine entsprechende Sulfinyl- oder Sulfonylverbindung übergeführt werden, oder
eine Verbindung der allgemeinen Formel I, die eine Nitrogruppe enthält, so kann diese mittels Reduktion in eine entsprechende Aminoverbindung übergeführt werden, oder
eine Verbindung der allgemeinen Formel I, in der R₄ eine durch eine Amino-, Alkylamino-, Aminoalkyl- oder N-Alkyl-aminogruppe substituierte Phenylgruppe darstellt, so kann diese anschliessend mittels Umsetzung mit einem entsprechenden Cyanat, Isocyanat oder Carbamoylhalogenid in eine entsprechende Harnstoffverbindung der allgemeinen Formel I übergeführt werden oder
eine Verbindung der allgemeinen Formel I, in der R₄ eine durch eine Amino-, Alkylamino-, Aminoalkyl- oder N-Alkyl-aminogruppe substituierte Phenylgruppe darstellt, so kann diese anschliessend mittels Umsetzung mit einer entsprechenden die Amidinogruppe übertragenden Verbindung oder durch Umsetzung mit einem entsprechenden Nitril in eine entsprechende Guanidinoverbindung der allgemeinen Formel I übergeführt werden.

Die anschließende Hydrolyse erfolgt vorzugsweise in einem wäßrigen Lösungsmittel, z.B. in Wasser, Methanol/Wasser, Ethanol/Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C.

Die anschließende reduktive Alkylierung wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid, Natriumcyanoborhydrid oder Lithiumaluminiumhydrid bei Temperaturen zwischen 0 und 100°C, vorzugsweise bei Temperaturen zwischen 20 und 80°C, durchgeführt.

Die anschließende Acylierung oder Sulfonylierung wird zweckmäßigerweise mit der entsprechenden freien Säure oder einer entsprechenden reaktionsfähigen Verbindung wie deren Anhydrid, Ester, Imidazolid oder Halogenid vorzugsweise in einem Lösungsmittel wie Methylenchlorid, Diethylether, Tetrahydrofuran, Toluol, Dioxan, Acetonitril, Dimethylsulfoxid oder Dimethylformamid gegebenenfalls in Gegenwart einer anorganischen oder einer tertiären organischen Base bei Temperaturen zwischen -20 und 200°C, vorzugsweise bei Temperaturen zwischen 20°C und der Siedetemperatur des verwendeten Lösungsmittel, durchgeführt. Die Umsetzung mit der freien Säure kann gegebenenfalls in Gegenwart eines die Säure aktivierenden Mittels oder eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluor-borat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylamino-pyridin, N-Methyl-morpholin oder Triethylamin zweckmäßigerweise bei Temperaturen zwischen 0 und 150°C, vorzugsweise bei Temperaturen zwischen 0 und 100°C, erfolgen. Die Umsetzung mit einer entsprechenden reaktionsfähigen Verbindung kann gegebenenfalls in Gegenwart einer tertiären organischen Base wie Triethylamin, N-Ethyl-diisopropylamin, N-Methyl-morpholin oder Pyridin oder bei Verwendung eines Anhydrids bei Gegenwart der entsprechenden Säure bei Temperaturen zwischen 0 und 150°C, vorzugsweise bei Temperaturen zwischen 50 und 100°C, erfolgen.

Die anschließende Veresterung oder Amidierung wird zweckmäßigerweise durch Umsetzung eines reaktionsfähigen entsprechenden Carbonsäurederivates mit einem entsprechenden Alkohol oder Amin wie vorstehend beschrieben durchgeführt.

Die anschließende Oxidation des Schwefelatoms wird vorzugsweise in einem Lösungsmittel oder Lösungsmittelgemisch, z.B. in Wasser, Wasser/Pyridin, Aceton, Methylenchlorid, Essigsäure, Essigsäure/Acetanhydrid, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer entsprechenden Sulfinylverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid, Chloroform oder Dioxan bei -20 bis 80°C, mit Natriummetaperjodat in wäßrigem Methanol oder Ethanol bei -15 bis 25°C, mit Brom in Eisessig oder wäßriger Essigsäure gegebenenfalls in Gegenwart einer schwachen Base wie Natriumacetat, mit N-Bromsuccinimid in Ethanol, mit tert.Butylhypochlorit in Methanol bei -80 bis -30°C, mit lodbenzodichlorid in wäßrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioether-Chlor-Komplex wird zweckmäßigerweise mit wäßrigem Ethanol hydrolysiert.

Zur Herstellung einer Sulfonylverbindung der allgemeinen Formel I wird die Oxidation ausgehend von einer entsprechenden Sulfinylverbindung zweckmäßigerweise mit einem oder mehr Äquivalenten des verwendeten Oxidationsmittels oder ausgehend von einer entsprechenden Mercaptoverbindung zweckmäßigerweise mit zwei oder mehr Äquivalenten des verwendeten Oxidationsmittels durchgeführt, z.B. mit Wasserstoffperoxid in Eisessig/Acetanhydrid, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure, Natriumperjodat oder Kaliumpermanganat in Essigsäure, Wasser/Schwefelsäure oder in Aceton bei 0 bis 20°C.

Die anschließende Reduktion einer Nitrogruppe erfolgt vorzugsweise hydrogenolytisch, z.B. mit Wasserstoff in Gegenwart eines Katalysators wie Palladium/Kohle oder Raney-Nickel in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure oder Eisessig bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die anschließende Herstellung einer entsprechenden Harnstoffverbindung der allgemeinen Formel I wird zweckmäßigerweise mit einem anorganischen Cyanat oder einem entsprechenden Isocyanat oder Carbamoylchlorid vorzugsweise in einem Lösungsmittel wie Dimethylformamid und gegebenenfalls in Gegenwart einer tertiären organischen Base wie Triethylamin bei Temperaturen zwischen 0 und 50°C, vorzugsweise bei der Raumtemperatur, durchgeführt.

Die anschließende Herstellung einer entsprechenden Guanidinoverbindung der allgemeinen Formel I wird zweckmäßigerweise durch Umsetzung mit einer die Amidinogruppe übertragenden Verbindung wie 3,5-Dimethylpyrazol-1-carbonsäure-amidin vorzugsweise in einem Lösungsmittel wie Dimethylformamid und gegebenenfalls in Gegenwart einer tertiären organischen Base wie Triethylamin bei Temperaturen zwischen 0 und 50°C, vorzugsweise bei der Raumtemperatur, durchgeführt.

Bei den vorstehend beschriebenen Umsetzungen können gegebenenfalls vorhandene reaktive Gruppen wie Carboxy-, Hydroxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Carboxygruppe die Trimethylsilyl-, Methyl-, Ethyl-, tert.Butyl-, Benzyl- oder Tetrahydropyranylgruppe und
als Schutzrest für eine Hydroxy-, Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure oder Eisessig bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan, Essigester oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können erhaltene chirale Verbindungen der allgemeinen Formel I in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes zwei asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen Gemisches diastereomerer Salze oder Derivate, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D-und L-Formen von Weinsäure, Dibenzoylweinsäure, Di-o-Tolylweinsäure, Apfelsäure, Mandelsäure, Camphersulfonsäure, Glutaminsäure, N-Acetylglutaminsäure, Asparaginsäure, N-Acetyl-asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure, Maleinsäure, Methansulfonsäure oder Ethansulfonsäure in Betracht.

Außerdem lassen sich die so erhaltenen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Die als Ausgangsprodukte verwendeten Verbindungen der allgemeinen Formeln II bis IX sind teilweise literaturbekannt oder man erhält diese nach literaturbekannten Verfahren oder können nach den vorstehend und in den Beispielen beschriebenen Verfahren erhalten werden. Beispielsweise sind die Verbindungen der allgemeinen Formel VIII aus den Verbindungen der allgemeinen Formel I, in denen R₄ eine durch eine Hydroxy-C₁₋₃-alkylgruppe substituierte Phenylgruppe darstellt, beispielsweise durch Umsetzung mit Alkyl- oder Arylsulfonylchloriden zugänglich. Wie bereits eingangs erwähnt, weisen die Verbindungen der allgemeinen Formel I, in der R₁ ein Wasserstoffatom oder einen Prodrugrest darstellt, wertvolle pharmakologische Eigenschaften auf, insbesondere eine inhibierende Wirkung auf verschiedene Kinasen, vor allem auf Rezeptor-Tyrosinkinasen wie VEGFR1, VEGFR2, VEGFR3, PDGFRα, PDGFRβ, FGFR1, FGFR3, EGFR, HER2, IGF1R und HGFR, sowie auf Komplexe von CDK's (Cyclin Dependent Kinases) wie CDK1, CDK2, CDK3, CDK4, CDK5, CDK6, CDK7, CDK8 und CDK9 mit ihren spezifischen Cyclinen (A, B1, B2, C, D1, D2, D3, E, F, G1, G2, H, I und K) und auf virales Cyclin, auf die Proliferation kultivierter humaner Zellen, insbesondere die von Endothelzellen, z.B. bei der Angiogenese, aber auch auf die Proliferation anderer Zellen, insbesondere von Tumorzellen.

Die biologischen Eigenschaften der Verbindungen wurde nach folgendem Standardverfahren wie folgt geprüft:
Humane Nabelschnur Endothelzellen (HUVEC) wurden in IMDM (Gibco BRL), supplementiert mit 10 % foetalem Rinderserum (FBS) (Sigma), 50 µM β-Mercaptoeethanol (Fluka), Standardantibiotika, 15 µg/ml Endothelzellwachstumsfaktor (ECGS, Collaborative Biomedical Products) und 100 µg/ml Heparin (Sigma) auf Gelatine-beschichteten Kulturflaschen (0.2 % Gelatine, Sigma) bei 37°C, 5 % CO₂ in wassergesättigter Atmosphäre kultiviert.

Zur Untersuchung der inhibitorischen Aktivität der Verbindungen wurden die Zellen für 16 Stunden "gehungert", d.h. in Kulturmedium ohne Wachstumsfaktoren (ECGS + Heparin) gehalten. Die Zellen wurden mittels Trypsin/EDTA von den Kulturflaschen abgelöst und einmal in serumhaltigem Medium gewaschen. Anschließend wurden 2,5 x 10³ Zellen pro well ausgesät.

Die Proliferation der Zellen wurde mit 5 ng/ml VEGF₁₆₅ (vascular endothelial growth factor; H. Weich, GBF Braunschweig) und 10 µg/ml Heparin stimuliert. Pro Platte wurden jeweils 6 wells als Kontrollwert nicht stimuliert.

Die Verbindungen wurden in 100 % Dimethylsulfoxid gelöst und in verschiedenen Verdünnungen als Dreifachbestimmungen den Kulturen zugefügt, wobei die maximale Dimethylsulfoxid-Konzentration 0.3 % betrug.

Die Zellen wurden für 76 Stunden bei 37°C inkubiert, dann wurde für weitere 16 Stunden ³H-Thymidin (0.1 µ Ci/well, Amersham) zugegeben, um die DNA Synthese zu bestimmen. Anschließend wurden die radioaktiv markierten Zellen auf Filtermatten immobilisiert und die eingebaute Radioaktivität in einem β-counter bestimmt. Zur Bestimmung der inhibitorischen Aktivität der Verbindungen wurde der Mittelwert der nicht-stimulierten Zellen vom Mittelwert der Faktor-stimulierten Zellen (in Anwesenheit oder Abwesenheit der erfindungsgemäßen Verbindungen) subtrahiert.

Die relative Zellproliferation wurde in Prozent der Kontrolle (HUVEC ohne Inhibitor) berechnet und die Wirkstoffkonzentration, die die Proliferation der Zellen zu 50 % hemmt (IC₅₀), abgeleitet.

Beispielhaft werden die Testergebnisse der folgenden Verbindungen (a) bis (bd) der allgemeinen Formel I angegeben:
(a) 3-(*Z*)-{1-[4-(Dimethylaminomethyl)-phenylamino]-1-(3,4-methylendioxyphenyl)-methylen}-6-chlor-2-indolinon
(b) 3-(*Z*)-{1-(4-[*N*-Methyl-*N*-(4-methylpiperazin-1-yl-methylcarbonyl)-amino]-phenylamino)-1-(chinoxalin-6-yl)-methylen}-6-chlor-2-indolinon
(c) 3-(*Z*)-{1-[4-(*N*-Ethyl-*N*-methyl-aminomethyl)-phenylamino]-1-(3,4-methylendioxyphenyl)-methylen}-6-chlor-2-indolinon
(d) 3-(*Z*)-{1-[4-(N-Methyl-N-{2-(dimethylamino)-ethyl-carbonyl}-amino)-phenylamino]-1-(3,4-ethylendioxyphenyl)-methylen}-6-fluor-2-indolinon
(e) 3-(*Z*)-{1-[4-(1,2,4-Triazol-1-yl-methyl)-phenylamino]-1-(1-methyl-benzimidazol-5-yl)-methylen}-6-fluor-2-indolinon
(f) 3-(*Z*)-{1-[4-(Dimethylaminomethyl)-phenylamino]-1-(1-methyl-benzimidazol-5-yl)-methylen}-6-chlor-2-indolinon
(g) 3-(*Z*)-{1-[4-(Dimethylaminomethyl)-phenylamino]-1-(3,4-ethylendioxyphenyl)-methylen}-6-chlor-2-indolinon
(h) 3-(*Z*)-{1-(4-[*N*-Methansulfonyl-*N*-(2-dimethylaminoethyl)-amino]-phenylamino)-1-(3,4-ethylendioxyphenyl)-methylen}-6-chlor-2-indolinon
(i) 3-(*Z*)-{1-[4-(Dimethylaminomethyl)-phenylamino]-1-(3,4-ethylendioxyphenyl)-methylen}-6-methoxycarbonyl-2-indolinon
(j) 3-(*Z*)-{1-(4-[*N*-Methyl-*N*-(4-methylpiperazin-1-yl-methylcarbonyl)-amino]-phenylamino)-1-(3,4-ethylendioxyphenyl)-methylen}-6-methoxycarbonyl-2-indolinon
(k) 3-(*Z*)-{1-(4-[*N*-Acetyl-*N*-(2-dimethylaminoethyl)-amino]-phenylamino)-1-(3,4-ethylendioxyphenyl)-methylen}-6-fluor-2-indolinon
(l) 3-(*Z*)-{1-[4-(Ethylaminomethyl)-phenylamino]-1-(3,4-ethylendioxyphenyl)-methylen}-6-fluor-2-indolinon
(m) 3-(*Z*)-{1-(4-[*N*-Acetyl-*N*-(3-dimethylaminopropyl)-amino]-phenylamino)-1-(3,4-ethylendioxyphenyl)-methylen}-6-chlor-2-indolinon
(n) 3-(*Z*)-{1-(4-[*N*-Propionyl-*N*-(3-dimethylaminopropyl)-amino]-phenylamino)-1-(3,4-ethylendioxyphenyl)-methylen}-6-chfor-2-indolinon
(o) 3-(*Z*)-{1-(4-[*N*-Propionyl-*N*-(2-dimethylaminoethyl)-amino]-phenylamino)-1-(3,4-ethylendioxyphenyl)-methylen}-6-chlor-2-indolinon
(p) 3-(*Z*)-{1-(4-[*N*-Acetyl-*N*-(2-dimethylaminoethyl)-amino]-phenylamino)-1-(3,4-ethylendioxyphenyl)-methylen}-6-chlor-2-indolinon
(q) 3-(*Z*)-{1-(4-[4-Methylpiperazin-1-yl-methyl]-phenylamino)-1-(3,4-methylendioxyphenyl)-methylen}-6-chlor-2-indolinon
(r) 3-(*Z*)-{1-(4-[*N*-Methansulfonyl-*N*-(2-dimethylaminoethyl)-amino]-phenylamino)-1-(3,4-ethylendioxyphenyl)-methylen}-6-methoxycarbonyl-2-indolinon
(s) 3-(*Z*)-{1-(4-[Pyrrolidin-1-yl-methyl]-phenylamino)-1-(3,4-ethylendioxyphenyl)-methylen}-6-methoxycarbonyl-2-indolinon
(t) 3-(*Z*}-{1-(4-[*N*-Methyl-*N*-(dimethylaminomethylcarbonyl)-amino]-phenylamino)-1-(3,4-ethylendioxyphenyl)-methylen}-6-methoxycarbonyl-2-indolinon
(u) 3-(*Z*)-{1-(4-[Ethylamino-methyl]-phenylamino)-1-(3,4-ethylendioxyphenyl)-methylen}-6-methoxycarbonyl-2-indolinon
(v) 3-(*Z*)-{1-(4-[4-Methylpiperazin-1-yl-methyl]-phenylamino)-1-(3,4-ethylendioxyphenyl)-methylen}-6-methoxycarbonyl-2-indolinon
(w) 3-(*Z*)-{1-(4-[Dimethylamino-methyl]-phenylamino)-1-(3,4-methylendioxyphenyl)-methylen}-6-methoxycarbonyl-2-indolinon
(x) 3-(*Z*)-{1-(4-[Diethylamino-methyl]-phenylamino)-1-(3,4-methylendioxyphenyl)-methylen}-6-methoxycarbonyl-2-indolinon
(y) 3-(*Z*)-{1-[4-(Dimethylaminocarbonyl)-phenylamino]-1-(1-methyl-benzimidazol-5-yl)-methylen}-6-fluor-2-indolinon
(z) 3-(*Z*)-{1-(4-[*N*-Propionyl-*N*-(3-dimethylaminopropyl)-amino]-phenylamino)-1-(3,4-ethylendioxyphenyl)-methylen}-6-fluor-2-indolinon
(aa) 3-(*Z*)-{1-(4-[*N*-Propionyl-*N*-(2-dimethylaminoethyl)-amino]-phenylamino)-1-(3,4-ethylendioxyphenyl)-methylen}-6-fluor-2-indolinon
(ab) 3-(*Z*)-{1-(4-[*N*-Methansulfonyl-*N*-(2-dimethylaminoethyl)-amino]-phenylamino)-1-(3,4-ethylendioxyphenyl)-methylen}-6-fluor-2-indolinon
(ac) 3-(*Z*)-{1-(4-[*N*-Acetyl-*N*-(2-dimethylaminoethyl)-amino]-phenylamino)-1-(3,4-ethylendioxyphenyl)-methylen}-6-methoxycarbonyl-2-indolinon
(ad) 3-(*Z*)-{1-(4-[N-Methyl-N-(2-dimethylaminoethyl)aminocarbonyl]-phenylamino)-1-(3,4-ethylendioxyphenyl)-methylen}-6-methoxycarbonyl-2-indolinon
(ae) 3-(*Z*)-{1-(4-[N-Methyl-N-(3-dimethylaminopropyl)aminocarbonyl]-phenylamino)-1-(3,4-ethylendioxyphenyl)-methylen}-6-methoxycarbonyl-2-indolinon
(af) 3-(*Z*)-{1-(4-[N-Methyl-N-(2-dimethylaminoethyl)aminocarbonyl]-phenylamino)-1-(3,4-methylendioxyphenyl)-methylen}-6-methoxycarbonyl-2-indolinon
(ag) 3-(*Z*)-{1-[4-(Dimethylaminomethyl)-phenylamino]-1-(benzimidazol-5-yl)-methylen}-2-indolinon
(ah) 3-(*Z*)-{1-(4-[1-Methyl-piperazin-4-yl-methyl]-phenylamino)-1-(3,4-ethylendioxy-phenyl)-methylen}-6-dimethylaminocarbonyl-2-indolinon
(ai) 3-(*Z*)-{1-[4-(Pyrrolidin-1-yl-methyl)-phenylamino]-1-(benzimidazol-5-yl)-methylen}-6-chlor-2-indolinon
(aj) 3-(*Z*)-{1-(4-[*N*-Methyl-*N*-(dimethylaminomethylcarbonyl)-amino]-phenylamino)-1-(3,4-methylendioxyphenyl)-methylen}-2-indolinon
(ak) 3-(*Z*)-{1-(4-[*N*-Methyl-*N*-(dimethylaminomethylcarbonyl)-amino]-phenylamino)-1-(1-methyl-benzimidazol-5-yl)-methylen}-6-chlor-2-indolinon
(al) 3-(*Z*)-{1-(4-[*N*-Acetyl-*N*-(2-dimethylaminoethyl)-amino]-phenylamino)-1-(3,4-(difluormethylendioxy)phenyl)-methylen}-6-chlor-2-indolinon
(am) 3-(*Z*)-{1-(4-[*N*-Methyl-*N*-(1-methyl-4-piperidinyl)-aminocarbonyl]-phenylamino)-1-(3,4-ethylendioxyphenyl)-methylenl-6-fluor-2-indolinon
(an) 3-(*Z*)-{1-(4-[*N*-Methyl-*N*-(dimethylaminomethylcarbonyl)-amino]-phenylamino)-1-(3-furyl)-methylen}-2-indolinon
(ao) 3-(*Z*)-{1-(4-[*N*-(3-Pyridylcarbonyl)-*N*-(2-dimethylaminoethyl)-amino]-phenylamino)-1-(3,4-methylendioxyphenyl)-methylen}-6-chlor-2-indolinon
(ap) 3-(*Z*)-{1-(4-[2-(Diethylamino)ethyl-sulfonyl]-phenylamino)-1-(3,4-methylendioxyphenyl)-methylen}-6-chlor-2-indolinon
(aq) 3-(*Z*)-{1-[4-(Dimethylaminomethyl)-phenylamino]-1-(1-methyl-benzimidazol-5-yl)-methylen}-2-indolinon
(ar) 3-(*Z*)-{1-(4-[1-Methylimidazol-2-yl]-phenylamino)-1-(chinoxalin-6-yl)-methylen}-6-chlor-2-indolinon
(as) 3-(*Z*)-{1-[4-(Pyrrolid-2-on-1-yl)-phenylamino]-1-(1-methyl-benzimidazol-5-yl)-methylen}-6-fluor-2-indolinon
(at) 3-(*Z*)-{1-[4-(3,5-Dimethyl-pyrazol-1-yl)-phenylamino]-1-(1-methyl-benzimidazol-5-yl)-methylen}-6-fluor-2-indolinon
(au) 3-(*Z*)-{1-[4-(N-Methyl-N-{2-dimethylamino-ethyl}-aminosulfonyl)-phenylamino]-1-(1-methyl-benzimidazol-5-yl)-methylen}-6-fluor-2-indolinon
(av) 3-(*Z*)-{1-[4-(Dimethylaminomethyl)-phenylamino]-1-(1-methyl-benzotriazol-5-yl)-methylen}-2-indolinon
(aw) 3-(*Z*)-{1-(4-[(1-Methyl-piperazin-4-yl)-carbonylmethyl]-phenylamino)-1-(3,4-ethylendioxyphenyl)-methylen}-6-fluor-2-indolinon
(ax) 3-(*Z*)-{1-(4-[*N*-(Propansulfonyl)-*N*-(2-dimethylaminoethyl)-amino]-phenylamino)-1-(4-pyridyl)-methylen}-6-chlor-2-indolinon
(ay) 3-(*Z*)-{1-[4-(Pyrrolidin-1-yl-methyl)-phenylamino]-1-(2-methyl-benzimidazol-5-yl)-methylen}-6-chlor-2-indolinon
(az) 3-(*Z*)-{1-(4-[*N*-Methyl-*N*-(4-methylpiperazin-1-yl-methylcarbonyl)-amino]-phenylamino)-1-(3,4-methylendioxyphenyl)-methylen}-6-chlor-2-indolinon
(ba) 6-Chlor-3-(Z)-[1-(4-{N-(2-dimethylamino-ethyl)-N-methansulfonyl-amino}-3-chlor-phenylamino)-1-(1,2-ethylendioxyphen-4-yl)-methylen]-2-indolinon
(bb) 6-Chlor-3-(Z)-{1-[4-(N-dimethylaminomethylcarbonyl-N-methyl-amino)-3-methoxy-phenylamino]-1-(1,2-ethylendioxyphen-4-yl)-methylen}-2-indolinon
(bc) 6-Fluor-3-(Z)-{1-[4-(N-dimethylaminomethylcarbonyl-N-methyl-amino)-3-methoxy-phenylamino]-1-(1,2-ethylendioxyphen-4-yl)-methylen}-2-indolinon
(bd) 6-Fluor-3-(Z)-[1-(4-{N-(2-dimethylamino-ethyl)-N-methansulfonyl-amino}-3-chlor-phenylamino)-1-(1,2-ethylendioxyphen-4-yl)-methylen]-2-indolinon

Die nachfolgende Tabelle enthält die gefundenen Ergebnisse:

| Verbindung | IC₅₀ [nM] |
|---|---|
| (a) | 11 |
| (b) | 4 |
| (c) | 2 |
| (d) | 3 |
| (e) | 16 |
| (f) | 2 |
| (g) | 9 |
| (h) | 3 |
| (i) | 0,5 |
| (j) | 0,2 |
| (k) | 0,7 |
| (l) | 10 |
| (m) | 0,3 |
| (n) | 1 |
| (o) | 1 |
| (p) | 2 |
| (q) | 2 |
| (r) | 0,2 |
| (s) | 0,6 |
| (t) | 0,6 |
| (u) | 0,8 |
| (v) | 1 |
| (w) | 0,5 |
| (x) | 1 |
| (y) | 14 |
| (z) | 0,5 |
| (aa) | 0,6 |
| (ab) | 1 |
| (ac) | 0,2 |
| (ad) | 0,4 |
| (ae) | 0,5 |
| (af) | 0,5 |
| (ag) | 31 |
| (ah) | 100 |
| (ai) | 3 |
| (aj) | 13 |
| (ak) | 30 |
| (al) | 83 |
| (am) | 5 |
| (an) | 58 |
| (ao) | 6 |
| (ap) | 120 |
| (aq) | 24 |
| (ar) | 71 |
| (as) | 18 |
| (at) | 30 |
| (au) | 13 |
| (av) | 94 |
| (aw) | 4 |
| (ax) | 37 |
| (ay) | 19 |
| (az) | 6 |
| (ba) | 30 |
| (bb) | 33 |
| (bc) | 5 |
| (bd) | 5 |

Auf Grund ihrer Hemmwirkung auf die Proliferation von Zellen, insbesondere von Endothelzellen und von Tumorzellen, eignen sich die Verbindungen der allgemeinen Formel I zur Behandlung von Krankheiten, in denen die Proliferation von Zellen, insbesondere die von Endothelzellen, eine Rolle spielt.

So stellt beispielsweise die Proliferation von Endothelzellen und die damit verbundene Neovaskularisierung einen entscheidenden Schritt bei der Tumorprogression dar (Folkman J. et al., Nature 339, 58-61, (1989); Hanahan D. und Folkman J., Cell 86, 353-365, (1996)). Weiterhin ist die Proliferation von Endothelzellen auch bei Hämangiomen, bei der Metastasierung, der rheumatischen Arthritis, der Psoriasis und der okularen Neovaskularisierung von Bedeutung (Folkman J., Nature Med. 1, 27-31, (1995)). Der therapeutische Nutzen von Inhibitoren der Endothelzellproliferation wurde im Tiermodell beispielsweise von O'Reilly et al. und Parangi et al. gezeigt (O'Reilly M.S. et al., Cell 88, 277-285, (1997); Parangi S. et al., Proc Natl Acad Sci USA 93, 2002-2007, (1996)).

Die Verbindungen der allgemeinen Formel I, deren Tautomeren, deren Stereoisomere oder deren physiologisch verträglichen Salze eignen sich somit beispielsweise zur Behandlung von Tumoren (z. B. Plattenepithelkarzinom, Astrozytom, Kaposi's Sarkom, Glioblastom, Lungenkrebs, Blasenkrebs, Hals- und Nacken-karzimom, Melanom, Ovarkarzinom, Prostatakarzinom, Brustkrebs, kleinzelliges Lungenkarzinom, Gliom, Colorektalkarzinom, urogenital Krebs und gastrointestinal Karzinom sowie hämatologischer Krebserkrankungen, wie multiples Myelom), Psoriasis, Arthritis (z. B. rheumatoide Arthritis), Hämangioma, Angiofibroma, Augenerkrankungen (z.B. diabetische Retinopathie), neovaskulares Glaukom, Nierenerkrankungen (z.B. Glomerulonephritis), diabetische Nephropathie, maligne Nephrosklerose, thrombische mikroangiopathische Syndrome, Transplantationsabstossungen und Glomerulopathie, fibrotische Erkrankungen (z. B. Leberzirrhose), mesangialzellproliferative Erkrankungen, Artheriosklerose, Verletzungen des Nervengewebes und zur Hemmung der Reocclusion von Gefässen nach Ballonkatheterbehandlung, bei der Gefässprothetik oder nach dem Einsetzen von mechanischen Vorrichtungen zum Offenhalten von Gefässen (z.B. Stents), oder anderen Erkrankungen, bei denen Zellproliferation oder Angiogenese eine Rolle spielen.

Auf Grund ihrer biologischen Eigenschaften können die Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin, Taxol), mit Nukleinsäuren interagierenden Verbindungen (z.B. cis-Platin, Cyclophosphamid, Adriamycin), Hormon-Antagonisten (z.B. Tamoxifen), inhibitoren metabolischer Prozelle (z.B. 5-FU etc.), Zytokinen (z.B. interferonen), Rezeptor-Tyrosin-Kinase- und linase-Inhibitoren, Antikörpern, oder auch in Kombination mit Strahlentherapie etc. Diese Kombinationen können entweder simultan oder sequentiell verabreicht werden.

Bei der pharmazeutischen Anwendung werden die Verbindungen in der Regel bei warmblütigen Wirbeltieren, insbesondere beim Menschen, in Dosierungen von 0,01-100 mg/kg Körpergewicht, vorzugsweise bei 0,1-20 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanzen wie Hartfett oder deren geeigneten Gemischen in übliche galenische Zubereitungen wie Tabletten, Dragees, Kapseln, Pulver, Injektionslösungen, Ampullen, Suspensionen, Lösungen, Sprays oder Zäpfchen eingearbeitet.

Die nachfolgenden Beispiele sollen die Erfindung näher erläutern:

### Verwendete Abkürzungen:

- DMF=: *N,N*-Dimethylformamid
- DMSO=: Dimethylsulfoxid
- HOBT =: 1-Hydroxy-1H-benzotriazol
- TBTU =: O-Benzotriazol-1-yl-N,N,N',N'-tetramethyluronium-tetrafluoroborat
- THF =: Tetrahydrofuran

### Herstellung der Ausgangsverbindungen:

### Beispiel I:

### 5-Carboxy-2-methyl-isoindol-1,3-dion

19.2 g Trimellithsäureanhydrid werden in 100 ml *N*-Methylformamid 4 Stunden bei 140 °C und anschließend bei Raumtemperatur über Nacht gerührt. Nach Zugabe von 300 ml Wasser wird weitere 12 Stunden gerührt; anschließend wird der Niederschlag abgesaugt, mit Wasser gewaschen und bei 80 °C getrocknet.
Ausbeute: 15.5 g (75 % der Theorie)
R_{f}-Wert: 0.59 (RP-8, Methanol / 5%ige Natriumchlorid-Lösung = 7:3) C₁₀H₇NO₄
Massenspektrum: m/z = 204 [M-H]⁻

### Beispiel II:

### 3,4-Ethylendioxybenzoesäure

Eine Suspension von 80.1 g Calciumhypochlorit in 360 ml Wasser und eine Suspension von 6.72 g Natriumhydroxid und 56.4 g Natriumcarbonat in 170 ml Wasser werden vereinigt und unter Rühren auf 50 °C erwärmt. Der Niederschlag wird durch Filtration entfernt und die erhaltene Lösung mit 25.0 g 1,4-Benzodioxan-6-yl-methylketon versetzt. Der Ansatz wird 15 h bei 60 °C gerührt, nach Abkühlung auf Raumtemperatur mit Ethylacetat extrahiert. Die wäßrige Phase wird durch Zusatz von konzentrierter Salzsäure unter Eiskühlung auf einen pH-Wert von 3 eingestellt. Das ausgefällte Produkt wird abgesaugt, mit Wasser gewaschen und bei 90 °C getrocknet.
Ausbeute: 18.8 g (74 % der Theorie)
R_{f}-Wert: 0.65 (Kieselgel, Dichlormethan/Methanol/Essigsäure = 90:10:1) C₉H₈O₄

### Beispiel III:

### 1-Methylbenzimidazol-5-carbonsäure

25.0 g 4-Methylamino-3-nitrobenzoesäure, gelöst in 200 ml DMF werden unter Zusatz von 2.5 g Palladium auf Aktivkohle (10 %) 5 Stunden lang bei 30 psi Wasserstoffdruck hydriert. Der Katalysator wird abgesaugt, das Lösungsmittel abdestilliert. Der Rückstand wird mit Diethylether verrührt , abgesaugt und getrocknet. Das so erhaltene Rohprodukt (19.7 g) wird 2 Stunden in 250 ml Ameisensäure unter Rückfluß erhitzt. Nach Abdestillieren des Lösungsmittels wird der Rückstand mit Diethylether verrührt, abgesaugt und getrocknet.
Ausbeute: 21 g (94 % der Theorie)
R_{f}-Wert: 0.25 (Kieselgel, Dichlormethan/Methanol/Essigsäure = 90:10:1) C₉H₈N₂O₂

### Beispiel IV:

### 2-Dibenzylaminooxazol-4-carbonsäure

21.6 g N-Benzylharnstoff und 28.7 g Brombrenztraubensäureethylester werden in 120 ml Ethanol 18 Stunden unter Rückfluß erhitzt. Der durch Abdestillieren des Lösungsmittels erhaltene ölige Rückstand wird mit Sodalösung (10 g in 170 ml Wasser) und 100 ml Diethylether versetzt und 2 Stunden gerührt. Nach Zugabe von 200 ml Ethylacetat werden die Phasen getrennt, die organische Phase zweimal mit Wasser extrahiert und zur Trockne eingedampft.

Das Zwischenprodukt (11.8 g) wird in 80 ml DMSO gelöst und mit 5.95 g Kalium-*tert*-butylat versetzt. Nach 90minütigem Rühren bei Raumtemperatur werden langsam 6.43 mal Benzylbromid zugetropft und der Ansatz weitere 3 Stunden gerührt. Der Ansatz wird auf 400 ml Eiswasser gegossen und zweimal mit Ethylacetat extrahiert. Die organischen Phasen werden vereinigt, mit Wasser gegenextrahiert und zur Trockne eingedampft.

Zur Esterverseifung wird der Rückstand in 200 ml Ethanol gelöst, mit 100 ml 1-molarer Natronlauge versetzt und 2 Stunden gerührt. Nach Abdestillieren des Ethanols wird mit Diethylether extrahiert, die wäßrige Phase mit 100 ml 1 molarer Salzsäure neutralisiert und mit Ethylacetat extrahiert. Die Ethylacetat-Phase wird mit Wasser gegenextrahiert, mit Natriumsulfat getrocknet und zur Trockne eingeengt. Das ölige Rohprodukt wird mit Petrolether/Diethylether verrührt, abgesaugt und bei 40 °C getrocknet.
Ausbeute: 11.6 g (28 % der Theorie)
R_{f}-Wert: 0.4 (Kieselgel, Dichlormethan/Methanol/Essigsäure 90:10:1) C₁₈H₁₆N₂O₃
Massenspektrum: m/z = 309 [M+H]⁺

### Beispiel V:

### 2-(4-Fluor-2-nitrophenyl)-malonsäuredimethylester

Zu einer Lösung von 188 ml Malonsäuredimethylester in 970 ml N-Methylpyrrolidon werden unter Eiskühlung 185 g Kalium-*tert*-butylat gegeben und der Ansatz 2 Stunden nachgerührt. Der entstandene Brei wird im Laufe von 30 Minuten tropfenweise mit 150 ml 2,5-Difluornitrobenzol versetzt und anschließend 6 Stunden bei 85 °C nachgerührt. Die Mischung wird auf 4 Liter Eiswasser und 250 ml konzentrierte Salzsäure gegossen und mit 2 Liter Ethylacatat extrahiert. Die organische Phase wird mit Natriumsulfat getrocknet und eingeengt. Der ölige Rückstand wird zweimal mit Wasser ausgerührt und anschließend in 600 ml Ethylacetat aufgenommen. Die Lösung wird mit Natriumsulfat getrocknet und zur Trockne eingeengt. Das kristallisierte Rohprodukt wird aus 600 ml Ethylacetat/Hexan = 2:8 umkristallisiert und getrocknet.
Ausbeute: 222 g (59 % der Theorie)
R_{f}-Wert: 0.4 (Kieselgel, Cyclohexan/Ethylacetat = 5:1) C₁₁H₁₀FNO₆
Massenspektrum: m/z = 270 [M-H]⁻
Analog Beispiel V wird folgende Verbindung hergestellt:
(V.1) 2-(4-Brom-2-nitrophenyl)-malonsäurediethylester
aus 2,5-Dibromnitrobenzol und Malonsäurediethylester
R_{f}-Wert: 0.40 (Kieselgel, Petrolether/Ethylacetat = 5:1)
C₁₃H₁₄BrNO₆
Massenspektrum: m/z = 359/361 [M]⁺

### Beispiel VI:

### 4-Fluor-2-nitrophenylessigsäure

50.0 g 2-(4-Fluor-2-nitrophenyl)-malonsäuredimethylester werden in 400 ml 6 molarer Salzsäure 20 Stunden bei 100 °C gerührt, anschließend mit 400 ml Wasser versetzt und auf 0 °C abgekühlt. Der entstandene Niederschlag wird abgesaugt, mit Wasser und 100 ml Petrolether gewaschen und getrocknet.
Ausbeute: 34.5 g (94 % der Theorie)
R_{f}-Wert: 0.3 (Kieselgel, Cyclohexan/Ethylacetat = 5:2
C₈H₆FNO₄
Massenspektrum: m/z = 244 [M+2Na-H]⁺

### Beispiel VII:

### 6-Fluor-2-indolinon

119 g 4-Fluor-2-nitrophenylessigsäure werden in 600 ml Essigsäure unter Zusatz von 20 g Palladium auf Aktivkohle (10%) unter 50 psi Wasserstoffdruck hydriert. Der Katalysator wird abgesaugt, das Lösungsmittel abdestilliert. Das Rohprodukt wird mit 500 ml Petrolether ausgerührt, abgesaugt, mit Wasser gewaschen und getrocknet.
Ausbeute: 82.5 g (91 % der Theorie)
R_{f}-Wert: 0.31 (Kieselgel, Petrolether/Ethylacetat = 1:1)
C₈H₆FNO
Massenspektrum: m/z = 210 [M+CH₃COO]⁻

### Analog Beispiel VII wird folgende Verbindung hergestellt:

(VII.1) 6-Brom-2-indolinon
aus 2-(4-Brom-2-nitrophenyl)-malonsäurediethylester (Edukt V.1) mit Raney-Nickel als Hydrierkatalysator
R_{f}-Wert: 0.45 (Kieselgel, Petrolether/Ethylacetat = 1:1)
C₈H₆BrNO
Massenspektrum: m/z = 210/212 [M-H]⁻

### Beispiel VIII:

### 1-Acetyl-6-Fluor-2-indolinon

82.5 g 6-Fluor-2-indolinon werden in 180 ml Essigsäureanhydrid 3 Stunden bei 130 °C gerührt. Nach Abkühlen auf Raumtemperatur wird der Niederschlag abgesaugt, mit 100 ml Petrolether gewaschen und getrocknet.
Ausbeute: 64.8 g (61 % der Theorie)
R_{f}-Wert: 0.75(Kieselgel, Petrolether/Ethylacetat = 1:1)
C₁₀H₈FNO₂
Massenspektrum: m/z = 192 [M-H]⁻
Analog Beispiel VIII werden folgende Verbindungen hergestellt:
(VIII.1) 1-Acetyl-2-indolinon
   aus 2-Indolinon und Essigsäureanhydrid
(VIII.2) 1-Acetyl-6-chlor-2-indolinon
   aus 6-Chlor-2-indolinon und Essigsäureanhydrid
(VIII.3) 1-Acetyl-6-methoxycarbonyl-2-indolinon
   aus 6-Methoxycarbonyl-2-indolinon und Essigsäureanhydrid
(VIII.4) 1-Acetyl-6-brom-2-indolinon
   aus 6-Brom-indolinon und Essigsäureanhydrid

### Beispiel IX

### 1-Acetyl-3-[1-hydroxy-1-(2-furyl)methylen]-2-indolinon

Eine eisgekühlte Lösung von 7.0 g 1-Acetyl-2-indolinon und 10.8 g DMAP in 60 ml DMF wird mit 4.4 ml Furan-2-carbonsäurechlorid versetzt. Der Ansatz wird 1 Stunde bei Raumtemperatur nachgerührt, dann auf 40 ml konz. Salzsäure und 500 ml Eiswasser gegossen. Der Niederschlag wird abgesaugt, nacheinander mit Ethanol und Diethylether gewaschen und getrocknet.
Ausbeute: 8.67 g (81 % der Theorie)
R_{f}-Wert: 0.6 (Kieselgel, Ethylacetat)
C₁₅H₁₁NO₄
Schmelzpunkt: 128-130 °C
Analog Beispiel IX wird folgende Verbindung hergestellt:
(IX.1) 1-Acetyl-6-chlor-3-[1-hydroxy-1-(2-pyrrolyl)methylen]-2-indolinon aus 1-Acetyl-6-chlor-2-indolinon und Pyrrol-2-carbonsäure

### Beispiel X

### 1-Acetyl-3-[1-hydroxy-1-(2-pyrazinyl)methylen]-2-indolinon

4.38 g 1-Acetyl-2-indolinon, 3.41 g Pyrazin-2-carbonsäure, 8.83 g TBTU, 4.21 g HOBT-hydrat und 21.8 ml Ethyldiisopropylamin werden in 70 ml DMF 15 Stunden bei Raumtemperatur gerührt. Der Ansatz wird auf 400 ml Eiswasser und 10 ml konz. Salzsäure gegossen und 1 Stunde gerührt. Der Niederschlag wird abgesaugt, mit Wasser gewaschen, mit Methanol verrührt, erneut abgesaugt, mit Methanol gewaschen und bei 100 °C getrocknet.
Ausbeute: 4.43 g (63 % der Theorie)
R_{f}-Wert: 0.2 (Kieselgel, Petrolether/Dichlormethan/Methanol = 5:4:1)
C₁₅H₁₁N₃O₃
Massenspektrum: m/z = 280 [M-H]⁻
Analog Beispiel X werden folgende Verbindungen hergestellt:
(X.1) 1-Acetyl-3-[1-hydroxy-1-(3,4-methylendioxyphenyl)methylen]-2-indolinon aus 1-Acetyl-2-indolinon und 3,4-Methylendioxybenzoesäure
(X.2) 1-Acetyl-3-[1-hydroxy-1-(3-thienyl)methylen]-2-indolinon aus 1-Acetyl-2-indolinon und Thiophen-3-carbonsäure
(X.3) 1-Acetyl-3-[1-hydroxy-1-(5-methylisoxazol-3-yl)methylen]-2-indolinon aus 1-Acetyl-2-indolinon und 5-Methylisoxazol-3-carbonsäure
(X.4) 1-Acetyl-3-[1-hydroxy-1-(3-methylpyrazol-5-yl)methylen]-2-indolinon aus 1-Acetyl-2-indolinon und 3-Methylpyrazol-5-carbonsäure
(X.5) 1-Acetyl-3-[1-hydroxy-1-(4-acetylamino-3-nitrophenyl)methylen]-2-indolinon aus 1-Acetyl-2-indolinon und 4-Acetylamino-3-nitrobenzoesäure
(X.6) 1-Acetyl-3-{1-hydroxy-1-[2-(dibenzylamino)oxazol-4-yl]methylen}-2-indolinon aus 1-Acetyl-2-indolinon und 2-(Dibenzylamino)oxazol-4-carbonsäure, wobei der nach Zugabe von Wasser und Salzsäure erhaltene Niederschlag chromatographisch an Kieselgel (Eluent: Petrolether/Dichlormethan/Ethylacetat = 5:4:1) gereinigt, anschließend mit Diethylether verrührt, abgesaugt und getrocknet wird.
(X.7) 1-Acetyl-3-[1-hydroxy-1-( 2-methyl-isoindol-1,3-dion-5-yl)methylen]-2-indolinon
   aus 1-Acetyl-2-indolinon und 2-Methyl-isoindol-1,3-dion-5-carbonsäure
(X.8) 1-Acetyl-6-chlor-3-[1-hydroxy-1-(4-acetylamino-3-nitrophenyl)methylen]-2-indolinon
   aus 1-Acetyl-6-chlor-2-indolinon und 4-Acetylamino-3-nitrobenzoesäure
(X.9) 1-Acetyl-6-chlor-3-[1-hydroxy-1-(4-pyridazinyl)methylen]-2-indolinon aus 1-Acetyl-6-chlor-2-indolinon und Pyridazin-4-carbonsäure
(X.10) 1-Acetyl-6-chlor-3-[1-hydroxy-1-(3,4-methylendioxyphenyl)methylen]-2-indolinon
   aus 1-Acetyl-6-chlor-2-indolinon und 3,4-Methylendioxybenzoesäure
(X.11) 1-Acetyl-6-chlor-3-[1-hydroxy-1-(3,4-ethylendioxyphenyl)methylen]-2-indolinon
   aus 1-Acetyl-6-chlor-2-indolinon und 3,4-Ethylendioxybenzoesäure
(X.12) 1-Acetyl-6-chlor-3-{1-hydroxy-1-[3,4-(difluormethylen)dioxyphenyl]methylen}-2-indolinon
   aus 1-Acetyl-6-chlor-2-indolinon und 3,4-(Difluormethylen)dioxybenzoesäure
(X.13) 1-Acetyl-6-fluor-3-[1-hydroxy-1-(3,4-ethylendioxyphenyl)methylen]-2-indolinon
   aus 1-Acetyl-6-fluor-2-indolinon und 3,4-Ethylendioxybenzoesäure
(X.14) 1-Acetyl-3-[1-hydroxy-1-(3-furyl)methylen]-2-indolinon
   aus 1-Acetyl-2-indolinon und Furan-3-carbonsäure, wobei vor Zugabe des Eiswassers das Lösungsmittel abdestilliert wird.
(X.15) 1-Acetyl-3-[1-hydroxy-1-(4-methylamino-3-nitrophenyl)methylen]-2-indolinon aus 1-Acetyl-2-indolinon und 4-Methylamino-3-nitrobenzoesäure
(X.16) 1-Acetyl-6-fluor-3-[1-hydroxy-1-(4-methylamino-3-nitrophenyl)methylen]-2-indolinon
   aus 1-Acetyl-6-fluor-2-indolinon und 4-Methylamino-3-nitrobenzoesäure
(X.17) 1-Acetyl-6-chlor-3-[1-hydroxy-1-(chinazolin-6-yl)methylen]-2-indolinon aus 1-Acetyl-6-chlor-2-indolinon und Chinazolin-6-carbonsäure (welche durch alkalische Verseifung des Methylesters hergestellt wird).
(X.18) 1-Acetyl-3-[1-hydroxy-1-(1-methyl-benzotriazol-5-yl)methylen]-2-indolinon ausl-Acetyl-2-indolinon und 1-Methylbenzotriazol-5-carbonsäure (welche analog der Darstellung von 1-Methylbenzotriazol, beschrieben in: A. Reissert, Chem. Ber. 47 (1914) 676, hergestellt wird).
(X.19) 1-Acetyl-6-brom-3-[1-hydroxy-1-(3,4-methylendioxyphenyl)methylen]-2-indolinon
   aus 1-Acetyl-6-brom-2-indolinon und 3,4-Methylendioxybenzoesäure
(X.20) 1-Acetyl-6-fluor-3-[1-hydroxy-1-(3,4-methylendioxyphenyl)methylen]-2-indolinon
   aus 1-Acetyl-6-fluor-2-indolinon und 3,4-Methylendioxybenzoesäure

### Beispiel XI

### 1-Acetyl-6-chlor-3-[1-hydroxy-1-(4-pyridyl)methylen]-2-indolinon

6.3 g 1-Acetyl-6-chlor-2-indolinon, 4.06 g Pyridin-4-carbonsäure, 10.6 g TBTU und 21 ml Triethylamin werden in 60 ml DMF 15 Stunden bei Raumtemperatur gerührt. Der Ansatz wird mit Wasser versetzt, anschließend durch Zugabe von 10 %iger Essigsäure auf pH 5 eingestellt Der Niederschlag wird abgesaugt, mit Wasser gewaschen und in Ethylacetat aufgenommen. Die erhaltene Lösung wird mit Natriumsulfat getrocknet und fast zur Trockne eingeengt. Der erhaltene Niederschlag wird abgesaugt und bei 100 °C getrocknet.
Ausbeute: 6.5 g (69 % der Theorie)
R_{f}-Wert: 0.6 (RP8, Methanoi/5%ige NaCl = 4:1)
C₁₆H₁₁ClN₂O₃
Massenspektrum: m/z = 313 [M-H]⁻
Schmelzpunkt: 215-217 °C
Analog Beispiel XI werden folgende Verbindungen hergestellt:
(XI.1) 1-Acetyl-6-chlor-3-[1-hydroxy-1-(3-pyridyl)methylen]-2-indolinon aus 1-Acetyl-6-chlor-2-indolinon und Pyridin-3-carbonsäure
(XI.2) 1-Acetyl-6-methoxycarbonyl-3-[1-hydroxy-1-(3,4-ethylendioxyphenyl)-methylen]-2-indolinon
   aus 1-Acetyl-6-methoxycarbonyl-2-indolinon und 3,4-Ethylendioxybenzoesäure
(XI.3) 1-Acetyl-3-[1-hydroxy-1-(4-imidazolyl)methylen]-2-indolinon
   aus 1-Acetyl-2-indolinon und Imidazol-4-carbonsäure, wobei vor der Wasserzugabe das Lösungsmittel abdestilliert wird.
(XI.4) 1-Acetyl-6-methoxycarbonyl-3-[1-hydroxy-1-(3,4-methylendioxyphenyl)-methylen]-2-indolinon
   aus 1-Acetyl-6-methoxycarbonyl-2-indolinon und 3,4-Methylendioxybenzoesäure

### Beispiel XII

### 1-Acetyl-6-chlor-3-[1-hydroxy-1-(1-methyl-5-benzimidazolyl)methylen]-2-indolinon

3.5 g 1-Acetyl-6-chlor-2-indolinon, 3.4 g 1-Methylbenzimidazol-5-carbonsäure, 6.1 g TBTU, 2.9 g HOBT-hydrat und 8.7 ml Ethyldiisopropylamin werden in 100 ml DMF 15 Stunden bei Raumtemperatur gerührt. Der Ansatz wird mit Wasser versetzt und mit Ethylacetat extrahiert, wobei das Rohprodukt teilweise ausfällt. Der durch Abdestillieren des Lösungsmittels erhaltene Rückstand wird in Dichlormethan und wenig Methanol aufgenommen und mit Wasser extrahiert. Die organische Phase wird eingeengt, der Rückstand nacheinander mit Ethylacetat und Diethylether verrührt, abgesaugt und getrocknet.
Ausbeute: 2.2 g (35 % der Theorie)
R_{f}-Wert: 0.17 (Kieselgel, Ethylacetat/Methanol/Ammoniaklösung = 80:20:1) C₁₉H₁₄ClN₃O₃
Massenspektrum: m/z = 368/390 [M+H]⁺
Analog Beispiel XII wird folgende Verbindung hergestellt:
(XII.1) 1-Acetyl-6-chlor-3-[1-hydroxy-1-(1-benzyl-5-imidazolyl)methylen]-2-indolinon aus 1-Acetyl-6-chlor-2-indolinon und 1-Benzylimidazol-5-carbonsäure, wobei das flüssig anfallende Produkt durch Einengen der Ethylacetatphase erhalten wird.

### Beispiel XIII

### 1-Acetyl-3-[1-chlor-1-(4-acetylamino-3-nitrophenyl)methylen]-2-indolinon

Eine Suspension von 11.4 g 1-Acetyl-3-(1-hydroxy-1-(4-acetylamino-3-nitrophenyl)-methylen)-2-indolinon und 9.37 g Phosphorpentachlorid in 200 ml Dioxan wird 4 Stunden bei 100 °C gerührt. Nach Zugabe von weiteren 2.0 g
Phosphorpentachlorid wird weitere 3 Stunden bei 100 °C gerührt. Anschließend wird das Lösungsmittel abdestilliert, der Rückstand mit 100 ml Ethylacetat verrührt, abgesaugt mit Ethylacetat gewaschen und bei 60 °C getrocknet.
Ausbeute: 6.40 g (53 % der Theorie)
R_{f}-Wert: 0.7 (Kieselgel, Dichlormethan/Ethylacetat = 9:1)
C₁₉H₁₄ClN₃O₅
Massenspektrum: m/z = 398/400 [M-H]⁻
Analog Beispiel XIII werden folgende Verbindungen hergestellt:
(XIII.1) 1-Acetyl-6-chlor-3-[1-chlor-1-(4-acetylamino-3-nitrophenyl)methylen]-2-indolinon
   aus 1-Acetyl-6-chlor-3-[1-hydroxy-1-(4-acetylamino-3-nitrophenyl)methylen]-2-indolinon
(XIII.2) 1-Acetyl-6-chlor-3-[1-chlor-1-(3-pyridyl)methylen]-2-indolinon aus 1-Acetyl-6-chlor-3-[1-hydroxy-1-(3-pyridyl)methylen]-2-indolinon
(XIII.3) 1-Acetyl-6-chlor-3-[1-chlor-1-(4-pyridyl)methylen]-2-indolinon aus 1-Acetyl-6-chlor-3-[1-hydroxy-1-(4-pyridyl)methylen]-2-indolinon
(XIII.4) 6-Chlor-3-[1-chlor-1-(1-methyl-5-benzimidazolyl)methylen]-2-indolinon aus 1-Acetyl-6-chlor-3-[1-hydroxy-1-(1-methyl-5-benzimidazolyl)methylen]-2-indolinon
(XIII.5) 1-Acetyl-6-chlor-3-[1-chlor-1-(1-benzyl-5-imidazolyl)methylen]-2-indolinon aus 1-Acetyl-6-chlor-3-[1-hydroxy-1-(1-benzyl-5-imidazolyl)methylen]-2-indolinon

### Beispiel XIV

### 1-Acetyl-6-chlor-3-[1-chlor-1-(2-pyrrolyl)methylen]-2-indolinon

Eine Suspension von 4.5 g 1-Acetyl-6-chlor-3-[1-hydroxy-1-(2-pyrrolyl)methylen]-2-indolinon und 3.4 g Phosphorpentachlorid in 50 ml Dioxan und 50 ml Toluol wird 3 Stunden bei 90 °C gerührt. Anschließend wird das Lösungsmittel abdestilliert und der Rückstand chromatographisch an Kieselgel (Fliessmittel: Toluol) gereinigt. Ausbeute: 2.2 g (46 % der Theorie)
R_{f}-Wert: 0.8 (Kieselgel, Toluol)
C₁₅H₁₀Cl₂N₂O₂
Analog Beispiel XIV wird folgende Verbindung hergestellt:
(XIV.1) 1-Acetyl-6-chlor-3-[1-chlor-1-(4-pyridazinyl)methylen]-2-indolinon aus 1-Acetyl-6-chlor-3-(1-hydroxy-1-(4-pyridazinyl)methylen)-2-indolinon, wobei als Fliessmittelsystem Dichlormethan/Methanol = 9:1 verwendet wird.

### Beispiel XV

### 1-Acetyl-3-[1-chlor-1-(5-methyl-3-isoxazolyl)methylen]-2-indolinon

3.00 g 1-Acetyl-3-[1-hydroxy-1-(5-methyl-3-isoxazolyl)methylen]-2-indolinon, 5.09 ml Tetrachlorkohlenstoff und 5.54 g Triphenylphosphin werden in 100 ml THF 8 Stunden unter Rückfluß erhitzt. Der nach Abdestillieren des Lösungsmittels erhaltene Rückstand wird chromatographisch an Kieselgel gereinigt (Fliessmittel:
Petrolether/Ethylacetat = 9:1).
Ausbeute: 2.53 g (79 % der Theorie)
R_{f}-Wert: 0.7 (Kieselgel, Petrolether/Dichlormethan/Methanol = 5:4:1)
C₁₅H₁₁ClN₂O₃
Massenspektrum: m/z = 302/304 [M˙]⁺
Analog Beispiel XV wird folgende Verbindung hergestellt:
(XV.1) 1-Acetyl-3-[1-chlor-1-(3-methyl-5-pyrazolyl)methylen]-2-indolinon aus 1-Acetyl-3-[1-hydroxy-1-(3-methyl-5-pyrazolyl)methylen]-2-indolinon

### Beispiel XVI

### 1-Acetyl-6-chlor-3-[1-methoxy-1-(3,4-methylendioxyphenyl)methylen]-2-indolinon

Eine Lösung von 20.9 g 1-Acetyl-6-chlor-3-[1-hydroxy-l-(3,4-methylendioxyphenyl)-methylen]-2-indolinon und 40 ml Ethyldiisopropylamin in 200 ml Dichlormethan wird portionsweise mit 17.3 g Trimethyloxoniumtetrafluoroborat versetzt, über Nacht gerührt und anschließend zweimal mit Wasser extrahiert. Die organische Phase wird mit Magnesiumsulfat getrocknet und zur Trockne eingeengt. Der Rückstand wird mit Methanol verrührt, abgesaugt, mit Methanol gewaschen und bei 80 °C getrocknet.
Ausbeute: 9.40 g (43 % der Theorie)
R_{f}-Wert: 0.7 (Kieselgel, Petrolether/Dichlormethan/Methanol = 5:4:1)
C₁₉H₁₄ClNO₅
Massenspektrum: m/z = 371/373 [M˙]⁺
Analog Beispiel XVI werden folgende Verbindungen hergestellt:
(XVI.1) 1-Acetyl-6-chlor-3-[1-methoxy-1-(3,4-ethylendioxyphenyl)methylen]-2-indolinon
   aus 1-Acetyl-6-chlor-3-[1-hydroxy-1-(3,4-ethylendioxyphenyl)methylen]-2-indolinon
(XVI.2) 1-Acetyl-6-chlor-3-{1-methoxy-1-[3,4-(difluormethylendioxy)phenyl]-methylen}-2-indolinon
   aus 1-Acetyl-6-chlor-3-{1-hydroxy-1-[3,4-(difluormethylendioxy)phenyl]methylen}-2-indolinon
(XVI.3) 1-Acetyl-3-[1-methoxy-1-(2-methyl-isoindol-1,3-dion-5-yl)methylen]-2-indolinon
   aus 1-Acetyl-3-[1-hydroxy-1-(2-methyl-isoindol-1,3-dion-5-yl)methylen]-2-indolinon
(XVI.4) 1-Acetyl-3-[1-methoxy-1-(3-furyl)methylen]-2-indolinon aus 1-Acetyl-3-[1-hydroxy-1-(3-furyl)methylen]-2-indolinon
(XVI.5) 1-Acetyl-3-[1-methoxy-1-(2-dibenzylamino-4-oxazolyl)methylen]-2-indolinon aus 1-Acetyl-3-[1-hydroxy-1-(2-dibenzylamino-4-oxazolyl)methylen]-2-indolinon
(XVI.6) 1-Acetyl-6-methoxycarbonyl-3-[1-methoxy-1-(3,4-ethylendioxyphenyl)-methylen]-2-indolinon
   aus 1-Acetyl-6-methoxycarbonyl-3-[1-hydroxy-1-(3,4-ethylendioxyphenyl)methylen]-2-indolinon
(XVI.7) 1-Acetyl-6-fluor-3-[1-methoxy-1-(3,4-ethylendioxyphenyl)methylen]-2-indolinon
   aus 1-Acetyl-6-fluor-3-[1-hydroxy-1-(3,4-ethylendioxyphenyl)methylen]-2-indolinon
(XVI.8) 1-Acetyl-3-[1-methoxy-1-(4-methylamino-3-nitrophenyl)methylen]-2-indolinon
   aus 1-Acetyl-3-[1-hydroxy-1-(4-methylamino-3-nitrophenyl)methylen]-2-indolinon
(XVI.9) 1-Acetyl-6-fluor-3-[1-methoxy-1-(4-methylamino-3-nitrophenyl)methylen]-2-indolinon
   aus 1-Acetyl-6-fluor-3-[1-hydroxy-1-(4-methylamino-3-nitrophenyl)methylen]-2-indolinon
(XVI.10) 1-Acetyl-6-chlor-3-[1-methoxy-1-(chinazolin-6-yl)-methylen]-2-indolinon aus 1-Acetyl-6-chlor-3-[1-hydroxy-1-(chinazolin-6-yl)methylen]-2-indolinon
(XVI.11) 1-Acetyl-8-methoxycarbonyl-3-[1-methoxy-1-(3,4-methylendioxyphenyl)-methylen]-2-indolinon
   aus 1-Acetyl-6-methoxycarbonyl-3-[1-hydroxy-1-(3,4-methylendioxyphenyl)-methylen]-2-indolinon
(XVI.12) 1-Acetyl-3-[1-methoxy-1-(1-methylbenzotriazol-5-yl)methylen]-2-indolinon aus 1-Acetyl-3-[1-hydroxy-1-(1-methylbenzotriazol-5-yl)methylen]-2-indolinon
(XVI.13) 1-Acetyl-8-brom-3-[1-methoxy-1-(3,4-methylendioxyphenyl)methylen]-2-indolinon
   aus 1-Acetyl-6-brom-3-[1-hydroxy-1-(3,4-methylendioxyphenyl)methylen]-2-indolinon
(XVI.14) 1-Acetyl-6-fluor-3-[1-methoxy-1-(3,4-methylendioxyphenyl)methylen]-2-indolinon
   aus 1-Acetyl-6-fluor-3-[1-hydroxy-1-(3,4-methylendioxyphenyl)methylen]-2-indolinon

### Beispiel XVII

### 6-Chlor-3-{1-[4-(4-methyl-1-piperazinyl-carbonyl)phenylaminol-1-(4-amino-3-nitrophenyl)methylen}-2-indolinon

2.61 g 1-Acetyl-6-chlor-3-[1-chlor-1-(4-acetylamino-3-nitrophenyl)methylen]-2-indolinon, 1.75 g 4-(4-Methyl-1-piperazinyl-carbonyl)anilin und 4.7 ml Ethyldiisopropylamin werden in 100 ml Dioxan 24 Stunden unter Rückfluß erhitzt. Der Ansatz wird heiß filtriert, das Filtrat zur Trockne eingeengt. Der Rückstand wird in Dichlormethan gelöst, dreimal mit Wasser extrahiert, mit Natriumsulfat getrocknet und zur Trockne eingeengt. Das halbfeste Zwischenprodukt wird in 10 ml DMF und 10 ml Methanol gelöst und nach Zugabe von 4 ml 6molarer Natronlauge 2 Stunden gerührt. Nach Zugabe von 300 ml Wasser wird der Niederschlag abgesaugt, getrocknet, mit 15 ml Ethylacetat/Methanol/Ammoniaklösung = 85:15:1.5 verrührt, erneut abgesaugt und bei 100 °C getrocknet.
Ausbeute: 1.47 g (46 % der Theorie)
R_{f}-Wert: 0.3 (Kieselgel, Ethylacetat/Methanol/Ammoniaklösung = 80:20:2) Schmelzpunkt: 190-195 °C
C₂₇H₂₅ClN₆O₄
Massenspektrum: m/z = 533/535 [M+H]⁺
Analog Beispiel XVII werden folgende Verbindungen hergestellt:
(XVII.1) 6-Chlor-3-{1-[4-(pyrrolidin-1-ylmethyl)phenylamino]-1-(4-amino-3-nitrophenyl)methylen}-2-indolinon
   aus 1-Acetyl-6-chlor-3-[1-chlor-1-(4-acetylamino-3-nitrophenyl)methylen]-2indolinon und 4-(Pyrrolidin-1-ylmethyl)-anilin
(XVII.2) 6-Chlor-3-{1-(4-[*N*-(Dimethylaminomethylcarbonyl)-*N*-methylamino]phenylamino)-1-(4-amino-3-nitrophenyl)methylen}-2-indolinon
   aus 1-Acetyl-6-chlor-3-[1-chlor-1-(4-acetylamino-3-nitrophenyl)methylen]-2-indolinon und *N*-(Dimethylaminomethylcarbonyl)-*N*-methyl-*p*-phenylendiamin
(XVII.3) 6-Chlor-3-{1-(4-[*N*-acetyl-*N*-(2-dimethylamino-ethyl)amino]phenylamino)-1-(4-amino-3-nitrophenyl)methylen}-2-indolinon
   aus 1-Acetyl-6-chlor-3-[1-chlor-1-(4-acetylamino-3-nitrophenyl)methylen]-2-indolinon und *N*-Acetyl-*N*-(2-dimethylamino-ethyl)-*p*-phenylendiamin
(XVII.4) 6-Chlor-3-{1-(4-[*N*-acetyl-*N*-(3-dimethylamino-propyl)amino]phenylamino)-1-(4-amino-3-nitrophenyl)methylen}-2-indolinon
   aus 1-Acetyl-6-chlor-3-[1-chlor-1-(4-acetylamino-3-nitrophenyl)methylen]-2-indolinon und *N*-Acetyl-*N*-(3-dimethylamino-propyl)-*p*-phenylendiamin
(XVII.5) 6-Chlor-3-{1-(4-[*N*-methansulfonyl-*N*-(2-dimethylamino-ethyl)amino]phenylamino)-1-(4-amino-3-nitrophenyl)methylen}-2-indolinon
   aus 1-Acetyl-6-chlor-3-[1-chlor-1-(4-acetylamino-3-nitrophenyl)methylen]-2-indolinon und *N*-Methansulfonyl-*N*-(2-dimethylamino-ethyl)-*p*-phenylendiamin
(XVII.6) 3-{1-[4-(Dimethylaminomethyl)phenylamino]-1-(4-amino-3-nitrophenyl)-methylen}-2-indolinon
   aus 1-Acetyl-3-[1-chlor-1-(4-acetylamino-3-nitrophenyl)methylen]-2-indolinon und 4-(Dimethylaminomethyl)-anilin

### Beispiel XVIII

### 6-Chlor-3-{1-[4-(4-methyl-1-piperazinyl-carbonyl)phenylamino]-1-(3,4-diaminophenyl)methylen}-2-indolinon

0.746 g 6-Chlor-3-{1-[4-(4-methyl-1-piperazinyl-carbonyl)phenylamino]-1-(4-amino-3-nitrophenyl)methylen}-2-indolinon werden in 30 ml Essigsäure unter Zusatz von 0.60 g Raney-Nickel unter 50 psi Wasserstoffdruck 4 Stunden hydriert. Nach Zugabe von weiteren 0.30 g Raney-Nickel wird unter gleichen Bedingungen weitere 4 Stunden hydriert, anschließend vom Katalysator abgesaugt. Der aus dem Filtrat durch Abdestillieren des Lösungsmittels erhaltene Rückstand wird in Wasser gelöst, die Lösung mit Sodalösung alkalisch gestellt. Das ausgefallene Rohprodukt wird abgesaugt, mit Wasser gewaschen, getrocknet, in 70 ml Dichlormethan/Methanol/ Ammoniaklösung = 70:30:3 gelöst und durch eine Kieselgelschicht filtriert. Das Filtrat wird eingedampft, der Rückstand mit 5 ml Essigester verrührt, abgesaugt und bei 80 °C getrocknet.
Ausbeute: 0.60 g (85 % der Theorie) R_{f}-Wert: 0.4 (Kieselgel, Dichlormethan/Methanol/Ammoniaklösung = 85:15:1.5) C₂₇H₂₇ClN₆O₂
Massenspektrum: m/z = 503/505 [M+H]⁺
Analog Beispiel XVIII werden folgende Verbindungen hergestellt:
(XVIII.1) 6-Chlor-3-{1-[4-(pyrrolidin-1-ylmethyl)phenylamino]-1-(3,4-diaminophenyl)-methylen}-2-indolinon
   aus 6-Chlor-3-{1-[4-(pyrrolidin-1-ylmethyl)phenylamino]-1-(4-amino-3-nitrophenyl)-methylen}-2-indolinon
(XVIII.2) 6-Chlor-3-{1-(4-[*N*-(Dimethylaminomethylcarbonyl)-*N*-methylamino]phenylamino)-1-(3,4-diaminophenyl)methylen}-2-indolinon
   aus 6-Chlor-3-{1-(4-[*N*-(Dimethylaminomethylcarbonyl)-*N*-methyl-amino]phenylamino)-1-(4-amino-3-nitrophenyl)methylen}-2-indolinon
(XVIII.3) 6-Chlor-3-{1-(4-[*N*-acetyl-*N*-(2-dimethylamino-ethyl)amino]phenylamino)-1-(3,4-diaminophenyl)methylen}-2-indolinon
   aus 6-Chlor-3-{1-(4-[*N*-acetyl-*N*-(2-dimethylamino-ethyl)amino]phenylamino)-1-(4-amino-3-nitrophenyl)methylen}-2-indolinon
(XVIII.4) 6-Chlor-3-{1-(4-[*N*-acetyl-*N*-(3-dimethylamino-propyl)amino]phenylamino)-1-(3,4-diaminophenyl)methylen}-2-indolinon
   aus 6-Chlor-3-{1-(4-[*N*-cetyl-*N*-(3-dimethylamino-propyl)amino]phenylamino)-1-(4-amino-3-nitrophenyl)methylen}-2-indolinon

### Beispiel XIX

### 1-Acetyl-6-fluor-3-[11-methoxy-1-(1-methylbenzimidazol-5-yl)methylen]-2-indolinon

0.40 g 1-Acetyl-6-fluor-3-[1-methoxy-1-(4-methylamino-3-nitrophenyl)methylen]-2-indolinon werden in 5.0 ml Ameisensäure unter Zusatz von 0.20 g Raney-Nickel 3 Stunden unter 50 psi Wasserstoffatmosphäre hydriert. Der Katalysator wird abgesaugt und das Filtrat eingeengt. Der Rückstand wird mit Diethylether verrührt, nach Kristallisation abgesaugt, und getrocknet.
Ausbeute: 0.30 g (79 % der Theorie)
R_{f}-Wert: 0.8 (Kieselgel, Dichlormethan/Methanol/konz. Ammoniak = 90:10:1) C₂₀H₁₆FN₃O₃
Massenspektrum: m/z = 366 [M⁺H]⁺
Analog Beispiel XIX wird folgende Verbindung hergestellt:
(XIX.1) 1-Acetyl-3-[1-methoxy-1-(1-methylbenzimidazol-5-yl)methylen]-2-indolinon aus 1-Acetyl-3-[1-methoxy-1-(4-methylamino-3-nitrophenyl)methylen]-2-indolinon

### Beispiel XX:

### 4-(1-Methyl-4-piperazinyl-carbonyl)-1-nitrobenzol

Zu einer Lösung von 12.0 ml N-Methylpiperazin und 30 ml Triethylamin in 600 ml Dichlormethan wird bei Raumtemperatur eine Lösung von 20.0 g 4-Nitrobenzoylchlorid in 100 ml Dichlormethan getropft. Die Reaktionslösung wird eine Stunde nachgerührt, anschließend dreimal mit Wasser gewaschen, mit Natriumsulfat getrocknet und zur Trockne eingeengt. Der Rückstand wird mit *tert*-Butylmethylether ausgerührt und bei 40 °C getrocket.
Ausbeute: 16.7 g (62 % der Theorie)
R_{f}-Wert: 0.35 (Kieselgel, Dichlormethan/Methanol = 9:1)
C₁₂H₁₅N₃O₃
Massenspektrum: m/z = 250 [M+H]⁺
Analog Beispiel XX werden folgende Verbindungen hergestellt:
(XX.1) 4-[*N*-Methyl-*N*-(1-methyl-4-piperidinyl)-aminocarbonyl]-1-nitrobenzol aus 4-Nitrobenzoylchlorid und 1-Methyl-4-methylamino-piperidin
(XX.2) 4-[*N*-Methyl-*N*-(2-dimethylamino-ethyl)-aminocarbonyl]-1-nitrobenzol
   aus 4-Nitrobenzoylchlorid und N,N,N'-Trimethyl-1,2-diaminoethan, wobei auf das Ausrühren mit *tert*-Butylmethylether verzichtet wird.
(XX.3) 4-[*N*-Methyl-*N*-(3-dimethylamino-propyl)-aminocarbonyl]-1-nitrobenzol
   aus 4-Nitrobenzoylchlorid und N,N,N'-Trimethyl-1,3-diaminopropan, wobei auf das Ausrühren mit *tert*-Butylmethylether verzichtet wird.
(XX.4) 4-(1-Methyl-4-piperazinyl-carbonyl-methyl)-1-nitrobenzol
   aus 4-Nitrophenylacetylchlorid und 1-Methylpiperazin
(XX.5) 4-(Dimethylamino-carbonyl)-1-nitrobenzol
   aus 4-Nitrobenzoylchlorid und Dimethylamin hydrochlorid
(XX.6) 4-[*N*-Methyl-*N*-(2-dimethylamino-ethyl)-aminosulfonyl]-1-nitrobenzol
   aus 4-Nitrobenzolsulfonylchlorid und N,N,N'-Trimethyl-1,2-diaminoethan, wobei auf das Ausrühren mit *tert*-Butylmethylether verzichtet wird.
(XX.7) 4-[*N*-Methyl-*N*-(3-dimethylamino-propyl)-aminosulfonyl]-1-nitrobenzol
   aus 4-Nitrobenzolsulfonylchlorid und N,N,N'-Trimethyl-1,3-diaminopropan, wobei auf das Ausrühren mit *tert*-Butylmethylether verzichtet wird.
(XX.8) 4-[*N*-Ethyl-*N*-(2-dimethylamino-ethyl)-aminocarbonyl]-1-nitrobenzol
   aus 4-Nitrobenzoylchlorid und N-Ethyl,N',N'-Dimethyl-1,2-diaminoethan, wobei auf das Ausrühren mit *tert*-Butylmethylether verzichtet wird.
(XX.9) 3-[*N*-Methyl-*N*-(2-dimethylamino-ethyl)-aminocarbonyl]-1-nitrobenzol
   aus 3-Nitrobenzoylchlorid und N,N,N'-Trimethyl-1,2-diaminoethan
(XX.10) 4-(1-*t*-Butoxycarbonyl-4-piperazinyl-carbonyl)-nitrobenzol
   aus 3-Nitrobenzoylchlorid und 1-*t*-Butoxycarbonyl-piperazin
(XX.11) 4-(1-*t*-Butoxycarbonyl-perhydro-1,4-diazepin-4-yl-carbonyl)-nitrobenzol
   aus 3-Nitrobenzoylchlorid und 1-*t*-Butoxycarbonyl-perhydro-1,4-diazepin
(XX.12) 4-[*N*-Methyl-*N*-(2-{N'-methyl-N'-*t*-butoxycarbonyl-amino}-ethyl)-aminocarbonyl]-1-nitrobenzol
   aus 4-Nitrobenzoylchlorid und N,N'-Dimethyl-N-*t*-butoxycarbonyl-1,2-diaminoethan

### Beispiel XXI:

### 4-(1-Methyl-4-piperazinyl-carbonyl)anilin

10.9 g 4-(1-Methyl-4-piperazinyl-carbonyl)-1-nitrobenzol werden in 90 ml Ethanol unter Zusatz von 1.1 g Raney-Nickel 35 Minuten bei Raumtemperatur unter 50 psi Wasserstoffatmosphäre hydriert. Vom Katalysator wird abgesaugt, das Filtrat eingeengt ,der Rückstand mit Diethylether verrührt und getrocknet.
Ausbeute: 8.43 g (88 % der Theorie)
R_{f}-Wert: 0.30 (Kieselgel, Dichlormethan/Methanol = 9:1)
C₁₂H₁₇N₃O
Massenspektrum: m/z = 220 [M+H]⁺
Analog Beispiel XXI werden folgende Verbindungen hergestellt:
(XXI.1) 4-[*N*-Methyl-*N*-(1-methyl-4-piperidinyl)-aminocarbonyl]-anilin
   aus 4-[*N*-Methyl-*N*-(1-methyl-4-piperidinyl)-aminocarbonyl]-1-nitrobenzol
(XXI.2) 4-[*N*-Methyl-*N*-(2-dimethylamino-ethyl)-aminocarbonyl]-1-anilin
   aus 4-[*N*-Methyl-*N*-(2-dimethylamino-ethyl)-aminocarbonyl]-1-nitrobenzol, wobei auf das Verrühren mit Diethylether verzichtet wird.
(XXI.3) 4-[*N*-Methyl-*N*-(3-dimethylamino-propyl)-aminocarbonyl]-1-anilin
   aus 4-[*N*-Methyl-*N*-(3-dimethylamino-propyl)-aminocarbonyl]-1-nitrobenzol, wobei auf das Verrühren mit Diethylether verzichtet wird.
(XXI.4) 4-(1-Methyl-4-piperazinyl-carbonyl-methyl)anilin
   aus 4-(1 1-Methyl-4-piperazinyl-carbonyl-methyl)-1-nitrobenzol
(XXI.5) N-Methoxyacetyl-N-methyl-4-amino-anilin
   aus N-Methoxyacetyl-N-methyl-4-amino-nitrobenzol
(XXI.6) N-Acetyl-N-methyl-4-amino-anilin
   aus N-Acetyl-N-methyl-4-amino-nitrobenzol
(XXI.7) 4-(Dimethylamino-carbonyl)-anilin
   aus 4-(Dimethylamino-carbonyl)-1-nitrobenzol
(XXI.8) 1-(4-Aminophenyl)-3,5-dimethylpyrazol
   aus 1-(4-Nitrophenyl)-3,5-dimethylpyrazol, welches dargestellt wird wie beschrieben in: K. v. Auwers, A. Kreuder, Chem. Ber. 58 (1925) 1981
(XXI.9) 4-[*N*-Methyl-*N*-(2-dimethylamino-ethyl)-aminosulfonyl]-1-anilin
   aus 4-[*N*-Methyl-*N*-(2-dimethylamino-ethyl)-aminosulfonyl]-1-nitrobenzol, wobei auf das Verrühren mit Diethylether verzichtet wird.
(XXI.10) N-4-Aminobenzoyl-N-methyl-aminoacetonitril
   aus N-4-Nitrobenzoyl-N-methyl-aminoacetonitril (hergestellt wie beschrieben in: D. Clerin et al., Tetrahedron 32 (1976) 1055-1059), wobei als Katalysator Pd/C (5%) und als Lösungsmittel Ethylacetat verwendet wird.
(XXI.11) 4-[*N*-Methyl-*N*-(3-dimethylamino-propyl)-aminosulfonyl]-1-anilin
   aus 4-[*N*-Methyl-*N*-(3-dimethylamino-propyl)-aminosulfonyl]-1-nitrobenzol, wobei auf das Verrühren mit Diethylether verzichtet wird.
(XXI.12) 4-[*N*-Ethyl-*N*-(2-dimethylamino-ethyl)-aminocarbonyl]-anilin
   aus 4-[*N*-Ethyl-*N*-(2-dimethylamino-ethyl)-aminocarbonyl]-1-nitrobenzol, wobei auf das Verrühren mit Diethylether verzichtet wird.
(XXI.13) 3-[*N*-Methyl-*N*-(2-dimethylamino-ethyl)-aminocarbonyl]-anilin
   aus 3-[*N*-Methyl-*N*-(2-dimethylamino-ethyl)-aminocarbonyl]-1-nitrobenzol
(XXI.14) 4-(1-*t*-Butoxycarbonyl-4-piperazinyl-carbonyl)-anilin
   aus 4-(1-*t*-Butoxycarbonyl-4-piperazinyl-carbonyl)-nitrobenzol
(XXI.15) 4-(1-*t*-Butoxycarbonyl-perhydro-1,4-diazepin-4-yl-carbonyl)-anilin
   aus 4-(1-*t*-Butoxycarbonyl-perhydro-1,4-diazepin-4-yl-carbonyl)-nitrobenzol
(XXI.16) 4-[*N*-Methyl-*N*-(2-{N'-methyl-N'-*t*-butoxycarbonyl-amino}-ethyl)-aminocarbonyl]-anilin
   aus 4-[*N*-Methyl-*N*-(2-(N'-methyl-N'-*t*-butoxycarbonyl-amino}-ethyl)-aminocarbonyl]-1-nitrobenzol

Die Synthesen folgender Verbindungen sind literaturbekannt:
(XXII) *N*-Acetyl-*N*-methyl-*p*-phenylendiamin
   wird dargestellt wie beschrieben in: G.T. Morgan, W.R. Grist, J. Chem. Soc. 113 (1918) 688-694
(XXIII) 4-(2-Dimethylamino-ethoxy)-anilin
   wird dargestellt wie beschrieben in: D.H. Hunter et al., Can. J. Chem. 62 (1984) 2015-2019
(XXIV) 4-(2-Diethylamino-ethylsulfonyl)-anilin
   wird dargestellt wie beschrieben in: J. Walker, J. Chem. Soc. (1945) 630-633
Analog Beispiel XXIV wird folgende Verbindung hergestellt:
(XXIV.1) 4-Methylsulfonyl-anilin
   aus 4-Acetamidophenylsulfinsäure und Dimethylsulfat

Die Synthesen folgender Verbindungen sind in der WO 01/27081 beschrieben:
(XXV) 4-(Pyrrolidin-1-ylmethyl)anilin
(XXV.1) N-(Dimethylaminomethylcarbonyl)-*N*-methyl-*p*-phenylendiamin
(XXV.2) *N*-Methyl-*N*-[(1-methylpiperazin-4-yl)methylcarbonyl]-*p*-phenylendiamin
(XXV.3) *N*-Acetyl-*N*-(2-dimethylamino-ethyl)-*p*-phenylendiamin
(XXV.4) *N*-Acetyl-*N*-(3-dimethylamino-propyl)-*p*-phenylendiamin
(XXV.5) *N*-Propionyl-*N*-(3-dimethylamino-propyl)-*p*-phenylendiamin wird analog Verbindung XXV.4 hergestellt.
(XXV.6) *N*-Methansulfonyl-*N*-(2-dimethylamino-ethyl)-*p*-phenylendiamin
(XXV.7) *N*-Propansulfonyl-*N*-(2-dimethylamino-ethyl)-*p*-phenylendiamin
(XXV.8) 4-(Dimethylaminomethyl)-anilin
(XXV.9) *N*-(2-Dimethylamino-ethyl)-*N*-(pyridin-3-carbonyl)-*p*-phenylendiamin
(XXV.10) 4-(*N*-Ethyl-*N*-methyl-aminomethyl)-anilin
(XXV.11) 4-[(4-Methylpiperazin-1-yl)-methyl]-anilin
(XXV.12) 4-(*N*-Ethyl-*N*-*tert*-butoxycarbonyl-aminomethyl)-anilin
(XXV.13) 4-(*N*-Methyl-*N*-*tert*-butoxycarbonyl-aminomethyl)-anilin
(XXV.14) 4-{[1-(*tert*-Butoxycarbonyl)-piperazin-4-yl]-methyl}-anilin
(XXV.15) 4-(1-Methylimidazol-2-yl)-anilin
(XXV.16) 4-(Diethylaminomethyl)-anilin.
(XXV.17) 4-(1,2,4-Triazol-1-yl-methyl)-anilin
(XXV.18) 4-(1,2,3-Triazol-1-yl-methyl)-anilin
(XXV.19) 4-(1,2,3-Triazol-2-yl-methyl)-anilin
(XXV.20) *N*-Methansulfonyl-*N*-methyl-*p*-phenylendiamin
(XXV.21) *N*-Methansulfonyl-*N*-(2-trifluoracetylamino-ethyl)-*p*-phenylendiamin
(XXV.22) 4-(4-Hydroxypiperidin-1-yl-methyl)-anilin
(XXV.23) N-[(2-Dimethylamino-ethyl)-carbonyl]-N-methyl-p-phenylendiamin
(XXV.24) 4-[N-(2-Hydroxyethyl)-N-methyl-amino-methyl]-anilin
(XXV.25) (*R*)-4-(3-Hydroxypyrrolidin-1-yl-methyl)-anilin
   wird dargestellt wie die beschriebene racemische Verbindung, wobei (*R*)-3-Hydroxypyrrolidin eingesetzt wird.
(XXV.26) (*S*)-4-(3-Hydroxypyrrolidin-1-yl-methyl)-anilin
   wird dargestellt wie die beschriebene racemische Verbindung, wobei (*S*)-3-Hydroxypyrrolidin eingesetzt wird.
(XXV.27) *N*-Ethansulfonyl-*N*-(2-dimethylamino-ethyl)-*p*-phenylendiamin
(XXV.28) *N*-Propionyl-*N*-(3-dimethylamino-propyl)-*p*-phenylendiamin
   wird dargestellt analog Verbindung XXV.4, wobei Propionsäureanhydrid anstelle von Essigsäureanhydrid verwendet wird.
(XXV.29) *N*-Propionyl-*N*-(2-dimethylamino-ethyl)-*p*-phenylendiamin
(XXV.30) 4-(1-Ethylimidazol-2-yl)-anilin
(XXV.31) 4-[N-(2-Methoxyethyl)-N-methyl-amino-methyl]-anilin
(XXV.32) (S)-4-([2-Hydroxymethyl-pyrrollidin-1-yl]-methyl)-anilin
   wird dargestellt analog der beschriebenen Verbindung 4-(3-Hydroxypyrrolidin-1-yl-methyl)-anilin ausgehend von (S)-Prolinol und 4-Nitrobenzylbromid
(XXV.33) 4-(Morpholin-4-yl-methyl)-anilin
(XXV.34) 4-(*N*-Isobutyl-*N-tert*-butoxycarbonyl-aminomethyl)-anilin
   wird dargestellt analog Verbindung XXV.12, wobei Isobutylamin anstelle von Ethylamin verwendet wird.
(XXV.35) *N*-Methansulfonyl-*N*-(2-dimethylamino-ethyl)-3-chlor-*p*-phenylendiamin

### Beispiel XXVI:

### N-Methoxyacetyl-N-methyl-4-amino-nitrobenzol

Eine Suspension von 15.4 g N-Methyl-4-nitroanilin in 100 ml Tetrahydrofuran und 38.5 ml Triethylamin wird tropfenweise mit einer Lösung von 10.0 g Methoxyacetylchlorid in 30 ml THF versetzt. Die Reaktionsmischung wird 15 Stunden gerührt, dann nochmals mit 10 g Methoxyacetylchlorid in 30 ml THF versetzt. Nach weiteren 5 Stunden wird das Lösungsmittel abdestilliert, der Rückstand in Dichlormethan aufgenommen und zweimal mit verdünnter Salzsäure extrahiert. Die organische Phase wird über Magnesiumsulfat getrocknet und eingeengt. Der Rückstand wird chromatografisch an Kieselgel gereinigt (Fliessittel: Dichlormethan/Ethylacetat 9:1, dann 8:2).
Ausbeute: 19.2 g (93 % der Theorie)
R_{f}-Wert: 0.66 (Kieselgel, Ethylacetat/Methanol = 9:1)
C₁₀H₁₂N₂O₄
Massenspektrum: m/z = 225 [M+H]⁺; 247 [M+Na]⁺
Analog Beispiel XXVI wird folgende Verbindung hergestellt:
(XXVI.1) N-Acetyl-N-methyl-4-amino-nitrobenzol

Die Synthesen folgender Verbindungen sind literaturbekannt:
(XXVII) *N*-(4-Aminophenyl)-2-pyrrolidon
   wird dargestellt wie beschrieben in: W. Reppe et al., Justus Liebigs Ann. Chem. 596 (1955) 204
(XXVIII) (4-Aminobenzyl)-pyridin-2-yl-amin
   wird dargestellt wie beschrieben in: V. Martinez-Barrasa, et al., Tetrahedron 56 (2000) 2481-2490

### Beispiel XXIX:

### N-[2-(Dimethylamino)-ethyl]-sufanilsäureamid

5.26 g Sulfanilsäurefluorid und 16.5 ml N,N-Dimethyl-ethylendiamin werden 4 Stunden bei 100 °C gerührt, anschließend in Ethylacetat gelöst und dreimal mit Kochsalzlösung extrahiert. Der durch Einengen der organischen Phase erhaltene Rückstand wird aus Diethylether kristallisiert und bei 60 °C getrocknet.
Ausbeute: 4.1 g (56 % der Theorie)
Schmelzpunkt: 86-88 °C
C₁₀H₁₇N₃O₂S
Massenspektrum: m/z = 244 [M+H]⁺; 242 [M-H]⁻
Analog Beispiel XXIX wird folgende Verbindung hergestellt:
(XXIX.1) N-[3-(Dimethylamino)-propyl]-sufanilsäureamid
   aus Sulfanilsäurefluorid und N,N-Dimethyl-1,3-diaminopropan

Die Synthese folgender Verbindung ist in der internationalen Anmeldung WO 01/27081 beschrieben:
(XXX) N-(Dimethylaminomethylcarbonyl)-N-methyl-3-methoxy-p-phenylendiamin

### Herstellung der Endverbindungen:

### Beispiel 1.0

### 3-(Z)-[1-{4-[N-Propionyl-N-(3-dimethylamino-propyl)-amino]-phenylamino}-1-(2-dibenzylamino-4-oxazolyl)-methylen]-2-indolinon

0.935 g 1-Acetyl-3-[1-methoxy-1-(2-dibenzylamino-4-oxazolyl)-methylen]-2-indolinon (Edukt XVI.5) und 0.549 g N-Propionyl-N-(3-dimethylamino-propyl)-*p-*phenylendiamin (Edukt XXV.5) werden in 10 ml Dimethylformamid gelöst und 3 Stunden bei 120 °C gerührt. Nach dem Abkühlen werden 5 ml Methanol und 2 ml 2 molare Natronlauge zugegeben und eine weitere Stunde bei Raumtemperatur gerührt. Nach Zugabe von 60 ml Wasser zweimal mit Essigester extrahiert. Der aus den vereinigten organischen Phasen durch entfernen des Lösungsmittels erhaltene Rückstand wird über eine Kieselgelsäule mit Dichlormethan/Methanol/konz. Ammoniak = 90:10:1 als Laufmittel chromatographisch gereinigt. Das so erhaltene Rohprodukt wird mit eiskaltem Diethylether verrührt, abgesaugt und bei 80 °C getrocknet.
Ausbeute: 0.40 g (31% der Theorie)
R_{f}-Wert:0.5 (Kieselgel, Methylenchlorid/Methanol/konz. Ammoniak = 85:15:1.5) Fp.: 204-205 °C
C₄₀H₄₂N₆O₃
Massenspektrum: m/z = 655 [M+H]⁺; m/z = 653 [M-H]⁻

Analog Beispiel 1.0 werden aus den jeweils angegebenen Edukten folgende Verbindungen der allgemeinen Formel I.1 hergestellt:

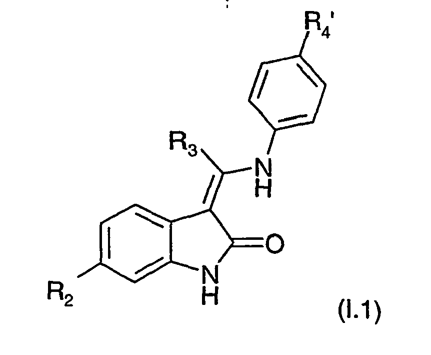

| Beispiel | R₂ | R₃ | R₄' | Edukte | Summenformel | Massen-spektrum | Fp. [°C] | R_{f-}Wert* |
|---|---|---|---|---|---|---|---|---|
| 1.1 | H | 3-Furyl- | -CH₂-NMe₂ | XVI.4 XXV.8 | C₂₂H₂₁N₃O₂ | 358 [M-H]⁻ | 220-222 | 0.5 (A) |
| 1.2 | H | 3-Furyl- | -N(CH₃)-CO-CH₂-NMe₂ | XVI.4 XXV.1 | C₂₄H₂₄N₄O₃ | 417 [M+H]⁺ 415 [M-H]⁻ | 243-245 | 0.6 (B) |
| 1.3 | H | 2-Methyl-isodinol-1,3-dion-5-yl- | -CH₂-NMe₂ | XVI.3 XXV.8 | C₂₇H₂₄N₄O₃ | 451 [M-H]⁻ | 248-251 | 0.08 (C) |
| 1.4 | H | 2-Methyl-isodinol-1,3-dion-5-yl- | -N(CH₃)-CO-CH₂-NMe₂ | XVI.3 XXV.1 | C₂₉H₂₇N₅O₄ | 510 [M+H]⁺ | 244-246 | 0.33 (D) |
| 1.5 | H | 2-Methyl-isodinol-1,3-dion-5-yl- | -N(COMe)-; (CH₂)₂-NMe₂ | XVI.3 XXV.3 | C₃₀H₂₉N₅O₄ | 524 [M+H]⁺ | 182-185 | 0.2 (C)- |
| 1.6 | Cl | 1,2-(Methylen-dioxy)-phen-4-yl- | -N(SO₂Me)-(CH₂)₂-NMe₂ | XVI XXV.6 | C₂₇H₂₇ClN₄O₅ S | 555/557 [M+H]⁺ 553/555 [M-H]⁻ | 235-237 | 0.4 (A) |
| 1.7 | Cl | 1,2-(Methylen-dioxy)-phen-4-yl- | -CH₂-NMe₂ | XVI XXV.8 | C₂₅H₂₂ClN₃O₃ | 446/448 [M-H]⁻ | 256-258 | 0.4 (A) |
| 1.8 | Cl | 1,2-(Methylen-dioxy)-phen-4-kl- | -N(CH₃)-CO-CH₂-NMe₂ | XVI XXV.1 | C₂₇H₂₅ClN₄O₄ | 505/507 [M+H]⁺ 503/505 [M-H]⁻ | 269-270 | 0.3 (A) |
| 1.9 | Cl | 1,2-(Methylen-dioxy)-phen-4-yl- | -N(COMe)-(CH₂)₃-NMe₂ | XVI XXV.4 | C₂₉H₂₉ClN₄O₄ | 533/535 [M+H]⁺ 531/533 [M-H]⁻ | 219-220 | 0.3 (A) |
| 1.10 | Cl | 1,2-(Methylen-dioxy)-phen-4-yl- | -N(COMe)-(CH₂)₂-NMe₂ | XVI XXV.3 | C₂₈H₂₇ClN₄O₄ | 519/521 [M+H]⁺ 517/519 [M-H]⁻ | 217-218 | 0.3 (A) |
| 1.11 | Cl | 1,2-(Methylen-dioxy)-phen-4-yl- | -CH₂-pyrrolidin-1-yl | XVI XXV | C₂₇H₂₄ClN₃O₃ | 474/476 [M+H]⁺ 472/474 [M-H]⁻ | 226-228 | 0.45 (A) |
| 1.12 | Cl | 1,2-(Methylen-dioxy)-phen-4-yl- | -CO-(1-methyl-piperazin-4-yl) | XVI XXI | C₂₆H₂₅ClN₄O₄ | 517/519 [M+H]⁺ 515/517 [M-H]⁻ | n.d. | 0.3 (A) |
| 1.13 | Cl | 1,2-(Methylen-dioxy)-phen-4-yl- | -N(CH₃)-CO-CH₂-(1-methylpiperazin-4-yl) | XVI XXV.2 | C₃₀H₃₀ClN₅O₄ | 560/562 [M+H]⁺ 558/560 [M-H]⁻ | 265-266 | 0.3 (A) |
| 1.14 | Cl | 1,2-(Methylen-dioxy)-phen-4-yl- | -N(CO-3-pyridyl)-(CH₂)₂-NMe₂ | XVI XXV.9 | C₃₂H₂₈ClN₅O₄ | 582/584 [M+H]⁺ 580/582 [M-H]⁻ | 241-242 | 0.3 (A) |
| 1.15 | Cl | 1,2-(Methylen-dioxy)-phen-4-yl- | -CH₂-N(Me)Et | XVI XXV.10 | C₂₆H₂₄ClN₃O₃ | 462/464 [M+H]⁺ | 200-203 | 0.45 (A) |
| 1.16 | Cl | 1,2-(Methylen-dioxy)-phen-4-yl- | -CH₂-(1-methylpiperazin-4-yl) | XVI XXV.11 | C₂₈H₂₇ClN₄O₃ | 503/505 [M+H]⁺ | 215-217 | 0.33 (A) |
| 1.17 | Cl | 1,2-(Methylen-dioxy)-phen-4-yl- | -CH₂-N(Et)-C(O)O-*t*Bu | XVI XXV.12 | C₃₀H₃₀ClN₃O₅ | 548/550 [M+H]⁺ | 234-237 | 0.37 (A) |
| 1.18 | Cl | 1,2-(Methylen-dioxy)-phen-4-yl- | -CH₂-N(Me)-C(O)O-*t*Bu | XVI XXV.13 | C₂₉H₂₈ClN₃O₅ | 534/536 [M+H]⁺ | 221-224 | 0.57 (A) |
| 1.19 | Cl | 1,2-(Methylen-dioxy)-phen-4-yl- | -CH₂-(1-(C(O)O*t*Bu)-piperazin-4-yl) | XVI XXV.14 | C₃₂H₃₃ClN₄O₅ | 589/591 [M+H]⁺ | 170-173 | 0.53 (A) |
| 1.20 | Cl | 1,2-(Methylen-dioxy)-phen-4-yl- | -SO₂-(CH₂)₂-NEt₂ | XVI XXIV | C₂₈H₂₈ClN₃O₅ S | 554/556 [M+H]⁺ 552/554 [M-H]⁻ | 215-217 | 0.4 (E) |
| 1.21 | Cl | 1,2-(Methylen-dioxy)-phen-4-yl- | -O-(CH₂)₂-NMe₂ | XVI XXIII | C₂₆H₂₄ClN₃O₄ | 476/478 [M-H]⁻ | 196-199 | 0.24 (A) |
| 1.22 | Cl | 1,2-(Ethylen-dioxy)-phen-4-yl- | -CH₂-NMe₂ | XVI.1 XXV.8 | C₂₆H₂₄ClN₃O₃ | 460/462 [M-H]⁻ | 195-197 | 0.10 (E) |
| 1.23 | Cl | 1,2-(Ethylen-dioxy)-phen-4-yl- | -N(SO₂Me)-(CH₂)₂-NMe₂ | XXV.6 | C₂₈H₂₉ClN₄O₅ S | 569/571 [M+H]⁺ | 220-223 | 0.17 (E) |
| 1.24 | Cl | 1,2-(Ethylen-dioxy)-phen-4-yl- | -N(CH₃)-CO-CH₂-NMe₂ | XVI.1 XXV.1 | C₂₈H₂₇ClN₄O₄ | 519/521 [M+H]⁺ | 281-284 | 0.32 (C) |
| 1.25 | Cl | 1,2-(Ethylen-dioxy)-phen-4-yl- | -CO-(1-methyl-piperazin-4-yl) | XVI.1 XXI | C₂₉H₂₇ClN₄O₄ | 531/533 [M+H]⁺ | 258-262 | 0.08 (C) |
| 1.26 | Cl | 1,2-(Ethylen-dioxy)-phen-4-yl- | -N(COMe)-(CH₂)₂-NMe₂ | XVI.1 XXV.3 | C₂₉H₂₉ClN₄O₄ | 533/535 [M+H]⁺ | 213-215 | 0.39 (C) |
| 1.27 | Cl | 1,2-(Difluor-methylen-dioxy)-phen-4-yl- | -CH₂-N(Me)-C(O)O-*t*Bu | XVI.2 XXV.13 | C₂₉H₂₆ClF₂N₃ O₅ | 592/594 [M+Na]⁺ | 185-187 | 0.89 (N) |
| 1.28 | Cl | 1,2-(Difluor-methylen-dioxy)-phen-4-yl- | -CO-(1-methyl-piperazin-4-yl) | XVI.2 XXI | C₂₈H₂₃ClF₂N₄ O₄ | 553/555 [M+H]⁺ | 237-239 | 0.18 (C) |
| 1.29 | Cl | 1,2-(Difluor-methylen-dioxy)-phen-4-yl- | -CH₂-NMe₂ | XVI.2 XXV.8 | C₂₅H₂₀ClF₂N₃ O₃ | 484/486 [M+H]⁺ | 225-228 | 0.22 (C) |
| 1.30 | Cl | 1,2-(Difluor-methylen-dioxy)-phen-4-yl- | -N(COMe)-(CH₂)₂-NMe₂ | XVI.2 XXV.3 | C₂₈H₂₅ClF₂N₄ O₄ | 555/557 [M+H]⁺ | 202-206 | 0.15 (C) |
| 1.31 | Cl | 1,2-(Difluor-methylen-dioxy)-phen-4-yl- | -N(CH₃)-CO-CH₂-NMe₂ | XVI.2 XXV.1 | C₂₇H₂₃ClF₂N₄ O₄ | 541/543 [M+H]⁺ | 254-259 | 0.19 (C) |
| 1.32 | COO Me | 1,2-(Ethylen-dioxy)-phen-4-yl- | -CH₂-NMe₂ | XVI.6 XXV.8 | C₂₈H₂₇N₃O₅ | 486 [M+H]⁺ 484 [M-H]⁻ | 220-222 | 0.35 (G) |
| 1.33 | COO Me | 1,2-(Ethylen-dioxy)-phen-4-yl- | -N(CH₃)-CO-CH₂-(1-methylpiperazin-4-yl) | XVI.6 XXV.2 | C₃₃H₃₅N₅O₆ | 598 [M+H]⁺ | 176-179 | 0.26 (G) |
| 1.34 | F | 1,2-(Ethylen-dioxy)-phen-4-yl- | -N(COMe)-(CH₂)₂-NMe₂ | XVI.7 XXV.3 | C₂₉H₂₉FN₄O₄ | 517 [M+H]⁺ | 142-144 | 0.47 (P) |
| 1.35 | F | 1,2-(Ethylen-dioxy)-phen-4-yl- | -CO-N(Me)-(1-methyl-piperidin-4-yl) | XVI.7 XXI.1 | C₃₁H₃₁FN₄O₄ | 543 [M+H]⁺ | 231-234 | 0.54 (P) |
| 1.36 | F | 1,2-(Ethylen-dioxy)-phen-4-yl- | -CH₂-NMe₂ | XVI.7 XXV.8 | C₂₆H₂₄FN₃O₃ | 446 [M+H]⁺ | 201-203 | 0.55 (A) |
| 1.37 | F | 1,2-(Ethylen-dioxy)-phen-4-yl- | -CH₂-N(Et)-C(O)O-*t*Bu | XVI.7 XXV.12 | C₃₁H₃₂FN₃O₅ | 568 [M+Na]⁺ | Nicht bestim mt | 0.67 (P) |
| 1.38 | F | 1,2-(Ethylen-dioxy)-phen-4-yl- | 1-Methyl-imidazol-2-yl | XVI.7 XXV.15 | C₂₇H₂₁FN₄O₃ | 469 [M+H]⁺ | 230-233 | 0.54 (A) |
| 1.39 | F | 1-Methyl-benzimidazo l-5-yl | -CH₂-NMe₂ | XIX XXV.8 | C₂₆H₂₄FN₅O | 442 [M+H]⁺ | 248-252 | 0.62 (P) |
| 1.40 | H | 1-Methyl-benzimidazo l-5-yl | -CH₂-NMe₂ | XIX.1 XXV.8 | C₂₆H₂₅N₅O | 424 [M+H]⁺ | 250-253 | 0.51 (P) |
| 1.41 | Cl | Chinoxalin-6-yl | -N(SO₂Me)-(CH₂)₂-NMe₂ | XVI.10 XXV.6 | C₂₈H₂₇ClN₆O₃ S | 563/565 [M+H]⁺ | 180-185 | 0.49 (A) |
| 1.42 | Cl | Chinoxalin-6-yl | -N(CH₃)-CO-CH₂-(1-methylpiperazin-4-yl) | XVI.10 XXV.2 | C₃₁H₃₀ClN₇O₂ | 568/570 [M+H]⁺ | 223-227 | 0.35 (A) |
| 1.43 | Cl | Chinoxalin-6-yl | -N(COMe)-(CH₂)₂-NMe₂ | XVI.10 XXV.3 | C₂₉H₂₇ClN₆O₂ | 527/529 [M+H]⁺ | 200-205 | 0.34 (A) |
| 1.44 | Cl | Chinoxalin-6-yl | -CO-(1-methyl-piperazin-4-yl) | XVI.10 XXI | C₂₉H₂₅ClN₆O₂ | 525/527 [M+H]⁺ | 235-240 | 0.48 (A) |
| 1.45 | Cl | Chinoxalin-6-yl | -CH₂-NMe₂ | XVI.10 XXV.8 | C₂₆H₂₂ClN₅O | 454/456 [M-H]⁻ | 258-260 | 0.33 (A) |
| 1.46 | Cl | Chinoxalin-6-yl | 1-Methyl-imidazol-2-yl | XVI.10 XXV.15 | C₂₇H₁₉ClN₆O | 477/479 [M-H]⁻ | 282-286 | 0.51 (A) |
| 1.47 | COO Me | 1,2-(Ethylen-dioxy)-phen-4-yl- | -CH₂-NEt₂ | XVI.6 XXV.16 | C₃₀H₃₁N₃O₅ | 512 [M-H]⁻ | 168-170 | 0.33 (Q) |
| 1.48 | COO Me | 1,2-(Ethylen-dioxy)-phen-4-yl- | -N(COMe)-(CH₂)₂-NMe₂ | XVI.6 XXV.3 | C₃₁H₃₂N₄O₆ | 557 [M+H]⁺ 555 [M-H]⁻ | 222-224 | 0.31 (G) |
| 1.49 | COO Me | 1,2-(Ethylen-dioxy)-phen-4-yl- | -CO-N(Me)-(CH₂)₂-NMe₂ | XVI.6 XXI.2 | C₃₁H₃₂N₄O₆ | 557 [M+H]⁺ 555 [M-H]⁻ | n.d. | 0.23 (G) |
| 1.50 | COO Me | 1,2-(Ethylen-dioxy)-phen-4-yl- | -CO-N(Me)-(CH₂)₃-NMe₂ | XVI.6 XXI.3 | C₃₂H₃₄N₄O₆ | 571 [M+H]⁺ 569 [M-H]⁻ | n.d. | 0.10 (G) |
| 1.51 | COO Me | 1,2-(Ethylen-dioxy)-phen-4-yl- | -CH₂-N(Et)-C(O)O-*t*Bu | XVI.6 XXV.12 | C₃₃H₃₆N₃O₇ | 586 [M+H]⁺ 584 [M-H]⁻ | 238 (Zerset zung) | 0.31 (G) |
| 1.52 | COO Me | 1,2-(Ethylen-dioxy)-phen-4-yl- | -CH₂-N(Me)Et | XVI.6 XXV.10 | C₂₉H₂₉N₃O₅ | 500 [M+H]⁺ | 192-193 | 0.26 (G) |
| 1.53 | COO Me | 1,2-(Ethylen-dioxy)-phen-4-yl- | -CH₂-pyrrolidin-1-yl | XVI.6 XXV | C₃₀H₂₉N₃O₅ | 512 [M+H]⁺ 510 [M-H]⁻ | 246-248 | 0.34 (G) |
| 1.54 | COO Me | 1,2-(Ethylen-dioxy)-phen-4-yl- | -N(CH₃)-CO-CH₂-NMe₂ | XVI.6 XXV.1 | C₃₀H₃₀N₄O₆ | 543 [M+H]⁺ 541 [M-H]⁻ | 231-233 | 0.35 (G) |
| 1.55 | COO Me | 1,2-(Ethylen-dioxy)-phen-4-yl- | -N(SO₂Me)-(CH₂)₂-NMe₂ | XVI.6 XXV.6 | C₃₀H₃₂N₄O₇S | 591 [M-H]⁻ | 244-246 | 0.39 (G) |
| 1.56 | COO Me | 1,2-(Ethylen-dioxy)-phen-4-yl- | -CH₂-(1-methylpiper azin-4-yl) | XVI.6 XXV.11 | C₃₁H₃₂N₄O₅ | 541 [M+H]⁺ 539 [M-H]⁻ | 258-259 | 0.39 (G) |
| 1.57 | COO Me | 1,2-(Ethylen-dioxy)-phen-4-yl- | -CO-(1-methyl-piperazin-4-yl) | XVI.6 XXI | C₃₁H₃₀N₄O₆ | 555 [M+H]⁺ 553 [M-H]⁻ | 271-273 | 0.35 (G) |
| 1.58 | COO Me | 1,2-(Methylen-dioxy)-phen-4-yl- | -CH₂-NEt₂ | XVI.11 XXV.16 | C₂₉H₂₉N₃O₅ | 498 [M-H]⁻ | 206 | 0.23 (G) |
| 1.59 | COO Me | 1,2-(Methylen-dioxy)-phen-4-yl- | -CH₂-NMe₂ | XVI.11 XXV.8 | C₂₇H₂₅N₃O₅ | 472 [M+H]⁺ | 234 | 0.20 (G) |
| 1.60 | COO Me | 1,2-(Methylen-dioxy)-phen-4-yl- | -N(CH₃)-CO-CH₂-(1-methylpipe-razin-4-yl) | XVI.11 XXV.2 | C₃₂H₃₃N₅O₆ | 584 [M+H]⁺ 582 [M-H]⁻ | 152 | 0.12 (G) |
| 1.61 | COO Me | 1,2-(Methylen-dioxy)-phen-4-yl- | -N(COMe)-(CH₂)₂-NMe₂ | XVI.11 XXV.3 | C₃₀H₃₀N₄O₆ | 543 [M+H]⁺ 541 [M-H]⁻ | 206 | 0.30 (G) |
| 1.62 | COO Me | 1,2-(Methylen-dioxy)-phen-4-yl- | -CH₂-N(Et)-C(O)O-*t*Bu | XVI.11 XXV.12 | C₃₂H₃₃N₃O₇ | 572 [M+H]⁺ 570 [M-H]⁻ | 233 | 0.29 (R) |
| 1.63 | COO Me | 1,2-(Methylen-dioxy)-phen-4-yl- | -O(CH₂)₂-NMe₂ | XVI.11 XXIII | C₂₈H₂₇N₃O₆ | 500 [M-H]⁻ | 243 | 0.29 (G) |
| 1.64 | COO Me | 1,2-(Methylen-dioxy)-phen-4-yl- | -CO-N(Me)-(CH₂)₂-NMe₂ | XVI.11 XXI.2 | C₃₀H₃₀N₄O₆ | 543 [M+H]⁺ 541 [M-H]⁻ | 173 | 0.30 (G) |
| 1.65 | F | 1-Methyl-benzimidazol-5-yl | -CH₂-(1,2,4-triazol-1-yl) | XIX XXV.17 | C₂₆H₂₀FN₇O | 466 [M+H]⁺ | 268-272 | 0.70 (P) |
| 1.66 | F | 1-Methyl-benzimidazo l-5-yl | -CH₂-(1,2,3-triazol-1-yl) | XIX XXV.18 | C₂₆H₂₀FN₇O | 466 [M+H]⁺ | 220-225 | 0.61 (P) |
| 1.67 | F | 1-Methyl-benzimidazo l-5-yl | -CH₂-(1,2,3-triazol-2-yl) | XIX XXV.19 | C₂₆H₂₀FN₇O | 466 [M+H]⁺ | 253-256 | 0.71 (P) |
| 1.68 | F | 1-Methyl-benzimida-zol-5-yl | -CH₂-CO-(1-methyl-pipe-razin-4-yl) | XIX XXI.4 | C₃₀H₂₉FN₆O₂ | 525 [M+H]⁺ | 158-165 | 0.55 (P) |
| 1.69 | F | 1-Methyl-benzimi-dazol-5-yl | -N(COMe)-(CH₂)₂-NMe₂ | XIX XXV.3 | C₂₉H₂₉FN₆O₂ | 513 [M+H]⁺ | 163-167 | 0.49 (P) |
| 1.70 | F | 1-Methyl-benzimidazo l-5-yl | 1-Methyl-imidazol-2-yl | XIX XXV.15 | C₂₇H₂₁FN₆O | 465 [M+H]⁺ | 279-284 (Zerset zung) | 0.59 (P) |
| 1.71 | F | 1-Methyl-benzimidazol-5-yl | -N(CH₃)-CO-CH₂-(1-methylpiperazin-4-yl) | XIX XXV.2 | C₃₁H₃₂FN₇O₂ | 554 [M+H]⁺ | 136-143 | 0.3 (P) |
| 1.72 | F | 1-Methyl-benzimidazol-5-yl | -N(CH₃)-CO-CH₂-OCH₃ | XIX XXI.5 | C₂₇H₂₄FN₅O₃ | 486 [M+H]⁺ | 168-172 | 0.5 (P) |
| 1.73 | F | 1-Methyl-benzimidazol-5-yl | 2-pyrrolidon-1-yl | XIX XXVII | C₂₇H₂₂FN₅O₂ | 468 [M+H]⁺ | 276-279 | 0.5 (P) |
| 1.74 | F | 1-Methyl-benzimidazol-5-yl | -N(CH₃)-COCH₃ | XIX XXI.6 | C₂₆H₂₂FN₅O₂ | 456 [M+H]⁺ | 322-326 | 0.6 (P) |
| 1.75 | F | 1-Methyl-benzimidazol-5-yl | -N(CH₃)-SO₂CH₃ | XIX XXV.20 | C₂₅H₂₂FN₅O₃ S | 492 [M+H]⁺ | 311-317 | 0.6 (P) |
| 1.76 | F | 1-Methyl-benzimidazol-5-yl | -SO₂CH₃ | XIX XXIV.1 | C₂₄H₁₉FN₄O₃ S | 463 [M+H]⁺ | 300-303 | 0.7 (P) |
| 1.77 | F | 1-Methyl-benzimidazol-5-yl | -CO-NMe₂ | XIX XXI.7 | C₂₆H₂₂FN₅O₂ | 456 [M+H]⁺ | 320 | 0.6 (P) |
| 1.78 | F | 1-Methyl-benzimidazol-5-yl | 3,5-dimethyl-pyrazol-1-yl | XIX XXI.8 | C₂₈H₂₃FN₆O | 479 [M+H]⁺ | 297-301 | 0.8 (P) |
| 1.79 | F | 1-Methyl-benzimidazol-5-yl | -CO-(1-methyl-piperazin-4-yl) | XIX XXI | C₂₉H₂₇FN₆O₂ | 511 [M+H]⁺ | 257-262 | 0.6 (P) |
| 1.80 | F | 1-Methyl-benzimidazol-5-yl | -N(CH₃)-CO-CH₂-NMe₂ | XIX XXV.1 | C₂₈H₂₇FN₆O₂ | 499 [M+H]⁺ | 248-257 | 0.5 (P) |
| 1.81 | F | 1-Methyl-benzimidazol-5-yl | -SO₂-N(Me)-(CH₂)₂-NMe₂ | XIX XXI.9 | C₂₈H₂₉FN₆O₃ S | 549 [M+H]⁺ | 134-138 | 0.4 (P) |
| 1.82 | F | 1-Methyl-benzimi-dazol-5-yl | -CO-N(Me)-CH₂CN | XIX XXI.10 | C₂₇H₂₁FN₆O₂ | 481 [M+H]⁺ | 128-134 | 0.9 (P) |
| 1.83 | F | 1-Methyl-benzimi-dazol-5-yl | -N(SO₂Me)-(CH₂)₂-NH₂ | XIX XXV.21 | C₂₆H₂₅FN₆O₃ S | 521 [M+H]⁺ | 205-211 | 0.6 (P) |
| 1.84 | H | 1-Methyl-benzotriazol-5-yl | -CH₂-NMe₂ | XVI.12 XXV.8 | C₂₅H₂₄FN₆O | 425 [M+H]⁺ | 285-297 | 0.19 (C) |
| 1.85 | Cl | 1,2-(Ethylen-dioxy)-phen-4-yl- | -CH₂-(4-hydroxy-piperidin-1-yl) | XVI.1 XXV.22 | C₂₉H₂₈ClN₃O₄ | 518/520 [M+H]⁺ | 260-263 | 0.19 (E) |
| 1.86 | Cl | 1,2-(Ethylen-dioxy)-phen-4-yl- | -N(CH₃)-CO-(CH₂)₂-NMe₂ | XVI.1 XXV.23 | C₂₉H₂₉ClN₄O₄ | 533/535 [M+H]⁺ | 246-248 | 0.05 (E) |
| 1.87 | Cl | 1,2-(Ethylen-dioxy)-phen-4-yl- | -CH₂-N(Me)Et | XVI.1 XXV.10 | C₂₇H₂₆ClN₃O₃ | 476/478 [M+H]⁺ | 192-195 | 0.19 (E) |
| 1.88 | Cl | 1,2-(Ethylen-dioxy)-phen-4-yl- | -CH₂-N(Me)-(CH₂)₂-OH | XVI.1 XXV.24 | C₂₇H₂₆ClN₃O₄ | 492/494 [M+H]⁺ | 128-133 | 0.17 (E) |
| 1.89 | Cl | 1,2-(Ethylen-dioxy)-phen-4-yl- | (*R*)-CH₂-(3-hydroxy-pyrrolidin-1-yl) | XVI.1 XXV.25 | C₂₈H₂₆ClN₃O₄ | 504/506 [M+H]⁺ | 148-151 | 0.17 (E) |
| 1.90 | Cl | 1,2-(Ethylen-dioxy)-phen-4-yl- | -N(SO₂Me)-(CH₂)₂-NMe₂ | XVI.1 XXV.6 | C₂₈H₂₉ClN₄O₅ S | 569/571 [M+H]⁺ | 219-223 | 0.18 (E) |
| 1.91 | Cl | 1,2-(Ethylen-dioxy)-phen-4-yl- | (S)-CH₂-(3-hydroxy-pyrrolidin-1-yl) | XVI.1 XXV.26 | C₂₈H₂₆ClN₃O₄ | 504/506 [M+H]⁺ | 148-151 | 0.17 (E) |
| 1.92 | Cl | 1,2-(Ethylen-dioxy)-phen-4-yl- | -CH₂-(1-methylpiperazin-4-yl) | XVI.1 XXV.11 | C₂₉H₂₉ClN₄O₃ | 517/519 [M+H]⁺ | 150 (Zerset zung) | 0.05 (E) |
| 1.93 | Cl | 1,2-(Ethylen-dioxy)-phen-4-yl- | -SO₂-N(Me)-(CH₂)₃-NMe₂ | XVI.1 XXI.11 | C₂₉H₃₁ClN₄O₅ S | 583/585 [M+H]⁺ | 200-210 | 0.10 (T) |
| 1.94 | Cl | 1,2-(Ethylen-dioxy)-phen-4-yl- | -N(SO₂Et)-(CH₂)₂-NMe₂ | XVI.1 XXV.27 | C₂₉H₃₁ClN₄O₅ S | 583/585 [M+H]⁺ | 185-188 | 0.18 (E) |
| 1.95 | Cl | 1,2-(Ethylendioxy)-phen-4-yl- | -SO₂-N(Me)-(CH₂)₂-NMe₂ | XVI.1 XXI.9 | C₂₈H₂₉ClN₄O₅ S | 569/571 [M+H]⁺ | 178-180 | 0.05 (E) |
| 1.96 | Cl | 1,2-(Ethylen-dioxy)-phen-4-yl- | -N(COMe)-(CH₂)₃-NMe₂ | XVI.1 XXV.4 | C₃₀H₃₁ClN₄O₄ | 547/549 [M+H]⁺ | 133-135 | 0.05 (T) |
| 1.97 | Cl | 1,2-(Ethylen-dioxy)-phen-4-yl- | -N(COEt)-(CH₂)₃-NMe₂ | XVI.1 XXV.28 | C₃₁H₃₃ClN₄O₄ | 561/563 [M+H]⁺ | sintert ab 120 | 0.13 (T) |
| 1.98 | Cl | 1,2-(Ethylen-dioxy)-phen-4-yl- | -N(COEt)-(CH₂)₂-NMe₂ | XVI.1 XXV.29 | C₃₀H₃₁ClN₄O₄ | 547/549 [M+H]⁺ | sintert ab 130 | 0.05 (E) |
| 1.99 | Cl | 1,2-(Ethylen-dioxy)-phen-4-yl- | -N(CH₃)-CO-CH₂-(1-methylpiperazin-4-yl) | XVI.1 XXV.2 | C₃₀H₃₁ClN₅O₄ | 572/574 [M-H]⁻ | 235 | 0.2 (G) |
| 1.100 | Cl | 1,2-(Ethylen-dioxy)-phen-4-yl- | -CO-N(Et)-(CH₂)₂-NMe₂ | XVI.1 XXI.12 | C₃₀H₃₁ClN₄O₄ | 547/549 [M+H]⁺ | 212 | 0.2 (G) |
| 1.101 | F | 1,2-(Ethylen-dioxy)-phen-4-yl- | 1-Ethylimidazol-2-yl | XVI.7 XXV.30 | C₂₈H₂₃FN₄O₃ | 483 [M+H]⁺ | 266-268 | 0.6 (P) |
| 1.102 | F | 1,2-(Ethylen-dioxy)-phen-4-yl- | -CH₂-NH-(2-pyridyl) | XVI.7 XXVIII | C₂₉H₂₃FN₄O₃ | 495 [M+H]⁺ | 137-139 | 0.6 (P) |
| 1.103 | F | 1,2-(Ethylen-dioxy)-phen-4-yl- | -N(CH₃)-CO-CH₂-NMe₂ | XVI.7 XXV.1 | C₂₈H₂₇FN₄O₄ | 503 [M+H]⁺ | 150-153 | 0.6 (P) |
| 1.104 | F | 1,2-(Ethylen-dioxy)-phen-4-yl- | -CO-(1-methyl-piperazin-4-yl) | XVI.7 XXI | C₂₉H₂₇FN₄O₄ | 515 [M+H]⁺ | 220-223 | 0.6 (P) |
| 1.105 | F | 1,2-(Ethylen-dioxy)-phen-4-yl- | -CH₂-NEt₂ | XVI.7 XXV.16 | C₂₈H₂₈FN₃O₃ | 474 [M+H]⁺ | 108-110 | 0.6 (P) |
| 1.106 | F | 1,2-(Ethylen-dioxy)-phen-4-yl- | -CH₂-(4-hydroxy-piperidin-1-yl) | XVI.7 XXV.22 | C₂₉H₂₈FN₃O₄ | 502 [M+H]⁺ | 237-245 | 0.6 (P) |
| 1.107 | F | 1,2-(Ethylen-dioxy)-phen-4-yl- | -N(CH₃)-CO-CH₂-(1-methylpiper azin-4-yl) | XVI.7 XXV.2 | C₃₁H₃₂FN₅O₄ | 558 [M+H]⁺ | 163-167 | 0.6 (P) |
| 1.108 | F | 1,2-(Ethylen-dioxy)-phen-4-yl- | -SO₂-N(Me)-(CH₂)₂-NMe₂ | XVI.7 XXI.9 | C₂₈H₂₉FN₄O₅ S | 553 [M+H]⁺ | 170-175 | 0.6 (P) |
| 1.109 | F | 1,2-(Ethylen-dioxy)-phen-4-yl- | -CO-N(Et)-(CH₂)₂-NMe₂ | XVI.7 XXI.12 | C₃₀H₃₁FN₄O₄ | 531 [M+H]⁺ | 237-242 | 0.6 (P) |
| 1.110 | F | 1,2-(Ethylen-dioxy)-phen-4-yl- | -CH₂-N(Me)Et | XVI.7 XXV.10 | C₂₇H₂₆FN₃O₃ | 460 [M+H]⁺ | 118-123 | 0.6 (P) |
| 1.111 | F | 1,2-(Ethylen-dioxy)-phen-4-yl- | -CH₂-N(Me)-(CH₂)₂-OH | XVI.7 XXV.24 | C₂₇H₂₆FN₃O₄ | 474 [M-H]⁻ | 181-188 | 0.6 (P) |
| 1.112 | F | 1,2-(Ethylen-dioxy)-phen-4-yl- | -N(SO₂Me)-(CH₂)₂-NMe₂ | XVI.7 XXV.6 | C₂₈H₂₉FN₄O₅ S | 553 [M+H]⁺ | 180-183 | 0.6 (P) |
| 1.113 | F | 1,2-(Ethylen-dioxy)-phen-4-yl- | -CH₂-N(Me)-(CH₂)₂-OMe | XVI.7 XXV.31 | C₂₈H₂₈FN₃O₄ | 490 [M+H]⁺ | 103-106 | 0.6 (P) |
| 1.114 | F | 1,2-(Ethylen-dioxy)-phen-4-yl- | (*R*)-CH₂-(3-hydroxy-pyrrolidin-1-yl) | XVI.7 XXV.25 | C₂₈H₂₆FN₃O₄ | 488 [M+H]⁺ | 145-148 | 0.6 (P) |
| 1.115 | F | 1,2-(Ethylen-dioxy)-phen-4-yl- | (*S*)-CH₂-(3-hydroxy-pyrrolidin-1-yl) | XVI.7 XXV.26 | C₂₈H₂₆FN₃O₄ | 488 [M+H]⁺ | 140-147 | 0.6 (P) |
| 1.116 | F | 1,2-(Ethylen-dioxy)-phen-4-yl- | (*S*)-CH₂-(2-[hydroxy-methyl]-pyrrolidin-1-yl) | XVI.7 XXV.32 | C₂₉H₂₈FN₃O₄ | 502 [M+H]⁺ | 149-156 | 0.6 (P) |
| 1.117 | F | 1,2-(Ethylen-dioxy)-phen-4-yl- | -N(CH₃)-CO-(CH₂)₂-NMe₂ | XVI.7 XXV.23 | C₂₉H₂₉FN₄O₄ | 517 [M+H]⁺ | 131-138 | 0.6 (P) |
| 1.118 | F | 1,2-(Ethylen-dioxy)-phen-4-yl- | -N(COEt)-(CH₂)₂-NMe₂ | XVI.7 XXV.29 | C₃₀H₃₁FN₄O₄ | 531 [M+H]⁺ | 143 | 0.6 (P) |
| 1.119 | F | 1,2-(Ethylen-dioxy)-phen-4-yl- | -N(COEt)-(CH₂)₃-NMe₂ | XVI.7 XXV.28 | C₃₁H₃₃FN₄O₄ | 545 [M+H]⁺ | 127 | 0.6 (P) |
| 1.120 | F | 1,2-(Ethylen-dioxy)-phen-4-yl- | -CH₂-(1-methylpiperazin-4-yl) | XVI.7 XXV.11 | C₂₉H₂₉FN₄O₃ | 501 [M+H]⁺ | 195-198 | 0.7 (P) |
| 1.121 | F | 1,2-(Ethylen-dioxy)-phen-4-yl- | -SO₂-N(Me)-(CH₂)₃-NMe₂ | XVI.7 XXI.11 | C₂₉H₃₁FN₄O₅ S | 567 [M+H]⁺ | 144-149 | 0.5 (P) |
| 1.122 | F | 1,2-(Ethylen-dioxy)-phen-4-yl- | -N(SO₂Et)-(CH₂)₂-NMe₂ | XVI.7 XXV.27 | C₂₉H₃₁FN₄O₅ S | 567 [M+H]⁺ | 177-178 | 0.7 (P) |
| 1.123 | F | 1,2-(Ethylen-dioxy)-phen-4-yl- | -CH₂-(morpholin-4-yl) | XVI.7 XXV.33 | C₂₈H₂₆FN₃O₄ | 486 [M-H]⁻ | 215-224 | 0.8 (P) |
| 1.124 | F | 1,2-(Ethylen-dioxy)-phen-4-yl- | -SO₂-(CH₂)₂-NEt₂ | XVI.7 XXIV | C₂₉H₃₀FN₃O₅ S | 552 [M+H]⁺ | 186-192 | 0.7 (P) |
| 1.125 | F | 1,2-(Ethylen-dioxy)-phen-4-yl- | -SO₂-NH-(CH₂)₂-NMe₂ | XVI.7 XXIX | C₂₇H₂₇FN₄O₅ S | 539 [M+H]⁺ | 184 | 0.1 (G) |
| 1.126 | F | 1,2-(Ethylen-dioxy)-phen-4-yl- | -SO₂-NH-(CH₂)₃-NMe₂ | XVI.7 XXIX.1 | C₂₈H₂₉FN₄O₅ S | 551 [M-H]⁻ | 223 | 0.1 (G) |
| 1.127 | F | 1,2-(Ethylen-dioxy)-phen-4-yl- | -CH₂-CO-(1-methyl-piperazin-4-yl) | XVI.7 XXI.4 | C₃₀H₂₉FN₄O₄ | 527 [M-H]⁻ | 237 | 0.3 (G) |
| 1.128 | F | 1,2-(Ethylen-dioxy)-phen-4-yl- | -N(COMe)-(CH₂)₃-NMe₂ | XVI.7 XXV.4 | C₃₀H₃₁FN₄O₄ | 529 [M-H]⁻ | 115 | 0.1 (G) |
| 1.134 | H | 1-Methyl-benzimidazol-5-yl | -N(COMe)-(CH₂)₂-NMe₂ | XIX.1 XXV.3 | C₂₉H₃₀N₆O₂ | 495 [M+H]⁺ | 270 (Zerset zung) | 0.67 (P) |
| 1.135 | H | 1-Methyl-benzimidazol-5-yl | -N(CH₃)-CO-CH₂-NMe₂ | XIX.1 XXV.1 | C₂₈H₂₈N₆O₂ | 481 [M+H]⁺ | 243-245 | 0.59 (P) |
| 1.136 | H | 1-Methyl-benzimidazol-5-yl | -N(CO-3-pyridyl)-(CH₂)₂-NMe₂ | XIX.1 XXV.9 | C₃₃H₃₁N₇O₂ | 558 [M+H]⁺ | 188 (Zerset zung) | 0.79 (U) |
| 1.137 | COO Me | 1,2-(Ethylen-dioxy)-phen-4-yl- | -N(COEt)-(CH₂)₂-NMe₂ | XVI.6 XXV.29 | C₃₂H₃₄N₄O₆ | 571 [M+H]⁺ | 212-214 | 0.55 (A) |
| 1.138 | COO Me | 1,2-(Ethylen-dioxy)-phen-4-yl- | -N(COEt)-(CH₂)₃-NMe₂ | XVI.6 XXV.28 | C₃₃H₃₆N₄O₆ | 585 [M+H]⁺ | 143-145 | 0.47 (A) |
| 1.139 | COO Me | 1,2-(Ethylen-dioxy)-phen-4-yl- | -N(COMe)-(CH₂)₃-NMe₂ | XVI.6 XXV.4 | C₃₂H₃₄N₄O₆ | 571 [M+H]⁺ | 238-239 | 0.52 (A) |
| 1.140 | F | 1,2-(Methylen-dioxy)-phen-4-yl- | -N(CH₃)-CO-CH₂-(1-methylpiperazin-4-yl) | XVI.14 XXV.2 | C₃₀H₃₀FN₅O₄ | 544 [M+H]⁺ | n.d. | 0.30 (A) |
| 1.141 | Br | 1,2-(Methylen-dioxy)-phen-4-yl- | -N(CH₃)-CO-CH₂-(1-methylpiper azin-4-yl) | XVI.13 XXV.2 | C₃₀H₃₀BrN₅O₄ | 604/606 [M+H]⁺ | n.d. | 0.30 (A) |

| | | | | | | | | |
|---|---|---|---|---|---|---|---|---|
| *Fließmittelgemische: (A): Kieselgel, Dichlormethan/Methanol/konz. Ammoniak (90:10:1) (B): Kieselgel, Dichlormethan/Methanol/konz. Ammoniak (85:15:1.5) (C): Kieselgel, Essigester/Methanol/konz. Ammoniak (80:20:1) (D): Kieselgel, Essigester/Methanol/konz. Ammoniak (90:10:2) (E): Kieselgel, Essigester/Methanol/konz. Ammoniak (90:10:1) (F): Kieselgel, Dichlormethan/Methanol (5:1) (G): Kieselgel, Dichlormethan/Methanol (9:1) (H): Kieselgel, Dichlormethan/Methanol/konz. Ammoniak (50:10:0.1) (I): Kieselgel, Dichlormethan/Methanol/konz. Ammoniak (90:10:0.1) (K): Alox, Dichlormethan/Ethanol (20:1) (L): Kieselgel, Dichlormethan/Methanol/konz. Ammoniak (80:20:2) (M): Alox, Essigester/Methanol/konz. Ammoniak (90:10:1) (N): Kieselgel, Essigester/Methanol/konz. Ammoniak (40:10:1) (O): Kieselgel, Essigester/Methanol (9:1) (P): Kieselgel, Dichlormethan/Methanol/konz. Ammoniak (80:20:1) (Q): Kieselgel, Dichlormethan/Methanol (50:1) (R): Kieselgel, Dichlormethan/Methanol (30:1) (S): Kieselgel, Dichlormethan/Methanol (4:1) (T): Kieselgel, Essigester/Methanol/konz. Ammoniak (70:30:1) (U): Kieselgel, Dichlormethan/Methanol/konz. Ammoniak (80:10:1) | | | | | | | | |

Ebenfalls analog Beispiel 1 werden folgende Verbindungen hergestellt:
(1.129) 6-Fluor-3-(Z)-[1-(3-{N-(2-dimethylaminoethyl)-N-methyl-aminocarbonyl}-phenylamino)-1-(1-methylbenzimidazol-5-yl)methylen]-2-indolinon aus den Edukten XIX und XXI.13
   R_{f}-Wert:0.6 (Kieselgel, Methylenchlorid/Methanol/konz. Ammoniak = 80:20:1) Fp.: 131-138 °C
   C₂₉H₂₉FN₆O₂
   Massenspektrum: m/z = 513 [M+H]⁺
(1.130) 6-Chlor-3-(Z)-[1-(4-{N-(2-dimethylamino-ethyl)-N-methansulfonyl-amino}-3-chlor-phenylamino)-1-(1,2-ethylendioxyphen-4-yl)-methylen]-2-indolinon aus den Edukten XVI.1 und XXV.35
   R_{f}-Wert:0.2 (Kieselgel, Methylenchlorid/Methanol = 9:1)
   Fp.: 153 °C
   C₂₈H₂₈Cl₂N₄O₅S
   Massenspektrum: m/z = 601/603/605 [M+H]⁺
(1.131) 6-Chlor-3-(Z)-{1-[4-(N-dimethylaminomethylcarbonyl-N-methyl-amino)-3-methoxy-phenylamino]-1-(1,2-ethylendioxyphen-4-yl)-methylen}-2-indolinon aus den Edukten XVI.1 und XXX
   R_{f}-Wert:0.3 (Kieselgel, Methylenchlorid/Methanol/konz. Ammoniak = 90:10:1)
   Fp.: 229 °C
   C₂₉H₂₉ClN₄O₅
   Massenspektrum: m/z = 549/551 [M+H]⁺
(1.132) 6-Fluor-3-(Z)-{1-[4-(N-dimethylaminomethylcarbonyl-N-methyl-amino)-3-methoxy-phenylamino]-1-(1,2-ethylendioxyphen-4-yl)-methylen}-2-indolinon aus den Edukten XVI.7 und XXX
   R_{f}-Wert:0.3 (Kieselgel, Methylenchlorid/Methanol = 9:1)
   Fp.: 142 °C
   C₂₉H₂₉FN₄O₅
   Massenspektrum: m/z = 533 [M+H]⁺
(1.133) 6-Fluor-3-(Z)-[1-(4-{N-(2-dimethylamino-ethyl)-N-methansulfonyl-amino}-3-chlor-phenylamino)-1-(1,2-ethylendioxyphen-4-yl)-methylen]-2-indolinon aus den Edukten XVI.7 und XXV.35
   R_{f}-Wert:0.2 (Kieselgel, Methylenchlorid/Methanol = 9:1)
   Fp.: 173 °C
   C₂₈H₂₈ClFN₄O₅S
   Massenspektrum: m/z = 687/589 [M+H]⁺

### Beispiel 2.0

### 3-(Z)-{1-[4-(N-Dimethylaminomethylcarbonyl-N-methyl-amino)-phenylamino]-1-(2-pyrazinyl)-methylen}-2-indolinon

0.281 g 1-Acetyl-3-(1-hydroxy-1-(2-pyrazinyl)-methylen-2-indolinon (Edukt X) und 0.416 g Phosphorpentachlorid werden in 10 ml Dioxan 3 Stunden unter Rückfluß erhitzt. Flüchtige Bestandteile werden abdestilliert, der Rückstand in 15 ml Dioxan aufgenommen und nach Zugabe von 0.415 g N-(Dimethylaminomethylcarbonyl)-N-methyl-p-phenylendiamin (Edukt XXV.1) wiederum 5 Stunden unter Rückfluß erhitzt. Nach Abkühlen und Zugabe von 50 ml Wasser wird fünfmal mit Dichlormethan extrahiert. Der aus den über Magnesiumsulfat getrockneten vereinigten organischen Phasen durch Abdestillieren des Lösungsmittels erhaltene Rückstand wird über eine Kieselgelsäule mit Dichlormethan/Methanol/konz. Ammoniak = 190:10:1 als Laufmittel chromatographisch gereinigt. Das so erhaltene Rohprodukt wird mit Diisopropylether verrührt, abgesaugt und bei 80 °C getrocknet.
Ausbeute: 0.07 g (16% der Theorie)
R_{f}-Wert:0.2 (Kieselgel, Methylenchlorid/Methanol/konz. Ammoniak = 90:10:1)
Fp.: 198-199 °CC₂₄H₂₄N₆O₂
Massenspektrum: m/z = 429 [M+H]⁺; m/z = 427 [M-H]⁻; m/z = 451 [M+Na]⁺

Analog Beispiel 2.0 werden aus den jeweils angegebenen Edukten folgende Verbindungen der allgemeinen Formel I .2 hergestellt:

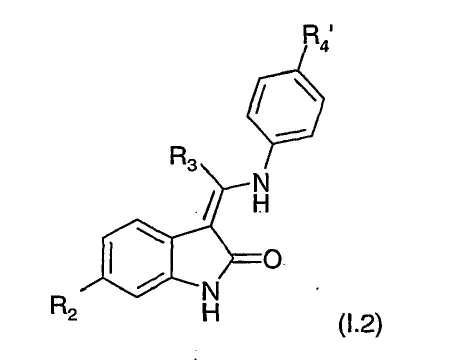

| Beispiel | R₂ | R₃ | R₄' | Edukte | Summenformel | Massenspektrum | Fp. [°C] | R_{f}- Wert* |
|---|---|---|---|---|---|---|---|---|
| 2.1 | H | 1,2-(Methylen-dioxy)-phen-4-yl- | -N(CH₃)-CO-CH₂-NMe₂ | X.1 XXV.1 | C₂₇H₂₆N₄O₄ | 471 [M+H]⁺ 469 [M-H]⁻ 493 [M+Na]⁺ | 221-222 | 0.3 (A) |
| 2.2 | H | 1,2-(Methylen-dioxy)-phen-4-yl- | -CH₂-NMe₂ | X.1 XXV.8 | C₂₅H₂₃N₃O₃ | 414 [M+H]⁺ 412 [M-H]⁻ 436 [M+Na]⁺ | 221-222 | 0.4 (A) |

| | | | | | | | | |
|---|---|---|---|---|---|---|---|---|
| *Fließmittelgemische: siehe Liste nach Beispiel 1 | | | | | | | | |

### Beispiel 3.0

### 6-Chlor-3-(Z)-[1-(4-{N-(dimethylaminomethylcarbonyl)-N-methyl-amino}-phenyl-amino)-1-(4-pyidy)-methyen]2-indolinon

0.270 g 1-Acetyl-6-chlor-3-[1-chlor-1-(4-pyridyl)-methylen]-2-indolinon (Edukt XIII.3), 0.232 g N-(Dimethylaminomethylcarbonyl)-N-methyl-*p*-phenylendiamin und 0.40 ml Triethylamin werden in 10 ml Tetrahydrofuran 15 Stunden unter Rückfluß erhitzt. Nach dem Abkühlen werden 0.50 ml Piperidin zugesetzt und 2 Stunden bei Raumtemperatur gerührt. Der durch Abdestillieren der flüchtigen Bestandteile erhaltene Rückstand wird über eine Kieselgelsäule mit Dichlormethan/Methanol = 9:1 als Laufmittel chromatographisch gereinigt. Das so erhaltene Rohprodukt wird mit Methanol verrührt, abgesaugt und bei 80 °C getrocknet.
Ausbeute: 0.075 g (20% der Theorie)
R_{f}-Wert:0.3 (Kieselgel, Dichlormethan/Methanol = 9:1)
Fp.: 274-276 °C
C₂₅H₂₄ClN₅O₂
Massenspektrum: m/z = 462/464 [M+H]⁺; m/z = 460/462 [M-H]⁻

Analog Beispiel 3.0 werden aus den jeweils angegebenen Edukten folgende Verbindungen der allgemeinen Formel I.3 hergestellt:

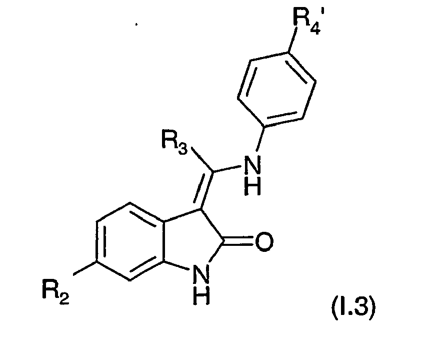

| Beispiel | R₂ | R₃ | R₄' | Edukte | Summenformel | Massenspektrum | Fp. [°C] | R_{f}-Wert* |
|---|---|---|---|---|---|---|---|---|
| 3.1 | Cl | 4-pyridyl- | -CH₂-NMe₂ | XIII.3 XXV.8 | C₂₃H₂₁ClN₄O | 405/407 [M+H]⁺ 403/405 [M-H]⁻ | 250-251 | 0.5 (F) |
| 3.2 | Cl | 4-pyridyl- | -N(SO₂- (CH₂)₂-CH₃)-(CH₂)₂-NMe₂ | XIII.3 XXV.7 | C₂₇H₃₀ClN₅O₃ S | 540/542 [M+H]⁺ 538/540 [M-H]⁻ | 228-232 | 0.3 (G) |
| 3.3 | H | 5-Methyl-isoxazol-3-yl- | -N(CH₃)-C(O)CH₃ | XV XXII | C₂₂H₂₀N₄O₃ | 388 [M⁻⁺] | 238-239 | 0.7 (A) |
| 3.4 | H | 5-Methyl-isoxazol-3-yl- | -N(SO₂-CH₃)-(CH₂)₂-NMe₂ | XV XXV.6 | C₂₄H₂₇N₅O₄S | 481 [M⁻⁺] | 241-242 | 0.3 (A) |
| 3.5 | H | 3-Methyl-pyrazol-5-yl- | -N(CH₃)-C(O)CH₃ | XV.1 XXII | C₂₂H₂₁N₅O₂ | 388 [M+H]⁺ 386 [M-H]⁻ 410 [M+Na]⁺ | 195-196 | 0.4 (A) |
| 3.6 | H | 3-Methyl-pyrazol-5-yl- | -N(SO₂-CH₃)-(CH₂)₂-NMe₂ | XV.1 XXV.6 | C₂₄H₂₈N₆O₃S | 480 [M⁻⁺] | 253-254 | 0.3 (A) |
| 3.7 | Cl | 2-Pyrrolyl- | -N(CH₃)-C(O)CH₂-NMe₂ | XIV XXV.1 | C₂₄H₂₄ClN₅O₂ | 450/452 [M+H]⁺ 448/450 [M-H]⁻ | 270-272 | 0.5 (G) |
| 3.8 | Cl | 2-Pyrrolyl- | -CH₂-NMe₂ | XIV XXV.8 | C₂₂H₂₁ClN₄O | 393/395 [M+H]⁺ 391/393 [M-H]⁻ | 201-203 | 0.3 (H) |
| 3.9 | Cl | 2-Pyrrolyl- | -N(C(O)-CH₃)-(CH₂)₂-NMe₂ | XIV XXV.3 | C₂₅H₂₆ClN₅O₂ | 464/462 [M+H]⁺ 462/464 [M-H]⁻ | 240-243 | 0.3 (I) |
| 3.10 | Cl | 2-Pyrrolyl- | -N(SO₂-(CH₂)₂-CH₃)-(CH₂)₂-NMe₂ | XIV XXV.7 | C₂₆H₃₀ClN₅O₃ S | 528/530 [M+H]⁺ 526/528 [M-H]⁻ | 203-205 | 0.3 (G) |
| 3.11 | Cl | 1-Benzyl-imidazol-5-yl- | -CH₂-NMe₂ | XIII.5 XXV.8 | C₂₈H₂₆ClN₅O | 482/484 [M-H]⁻ 506/508 [M+Na]⁺ | 270-272 (Fp. des Hydro-chlorids | 0.5 (K) |
| 3.12 | Cl | 3-Pyridyl- | -CH₂-NMe₂ | XIII.2 XXV.8 | C₂₃H₂₁ClN₄O | 403/405 [M-H]⁻ | 205-208 | 0.3 (F) |
| 3.13 | Cl | 3-Pyridyl- | -N(SO₂-(CH₂)₂-CH₃)-(CH₂)₂-NMe₂ | XIII.2 XXV.7 | C₂₇H₃₀ClN₅O₃ S | 464/462 [M+H]⁺ | 204-205 | 0.7 (F) |
| 3.14 | Cl | 3-Pyridyl- | -N(CH₃)-C(O)CH₂-NMe₂ | XIII.2 XXV.1 | C₂₅H₂₄ClN₅O₂ | 462/464 [M+H]⁺ | 243-245 | 0.6 (F) |

| | | | | | | | | |
|---|---|---|---|---|---|---|---|---|
| *Fließmittelgemische: siehe Liste nach Beispiel 1 | | | | | | | | |

### Beispiel 4.0

### 6-Chlor-3-(Z)-{1-[4-(pyrrolidin-1-ylmethyl)phenylamino]-1-(benzimidazol-5-yl)-methylen}-2-indolinon

0.184 6-Chlor-3-(1-(4-(pyrrolidin-1-ylmethyl)phenylamino)-1-(3,4-diaminophenyl)-methylen)-2-indolinon (Edukt XVIII.1), werden in 5 ml Ameisensäure 1 Stunde unter Rückfluß erhitzt. Der durch Abdestillieren des Lösungsmittels erhaltene Rückstand wird in Wasser suspendiert, durch Zugabe von Sodalösung alkalisch gestellt und mit Essigester extrahiert. Die Essigesterphase wird dreimal mit Wasser gegenextrahiert und über Natriumsulfat getrocknet. Der durch Abdestillieren des Lösungsmittels erhaltene Rückstand wird mit Diethylether verrührt, abgesaugt, mit Diethylether gewaschen und bei 80 °C getrocknet.
Ausbeute: 0.150 g (80% der Theorie)
R_{f}-Wert:0.5 (Kieselgel, Methylenchlorid/Methanol/konz. Ammoniak = 85:15:1.5)
Fp.: 277-279 °C
C₂₇H₂₄ClN₅O
Massenspektrum: m/z = 468/470 [M-H]⁻

Analog Beispiel 4.0 werden unter den jeweils angegebenen Reaktionsbedingungen folgende Verbindungen der Allgemeinen Formel I.4 hergestellt:

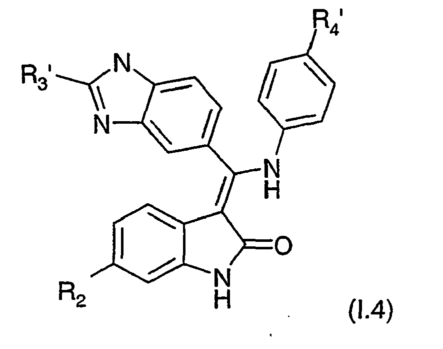

| Beispiel | R₂ | R₃' Solvens Reaktions-dauer | R₄' | Edukt | Summenformel | Massenspektrum | Fp. [°C] | Rr Wert* |
|---|---|---|---|---|---|---|---|---|
| 4.1 | Cl | CH₃-Essigsäure 1 h | -CH₂-pyrrolidin-1-yl- | XVIII.1 | C₂₈H₂₆ClN₅O | 484/486 [M+H]⁺ 482/484 [M-H]⁻ | 295-297 (Zersetzung) | 0.5 (B) |
| 4.2 | Cl | H-Ameisensäure 1 h | -N(CH₃)-C(O)CH₂-NMe₂ | XVIII.2 | C₂₇H₂₅ClN₆O₂ | 500/502 [M^{*}] 501/503 [M+H]⁺ 499/501 [M-H]⁻ | 285-288 (Zersetzung) | 0.3 (B) |
| 4.3 | Cl | CH₃-Essigsäure 7.5 h | -N(CH₃)-C(O)CH₂-NMe₂ | XVIII.2 | C₂₈H₂₇ClN₆O₂ | 515/517 [M+H]⁺ 513/515 [M-H]⁻ | 230-235 (Zersetzung; sintert ab 200) | 0.4 (B) |
| 4.4 | Cl | H-Ameisensäure 7 h | -C(O)-(1-methylpiperazin-4-yl) | XVIII | C₂₈H₂₅ClN₆O₂ | 515/515 [M+H]⁺ 511/513 [M-H]⁻ | 240-243, sintert 215-220 | 0.4 (B) |
| 4.5 | Cl | H-Ameisensäure 2.5 h | -N(C(O)-CH₃)-(CH₂)₂-NMe₂ | XVIII.3 | C₂₈H₂₇ClN₆O₂ | 513/515 [M-H]⁻ | 283-287 (Zersetzung) | 0.4 (B) |
| 4.6 | Cl | CH₃-Essigsäure 7h | -N(C(O)-CH₃)-(CH₂)₂-NMe₂ | XVIII.3 | C₂₉H₂₉ClN₆O₂ | 527/529 [M-H]⁻ | 266-268 | 0.4 (B) |
| 4.7 | Cl | H-Ameisensäure 4h | -N(C(O)-CH₃)-(CH₂)₃-NMe₂ | XVIII.4 | C₂₉H₂₉ClN₆O₂ | 529/531 [M+H]⁺ 527/529 [M-H]⁻ | 232-234 (sintert bei 180) | 0.3 (L) |
| 4.8 | Cl | CH₃-Essigsäure 9h | -N(C(O)-CH₃)-(CH₂)₃-NMe₂ | XVIII.4 | C₂₉H₂₉ClN₆O₂ | 543/545 [M+H]⁺ 541/543 [M-H]⁻ | 298-299 (Zersetzu ng) | 0.4 (M) |
| 4.9 | Cl | CH₃-Essigsäure 4.5 h | -C(O)-(1-methylpiperazin-4-yl) | XVIII | C₂₉H₂₇ClN₆O₂ | 527/529 [M+H]⁺ | 265-268 | 0.5 (B) |

| | | | | | | | | |
|---|---|---|---|---|---|---|---|---|
| *Fließmittelgemische: siehe Liste nach Beispiel 1 | | | | | | | | |

### Beispiel 5.0

### 3-(Z)-{1-[4-(Dimethylaminomethyl)phenylamino]-1-(benzimidazol-5-yl)methylen}-2-indolinon

0.429 g 3-(1-(4-(Dimethylaminomethyl)phenylamino)-1-(4-amino-3-nitrophenyl)-methylen)-2-indolinon (Edukt XVII.6), werden in 30 ml Ameisensäure unter Zusatz von 0.50 g Raney-Nickel bei Raumtemperatur 3.5 Stunden unter 50 psi Wasserstoffdruck hydriert. Der Katalysator wird abgesaugt, die Lösung eingeengt und der Rückstand in Dichlormethan/Methanol/Ammoniak = 50:50:1 gelöst. Die Lösung wird durch Kieselgel filtriert, der durch Abdestillieren des Lösungsmittels erhaltene Rückstand wird in Sodalösung aufgenommen und über Nacht gerührt. Der entstandene Niederschlag wird abfiltriert, mit Wasser gewaschen, getrocknet, mit Diethylether verrührt, erneut abfiltriert und bei 100 °C getrocknet.
Ausbeute: 0.320 g (78% der Theorie)
R_{f}-Wert:0.4 (Kieselgel, Methylenchlorid/Methanol/konz. Ammoniak = 80:20:2)
Fp.: 273-277 °C (Zersetzung)
C₂₅H₂₃N₅O
Massenspektrum: m/z = 408 [M-H]⁻

Analog Beispiel 5.0 wird folgende Verbindung hergestellt:
(5.1) 6-Chlor-3-(Z)-[1-(4-{N-methansulfonyl-N-(2-dimethylamino-ethyl)-amino}-phenylamino)-1-(benzimidazol-5-yl)methylen]-2-indolinon
   aus 6-Chlor-3-[1-(4-{N-methansulfonyl-N-(2-dimethylamino-ethyl)-amino}-phenylamino)-1-(4-amino-3-nitrophenyl)-methylen]-2-indolinon.
   R_{f}-Wert:0.5 (Kieselgel, Methylenchlorid/Methanol/konz. Ammoniak = 85:15:1.5)
   Fp: 160 °C (sintert)
   C₂₇H₂₇ClN₆O₃S
   Massenspektrum: m/z = 551/553 [M+H]⁺; m/z = 549/551 [M-H]⁻

### Beispiel 6.0

### 3-(Z)-[1-(1-Methylpiperidin-4-ylamino)-1-(3-furyl)-methylen]-2-indolinon

4.30 g 1-Acetyl-3-[1-hydroxy-1-(3-furyl)-methylen]-2-indolinon (Edukt X.14) und 3.6 g 4-Amino-1-methylpiperidin werden 1.5 Stunden auf 140 °C erhitzt. Nach dem Abkühlen werden 2 N Salzsäure und Essigester zugegeben und der Ansatz bis zur vollständigen Lösung gerührt. Die wässrige Phase wird abgetrennt, mit konz. Ammoniaklösung alkalisch gestellt. Der entstandene Niederschlag wird abgesaugt, mit Eiswasser gewaschen, getrocknet, mit Aceton verrührt, erneut abgesaugt und wiederum getrocknet.
Ausbeute: 0.287 g (5.6% der Theorie)
R_{f}-Wert:0.5 (Kieselgel, Dichlormethan/Methanol/konz. Ammoniak = 50:10:0.1) Fp.: 255-260 °C
C₁₉H₂₁N₃O₂
Massenspektrum: m/z = 323 [M⁻]⁺
Analog Beispiel 6.0 werden folgende Verbindungen hergestellt:
(6.1) 3-(Z)-[1-(1-Methylpiperidin-4-ylamino)-1-(3-thienyl)methylen]-2-indolinon
   aus 1-Acetyl-3-(1-hydroxy-1-(3-thienyl)-methylen-2-indolinon (Edukt X.2).
   Das Produkt fällt bei der Aufarbeitung als Hydrochlorid aus, welches gewaschen und getrocknet wird.
   R_{f}-Wert:0.55 (Kieselgel, Dichlormethan/Methanol/konz. Ammoniak = 50:10:0.1)
   Fp.: 360 °C
   C₁₉H₂₁N₃OS
   Massenspektrum: m/z = 339 [M⁻]⁺
(6.2) 3-(Z)-[1-(1-Methylpiperidin-4-ylamino)-1-(4-imidazolyl)-methylen]-2-indolinon aus 1-Acetyl-3-[1-hydroxy-1-(4-imidazolyl)-methylen]-2-indolinon (Edukt XI.3).
   R_{f}-Wert: 0.18 (Kieselgel, Essigester/Methanol/konz. Ammoniak 5:5:1)
   Fp.: 274-279 °C
   C₁₈H₁₈ClN₃O
   Massenspektrum: m/z = 324 [M+H]⁺; m/z = 322 [M-H]⁻
(6.3) 3-(Z)-[1-(1-Methylpiperidin-4-ylamino)-1-(2-furyl)-methylen]-2-indolinon aus 1-Acetyl-3-[1-hydroxy-1-(2-furyl)-methylen]-2-indolinon
   R_{f}-Wert: 0.45 (Kieselgel, Dichlormethan/Methanol/konz. Ammoniak = 50:10:1)
   Fp.: 255-260 °C
   C₁₉H₂₁N₃O₂
   Massenspektrum: m/z = 323 [M⁻]⁺

### Beispiel 7.0

### 6-Chlor-3-(Z)-{1-[4-(dimethylaminomethyl)-phenylamino]-1-(1-methylbenzimidazol-5-yl)-methylen}-2-indolinon

0.344 g 6-Chlor-3-[1-chlor-1-(1-methylbenzimidazol-5-yl)-methylen]-2-indolinon (Edukt XIII.4), 0.195 g 4-(Dimethylaminomethyl)-anilin und 0.513 ml Ethyldiisopropylamin werden in 5 ml DMF 6 Stunden bei 100 °C gerührt. Nach weiterer Zugabe von 0.090 g 4-(Dimethylaminomethyl)-anilin und 0.34 ml Ethyl-diisopropylamin wird weitere 6 Stunden bei 100 °C gerührt. Nach Abkühlen und Zugabe von Wasser wird der entstandene Niederschlag abgesaugt und mit Wasser gewaschen. Das so erhaltene Rohprodukt wird über eine Alox-Säule mit Essigester/Methanol = 95:5 als Laufmittel chromatographisch gereinigt. Das Produkt wird mit eiskaltem Diethylether verrührt, abgesaugt und bei 80 °C getrocknet. Ausbeute: 0.060 g (13% der Theorie)
R_{f}-Wert:0.52 (Alox, Ethylacetat/Methanol = 95:5)
Fp.: 288 °C (Zersetzung)
C₂₆H₂₄ClN₅O
Massenspektrum: m/z = 458/460 [M+H]⁺
Analog Beispiel 7.0 werden folgende Verbindungen der Allgemeinen Formel I.7 hergestellt:

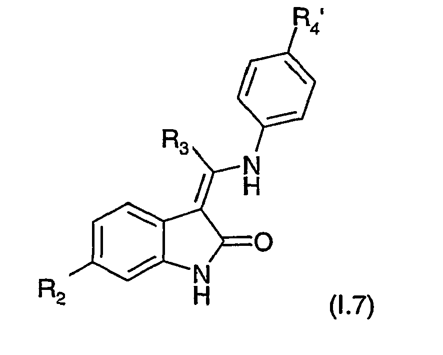

| Beispiel | R₂ | R₃ | R₄' | Edukte | Summenformel | Massenspektrum | Fp. [°C] | R_{f}-Wert* |
|---|---|---|---|---|---|---|---|---|
| 7.1 | Cl | 1-Methylbenzim idazol-5-yl- | -CO-(1-methyl-piperazin-4-yl) | XIII.4 XXI | C₂₉H₂₇ClN₆O₂ | 527/529 [M+H]⁺ | 292 | 0.4 (O) |
| 7.2 | Cl | 1-Methylbenzimidazol-5-yl- | -N(CH₃)-C(O)CH₂-NMe₂ | XIII.4 XXV.1 | C₂₈H₂₇ClN₆O₂ | 515/517 [M+H]⁺ | 270 | 0.41 (O) |
| 7.3 | Cl | Pyridazin-4-yl- | -N(CO-Me)-(CH₂)₂-NMe₂ | XIV.1 XXV.3 | C₂₅H₂₅ClN₆O₂ | 477/479 [M+H]⁺ 475/477 [M-H]⁻ 476/478 [M⁻]⁺ | n.d. | 0.3 (A) |

| | | | | | | | | |
|---|---|---|---|---|---|---|---|---|
| *Fließmittelgemische: siehe Liste nach Beispiel 1 | | | | | | | | |

### Beispiel 8.0

### 6-Chlor-3-(Z)-{1-[4-(ethylaminomethyl)-phenylamino]-1-(3,4-methylendioxyphenyl)-methylen}-2-indolinon

0.8 g 1-Acetyl-6-chlor-3-(Z)-{1-[4-(N-*tert*-butoxycarbonyl-N-ethyl-aminomethyl)-phenylamino]-1-(3,4-methylendioxyphenyl)-methylen}-2-indolinon (Beispiel 1.17) werden in 7 ml Trifluoressigsäure und 14 ml Dichlormethan 1.5 Stunden bei Raumtemperatur gerührt. Der nach Abdestillieren der flüchtigen Bestandteile erhaltene Rückstand wird mit Diethylether verrieben, der erhaltene Niederschlag abgesaugt und bei 100 °C getrocknet.
Ausbeute: 0.78 g (95% der Theorie)
R_{f}-Wert:0.42 (Kieselgel, Dichlormethan/Methanol/konz. Ammoniak = 90:10:1)
Fp.: 248-250 °C
C₂₅H₂₂ClN₃O₃ · C₂HF₃O₂
Massenspektrum: m/z = 470/472 [M+Na]⁺
Analog Beispiel 8.0 werden folgende Verbindungen der allgemeinen Formel I.8 hergestellt:

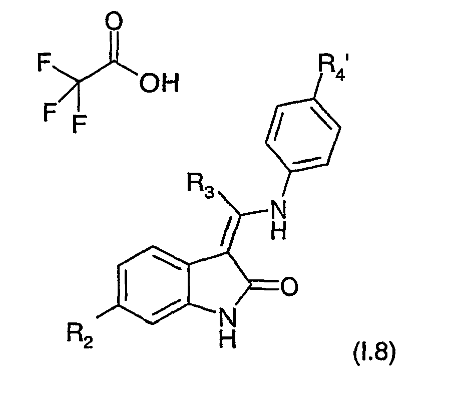

| Beispiel | R₂ | R₃ | R₄' | Edukt | Summenformel | Massenspektrum | Fp. [°C] | R_{f}-Wert* |
|---|---|---|---|---|---|---|---|---|
| 8.1 | Cl | 1,2-(Methylen-dioxy)-phen-4-yl- | -CH₂-NHMe | 1.18 | C₂₄H₂₀ClN₃O₃ · C₂HF₃O₂ | 456/458 [M+Na]⁺ | 252-254 | 0.13 (A) |
| 8.2 | Cl | 1,2-(Methylen-dioxy)-phen-4-yl- | -CH₂-1-piperazinyl | 1.19 | C₂₇H₂₅ClN₄O₃ · C₂HF₃O₂ | 489/491 [M+H]⁺ | 201 | 0.32 (A) |
| 8.3 | Cl | 1,2-(Difluormethylendioxy)-phen-4-yl- | -CH₂-NHMe | 1.27 | C₂₄H₁₈ClF₂N₃ O₃ · C₂HF₃O₂ | 470/472 [M+H]⁺ | 267-271 | 0.1 (C) |
| 8.4 | F | 1,2-(Ethylendioxy)-phen-4-yl- | -CH₂-NHEt | 1.37 | C₂₆H₂₄FN₃O₃· C₂HF₃O₂ | 468 [M+Na]⁺ | 153-154 | 0.66 (A) |
| 8.5 | CO OMe | 1,2-(Ethylen-dioxy)-phen-4-yl- | -CH₂-NHEt | 1.51 | C₂₈H₂₇FN₃O₅· C₂HF₃O₂ | 486 [M+H]⁺ 484 [M-H]⁻ | 266 | 0.12 (G) |
| 8.6 | CO OMe | 1,2-(Methylendioxy)-phen-4-yl- | -CH₂-NHEt | 1.62 | C₂₇H₂₅FN₃O₅· C₂HF₃O₂ | 472 [M+H]⁺ | 258 | 0.55 (S) |

| | | | | | | | | |
|---|---|---|---|---|---|---|---|---|
| *Fließmittelgemische: siehe Liste nach Beispiel 1 | | | | | | | | |

### Beispiel 9.0

### 6-Carboxy-3-(Z)-{1-[4-(1-methylpiperazin-4-yl-methyl)-phenylamino]-1-(3,4-ethylen-dioxyphenyl)-methylen}-2-indolinon

0.36 g 6-Methoxycarbonyl-3-(Z)-{1-[4-(1-methylpiperazin-4-yl-methyl)-phenylamino]-1-(3,4-ethylendioxyphenyl)-methylen}-2-indolinon (Beispiel 1.57) in 5 ml Ethanol werden bei 80 °C mit 1.6 ml 1 molarer Natronlauge versetzt und 2 Stunden bei 80 °C gerührt. Nach Zugabe von 1.6 ml 1 molarer Salzsäure und 2 ml Wasser wird der Ansatz ohne Heizung über Nacht weitergerührt. Der entstandene Niederschlag wird abgesaugt, hintereinander mit Wasser, wenig Ethanol und zuletzt mit Diethylether gewaschen und anschließend bei 80 °C getrocknet. Ausbeute: 0.32 g (91 % der Theorie)
R_{f}-Wert: 0.15 (Kieselgel, Dichlormethan/Methanol =4:1)
C₃₀H₃₀N₄O₅
Massenspektrum: m/z = 527 [M+H]⁺; m/z = 525 [M-H]⁻

### Beispiel 10.0

### 6-Methylaminocarbonyl-3-(Z)-{1-[4-(1-methylpiperazin-4-yl-methyl)-phenylamino]-1-(3,4-ethylendioxyphenyl)-methylen}-2-indolinon

0.100 g 6-Carboxy-3-(Z)-{1-[4-(1-methylpiperazin-4-yl-methyl)-phenylamino]-1-(3,4-ethylendioxyphenyl)-methylen}-2-indolinon, 0.103 g Dimethylamin hydrochlorid, 73 mg TBTU, 35 mg HOBT und 0.416 ml Triethylamin werden in 3 ml DMF 6 Stunden bei Raumtemperatur gerührt. Das Reaktionsgemisch wird auf Wasser gegossen und dreimal mit Dichlromethan/Methanol 19:1 extrahiert. Die vereinigten organischen Phasen werden mit Wasser gewaschen und über Natriumsulfat getrocknet. Ungelöste Bestandteile werden abfiltriert und das Filtrat im Vakuum zur Trockne eingedampft. Der Rückstand wird mit Diethylether verrieben, abgesaugt und bei 80 °C getrocknet.
Ausbeute: 0.075 g (73% der Theorie)
R_{f}-Wert: 0.38 (Kieselgel, Dichlormethan/Methanol = 9:1)
Fp.: 172 °C
C₃₁H₃₃N₅O₄
Massenspektrum: m/z = 540 [M+H]⁺; m/z = 538 [M-H]⁻
Analog Beispiel 10.0 wird folgende Verbindung hergestellt:
(10.1) 6-Dimethylaminocarbonyl-3-(Z)-{1-[4-(1-methylpiperazin-4-yl-methyl)-phenylamino]-1-(3,4-ethylendioxyphenyl)-methylen}-2-indolinon unter Verwendung von Dimethylamin hydrochlorid.
   R_{f}-Wert: 0.51 (Kieselgel, Dichlormethan/Methanol = 9:1)
   Fp.: 257 °C
   C₃₂H₃₅N₅O₄
   Massenspektrum: m/z = 554 [M+H]⁺; m/z = 552 [M-H]⁻

### Beispiel 11

### 6-Fluor-3-(Z)-{1-[4-(ethylaminomethyl)-phenylamino]-1-(1-methylbenzimidazol-5-yl)-methylen}-2-indolinon

Eine Lösung von 0.30 g 1-Acetyl-6-fluor-3-[1-methoxy-1-(1-methylbenzimidazol-5-yl)methylen]-2-indolinon (Beispiel XIX) und 0.206 g 4-(*N*-Ethyl-*N*-*tert-*butoxycarbonyl-aminomethyl)-anilin (Beispiel XXV.12) in 5 ml DMF wird 15 Stunden bei 115 °C gerührt. Der durch abdestillieren des Lösungsmittels erhaltene Rückstand wird in 5 ml Methanol aufgenommen und nach Zugabe von 1 ml 1 M Natronlauge 1 Stunde gerührt. Die Lösung wird durch Zugabe von Ammoniumchloridlösung neutralisiert und anschließend zur Trockne eingedampft. Der Rückstand wird chromatographisch über eine Kieselgelsäule mit Dichlormethan/Methanol/Ammoniak = 90:10:1 gereinigt. Das so erhaltene Zwischenprodukt wird in 5 ml Dichlormethan gelöst, mit 1 ml Trifluoressigsäure versetzt und 30 Minuten gerührt. Nach Abdestillieren der flüchtigen Bestandteile wird der Rückstand aus Diethylether kristallisiert und anschließend bei 80 °C getrocknet.
Ausbeute: 0.143 g (31 % der Theorie)
R_{f}-Wert: 0.2 (Kieselgel, Dichlormethan/Methanol/konz. Ammoniaklösung = 80:20:1)
Fp.: 226-228 °C
C₂₆H₂₄FN₅O · C₂HF₃O₂
Analog Beispiel 11.0 werden folgende Verbindungen der allgemeinen Formel I.11 hergestellt:

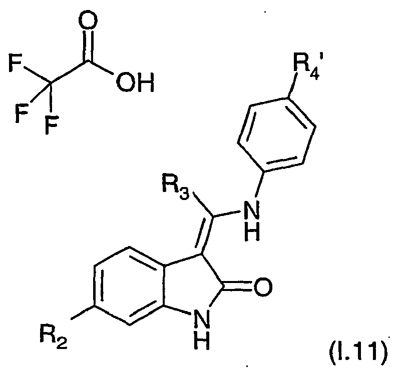

| Beispiel | R₂ | R₃ | R₄' | Edukte | Summenformel | Massenspektrum | Fp. [°C] | R_{f}-Wert* |
|---|---|---|---|---|---|---|---|---|
| 11.1 | Cl | 1,2-(Ethylen-dioxy)-phen-4-yl- | -CH₂-NHMe | XVI.1 XXV.13 | C₂₅H₂₂CIN₃O₃ · C₂HF₃O₂ | 446/448 [M-H]⁻ | 260 (Zersetzung) | 0.07 (E) |
| 11.2 | Cl | 1,2-(Ethylen-dioxy)-phen-4-yl- | -CH₂-NHEt | XVI.1 XXV.12 | C₂₆H₂₄CIN₃O₃ · C₂HF₃O₂ | 460/462 [M-H]⁻ | 247 (Zersetzung) | 0.11 (E) |
| 11.3 | Cl | 1,2-(Ethylen-dioxy)-phen-4-yl- | -CH₂-NH/Bu | XVI.1 XXV.34 | C₂₈H₂₈ClN₃O₃ · C₂HF₃O₂ | 490/492 [M+H]⁺ | 248 (Zersetzung) | 0.38 (E) |
| 11.4 | F | 1,2-(Ethylen-dioxy)-phen-4-yl- | -CH₂-NHMe | XVI.7 XXV.13 | C₂₅H₂₂FN₃O₃ · C₂HF₃O₂ | 430 [M-H]⁻ | 125-133 | 0.6 (P) |
| 11.5 | F | 1,2-(Ethylen-dioxy)-phen-4-yl- | -CH₂-NH/Bu | XVI.7 XXV.34 | C₂₈H₂₈FN₃O₃· C₂HF₃O₂ | 474 [M+H]⁺ | 239-242 | 0.6 (P) |
| 11.6 | F | 1,2-(Ethylen-dioxy)-phen-4-yl- | -CO-(perhydro-diazepin-1-yl) | XVI.7 XXI.15 | C₂₉H₂₇FN₄O₄· C₂HF₃O₂ | 515 [M+H]⁺ | 247-253 | 0.6 (P) |
| 11.7 | Cl | 1,2-(Ethylen-dioxy)-phen-4-yl- | -CO-NMe-(CH₂)₂-NHMe | XVI.1 XXI.16 | C₂₈H₂₇ClN₄O₄ · C₂HF₃O₂ | 519/521 [M+H]⁺ | 185 (Zersetzung) | 0.05 (E) |
| 11.8 | F | 1,2-(Ethylen-dioxy)-phen-4-yl- | -CO-(piperazin-1-yl) | XVI.7 XXI.14 | C₂₈H₂₅FN₄O₄· C₂HF₃O₂ | 501 [M+H]⁺ | 256-263 | 0.6 (P) |
| 11.9 | F | 1,2-(Ethylen-dioxy)-phen-4-yl- | -CO-NMe-(CH₂)₂-NHMe | XVI.7 XXI.16 | C₂₈H₂₇FN₄O₄ · C₂HF₃O₂ | 503 [M+H]⁺ | 166 | 0.1 (G) |

| | | | | | | | | |
|---|---|---|---|---|---|---|---|---|
| *Fließmittelgemische: siehe Liste nach Beispiel 1 | | | | | | | | |

### Beispiel 13

### Trockenampulle mit 75 mg Wirkstoff pro 10 ml

| Zusammensetzung: | |
|---|---|
| Wirkstoff | 75.0 mg |
| Mannitol | 50.0 mg |
| Wasser für Injektionszwecke | ad 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel 14

### Trockenampulle mit 35 mg Wirkstoff pro 2 ml

| Zusammensetzung: | |
|---|---|
| Wirkstoff | 35.0 mg |
| Mannitol | 100.0 mg |
| Wasser für Injektionszwecke | ad 2.0 ml |

### Herstellung:

Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

### Beispiel 15

### Tablette mit 50 mg Wirkstoff

| Zusammensetzung: | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215.0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

### Beispiel 16

### Tablette mit 350 mg Wirkstoff

| Zusammensetzung: | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

### Beispiel 17

### Kapseln mit 50 mg Wirkstoff

| Zusammensetzung: | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 3 abgefüllt.

### Beispiel 18

### Kapseln mit 350 mg Wirkstoff

| Zusammensetzung: | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

### Beispiel 19

### Suppositorien mit 100 mg Wirkstoff

| 1 Zäpfchen enthält: | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2 000.0 mg |

### Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

Analog den vorstehenden Beispielen können folgende Verbindungen hergestellt werden:
(1) 3-(*Z*)-{1-[4-(Dimethylaminomethyl)-phenylamino]-1-(3,4-methylendioxyphenyl)-methylen}-6-cyano-2-indolinon
(2) 3-(*Z*)-{1-[4-(Diethylaminomethyl)-phenylamino]-1-(3,4-methylendioxyphenyl)-methylen}-6-cyano-2-indolinon
(3) 3-(*Z*)-{1-[4-(1-Methylpiperazin-4-yl-methyl)-phenylamino]-1-(3,4-methylendioxyphenyl)-methylen}-6-cyano-2-indolinon
(4) 3-(*Z*)-{1-[4-(Ethylaminomethyl)-phenylamino]-1-(3,4-methylendioxyphenyl)-methylen}-6-cyano-2-indolinon
(5) 3-(*Z*)-{1-[4-(N-Methyl-N-{dimethylaminomethyl-carbonyl}-amino)-phenylamino]-1-(3,4-methylendioxyphenyl)-methylen}-6-cyano-2-indolinon
(6) 3-(*Z*)-{1-[4-(N-Methyl-N-{1-methylpiperazin-4-yl-methylcarbonyl}-amino)-phenylamino]-1-(3,4-methylendioxyphenyl)-methylen}-6-cyano-2-indolinon
(7) 3-(*Z*)-{1-[4-(N-Acetyl-N-{2-dimethylamino-ethyl}-amino)-phenylamino]-1-(3,4-methylendioxyphenyl)-methylen}-6-cyano-2-indolinon
(8) 3-(*Z*)-{1-[4-(N-Acetyl-N-{3-dimethylamino-propyl}-amino)-phenylamino]-1-(3,4-methylendioxyphenyl)-methylen}-6-cyano-2-indolinon
(9) 3-(*Z*)-{1-[4-(N-Methyl-N-{2-dimethylamino-ethyl}-aminocarbonyl)-phenylamino]-1-(3,4-methylendioxyphenyl)-methylen}-6-cyano-2-indolinon
(10) 3-(*Z*)-{1-[4-(N-Methansulfonyl-N-{2-dimethylamino-ethyl}-amino)-phenylamino]-1-(3,4-methylendioxyphenyl)-methylen}-6-cyano-2-indolinon
(11) 3-(*Z*)-{1-[4-(1-Methylpiperazin-4-yl-carbonylmethyl)-phenylamino]-1-(3,4-methylendioxyphenyl)-methylen}-6-cyano-2-indolinon
(12) 3-(*Z*)-{1-[4-(N-Methyl-N-{2-dimethylamino-ethyl-carbonyl}-amino)-phenylamino]-1-(3,4-methylendioxyphenyl)-methylen}-6-cyano-2-indolinon
(13) 3-(*Z*)-{1-[4-(1-Methyl-imidazol-2-yl)-phenylamino]-1-(3,4-methylendioxyphenyl)-methylen}-6-cyano-2-indolinon
(14) 3-(*Z*)-{1-[4-(Dimethylaminomethyl)-phenylamino]-1-(3,4-ethylendioxyphenyl)-methylen}-6-cyano-2-indolinon
(15) 3-(*Z*)-{1-[4-(Diethylaminomethyl)-phenylamino]-1-(3,4-ethylendioxyphenyl)-methylen}-6-cyano-2-indolinon
(16) 3-(*Z*)-{1-[4-(1-Methylpiperazin-4-yl-methyl)-phenylamino]-1-(3,4-ethylendioxyphenyl)-methylen}-6-cyano-2-indolinon
(17) 3-(*Z*)-{1-[4-(Ethylaminomethyl)-phenylamino]-1-(3,4-ethylendioxyphenyl)-methylen}-6-cyano-2-indolinon
(18) 3-(*Z*)-{1-[4-(N-Methyl-N-{dimethylaminomethyl-carbonyl}-amino)-phenylamino]-1-(3,4-ethylendioxyphenyl)-methylen}-6-cyano-2-indolinon
(19) 3-(*Z*)-{1-[4-(N-Methyl-N-{1-methylpiperazin-4-yl-methylcarbonyl}-amino)-phenylamino]-1-(3,4-ethylendioxyphenyl)-methylen}-6-cyano-2-indolinon
(20) 3-(*Z*)-{1-[4-(N-Acetyl-N-{2-dimethylamino-ethyl}-amino)-phenylamino]-1-(3,4-ethylendioxyphenyl)-methylen}-6-cyano-2-indolinon
(21) 3-(*Z*)-{1-[4-(N-Acetyl-N-{3-dimethylamino-propyl}-amino)-phenylamino]-1-(3,4-ethylendioxyphenyl)-methylen}-6-cyano-2-indolinon
(22) 3-(*Z*)-{1-[4-(N-Methyl-N-{2-dimethylamino-ethyl}-aminocarbonyl)-phenylamino]-1-(3,4-ethylendioxyphenyl)-methylen}-6-cyano-2-indolinon
(23) 3-(*Z*)-{1-[4-(N-Methansulfonyl-N-{2-dimethylamino-ethyl}-amino)-phenylamino]-1-(3,4-ethylendioxyphenyl)-methylen}-6-cyano-2-indolinon
(24) 3-(*Z*)-{1-[4-(1-Methylpiperazin-4-yl-carbonylmethyl)-phenylamino]-1-(3,4-ethylendioxyphenyl)-methylen}-6-cyano-2-indolinon
(25) 3-(*Z*)-{1-[4-(N-Methyl-N-{2-dimethylamino-ethyl-carbonyl}-amino)-phenylamino]-1-(3,4-ethylendioxyphenyl)-methylen}-6-cyano-2-indolinon
(26) 3-(*Z*)-{1-[4-(1-Methyl-imidazol-2-yl)-phenylamino]-1-(3,4-ethylendioxyphenyl)-methylen}-6-cyano-2-indolinon
(27) 3-(*Z*)-{1-[4-(Dimethylaminomethyl)-phenylamino]-1-(3,4-methylendioxyphenyl)-methylen}-6-nitro-2-indolinon
(28) 3-(*Z*)-{1-[4-(Diethylaminomethyl)-phenylamino]-1-(3,4-methylendioxyphenyl)-methylen}-6-nitro-2-indolinon
(29) 3-(*Z*)-{1-[4-(1-Methylpiperazin-4-yl-methyl)-phenylamino]-1-(3,4-methylendioxyphenyl)-methylen}-6-nitro-2-indolinon
(30) 3-(*Z*)-{1-[4-(Ethylaminomethyl)-phenylamino]-1-(3,4-methylendioxyphenyl)-methylen}-6-nitro-2-indolinon
(31) 3-(*Z*)-{1-[4-(N-Methyl-N-{dimethylaminomethyl-carbonyl}-amino)-phenylamino]-1-(3,4-methylendioxyphenyl)-methylen}-6-nitro-2-indolinon
(32) 3-(*Z*)-{1-[4-(N-Methyl-N-{1-methylpiperazin-4-yl-methylcarbonyl}-amino)-phenylamino]-1-(3,4-methylendioxyphenyl)-methylen}-6-nitro-2-indolinon
(33) 3-(*Z*)-{1-[4-(N-Acetyl-N-{2-dimethylamino-ethyl}-amino)-phenylamino]-1-(3,4-methylendioxyphenyl)-methylen}-6-nitro-2-indolinon
(34) 3-(*Z*)-{1-[4-(N-Acetyl-N-{3-dimethylamino-propyl}-amino)-phenylamino]-1-(3,4-methylendioxyphenyl)-methylen}-6-nitro-2-indolinon
(35) 3-(*Z*)-{1-[4-(N-Methyl-N-{2-dimethylamino-ethyl}-aminocarbonyl)-phenylamino]-1-(3,4-methylendioxyphenyl)-methylen}-6-nitro-2-indolinon
(36) 3-(*Z*)-{1-[4-(N-Methansulfonyl-N-{2-dimethylamino-ethyl}-amino)-phenylamino]-1-(3,4-methylendioxyphenyl)-methylen}-6-nitro-2-indolinon
(37) 3-(*Z*)-{1-[4-(-Methylpiperazin-4-yl-carbonylmethyl)-phenylamino]-1-(3,4-methylendioxyphenyl)-methylen}-6-nitro-2-indolinon
(38) 3-(*Z*)-{1-[4-(N-Methyl-N-{2-dimethylamino-ethyl-carbonyl}-amino)-phenylamino]-1-(3,4-methylendioxyphenyl)-methylen}-6-nitro-2-indolinon
(39) 3-(*Z*)-{1-[4-(1-Methyl-imidazol-2-yl)-phenylamino]-1-(3,4-methylendioxyphenyl)-methylen}-6-nitro-2-indolinon
(40) 3-(*Z*)-{1-[4-(Dimethylaminomethyl)-phenylamino]-1-(3,4-ethylendioxyphenyl)-methylen}-6-nitro-2-indolinon
(41) 3-(*Z*)-{1-[4-(Diethylaminomethyl)-phenylamino]-1-(3,4-ethylendioxyphenyl)methylen}-6-nitro-2-indolinon
(42) 3-(*Z*)-{1-[4-(1-Methylpiperazin-4-yl-methyl)-phenylamino]-1-(3,4-ethylendioxyphenyl)-methylen}-6-nitro-2-indolinon
(43) 3-(*Z*)-{1-[4-(Ethylaminomethyl)-phenylamino]-1-(3,4-ethylendioxyphenyl)-methylen}-6-nitro-2-indolinon
(44) 3-(*Z*)-{1-[4-(N-Methyl-N-{dimethylaminomethyl-carbonyl}-amino)-phenylamino]-1-(3,4-ethylendioxyphenyl)-methylen}-6-nitro-2-indolinon
(45) 3-(*Z*)-{1-[4-(N-Methyl-N-{1-methylpiperazin-4-yl-methylcarbonyl}-amino)-phenylamino]-1-(3,4-ethylendioxyphenyl)-methylen}-6-nitro-2-indolinon
(46) 3-(*Z*)-{1-[4-(N-Acetyl-N-{2-dimethylamino-ethyl}-amino)-phenylamino]-1-(3,4-ethylendioxyphenyl)-methylen}-6-nitro-2-indolinon
(47) 3-(*Z*)-{1-[4-(N-Acetyl-N-{3-dimethylamino-propyl}-amino)-phenylamino]-1-(3,4-ethylendioxyphenyl)-methylen}-6-nitro-2-indolinon
(48) 3-(*Z*)-{1-[4-(N-Methyl-N-{2-dimethylamino-ethyl}-aminocarbonyl)-phenylamino]-1-(3,4-ethylendioxyphenyl)-methylen}-6-nitro-2-indolinon
(49) 3-(*Z*)-{1-[4-(N-Methansulfonyl-N-{2-dimethylamino-ethyl}-amino)-phenylamino]-1-(3,4-ethylendioxyphenyl)-methylen}-6-nitro-2-indolinon
(50) 3-(*Z*)-{1-[4-(1-Methylpiperazin-4-yl-carbonylmethyl)-phenylamino]-1-(3,4-ethylendioxyphenyl)-methylen}-6-nitro-2-indolinon
(51) 3-(*Z*)-{1-[4-(N-Methyl-N-{2-dimethylamino-ethyl-carbonyl}-amino)-phenylamino]-1-(3,4-ethylendioxyphenyl)-methylen}-6-nitro-2-indolinon
(52) 3-(*Z*)-{1-[4-(1-Methyl-imidazol-2-yl)-phenylamino]-1-(3,4-ethylendioxyphenyl)-methylen}-6-nitro-2-indolinon
(53) 3-(*Z*)-{1-[4-(Dimethylaminomethyl)-phenylamino]-1-(2-thienyl)-methylen}-6-methoxycarbonyl-2-indolinon
(54) 3-(*Z*)-{1-[4-(Diethylaminomethyl)-phenylamino]-1-(2-thienyl)-methylen}-6-methoxycarbonyl-2-indolinon
(55) 3-(*Z*)-{1-[4-(1-Methylpiperazin-4-yl-methyl)-phenylamino]-1-(2-thienyl)-methylen}-6-methoxycarbonyl-2-indolinon
(56) 3-(*Z*)-{1-[4-(Ethylaminomethyl)-phenylamino]-1-(2-thienyl)-methylen}-6-methoxycarbonyl-2-indolinon
(57) 3-(*Z*)-{1-[4-(N-Methyl-N-{dimethylaminomethyl-carbonyl}-amino)-phenylamino]-1-(2-thienyl)-methylen}-6-methoxycarbonyl-2-indolinon
(58) 3-(*Z*)-{1-[4-(N-Methyl-N-{1-methylpiperazin-4-yl-methylcarbonyl}-amino)phenylamino]-1-(2-thienyl)-methylen}-6-methoxycarbonyl-2-indolinon
(59) 3-(*Z*)-{1-[4-(N-Acetyl-N-{2-dimethylamino-ethyl}-amino)-phenylamino]-1-(2-thienyl)-methylen}-6-methoxycarbonyl-2-indolinon
(60) 3-(*Z*)-{1-[4-(N-Acetyl-N-{3-dimethylamino-propyl}-amino)-phenylamino]-1-(2-thienyl)-methylen}-6-methoxycarbonyl-2-indolinon
(61) 3-(*Z*)-{1-[4-(N-Methyl-N-{2-dimethylamino-ethyl}-aminocarbonyl)-phenylamino]-1-(2-thienyl)-methylen}-6-methoxycarbonyl-2-indolinon
(62) 3-(*Z*)-{1-[4-(N-Methansulfonyl-N-{2-dimethylamino-ethyl}-amino)-phenylamino]-1-(2-thienyl)-methylen}-6-methoxycarbonyl-2-indolinon
(63) 3-(*Z*)-{1-[4-(1-Methylpiperazin-4-yl-carbonylmethyl)-phenylamino]-1-(2-thienyl)-methylen}-6-methoxycarbonyl-2-indolinon
(64) 3-(*Z*)-{1-[4-(N-Methyl-N-{2-dimethylamino-ethyl-carbonyl}-amino)-phenylamino]-1-(2-thienyl)-methylen}-6-methoxycarbonyl-2-indolinon
(65) 3-(*Z*)-{1-[4-(1-Methyl-imidazol-2-yl)-phenylamino]-1-(2-thienyl)-methylen}-6-methoxycarbonyl-2-indolinon
(66) 3-(*Z*)-{1-[4-(Dimethylaminomethyl)-phenylamino]-1-(3-thienyl)-methylen}-6-methoxycarbonyl-2-indolinon
(67) 3-(*Z*)-{1-[4-(Diethylaminomethyl)-phenylamino]-1-(3-thienyl)-methylen}-6-methoxycarbonyl-2-indolinon
(68) 3-(*Z*)-{1-[4-(1-Methylpiperazin-4-yl-methyl)-phenylamino]-1-(3-thienyl)-methylen}-6-methoxycarbonyl-2-indolinon
(69) 3-(*Z*)-{1-[4-(Ethylaminomethyl)-phenylamino]-1-(3-thienyl)-methylen}-6-methoxycarbonyl-2-indolinon
(70) 3-(*Z*)-{1-[4-(N-Methyl-N-{dimethylaminomethyl-carbonyl}-amino)-phenylamino]-1-(3-thienyl)-methylen}-6-methoxycarbonyl-2-indolinon
(71) 3-(*Z*)-{1-[4-(N-Methyl-N-{1-methylpiperazin-4-yl-methylcarbonyl}-amino)phenylamino]-1-(3-thienyl)-methylen}-6-methoxycarbonyl-2-indolinon
(72) 3-(*Z*)-{1-[4-(N-Acety)-N-{2-dimethylamino-ethyl}-amino)-phenylamino]-1-(3-thienyl)-methylen}-6-methoxycarbonyl-2-indolinon
(73) 3-(*Z*)-{1-[4-(N-Acetyl-N-(3-dimethylamino-propyl}-amino)-phenylamino]-1-(3-thienyl)-methylen}-6-methoxycarbonyl-2-indolinon
(74) 3-(*Z*)-{1-[4-(N-Methyl-N-{2-dimethylamino-ethyl}-aminocarbonyl)-phenylamino]-1-(3-thienyl)-methylen}-6-methoxycarbonyl-2-indolinon
(75) 3-(*Z*)-{1-[4-(N-Methansulfonyl-N-{2-dimethylamino-ethyl}-amino)-phenylamino]-1-(3-thienyl)-methylen}-6-methoxycarbonyl-2-indolinon
(76) 3-(*Z*)-{1-[4-(1-Methylpiperazin-4-yl-carbonylmethyl)-phenylamino]-1-(3thienyl)-methylen}-6-methoxycarbonyl-2-indolinon
(77) 3-(*Z*)-{1-[4-(N-Methyl-N-{2-dimethylamino-ethyl-carbonyl}-amino)-phenylamino]-1-(3-thienyl)-methylen}-6-methoxycarbonyl-2-indolinon
(78) 3-(*Z*)-{1-[4-(1-Methyl-imidazol-2-yl)-phenylamino]-1-(3-thienyl)-methylen}-6-methoxycarbonyl-2-indolinon
(79) 3-(*Z*)-{1-[4-(Dimethylaminomethyl)-phenylamino]-1-(3,4-methylendioxyphenyl)-methylen}-6-ethoxycarbonyl-2-indolinon
(80) 3-(*Z*)-{1-[4-(Diethylaminomethyl)-phenylamino]-1-(3,4-methylendioxyphenyl)-methylen}-6-ethoxycarbonyl-2-indolinon
(81) 3-(*Z*)-{1-[4-(1-Methylpiperazin-4-yl-methyl)-phenylamino]-1-(3,4-methylendioxyphenyl)-methylen}-6-ethoxycarbonyl-2-indolinon
(82) 3-(*Z*)-{1-[4-(Ethylaminomethyl)-phenylamino]-1-(3,4-methylendioxyphenyl)-methylen}-6-ethoxycarbonyl-2-indolinon
(83) 3-(*Z*)-{1-[4-(N-Methyl-N-{dimethylaminomethyl-carbonyl}-amino)-phenylamino]-1-(3,4-methylendioxyphenyl)-methylen}-6-ethoxycarbonyl-2-indolinon
(84) 3-(*Z*)-{1-[4-(N-Methyl-N-{1-methylpiperazin-4-yl-methylcarbonyl}-amino)-phenylamino]-1-(3,4-methylendioxyphenyl)-methylen}-6-ethoxycarbonyl-2-indolinon
(85) 3-(*Z*)-{1-[4-(N-Acetyl-N-{2-dimethylamino-ethyl}-amino)-phenylamino]-1-(3,4-methylendioxyphenyl)-methylenl-6-ethoxycarbonyl-2-indolinon
(86) 3-(*Z*)-{1-[4-(N-Acetyl-N-{3-dimethylamino-propyl}-amino)-phenylamino]-1-(3,4-methylendioxyphenyl)-methylen}-6-ethoxycarbonyl-2-indolinon
(87) 3-(*Z*)-{1-[4-(N-Methyl-N-{2-dimethylamino-ethyl}-aminocarbonyl)-phenyl-amino]-1-(3,4-methylendioxyphenyl)-methylen}-6-ethoxycarbonyl-2-indolinon
(88) 3-(*Z*)-{1-[4-(N-Methansulfonyl-N-{2-dimethylamino-ethyl}-amino)-phenylamino]-1-(3,4-methylendioxyphenyl)-methylen}-6-ethoxycarbonyl-2-indolinon
(89) 3-(*Z*)-{1-[4-(1-Methylpiperazin-4-yl-carbonylmethyl)-phenylamino]-1-(3,4-methylendioxyphenyl)-methylen}-6-ethoxycarbonyl-2-indolinon
(90) 3-(*Z*)-{1-[4-(N-Methyl-N-{2-dimethylamino-ethyl-carbonyl}-amino)-phenylamino]-1-(3,4-methylendioxyphenyl)-methylen}-6-ethoxycarbonyl-2-indolinon
(91) 3-(*Z*)-{1-[4-(1-Methyl-imidazol-2-yl)-phenylamino]-1-(3,4-methylendioxyphenyl)-methylen}-6-ethoxycarbonyl-2-indolinon
(92) 3-(*Z*)-{1-[4-(Dimethylaminomethyl)-phenylamino]-1-(3,4-ethylendioxy-phenyl)-methylen}-6-ethoxycarbonyl-2-indolinon
(93) 3-(*Z*)-{1-[4-(Diethylaminomethyl)-phenylamino]-1-(3,4-ethylendioxyphenyl)-methylen}-6-ethoxycarbonyl-2-indolinon
(94) 3-(*Z*)-{1-[4-(1-Methylpiperazin-4-yl-methyl)-phenylamino]-1-(3,4-ethylen-dioxyphenyl)-methylen}-6-ethoxycarbonyl-2-indolinon
(95) 3-(*Z*)-{1-[4-(Ethylaminomethyl)-phenylamino]-1-(3,4-ethylendioxyphenyl)-methylen}-6-ethoxycarbonyl-2-indolinon
(96) 3-(*Z*)-{1-[4-(N-Methyl-N-{dimethylaminomethyl-carbonyl}-amino)-phenylamino]-1-(3,4-ethylendioxyphenyl)-methylen}-6-ethoxycarbonyl-2-indolinon
(97) 3-(*Z*)-{1-[4-(N-Methyl-N-{1-methylpiperazin-4-yl-methylcarbonyl}-amino)-phenylamino]-1-(3,4-ethylendioxyphenyl)-methylen}-6-ethoxycarbonyl-2-indolinon
(98) 3-(*Z*)-{1-[4-(N-Acetyl-N-{2-dimethylamino-ethyl}-amino)-phenylamino]-1-(3,4-ethylendioxyphenyl)-methylen}-6-ethoxycarbonyl-2-indolinon
(99) 3-(*Z*)-{1-[4-(N-Acetyl-N-{3-dimethylamino-propyl}-amino)-phenylamino]-1-(3,4-ethylendioxyphenyl)-methylen}-6-ethoxycarbonyl-2-indolinon
(100) 3-(*Z*)-{1-[4-(N-Methyl-N-{2-dimethylamino-ethyl}-aminocarbonyl)-phenylamino]-1-(3,4-ethylendioxyphenyl)-methylen}-6-ethoxycarbonyl-2-indolinon
(101) 3-(*Z*)-{1-[4-(N-Methansulfonyl-N-{2-dimethylamino-ethyl}-amino)-phenylamino]-1-(3,4-ethylendioxyphenyl)-methylen}-6-ethoxycarbonyl-2-indolinon
(102) 3-(*Z*)-{1-[4-(1-Methylpiperazin-4-yl-carbonylmethyl)-phenylamino]-1-(3,4-ethylendioxyphenyl)-methylen}-6-ethoxycarbonyl-2-indolinon
(103) 3-(*Z*)-{1-[4-(N-Methy)-N-{2-dimethylamino-ethyl-carbonyl}-amino)-phenylamino]-1-(3,4-ethylendioxyphenyl)-methylen}-6-ethoxycarbonyl-2-indolinon
(104) 3-(*Z*)-{1-[4-(1-Methyl-imidazol-2-yl)-phenylamino]-1-(3,4-ethylendioxy-phenyl)-methylen}-6-ethoxycarbonyl-2-indolinon
(105) 3-(*Z*)-{1-[4-(Dimethylaminomethyl)-phenylamino]-1-(chinoxalin-6-yl)-methylen)-6-methoxycarbonyl-2-indolinon
(106) 3-(*Z*)-{1-[4-(Diethylaminomethyl)-phenylamino]-1-(chinoxalin-6-yl)-methylen}-6-methoxycarbonyl-2-indolinon
(107) 3-(*Z*)-{1-[4-(1-Methylpiperazin-4-yl-methyl)-phenylamino]-1-(chinoxalin-6-yl)-methylen}-6-methoxycarbonyl-2-indolinon
(108) 3-(*Z*)-{1-[4-(Ethylaminomethyl)-phenylamino]-1-(chinoxalin-6-yl)-methylen}-6-methoxycarbonyl-2-indolinon
(109) 3-(*Z*)-{1-[4-(N-Methyl-N-{dimethylaminomethyl-carbonyl}-amino)-phenylamino]-1-(chinoxalin-6-yl)-methylen}-6-methoxycarbonyl-2-indolinon
(110) 3-(*Z*)-{1-[4-(N-Methyl-N-{1-methylpiperazin-4-yl-methylcarbonyl}-amino)-phenylamino]-1-(chinoxalin-6-yl)-methylen}-6-methoxycarbonyl-2-indolinon
(111) 3-(*Z*)-{1-[4-(N-Acetyl-N-{2-dimethylamino-ethyl}-amino)-phenylamino]-1-(chinoxalin-6-yl)-methylen}-6-methoxycarbonyl-2-indolinon
(112) 3-(*Z*)-{1-[4-(N-Acetyl-N-{3-dimethylamino-propyl}-amino)-phenylamino]-1-(chinoxalin-6-yl)-methylen}-6-methoxycarbonyl-2-indolinon
(113) 3-(2)-{1-[4-(N-Methyl-N-{2-dimethylamino-ethyl}-aminocarbonyl)-phenylamino]-1-(chinoxalin-6-yl)-methylen}-6-methoxycarbonyl-2-indolinon
(114) 3-(*Z*)-{1-[4-(N-Methansulfonyl-N-{2-dimethylamino-ethyl}-amino)-phenylamino]-1-(chinoxalin-6-yl)-methylen}-6-methoxycarbonyl-2-indolinon
(115) 3-(*Z*)-{1-[4-(1-Methylpiperazin-4-yl-carbonylmethyl)-phenylamino]-1-(chinoxalin-6-yl)-methylen}-6-methoxycarbonyl-2-indolinon
(116) 3-(*Z*)-{1-[4-(N-Methyl-N-{2-dimethylamino-ethyl-carbonyl}-amino)-phenylamino]-1-(chinoxalin-6-yl)-methylen}-6-methoxycarbonyl-2-indolinon
(117) 3-(*Z*)-{1-[4-(1-Methyl-imidazol-2-yl)-phenylamino]-1-(chinoxalin-6-yl)-methylen}-6-methoxycarbonyl-2-indolinon
(118) 3-(*Z*)-{1-[4-(Dimethylaminomethyl)-phenylamino]-1-(chinoxalin-6-yl)-methylen}-6-fluor-2-indolinon
(119) 3-(*Z*)-{1-[4-(Diethylaminomethyl)-phenylamino]-1-(chinoxalin-6-yl)-methylen}-6-fluor-2-indolinon
(120) 3-(*Z*)-{1-[4-(1-Methylpiperazin-4-yl-methyl)-phenylamino]-1-(chinoxalin-6-yl)-methylen}-6-fluor-2-indolinon
(121) 3-(*Z*)-{1-[4-(Ethylaminomethyl)-phenylamino]-1-(chinoxalin-6-yl)-methylen}-6-fluor-2-indolinon
(122) 3-(*Z*)-{1-[4-(N-Methyl-N-{dimethylaminomethyl-carbonyl}-amino)-phenylamino]-1-(chinoxalin-6-yl)-methylen}-6-fluor-2-indolinon
(123) 3-(*Z*)-{1-[4-(N-Methyl-N-{1-methylpiperazin-4-yl-methylcarbonyl}-amino)-phenylamino]-1-(chinoxalin-6-yl)-methylen}-6-fluor-2-indolinon
(124) 3-(*Z*)-{1-[4-(N-Acetyl-N-{2-dimethylamino-ethyl}-amino)-phenylamino]-1-(chinoxalin-6-yl)-methylen}-6-fluor-2-indolinon
(125) 3-(*Z*)-{1-[4-(N-Acetyl-N-{3-dimethylamino-propyl}-amino)-phenylamino]-1-(chinoxalin-6-yl)-methylen}-6-fluor-2-indolinon
(126) 3-(*Z*)-{1-[4-(N-Methyl-N-{2-dimethylamino-ethyl}-aminocarbonyl)-phenylamino]-1-(chinoxalin-6-yl)-methylen}-6-fluor-2-indolinon
(127) 3-(*Z*)-{1-[4-(N-Methansulfonyl-N-{2-dimethylamino-ethyl}-amino)-phenyl-amino]-1-(chinoxalin-6-yl)-methylen}-6-fluor-2-indolinon
(128) 3-(*Z*)-{1-[4-(1-Methylpiperazin-4-yl-carbonylmethyl)-phenylamino]-1-(chinoxalin-6-yl)-methylen}-6-fluor-2-indolinon
(129) 3-(*Z*)-{1-[4-(N-Methyl-N-{2-dimethylamino-ethyl-carbonyl}-amino)-phenylamino]-1-(chinoxalin-6-yl)-methylen}-6-fluor-2-indolinon
(130) 3-(*Z*)-{1-[4-(1-Methyl-imidazol-2-yl)-phenylamino]-1-(chinoxalin-6-yl)-methylen}-6-fluor-2-indolinon
(131) 3-(*Z*)-{1-[4-(Diethylaminomethyl)-phenylamino]-1-(chinoxalin-6-yl)-methylen}-6-chlor-2-indolinon
(132) 3-(*Z*)-{1-[4-(1-Methylpiperazin-4-yl-methyl)-phenylamino]-1-(chinoxalin-6-yl)-methylen}-6-chlor-2-indolinon
(133) 3-(*Z*)-{1-[4-(Ethylaminomethyl)-phenylamino]-1-(chinoxalin-6-yl)-methylen}-6-chlor-2-indolinon
(134) 3-(*Z*)-{1-[4-(N-Methyl-N-{dimethylaminomethyl-carbonyl}-amino)-phenyl-amino]-1-(chinoxalin-6-yl)-methylen}-6-chlor-2-indolinon
(135) 3-(*Z*)-{1-[4-(N-Acetyl-N-{3-dimethylamino-propyl}-amino)-phenylamino]-1-(chinoxalin-6-yl)-methylen}-6-chlor-2-indolinon
(136) 3-(*Z*)-{1-[4-(N-Methyl-N-{2-dimethylamino-ethyl}-aminocarbonyl)-phenyl-amino]-1-(chinoxalin-6-yl)-methylen}-6-chlor-2-indolinon
(137) 3-(*Z*)-{1-[4-(1-Methylpiperazin-4-yl-carbonylmethyl)-phenylamino]-1-(chinoxalin-6-yl)-methylen}-6-chlor-2-indolinon
(138) 3-(*Z*)-{1-[4-(N-Methyl-N-{2-dimethylamino-ethyl-carbonyl}-amino)-phenylamino]-1-(chinoxalin-6-yl)-methylen}-6-chlor-2-indolinon
(139) 3-(*Z*)-{1-[4-(Dimethylaminomethyl)-phenylamino]-1-(benzo[1,4]dioxin-6-yl)-methylen}-6-methoxycarbonyl-2-indolinon
(140) 3-(*Z*)-{1-[4-(Diethylaminomethyl)-phenylamino]-1-(benzo[1,4]dioxin-6-yl)-methylen}-6-methoxycarbonyl-2-indolinon
(141) 3-(*Z*)-{1-[4-(1-Methylpiperazin-4-yl-methyl)-phenylamino]-1-(benzo[1,4]dioxin-6-yl)-methylen}-6-methoxycarbonyl-2-indolinon
(142) 3-(*Z*)-{1-[4-(Ethylaminomethyl)-phenylamino]-1-(benzo[1,4]dioxin-6-yl)-methylen}-6-methoxycarbonyl-2-indolinon
(143) 3-(*Z*)-{1-[4-(N-Methyl-N-{dimethylaminomethyl-carbonyl}-amino)-phenylamino]-1-(benzo[1,4]dioxin-6-yl)-methylen}-6-methoxycarbonyl-2-indolinon
(144) 3-(*Z*)-{1-[4-(N-Methyl-N-{1-methylpiperazin-4-yl-methylcarbonyl}-amino)-phenylamino]-1-(benzo[1,4]dioxin-6-yl)-methylen}-6-methoxycarbonyl-2-indolinon
(145) 3-(*Z*)-{1-[4-(N-Acetyl-N-{2-dimethylamino-ethyl}-amino)-phenylamino]-1-(benzo[1,4]dioxin-6-yl)-methylen}-6-methoxycarbonyl-2-indolinon
(146) 3-(*Z*)-{1-[4-(N-Acetyl-N-{3-dimethylamino-propyl}-amino)-phenylamino]-1-(benzo[1,4]dioxin-6-yl)-methylen}-6-methoxycarbonyl-2-indolinon
(147) 3-(*Z*)-{1-[4-(N-Methyl-N-{2-dimethylamino-ethyl}-aminocarbonyl)-phenylamino]-1-(benzo[1,4]dioxin-6-yl)-methylen}-6-methoxycarbonyl-2-indolinon
(148) 3-(*Z*)-{1-[4-(N-Methansulfonyl-N-{2-dimethylamino-ethyl}-amino)-phenylamino]-1-(benzo[1,4]dioxin-6-yl)-methylen}-6-methoxycarbonyl-2-indolinon
(149) 3-(*Z*)-{1-[4-(1-Methylpiperazin-4-yl-carbonylmethyl)-phenylamino]-1-(benzo[1,4]dioxin-6-yl)-methylen}-6-methoxycarbonyl-2-indolinon
(150) 3-(*Z*)-{1-[4-(N-Methyl-N-{2-dimethylamino-ethyl-carbonyl}-amino)-phenylamino]-1-(benzo[1,4]dioxin-6-yl)-methylen}-6-methoxycarbonyl-2-indolinon
(151) 3-(*Z*)-{1-[4-(1-Methyl-imidazol-2-yl)-phenylamino]-1-(benzo[1,4]dioxin-6-yl)-methylen}-6-methoxycarbonyl-2-indolinon
(152) 3-(*Z*)-{1-[4-(Dimethylaminomethyl)-phenylamino]-1-(benzo[1,4]dioxin-6-yl)-methylen}-6-fluor-2-indolinon
(153) 3-(*Z*)-{1-[4-(Diethylaminomethyl)-phenylamino]-1-(benzo[1,4]dioxin-6-yl)-methylen}-6-fluor-2-indolinon
(154) 3-(*Z*)-{1-[4-(1-Methylpiperazin-4-yl-methyl)-phenylamino]-1-(benzo[1,4]dioxin-6-yl)-methylen}-6-fluor-2-indolinon
(155) 3-(*Z*)-{1-[4-(Ethylaminomethyl)-phenylamino]-1-(benzo[1,4]dioxin-6-yl)-methylen}-6-fluor-2-indolinon
(156) 3-(*Z*)-{1-[4-(N-Methyl-N-{dimethylaminomethyl-carbonyl}-amino)-phenylamino]-1-(benzo[1,4]dioxin-6-yl)-methylen}-6-fluor-2-indolinon
(157) 3-(*Z*)-{1-[4-(N-Methyl-N-{1-methylpiperazin-4-yl-methylcarbonyl}-amino)-phenylamino]-1-(benzo[1,4]dioxin-6-yl)-methylen}-6-fluor-2-indolinon
(158) 3-(*Z*)-{1-[4-(N-Acetyl-N-{2-dimethylamino-ethyl}-amino)-phenylamino]-1-(benzo[1,4]dioxin-6-yl)-methylen}-6-fluor-2-indolinon
(159) 3-(*Z*)-{1-[4-(N-Acetyl-N-{3-dimethylamino-propyl}-amino)-phenylamino]-1-(benzo[1,4]dioxin-6-yl)-methylen}-6-fluor-2-indolinon
(160) 3-(*Z*)-{1-[4-(N-Methyl-N-{2-dimethylamino-ethyl}-aminocarbonyl)-phenylamino]-1-(benzo[1,4]dioxin-6-yl)-methylen}-6-fluor-2-indolinon
(161) 3-(*Z*)-{1-[4-(N-Methansulfonyl-N-{2-dimethylamino-ethyl}-amino)-phenylamino]-1-(benzo[1,4]dioxin-6-yl)-methylen}-6-fluor-2-indolinon
(162) 3-(*Z*)-{1-[4-(1-Methylpiperazin-4-yl-carbonylmethyl)-phenylamino]-1-(benzo[1,4]dioxin-6-yl)-methylen}-6-fluor-2-indolinon
(163) 3-(*Z*)-{1-[4-(N-Methy)-N-{2-dimethylamino-ethyl-carbonyl}-amino)-phenyl-amino]-1-(benzo[1,4]dioxin-6-yl)-methylen}-6-fluor-2-indolinon
(164) 3-(*Z*)-{1-[4-(1-Methyl-imidazol-2-yl)-phenylamino]-1-(benzo[1,4]dioxin-6-yl)-methylen}-6-fluor-2-indolinon
(165) 3-(*Z*)-{1-[4-(Dimethylaminomethyl)-phenylamino]-1-(benzo[1,4]dioxin-6-yl)-methylen}-6-chlor-2-indolinon
(166) 3-(*Z*)-{1-[4-(Diethylaminomethyl)-phenylamino]-1-(benzo[1,4]dioxin-6-yl)-methylen}-6-chlor-2-indolinon
(167) 3-(*Z*)-{1-[4-(1-Methylpiperazin-4-yl-methyl)-phenylamino]-1-(benzo[1,4]dioxin-6-yl)-methylen}-6-chlor-2-indolinon
(168) 3-(*Z*)-{1-[4-(Ethylaminomethyl)-phenylamino]-1-(benzo[1,4]dioxin-6-yl)-methylen}-6-chlor-2-indolinon
(169) 3-(*Z*)-{1-[4-(N-Methyl-N-{dimethylaminomethyl-carbonyl}-amino)-phenylamino]-1-(benzo[1,4]dioxin-6-yl)-methylen}-6-chlor-2-indolinon
(170) 3-(*Z*)-{1-[4-(N-Methyl-N-{1-methylpiperazin-4-yl-methylcarbonyl}-amino)-phenylamino]-1-(benzo[1,4]dioxin-6-yl)-methylen}-6-chlor-2-indolinon
(171) 3-(*Z*)-{1-[4-(N-Acetyl-N-{2-dimethylamino-ethyl}-amino)-phenylamino]-1-(benzo[1,4]dioxin-6-yl)-methylen}-6-chlor-2-indolinon
(172) 3-(*Z*)-{1-[4-(N-Acetyl-N-{3-dimethylamino-propyl}-amino)-phenylamino]-1-(benzo[1,4]dioxin-6-yl)-methylen}-6-chlor-2-indolinon
(173) 3-(*Z*)-{1-[4-(N-Methyl-N-{2-dimethylamino-ethyl}-aminocarbonyl)-phenylamino]-1-(benzo[1,4]dioxin-6-yl)-methylen}-6-chlor-2-indolinon
(174) 3-(*Z*)-{1-[4-(N-Methansulfonyl-N-{2-dimethylamino-ethyl}-amino)-phenylamino]-1-(benzo[1,4]dioxin-6-yl)-methylen}-6-chlor-2-indolinon
(175) 3-(*Z*)-{1-[4-(1-Methylpiperazin-4-yl-carbonylmethyl)-phenylamino]-1-(benzo[1,4]dioxin-8-yl)-methylen}-6-chlor-2-indolinon
(176) 3-(*Z*)-{1-[4-(N-Methyl-N-{2-dimethylamino-ethyl-carbonyl}-amino)-phenylamino]-1-(benzo[1,4]dioxin-6-yl)-methylen}-6-chlor-2-indolinon
(177) 3-(*Z*)-{1-[4-(1-Methyl-imidazol-2-yl)-phenylamino]-1-(benzo[1,4]dioxin-6-yl)-methylen}-6-chlor-2-indolinon
(178) 3-(*Z*)-{1-[4-(Dimethylaminomethyl)-phenylamino]-1-(3-methyl-benzoxazol-2-on-6-yl)-methylen}-6-chlor-2-indolinon
(179) 3-(*Z*)-{1-[4-(Diethylaminomethyl)-phenylamino]-1-(3-methyl-benzoxazol-2-on-6-yl)-methylen}-6-chlor-2-indolinon
(180) 3-(*Z*)-{1-[4-(1-Methylpiperazin-4-yl-methyl)-phenylamino]-1-(3-methyl-benzoxazol-2-on-6-yl)-methylen}-6-chlor-2-indolinon
(181) 3-(*Z*)-{1-[4-(Ethylaminomethyl)-phenylamino]-1-(3-methyl-benzoxazol-2-on-6-yl)-methylen}-6-chlor-2-indolinon
(182) 3-(*Z*)-{1-[4-(N-Methy)-N-{dimethylaminomethyl-carbonyl}-amino)-phenylamino]-1-(3-methyl-benzoxazol-2-on-6-yl)-methylen}-6-chlor-2-indolinon
(183) 3-(*Z*)-{1-[4-(N-Methyl-N-{1-methylpiperazin-4-yl-methylcarbonyl}-amino)-phenylamino]-1-(3-methyl-benzoxazol-2-on-6-yl)-methylen}-6-chlor-2-indolinon
(184) 3-(*Z*)-{1-[4-(N-Acetyl-N-{2-dimethylamino-ethyl}-amino)-phenylamino]-1-(3-methyl-benzoxazol-2-on-6-yl)-methylen}-6-chlor-2-indolinon
(184) 3-(*Z*)-{1-[4-(N-Acetyl-N-{3-dimethylamino-propyl}-amino)-phenylamino]-1-(3-methyl-benzoxazol-2-on-6-yl)-methylen}-6-chlor-2-indolinon
(185) 3-(*Z*)-{1-[4-(N-Methyl-N-{2-dimethylamino-ethyl}-aminocarbonyl)-phenylamino]-1-(3-methyl-benzoxazol-2-on-6-yl)-methylen}-6-chlor-2-indolinon
(186) 3-(*Z*)-{1-[4-(N-Methansulfonyl-N-{2-dimethylamino-ethyl}-amino)-phenylamino]-1-(3-methyl-benzoxazol-2-on-6-yl)-methylen}-6-chlor-2-indolinon
(187) 3-(*Z*)-{1-[4-(1-Methylpiperazin-4-yl-carbonylmethyl)-phenylamino]-1-(3-methyl-benzoxazol-2-on-6-yl)-methylen}-6-chlor-2-indolinon
(188) 3-(*Z*)-{1-[4-(N-Methyl-N-{2-dimethylamino-ethyl-carbonyl}-amino)-phenylamino]-1-(3-methyl-benzoxazol-2-on-6-yl)-methylen}-6-chlor-2-indolinon
(189) 3-(*Z*)-{1-[4-(1-Methyl-imidazol-2-yl)-phenylamino]-1-(3-methyl-benzoxazol-2-on-6-yl)-methylen}-6-chlor-2-indolinon
(190) 3-(*Z*)-{1-[4-(Dimethylaminomethyl)-phenylamino]-1-(benzoxazol-2-on-6-yl)-methylen}-6-chlor-2-indolinon
(191) 3-(*Z*)-{1-[4-(Diethylaminomethyl)-phenylamino]-1-(benzoxazol-2-on-6-yl)-methylen}-6-chlor-2-indolinon
(192) 3-(*Z*)-{1-[4-(1-Methylpiperazin-4-yl-methyl)-phenylamino]-1-(benzoxazol-2-on-6-yl)-methylen}-6-chlor-2-indolinon
(193) 3-(*Z*)-{1-[4-(Ethylaminomethyl)-phenylamino]-1-(benzoxazo1-2-on-6-yl)-methylen}-6-chlor-2-indolinon
(194) 3-(*Z*)-{1-[4-(N-Methyl-N-{dimethylaminomethyl-carbonyl}-amino)-phenyl-amino]-1-(benzoxazol-2-on-6-yl)-methylen}-6-chlor-2-indolinon
(195) 3-(*Z*)-{1-[4-(N-Methyl-N-{1-methylpiperazin-4-yl-methylcarbonyl}-amino)-phenylamino]-1-(benzoxazol-2-on-6-yl)-methylen}-6-chlor-2-indolinon
(196) 3-(*Z*)-{1-[4-(N-Acetyl-N-{2-dimethylamino-ethyl}-amino)-phenylamino]-1-(benzoxazol-2-on-6-yl)-methylen}-6-chlor-2-indolinon
(197) 3-(*Z*)-{1-[4-(N-Acetyl-N-{3-dimethylamino-propyl}-amino)-phenylamino]-1-(benzoxazol-2-on-6-yl)-methylen}-6-chlor-2-indolinon
(198) 3-(*Z*)-{1-[4-(N-Methyl-N-{2-dimethylamino-ethyl}-aminocarbonyl)-phenylamino]-1-(benzoxazol-2-on-6-yl)-methylen}-6-chlor-2-indolinon
(199) 3-(*Z*)-{1-[4-(N-Methansulfonyl-N-{2-dimethylamino-ethyl}-amino)-phenylamino]-1-(benzoxazol-2-on-6-yl)-methylen}-6-chlor-2-indolinon
(200) 3-(*Z*)-{1-[4-(1-Methylpiperazin-4-yl-carbonylmethyl)-phenylamino}-1-(benzoxazol-2-on-6-yl)-methylen}-6-chlor-2-indolinon
(201) 3-(*Z*)-{1-[4-(N-Methyl-N-{2-dimethylamino-ethyl-carbonyl}-amino)-phenyl-amino]-1-(benzoxazol-2-on-6-yl)-methylen}-6-chlor-2-indolinon
(202) 3-(*Z*)-{1-[4-(1-Methyl-imidazol-2-yl)-phenylamino]-1-(benzoxazol-2-on-6-yl)-methylen}-6-chlor-2-indolinon
(203) 3-(*Z*)-{1-[4-(Dimethylaminomethyl)-phenylamino]-1-(benzoxazol-2-on-5-yl)-methylen}-6-chlor-2-indolinon
(204) 3-(*Z*)-{1-[4-(Diethylaminomethyl)-phenylamino]-1-(benzoxazol-2-on-5-yl)-methylen}-6-chlor-2-indolinon
(205) 3-(*Z*)-{1-[4-(1-Methylpiperazin-4-yl-methyl)-phenylamino]-1-(benzoxazol-2-on-5-yl)-methylen}-6-chlor-2-indolinon
(206) 3-(*Z*)-{1-[4-(Ethylaminomethyl)-phenylamino]-1-(benzoxazol-2-on-5-yl)-methylen}-6-chlor-2-indolinon
(207) 3-(*Z*)-{1-[4-(N-Methyl-N-{dimethylaminomethyl-carbonyl}-amino)-phenylamino]-1-(benzoxazol-2-on-5-yl)-methylen}-6-chlor-2-indolinon
(208) 3-(*Z*)-{1-[4-(N-Methyl-N-{1-methylpiperazin-4-yl-methylcarbonyl}-amino)-phenylamino]-1-(benzoxazol-2-on-5-yl)-methylen}-6-chlor-2-indolinon
(209) 3-(*Z*)-{1-[4-(N-Acetyl-N-{2-dimethylamino-ethyl}-amino)-phenylamino]-1-(benzoxazol-2-on-5-yl)-methylen}-6-chlor-2-indolinon
(210) 3-(*Z*)-{1-[4-(N-Acetyl-N-{3-dimethylamino-propyl}-amino)-phenylamino]-1-(benzoxazol-2-on-5-yl)-methylen}-6-chlor-2-indolinon
(211) 3-(*Z*)-{1-[4-(N-Methyl-N-{2-dimethylamino-ethyl}-aminocarbonyl)-phenylamino]-1-(benzoxazol-2-on-5-yl)-methylen}-6-chlor-2-indolinon
(212) 3-(*Z*)-{1-[4-(N-Methansulfonyl-N-{2-dimethylamino-ethyl}-amino)-phenylamino]-1-(benzoxazol-2-on-5-yl)-methylen}-6-chlor-2-indolinon
(213) 3-(*Z*)-{1-[4-(1-Methylpiperazin-4-yl-carbonylmethyl)-phenylamino]-1-(benzoxazol-2-on-5-yl)-methylen}-6-chlor-2-indolinon
(214) 3-(*Z*)-{1-[4-(N-Methyl-N-{2-dimethylamino-ethyl-carbonyl}-amino)-phenyl-amino]-1-(benzoxazol-2-on-5-yl)-methylen}-6-chlor-2-indolinon
(215) 3-(*Z*)-{1-[4-(1-Methyl-imidazol-2-yl)-phenylamino]-1-(benzoxazol-2-on-5-yl)-methylen}-6-chlor-2-indolinon
(216) 3-(*Z*)-{1-[4-(Dimethylaminomethyl)-phenylamino]-1-(chinolin-7-yl)-methylen}-6-chlor-2-indolinon
(217) 3-(*Z*)-{1-[4-(Diethylaminomethyl)-phenylamino]-1-(chinolin-7-yl)-methylen}-6-chlor-2-indolinon
(218) 3-(*Z*)-{1-[4-(1-Methylpiperazin-4-yl-methyl)-phenylamino]-1-(chinolin-7-yl)-methylen}-6-chlor-2-indolinon
(219) 3-(*Z*)-{1-[4-(Ethylaminomethyl)-phenylamino]-1-(chinolin-7-yl)-methylen}-6-chlor-2-indolinon
(220) 3-(*Z*)-{1-[4-(N-Methyl-N-{dimethylaminomethyl-carbonyl}-amino)-phenylamino]-1-(chinolin-7-yl)-methylen}-6-chlor-2-indolinon
(221) 3-(*Z*)-{1-[4-(N-Methyl-N-{1-methylpiperazin-4-yl-methylcarbonyl}-amino)-phenylamino]-1-(chinolin-7-yl)-methylen}-6-chlor-2-indolinon
(222) 3-(*Z*)-{1-[4-(N-Acetyl-N-{2-dimethylamino-ethyl}-amino)-phenylamino]-1-(chinolin-7-yl)-methylen}-6-chlor-2-indolinon
(223) 3-(*Z*)-{1-[4-(N-Acetyl-N-{3-dimethylamino-propyl}-amino)-phenylamino]-1-(chinolin-7-yl)-methylen}-6-chlor-2-indolinon
(224) 3-(*Z*)-{1-[4-(N-Methyl-N-{2-dimethylamino-ethyl}-aminocarbonyl)-phenylamino]-1-(chinolin-7-yl)-methylen}-6-chlor-2-indolinon
(225) 3-(*Z*)-{1-[4-(N-Methansulfonyl-N-{2-dimethylamino-ethyl}-amino)-phenylamino]-1-(chinolin-7-yl)-methylen}-6-chlor-2-indolinon
(226) 3-(*Z*)-{1-[4-(1-Methylpiperazin-4-yl-carbonylmethyl)-phenylamino]-1-(chinolin-7-yl)-methylen}-6-chlor-2-indolinon
(227) 3-(*Z*)-{1-[4-(N-Methyl-N-{2-dimethylamino-ethyl-carbonyl}-amino)-phenylamino]-1-(chinolin-7-yl)-methylen}-6-chlor-2-indolinon
(228) 3-(*Z*)-{1-[4-(1-Methyl-imidazol-2-yl)-phenylamino]-1-(chinolin-7-yl)-methylen}-6-chlor-2-indolinon
(229) 3-(*Z*)-{1-[4-(Dimethylaminomethyl)-phenylamino]-1-(chinolin-6-yl)-methylen}-6-chlor-2-indolinon
(230) 3-(*Z*)-{1-[4-(Diethylaminomethyl)-phenylamino]-1-(chinolin-6-yl)-methylen}-6-chlor-2-indolinon
(231) 3-(*Z*)-{1-[4-(1-Methylpiperazin-4-yl-methyl)-phenylamino]-1-(chinolin-6-yl)-methylen}-6-chlor-2-indolinon
(232) 3-(*Z*)-{1-[4-(Ethylaminomethyl)-phenylamino]-1-(chinolin-6-yl)-methylen}-6-chlor-2-indolinon
(233) 3-(*Z*)-{1-[4-(N-Methyl-N-{dimethylaminomethyl-carbonyl}-amino)-phenylamino]-1-(chinolin-6-yl)-methylen}-6-chlor-2-indolinon
(234) 3-(*Z*)-{1-[4-(N-Methyl-N-{1-methylpiperazin-4-yl-methylcarbonyl}-amino)-phenylamino]-1-(chinolin-6-yl)-methylen}-6-chlor-2-indolinon
(235) 3-(*Z*)-{1-[4-(N-Acetyl-N-{2-dimethylamino-ethyl}-amino)-phenylamino]-1-(chinolin-6-yl)-methylen}-6-chlor-2-indolinon
(236) 3-(*Z*)-{1-[4-(N-Acetyl-N-{3-dimethylamino-propyl}-amino)-phenylamino]-1-(chinolin-6-yl)-methylen}-6-chlor-2-indolinon
(237) 3-(*Z*)-{1-[4-(N-Methyl-N-{2-dimethylamino-ethyl}-aminocarbonyl)-phenylamino]-1-(chinolin-6-yl)-methylen}-6-chlor-2-indolinon
(238) 3-(*Z*)-{1-[4-(N-Methansulfonyl-N-{2-dimethylamino-ethyl}-amino)-phenylamino]-1-(chinolin-6-yl)-methylen}-6-chlor-2-indolinon
(239) 3-(*Z*)-{1-[4-(1-Methylpiperazin-4-yl-carbonylmethyl)-phenylamino]-1-(chinolin-6-yl)-methylen}-6-chlor-2-indolinon
(240) 3-(*Z*)-{1-[4-(N-Methyl-N-{2-dimethylamino-ethyl-carbonyl}-amino)-phenylamino]-1-(chinolin-6-yl)-methylen}-6-chlor-2-indolinon
(241) 3-(*Z*)-{1-[4-(1-Methyl-imidazol-2-yl)-phenylamino]-1-(chinolin-6-yl)-methylen}-6-chlor-2-indolinon
(242) 3-(*Z*)-{1-[4-(Dimethylaminomethyl)-phenylamino]-1-(benzothiazol-5-yl)-methylen}-6-methoxycarbonyl-2-indolinon
(243) 3-(*Z*)-{1-[4-(Diethylaminomethyl)-phenylamino]-1-(benzothiazol-5-yl)-methylen}-6-methoxycarbonyl-2-indolinon
(244) 3-(*Z*)-{1-[4-(1-Methylpiperazin-4-yl-methyl)-phenylamino]-1-(benzothiazol-5-yl)-methylen}-6-methoxycarbonyl-2-indolinon
(245) 3-(*Z*)-{1-[4-(Ethylaminomethyl)-phenylamino]-1-(benzothiazol-5-yl)-methylen}-6-methoxycarbonyl-2-indolinon
(246) 3-(*Z*)-{1-[4-(N-Methyl-N-{dimethylaminomethyl-carbonyl}-amino)-phenylamino]-1-(benzothiazol-5-yl)-methylen}-6-methoxycarbonyl-2-indolinon
(247) 3-(*Z*)-{1-[4-(N-Methyl-N-{1-methylpiperazin-4-yl-methylcarbonyl}-amino)-phenylamino]-1-(benzothiazol-5-yl)-methylen}-6-methoxycarbonyl-2-indolinon
(248) 3-(*Z*)-{1-[4-(N-Acetyl-N-{2-dimethylamino-ethyl}-amino)-phenylamino]-1-(benzothiazol-5-yl)-methylen}-6-methoxycarbonyl-2-indolinon
(249) 3-(*Z*)-{1-[4-(N-Acetyl-N-{3-dimethylamino-propyl}-amino)-phenylamino]-1-(benzothiazol-5-yl)-methylen}-6-methoxycarbonyl-2-indolinon
(250) 3-(*Z*)-{1-[4-(N-Methyl-N-{2-dimethylamino-ethyl}-aminocarbonyl)-phenylamino]-1-(benzothiazol-5-yl)-methylen}-6-methoxycarbonyl-2-indolinon
(251) 3-(*Z*)-{1-[4-(N-Methansulfonyl-N-{2-dimethylamino-ethyl}-amino)-phenylamino]-1-(benzothiazol-5-yl)-methylen}-6-methoxycarbonyl-2-indolinon
(252) 3-(*Z*)-{1-[4-(1-Methylpiperazin-4-yl-carbonylmethyl)-phenylamino]-1-(benzo-thiazol-5-yl)-methylen}-6-methoxycarbonyl-2-indolinon
(253) 3-(*Z*)-{1-[4-(N-Methyl-N-{2-dimethylamino-ethyl-carbonyl}-amino)-phenylamino]-1-(benzothiazol-5-yl)-methylen}-6-methoxycarbonyl-2-indolinon
(254) 3-(*Z*)-{1-[4-(1-Methyl-imidazol-2-yl)-phenylamino]-1-(benzothiazol-5-yl)-methylen}-6-methoxycarbonyl-2-indolinon
(255) 3-(*Z*)-{1-[4-(Dimethylaminomethyl)-phenylamino]-1-(benzothiazol-6-yl)-methylen}-6-methoxycarbonyl-2-indolinon
(256) 3-(*Z*)-{1-[4-(Diethylaminomethyl)-phenylamino]-1-(benzothiazol-6-yl)-methylen}-6-methoxycarbonyl-2-indolinon
(257) 3-(*Z*)-{1-[4-(1-Methylpiperazin-4-yl-methyl)-phenylamino]-1-(benzothiazol-6-yl)-methylen}-6-methoxycarbonyl-2-indolinon
(258) 3-(*Z*)-{1-[4-(Ethylaminomethyl)-phenylamino]-1-(benzothiazol-6-yl)-methylen}-6-methoxycarbonyl-2-indolinon
(259) 3-(*Z*)-{1-[4-(N-Methyl-N-{dimethylaminomethyl-carbonyl}-amino)-phenyl-amino]-1-(benzothiazol-6-yl)-methylen}-6-methoxycarbonyl-2-indolinon
(260) 3-(*Z*)-{1-[4-(N-Methyl-N-{1-methylpiperazin-4-yl-methylcarbonyl}-amino)-phenylamino]-1-(benzothiazol-6-yl)-methylen}-6-methoxycarbonyl-2-indolinon
(261) 3-(*Z*)-{1-[4-(N-Acetyl-N-{2-dimethylamino-ethyl}-amino)-phenylamino]-1-(benzothiazol-6-yl)-methylen}-6-methoxycarbonyl-2-indolinon
(262) 3-(*Z*)-{1-[4-(N-Acetyl-N-{3-dimethylamino-propyl}-amino)-phenylamino]-1-(benzothiazol-6-yl)-methylenl-6-methoxycarbonyl-2-indolinon
(263) 3-(*Z*)-{1-[4-(N-Methyl-N-{2-dimethylamino-ethyl}-aminocarbonyl)-phenyl-amino]-1-(benzothiazol-6-yl)-methylen}-6-methoxycarbonyl-2-indolinon
(264) 3-(*Z*)-{1-[4-(N-Methansulfonyl-N-{2-dimethylamino-ethyl}-amino)-phenylamino]-1-(benzothiazol-6-yl)-methylen}-6-methoxycarbonyl-2-indolinon
(265) 3-(*Z*)-{1-[4-(1-Methylpiperazin-4-yl-carbonylmethyl)-phenylamino]-1-(benzo-thiazol-6-yl)-methylen}-6-methoxycarbonyl-2-indolinon
(266) 3-(*Z*)-{1-[4-(N-Methyl-N-{2-dimethylamino-ethyl-carbonyl}-amino)-phenylamino]-1-(benzothiazol-6-yl)-methylen}-6-methoxycarbonyl-2-indolinon
(267) 3-(*Z*)-{1-[4-(1-Methyl-imidazol-2-yl)-phenylamino]-1-(benzothiazol-6-yl)-methylen}-6-methoxycarbonyl-2-indolinon
(268) 3-(*Z*)-{1-[4-(Dimethylaminomethyl)-phenylamino]-1-(indol-5-yl)-methylen}-6-methoxycarbonyl-2-indolinon
(269) 3-(*Z*)-{1-[4-(Diethylaminomethyl)-phenylamino]-1-(indol-5-yl)-methylen}-6-methoxycarbonyl-2-indolinon
(270) 3-(*Z*)-{1-[4-(1-Methylpiperazin-4-yl-methyl)-phenylamino]-1-(indol-5-yl)-methylen}-6-methoxycarbonyl-2-indolinon
(271) 3-(*Z*)-{1-[4-(Ethylaminomethyl)-phenylamino]-1-(indol-5-yl)-methylen}-6-methoxycarbonyl-2-indolinon
(272) 3-(*Z*)-{1-[4-(N-Methyl-N-{dimethylaminomethyl-carbonyl}-amino)-phenylamino]-1-(indol-5-yl)-methylen}-6-methoxycarbonyl-2-indolinon
(273) 3-(*Z*)-{1-[4-(N-Methyl-N-{1-methylpiperazin-4-yl-methylcarbonyl}-amino)-phenylamino]-1-(indol-5-yl)-methylen}-6-methoxycarbonyl-2-indolinon
(274) 3-(*Z*)-{1-[4-(N-Acetyl-N-{2-dimethylamino-ethyl}-amino)-phenylamino]-1-(indol-5-yl)-methylen}-6-methoxycarbonyl-2-indolinon
(275) 3-(*Z*)-{1-[4-(N-Acetyl-N-{3-dimethylamino-propyl}-amino)-phenylamino]-1-(indol-5-yl)-methylen}-6-methoxycarbonyl-2-indolinon
(276) 3-(*Z*)-{1-[4-(N-Methyl-N-{2-dimethylamino-ethyl}-aminocarbonyl)-phenylamino]-1-(indol-5-yl)-methylen}-6-methoxycarbonyl-2-indolinon
(277) 3-(*Z*)-{1-[4-(N-Methansulfonyl-N-{2-dimethylamino-ethyl}-amino)-phenylamino]-1-(indol-5-yl)-methylen}-6-methoxycarbonyl-2-indolinon
(278) 3-(*Z*)-{1-[4-(1-Methylpiperazin-4-yl-carbonylmethyl)-phenylamino]-1-(indol-5-yl)-methylen}-6-methoxycarbonyl-2-indolinon
(279) 3-(*Z*)-{1-[4-(N-Methyl-N-{2-dimethylamino-ethyl-carbonyl}-amino)-phenylamino]-1-(indol-5-yl)-methylen}-6-methoxycarbonyl-2-indolinon
(280) 3-(*Z*)-{1-[4-(1-Methyl-imidazol-2-yl)-phenylamino]-1-(indol-5-yl)-methylen}-6-methoxycarbonyl-2-indolinon
(281) 3-(*Z*)-{1-[4-(Dimethylaminomethyl)-phenylamino]-1-(phthalazin-6-yl)-methylen}-6-methoxycarbonyl-2-indolinon
(282) 3-(*Z*)-{1-[4-(Diethylaminomethyl)-phenylamino]-1-(phthalazin-6-yl)-methylen}-6-methoxycarbonyl-2-indolinon
(283) 3-(*Z*)-{1-[4-(1-Methylpiperazin-4-yl-methyl)-phenylamino]-1-(phthalazin-6-yl)-methylen}-6-methoxycarbonyl-2-indolinon
(284) 3-(*Z*)-{1-[4-(Ethylaminomethyl)-phenylamino]-1-(phthalazin-6-yl)-methylen}-6-methoxycarbonyl-2-indolinon
(285) 3-(*Z*)-{1-[4-(N-Methyl-N-{dimethylaminomethyl-carbonyl}-amino)-phenylamino]-1-(phthalazin-6-yl)-methylen}-6-methoxycarbonyl-2-indolinon
(286) 3-(*Z*)-{1-[4-(N-Methyl-N-{1-methylpiperazin-4-yl-methylcarbonyl}-amino)-phenylamino]-1-(phthalazin-6-yl)-methylen}-6-methoxycarbonyl-2-indolinon
(287) 3-(*Z*)-{1-[4-(N-Acetyl-N-{2-dimethylamino-ethyl}-amino)-phenylamino]-1-(phthalazin-6-yl)-methylen}-6-methoxycarbonyl-2-indolinon
(288) 3-(*Z*)-{1-[4-(N-Acetyl-N-{3-dimethylamino-propyl}-amino)-phenylamino]-1-(phthalazin-6-yl)-methylen}-6-methoxycarbonyl-2-indolinon
(289) 3-(*Z*)-{1-[4-(N-Methyl-N-{2-dimethylamino-ethyl}-aminocarbonyl)-phenylamino]-1-(phthalazin-6-yl)-methylen}-6-methoxycarbonyl-2-indolinon
(290) 3-(*Z*)-{1-[4-(N-Methansulfonyl-N-{2-dimethylamino-ethyl}-amino)-phenylamino]-1-(phthalazin-6-yl)-methylen}-6-methoxycarbonyl-2-indolinon
(291) 3-(*Z*)-{1-[4-(1-Methylpiperazin-4-yl-carbonylmethyl)-phenylamino]-1-(phthalazin-6-yl)-methylen}-6-methoxycarbonyl-2-indolinon
(292) 3-(*Z*)-{1-[4-(N-Methyl-N-{2-dimethylamino-ethyl-carbonyl}-amino)-phenylamino]-1-(phthalazin-6-yl)-methylen}-6-methoxycarbonyl-2-indolinon
(293) 3-(*Z*)-{1-[4-(1-Methyl-imidazol-2-yl)-phenylamino]-1-(phthalazin-6-yl)-methylen}-6-methoxycarbonyl-2-indolinon
(294) 3-(*Z*)-{1-[4-(Dimethylaminomethyl)-phenylamino]-1-(4-methyl-3,4-dihydro-2*H*-benzo[1,4]oxazin-7-yl)-methylen}-6-chlor-2-indolinon
(295) 3-(*Z*)-{1-[4-(Diethylaminomethyl)-phenylamino]-1-(4-methyl-3,4-dihydro-2*H-*benzo[1,4]oxazin-7-yl)-methylen}-6-chlor-2-indolinon
(296) 3-(*Z*)-{1-[4-(1-Methylpiperazin-4-yl-methyl)-phenylamino]-1-(4-methyl-3,4-dihydro-2*H*-benzo[1,4]oxazin-7-yl)-methylen}-6-chlor-2-indolinon
(297) 3-(*Z*)-{1-[4-(Ethylaminomethyl)-phenylamino]-1-(4-methyl-3,4-dihydro-2*H-*benzo[1,4]oxazin-7-yl)-methylen}-6-chlor-2-indolinon
(298) 3-(*Z*)-{1-[4-(N-Methyl-N-{dimethylaminomethyl-carbonyl}-amino)-phenylamino]-1-(4-methyl-3,4-dihydro-2*H*-benzo[1,4]oxazin-7-yl)-methylen}-6-chlor-2-indolinon
(299) 3-(*Z*)-{1-[4-(N-Methyl-N-{1-methylpiperazin-4-yl-methylcarbonyl}-amino)-phenylamino]-1-(4-methyl-3,4-dihydro-2*H*-benzo[1,4]oxazin-7-yl)-methylen}-6-chlor-2-indolinon
(300) 3-(*Z*)-{1-[4-(N-Acetyl-N-{2-dimethylamino-ethyl}-amino)-phenylamino]-1-(4-methyl-3,4-dihydro-2*H*-benzo[1,4]oxazin-7-yl)-methylen}-6-chlor-2-indolinon
(301) 3-(*Z*)-{1-[4-(N-Acetyl-N-{3-dimethylamino-propyl}-amino)-phenylamino]-1-(4-methyl-3,4-dihydro-2*H*-benzo[1,4]oxazin-7-yl)-methylen}-6-chlor-2-indolinon
(302) 3-(*Z*)-{1-[4-(N-Methyl-N-{2-dimethylamino-ethyl}-aminocarbonyl)-phenylamino]-1-(4-methyl-3,4-dihydro-2*H*-benzo[1,4]oxazin-7-yl)-methylen}-6-chlor-2-indolinon
(303) 3-(*Z*)-{1-[4-(N-Methansulfonyl-N-{2-dimethylamino-ethyl}-amino)-phenyl-amino]-1-(4-methyl-3,4-dihydro-2*H*-benzo[1,4]oxazin-7-yl)-methylen}-6-chlor-2-indolinon
(304) 3-(*Z*)-{1-[4-(1-Methylpiperazin-4-yl-carbonylmethyl)-phenylamino]-1-(4-methyl-3,4-dihydro-2*H*-benzo[1,4]oxazin-7-yl)-methylen}-6-chlor-2-indolinon
(305) 3-(*Z*)-{1-[4-(N-Methyl-N-{2-dimethylamino-ethyl-carbonyl}-amino)-phenylamino]-1-(4-methyl-3,4-dihydro-2*H*-benzo[1,4]oxazin-7-yl)-methylen}-6-chlor-2-indolinon
(306) 3-(*Z*)-{1-[4-(1-Methyl-imidazol-2-yl)-phenylamino]-1-(4-methyl-3,4-dihydro-2*H*-benzo[1,4]oxazin-7-yl)-methylen}-6-chlor-2-indolinon
(307) 3-(*Z*)-{1-[4-(Dimethylaminomethyl)-phenylamino]-1-(4-pyridyl)-methylen}-6-methoxycarbonyl-2-indolinon
(308) 3-(*Z*)-{1-[4-(Diethylaminomethyl)-phenylamino]-1-(4-pyridyl)-methylen}-6-methoxycarbonyl-2-indolinon
(309) 3-(*Z*)-{1-[4-(1-Methylpiperazin-4-yl-methyl)-phenylamino]-1-(4-pyridyl)-methylen}-6-methoxycarbonyl-2-indolinon
(310) 3-(*Z*)-{1-[4-(Ethylaminomethyl)-phenylamino]-1-(4-pyridyl)-methylen}-6-methoxycarbonyl-2-indolinon
(311) 3-(*Z*)-{1-[4-(N-Methyl-N-{dimethylamimomethyl-carbonyl}-amino)-phenylamino]-1-(4-pyridyl)-methylen}-6-methoxycarbonyl-2-indolinon
(312) 3-(*Z*)-{1-[4-(N-Methyl-N-{1-methylpiperazin-4-yl-methylcarbonyl}-amino)-phenylamino]-1-(4-pyridyl)-methylen}-6-methoxycarbonyl-2-indolinon
(313) 3-(*Z*)-{1-[4-(N-Acetyl-N-{2-dimethylamino-ethyl}-amino)-phenylamino]-1-(4-pyridyl)-methylen}-6-methoxycarbonyl-2-indolinon
(314) 3-(*Z*)-{1-[4-(N-Acetyl-N-{3-dimethylamino-propyl}-amino)-phenylamino]-1-(4-pyridyl)-methylen}-6-methoxycarbonyl-2-indolinon
(315) 3-(*Z*)-{1-[4-(N-Methyl-N-{2-dimethylamino-ethyl}-aminocarbonyl)-phenylamino]-1-(4-pyridyl)-methylen}-6-methoxycarbonyl-2-indolinon
(316) 3-(*Z*)-{1-[4-(N-Methansulfonyl-N-{2-dimethylamino-ethyl}-amino)-phenylamino]-1-(4-pyridyl)-methylen}-6-methoxycarbonyl-2-indolinon
(317) 3-(*Z*)-{1-[4-(1-Methylpiperazin-4-yl-carbonylmethyl)-phenylamino]-1-(4-pyridyl)-methylen}-6-methoxycarbonyl-2-indolinon
(318) 3-(*Z*)-{1-[4-(N-Methyl-N-{2-dimethylamino-ethyl-carbonyl}-amino)-phenylamino]-1-(4-pyridyl)-methylen}-6-methoxycarbonyl-2-indolinon
(319) 3-(*Z*)-{1-[4-(1-Methyl-imidazol-2-yl)-phenylamino]-1-(4-pyridyl)-methylen}-6-methoxycarbonyl-2-indolinon
(320) 3-(*Z*)-{1-[4-(Dimethylaminomethyl)-phenylamino]-1-(3-pyridyl)-methylen}-6-methoxycarbonyl-2-indolinon
(321) 3-(*Z*)-{1-[4-(Diethylaminomethyl)-phenylamino]-1-(3-pyridyl)-methylen}-6-methoxycarbonyl-2-indolinon
(322) 3-(*Z*)-{1-[4-(1-Methylpiperazin-4-yl-methyl)-phenylamino]-1-(3-pyridyl)-methylen}-6-methoxycarbonyl-2-indolinon
(323) 3-(*Z*)-{1-[4-(Ethylaminomethyl)-phenylamino]-1-(3-pyridyl)-methylen}-6-methoxycarbonyl-2-indolinon
(324) 3-(*Z*)-{1-[4-(N-Methyl-N-{dimethylaminomethyl-carbonyl}-amino)-phenylamino]-1-(3-pyridyl)-methylen}-6-methoxycarbonyl-2-indolinon
(325) 3-(*Z*)-{1-[4-(N-Methyl-N-{1-methylpiperazin-4-yl-methylcarbonyl}-amino)-phenylamino]-1-(3-pyridyl)-methylen}-6-methoxycarbonyl-2-indolinon
(326) 3-(*Z*)-{1-[4-(N-Acetyl-N-{2-dimethylamino-ethyl}-amino)-phenylamino]-1-(3-pyridyl)-methylen}-6-methoxycarbonyl-2-indolinon
(327) 3-(*Z*)-{1-[4-(N-Acetyl-N-{3-dimethylamino-propyl}-amino)-phenylamino]-1-(3-pyridyl)-methylen}-6-methoxycarbonyl-2-indolinon
(328) 3-(*Z*)-{1-[4-(N-Methyl-N-{2-dimethylamino-ethyl}-aminocarbonyl)-phenylamino]-1-(3-pyridyl)-methylen}-6-methoxycarbonyl-2-indolinon
(329) 3-(*Z*)-{1-[4-(N-Methansulfonyl-N-{2-dimethylamino-ethyl}-amino)-phenylamino]-1-(3-pyridyl)-methylen}-6-methoxycarbonyl-2-indolinon
(330) 3-(*Z*)-{1-[4-(1-Methylpiperazin-4-yl-carbonylmethyl)-phenylamino]-1-(3-pyridyl)-methylen}-6-methoxycarbonyl-2-indolinon
(331) 3-(*Z*)-{1-[4-(N-Methyl-N-{2-dimethylamino-ethyl-carbonyl}-amino)-phenylamino]-1-(3-pyridyl)-methylen}-6-methoxycarbonyl-2-indolinon
(332) 3-(*Z*)-{1-[4-(1-Methyl-imidazol-2-yl)-phenylamino]-1-(3-pyridyl)-methylen}-6-methoxycarbonyl-2-indolinon
(333) 3-(*Z*)-{1-[3-Fluor-4-(N-methyl-N-{dimethylaminomethyl-carbonyl}-amino)-phenylamino]-1-(3,4-methylendioxyphenyl)-methylen}-6-chlor-2-indolinon
(334) 3-(*Z*)-{1-[3-Fluor-4-(N-methyl-N-{dimethylaminomethyl-carbonyl}-amino)-phenylamino]-1-(3,4-methylendioxyphenyl)-methylen}-6-methoxycarbonyl-2-indolinon
(335) 3-(*Z*)-{1-[3-Fluor-4-(N-methyl-N-{dimethylaminomethyl-carbonyl}-amino)-phenylamino]-1-(3,4-ethylendioxyphenyl)-methylen}-6-chlor-2-indolinon
(336) 3-(*Z*)-{1-[3-Fluor-4-(N-methyl-N-{dimethylaminomethyl-carbonyl}-amino)-phenylamino]-1-(3,4-ethylendioxyphenyl)-methylen}-6-methoxycarbonyl-2-indolinon
(337) 3-(*Z*)-{1-[3-Fluor-4-(N-methyl-N-{dimethylaminomethyl-carbonyl}-amino)-phenylamino]-1-(2-thienyl)-methylen}-6-chlor-2-indolinon
(338) 3-(*Z*)-{1-[3-Fluor-4-(N-methyl-N-{dimethylaminomethyl-carbonyl}-amino)-phenylamino]-1-(2-thienyl)-methylen}-6-methoxycarbonyl-2-indolinon
(339) 3-(*Z*)-{1-[3-Fluor-4-(N-methyl-N-{dimethylaminomethyl-carbonyl}-amino)-phenylamino]-1-(3-thienyl)-methylen}-6-chlor-2-indolinon
(340) 3-(*Z*)-{1-[3-Fluor-4-(N-methyl-N-{dimethylaminomethyl-carbonyl}-amino)-phenylamino]-1-(3-thienyl)-methylen}-6-methoxycarbonyl-2-indolinon
(341) 3-(*Z*)-{1-[3-Fluor-4-(N-methyl-N-{dimethylaminomethyl-carbonyl}-amino)-phenylamino]-1-(chinoxalin-6-yl)-methylen}-6-chlor-2-indolinon
(342) 3-(*Z*)-{1-[3-Fluor-4-(N-methyl-N-{dimethylaminomethyl-carbonyl}-amino)-phenylamino]-1-(chinoxalin-6-yl)-methylen}-6-methoxycarbonyl-2-indolinon
(343) 3-(*Z*)-{1-[3-Fluor-4-(N-methansulfonyl-N-{2-dimethylamino-ethyl}-amino)-phenylamino]-1-(3,4-methylendioxyphenyl)-methylen}-6-chlor-2-indolinon
(344) 3-(Z)-{1-[3-Fluor-4-(N-methansulfonyl-N-{2-dimethylamino-ethyl}-amino)-phenylamino]-1-(3,4-methylendioxyphenyl)-methylen}-6-methoxycarbonyl-2-indolinon
(345) 3-(*Z*)-{1-[3-Fluor-4-(N-methansulfonyl-N-{2-dimethylamino-ethyl}-amino)-phenylamino]-1-(3,4-ethylendioxyphenyl)-methylen}-6-chlor-2-indolinon
(346) 3-(*Z*)-{1-[3-Fluor-4-(N-methansulfonyl-N-{2-dimethylamino-ethyl}-amino)-phenylamino]-1-(3,4-ethylendioxyphenyl)-methylen}-6-methoxycarbonyl-2-indolinon
(347) 3-(*Z*)-{1-[3-Fluor-4-(N-methansulfonyl-N-{2-dimethylamino-ethyl}-amino)-phenylamino]-1-(2-thienyl)-methylen}-6-chlor-2-indolinon
(348) 3-(*Z*)-{1-[3-Fluor-4-(N-methansulfonyl-N-{2-dimethylamino-ethyl}-amino)-phenylamino]-1-(2-thienyl)-methylen}-6-methoxycarbonyl-2-indolinon
(349) 3-(*Z*)-{1-[3-Fluor-4-(N-methansulfonyl-N-{2-dimethylamino-ethyl}-amino)-phenylamino]-1-(3-thienyl)-methylen}-6-chlor-2-indolinon
(350) 3-(*Z*)-{1-[3-Fluor-4-(N-methansulfonyl-N-{2-dimethylamino-ethyl}-amino)-phenylamino]-1-(3-thienyl)-methylen}-6-methoxycarbonyl-2-indolinon
(351) 3-(*Z*)-{1-[3-Fluor-4-(N-methansulfonyl-N-{2-dimethylamino-ethyl}-amino)-phenylamino]-1-(chinoxalin-6-yl)-methylen}-6-chlor-2-indolinon
(352) 3-(*Z*)-{1-[3-Fluor-4-(N-methansulfonyl-N-{2-dimethylamino-ethyl}-amino)-phenylamino]-1-(chinoxalin-6-yl)-methylen}-6-methoxycarbonyl-2-indolinon
(353) 3-(*Z*)-{1-[1-Methyl-2-(N-{2-dimethylamino-ethyl}-N-methyl-aminocarbonyl)-pyrrol-4-yl-amino]-1-(3,4-methylendioxyphenyl)-methylen}-6-chlor-2-indolinon
(354) 3-(*Z*)-{1-[1-Methyl-2-(N-{2-dimethylamino-ethyl}-N-methyl-aminocarbonyl)-pyrrol-4-yl-amino]-1-(3,4-methylendioxyphenyl)-methylen}-6-methoxycarbonyl-2-indolinon
(355) 3-(*Z*)-{1-[1-Methyl-2-(N-{2-dimethylamino-ethyl}-N-methyl-aminocarbonyl)-pyrrol-4-yl-amino]-1-(3,4-ethylendioxyphenyl)-methylen}-6-chlor-2-indolinon
(356) 3-(*Z*)-{1-[1-Methyl-2-(N-{2-dimethylamino-ethyl}-N-methyl-aminocarbonyl)-pyrrol-4-yl-amino]-1-(3,4-ethylendioxyphenyl)-methylen}-6-methoxycarbonyl-2-indolinon
(357) 3-(*Z*)-{1-[1-Methyl-2-(N-{2-dimethylamino-ethyl}-N-methyl-aminocarbonyl)-pyrrol-4-yl-amino]-1-(2-thienyl)-methylen}-6-chlor-2-indolinon
(358) 3-(*Z*)-{1-[1-Methyl-2-(N-{2-dimethylamino-ethyl}-N-methyl-aminocarbonyl)-pyrrol-4-yl-amino]-1-(2-thienyl)-methylen}-6-methoxycarbonyl-2-indolinon
(359) 3-(*Z*)-{1-[1-Methyl-2-(N-{2-dimethylamino-ethyl}-N-methyl-aminocarbonyl)-pyrrol-4-yl-amino]-1-(3-thienyl)-methylen}-6-chlor-2-indolinon
(360) 3-(*Z*)-{1-[1-Methyl-2-(N-{2-dimethylamino-ethyl}-N-methyl-aminocarbonyl)-pyrrol-4-y1-amino]-1-(3-thienyl)-methylen}-6-methoxycarbonyl-2-indolinon
(361) 3-(*Z*)-{1-[1-Methyl-2-(N-{2-dimethylamino-ethyl}-N-methyl-aminocarbonyl)-pyrrol-4-yl-amino]-1-(chinoxalin-6-yl)-methylen}-6-chlor-2-indolinon
(362) 3-(*Z*)-{1-[1-Methyl-2-(N-{2-dimethylamino-ethyl}-N-methyl-aminocarbonyl)-pyrrol-4-yl-amino]-1-(chinoxalin-6-yl)-methylen}-6-methoxycarbonyl-2-indolinon
(363) 3-(*Z*)-{1-[4-(Dimethylaminomethyl)-phenylamino]-1-(3,4-methylendioxy-phenyl)-methylen}-6-fluor-2-indolinon
(364) 3-(*Z*)-{1-[4-(Diethylaminomethyl)-phenylamino]-1-(3,4-methylendioxyphenyl)-methylen}-6-fluor-2-indolinon
(365) 3-(*Z*)-{1-[4-(1-Methylpiperazin-4-yl-methyl)-phenylamino]-1-(3,4-methylendioxyphenyl)-methylen}-6-fluor-2-indolinon
(366) 3-(*Z*)-{1-[4-(Ethylaminomethyl)-phenylamino]-1-(3,4-methylendioxyphenyl)-methylen}-6-fluor-2-indolinon
(367) 3-(*Z*)-{1-[4-(N-Methyl-N-{dimethylaminomethyl-carbonyl}-amino)-phenylamino]-1-(3,4-methylendioxyphenyl)-methylen}-6-fluor-2-indolinon
(368) 3-(*Z*)-{1-[4-(N-Methyl-N-{1-ethylpiperazin-4-yl-methylcarbonyl}-amino)-phenylamino]-1-(3,4-methylendioxyphenyl)-methylen}-6-chlor-2-indolinon
(369) 3-(*Z*)-{1-[4-(N-Acetyl-N-{2-dimethylamino-ethyl}-amino)-phenylamino]-1-(3,4-methylendioxyphenyl)-methylen}-6-fluor-2-indolinon
(370) 3-(*Z*)-{1-[4-(N-Acetyl-N-{3-dimethylamino-propyl}-amino)-phenylamino]-1-(3,4-methylendioxyphenyl)-methylen}-6-fluor-2-indolinon
(371) 3-(*Z*)-{1-[4-(N-Methyl-N-{2-dimethylamino-ethyl}-aminocarbonyl)-phenylamino]-1-(3,4-methylendioxyphenyl)-methylen}-6-fluor-2-indolinon
(372) 3-(*Z*)-{1-[4-(N-Methansulfonyl-N-{2-dimethylamino-ethyl}-amino)-phenylamino]-1-(3,4-methylendioxyphenyl)-methylen}-6-fluor-2-indolinon
(373) 3-(*Z*)-{1-[4-(1-Methylpiperazin-4-yl-carbonylmethyl)-phenylamino]-1-(3,4-methylendioxyphenyl)-methylen}-6-fluor-2-indolinon
(374) 3-(*Z*)-{1-[4-(N-Methyl-N-{2-dimethylamino-ethyl-carbonyl}-amino)-phenylamino]-1-(3,4-methylendioxyphenyl)-methylen}-6-fluor-2-indolinon
(375) 3-(*Z*)-{1-[4-(1-Methyl-imidazol-2-yl)-phenylamino]-1-(3,4-methylendioxyphenyl)-methylen}-6-fluor-2-indolinon
(376) 3-(*Z*)-{1-[4-(Dimethylaminomethyl)-phenylamino]-1-(3,4-methylendioxyphenyl)-methylen}-6-brom-2-indolinon
(377) 3-(*Z*)-{1-[4-(Diethylaminomethyl)-phenylamino]-1-(3,4-methylendioxyphenyl)-methylen}-6-brom-2-indolinon
(378) 3-(*Z*)-{1-[4-(1-Methylpiperazin-4-yl-methyl)-phenylamino]-1-(3,4-methylendioxyphenyl)-methylen}-6-brom-2-indolinon
(379) 3-(*Z*)-{1-[4-(Ethylaminomethyl)-phenylamino]-1-(3,4-methylendioxyphenyl)-methylen}-6-brom-2-indolinon
(380) 3-(*Z*)-{1-[4-(N-Methyl-N-{dimethylaminomethyl-carbonyl}-amino)-phenylamino]-1-(3,4-methylendioxyphenyl)-methylen}-6-brom-2-indolinon
(381) 3-(*Z*)-{1-[4-(N-Ethyl-N-{1-methylpiperazin-4-yl-methylcarbonyl}-amino)-phenylamino]-1-(3,4-methylendioxyphenyl)-methylen}-6-chlor-2-indolinon
(382) 3-(*Z*)-{1-[4-(N-Acetyl-N-{2-dimethylamino-ethyl}-amino)-phenylamino]-1-(3,4-methylendioxyphenyl)-methylen}-6-brom-2-indolinon
(383) 3-(*Z*)-{1-[4-(N-Acetyl-N-{3-dimethylamino-propyl}-amino)-phenylamino]-1-(3,4-methylendioxyphenyl)-methylen}-6-brom-2-indolinon
(384) 3-(*Z*)-{1-[4-(N-Methyl-N-{2-dimethylamino-ethyl}-aminocarbonyl)-phenylamino]-1-(3,4-methylendioxyphenyl)-methylen}-6-brom-2-indolinon
(385) 3-(*Z*)-{1-[4-(N-Methansulfonyl-N-{2-dimethylamino-ethyl}-amino)-phenylamino]-1-(3,4-methylendioxyphenyl)-methylen}-6-brom-2-indolinon
(386) 3-(*Z*)-{1-[4-(1-Methylpiperazin-4-yl-carbonylmethyl)-phenylamino]-1-(3,4-methylendioxyphenyl)-methylen}-6-brom-2-indolinon
(387) 3-(*Z*)-{1-[4-(N-Methyl-N-{2-dimethylamino-ethyl-carbonyl}-amino)-phenylamino]-1-(3,4-methylendioxyphenyl)-methylen}-6-brom-2-indolinon
(388) 3-(*Z*)-{1-[4-(1-Methyl-imidazol-2-yl)-phenylamino]-1-(3,4-methylendioxyphenyl)-methylen}-6-brom-2-indolinon
(389) 3-(*Z*)-{1-[4-(Dimethylaminomethyl)-phenylamino]-1-(2-[2-carboxyethyl]-thien-5-yl)-methylen}-6-fluor-2-indolinon
(390) 3-(*Z*)-{1-[4-(Dimethylaminomethyl)-phenylamino]-1-(3-[2-carboxyethyl]-thien-5-yl)-methylen}-6-fluor-2-indolinon
(391) 3-(*Z*)-{1-[4-(Dimethylaminomethyl)-phenylamino]-1-(2-[2-carboxyethyl]-thien-4-yl)-methylen}-6-fluor-2-indolinon
(392) 3-(*Z*)-{1-[4-(Dimethylaminomethyl)-phenylamino]-1-(2-[2-carboxyethyl]-pyridin-4-yl)-methylen}-6-fluor-2-indolinon
(393) 3-(*Z*)-{1-[4-(Dimethylaminomethyl)-phenylamino]-1-(3-[2-carboxyethyl]-pyridin-5-yl)-methylen}-6-fluor-2-indolinon
(394) 3-(*Z*)-{1-[4-(Dimethylaminomethyl)-phenylamino]-1-(2-[2-carboxyethyl]-pyridin-5-yl)-methylen}-6-fluor-2-indolinon
(395) 3-(*Z*)-{1-[4-(Dimethylaminomethyl)-phenylamino]-1-(2-[2-carboxyethyl]-thien-5-yl)-methylen}-8-chlor-2-indolinon
(396) 3-(*Z*)-{1-[4-(Dimethylaminomethyl)-phenylamino]-1-(3-[2-carboxyethyl]-thien-5-yl)-methylen}-8-chlor-2-indolinon
(397) 3-(*Z*)-{1-[4-(Dimethylaminomethyl)-phenylamino]-1-(2-[2-carboxyethyl]-thien-4-yl)-methylen}-6-chlor-2-indolinon
(398) 3-(*Z*)-{1-[4-(Dimethylaminomethyl)-phenylamino]-1-(2-[2-carboxyethyl]-pyridin-4-yl)-methylen}-6-chlor-2-indolinon
(399) 3-(*Z*)-{1-[4-(Dimethylaminomethyl)-phenylamino]-1-(3-[2-carboxyethyl]-pyridin-5-yl)-methylen}-6-chlor-2-indolinon
(400) 3-(*Z*)-{1-[4-(Dimethylaminomethyl)-phenylamino]-1-(2-[2-carboxyethyl]-pyridin-5-yl)-methylen}-6-chlor-2-indolinon
(401) 3-(*Z*)-{1-[4-(Dimethylaminomethyl)-phenylamino]-1-(2-[2-carboxyethyl]-thien-5-yl)-methylen}-8-cyano-2-indolinon
(402) 3-(*Z*)-{1-[4-(Dimethylaminomethyl)-phenylamino]-1-(3-[2-carboxyethyl]-thien-5-yl)-methylen}-6-cyano-2-indolinon
(403) 3-(*Z*)-{1-[4-(Dimethylaminomethyl)-phenylamino]-1-(2-[2-carboxyethyl]-thien-4-yl)-methylen}-6-cyano-2-indolinon
(404) 3-(*Z*)-{1-[4-(Dimethylaminomethyl)-phenylamino]-1-(2-[2-carboxyethyl]-pyridin-4-yl)-methylen}-6-cyano-2-indolinon
(405) 3-(*Z*)-{1-[4-(Dimethylaminomethyl)-phenylamino]-1-(3-[2-carboxyethyl]-pyridin-5-yl)-methylen}-6-cyano-2-indolinon
(406) 3-(*Z*)-{1-[4-(Dimethylaminomethyl)-phenylamino]-1-(2-[2-carboxyethyl]-pyridin-5-yl)-methylen}-6-cyano-2-indolinon
(407) 3-(*Z*)-{1-[4-(Dimethylaminomethyl)-phenylamino]-1-(2-[2-carboxyethyl]-thien-5-yl)-methylen}-6-methoxycarbonyl-2-indolinon
(408) 3-(*Z*)-{1-[4-(Dimethylaminomethyl)-phenylamino]-1-(3-[2-carboxyethyl]-thien-5-yl)-methylen}-6-methoxycarbonyl-2-indolinon
(409) 3-(*Z*)-{1-[4-(Dimethylaminomethyl)-phenylamino]-1-(2-[2-carboxyethyl]-thien-4-yl)-methylen}-6-methoxycarbonyl-2-indolinon
(410) 3-(*Z*)-{1-[4-(Dimethylaminomethyl)-phenylamino]-1-(2-[2-carboxyethyl]-pyridin-4-yl)-methylen}-6-methoxycarbonyl-2-indolinon
(411) 3-(*Z*)-{1-[4-(Dimethylaminomethyl)-phenylamino]-1-(3-[2-carboxyethyl]-pyridin-5-yl)-methylen}-6-methoxycarbonyl-2-indolinon
(412) 3-(*Z*)-{1-[4-(Dimethylaminomethyl)-phenylamino]-1-(2-[2-carboxyethyl]-pyridin-5-yl)-methylen}-6-methoxycarbonyl-2-indolinon
(413) 3-(*Z*)-{1-[4-(Dimethylaminomethyl)-phenylamino]-1-(2,3-dihydrobenzofuran-6-yl)-methylen}-6-fluor-2-indolinon
(414) 3-(*Z*)-{1-[4-(N-Methyl-N-{1-methylpiperazin-4-yl-methylcarbonyl}-amino)-phenylamino]-1-(2,3-dihydrobenzofuran-6-yl)-methylen}-6-fluor-2-indolinon
(415) 3-(*Z*)-{1-[4-(N-Methansulfonyl-N-{2-dimethylamino-ethyl}-amino)-phenylamino]-1-(2,3-dihydrobenzofuran-6-yl)-methylen}-6-fluor-2-indolinon
(416) 3-(*Z*)-{1-[4-(Dimethylaminomethyl)-phenylamino]-1-(2,3-dihydrobenzofuran-5-yl)-methylen}-6-fluor-2-indolinon
(417) 3-(*Z*)-{1-[4-(N-Methyl-N-{1-methylpiperazin-4-yl-methylcarbonyl}-amino)-phenylamino]-1-(2,3-dihydrobenzofuran-5-yl)-methylen}-6-fluor-2-indolinon
(418) 3-(*Z*)-{1-[4-(N-Methansulfonyl-N-{2-dimethylamino-ethyl}-amino)-phenylamino]-1-(2,3-dihydrobenzofuran-5-yl)-methylen}-6-fluor-2-indolinon
(419) 3-(*Z*)-{1-[4-(Dimethylaminomethyl)-phenylamino]-1-(imidazo[1,2-a]pyridin-7-yl)-methylen}-6-fluor-2-indolinon
(420) 3-(*Z*)-{1-[4-(N-Methyl-N-{1-methylpiperazin-4-yl-methylcarbonyl}-amino)-phenylamino]-1-(imidazo[1,2-a]pyridin-7-yl)-methylen}-6-fluor-2-indolinon
(421) 3-(*Z*)-{1-[4-(N-Methansulfonyl-N-{2-dimethylamino-ethyl}-amino)-phenylamino]-1-(imidazo[1,2-a]pyridin-7-yl)-methylen}-6-fluor-2-indolinon
(422) 3-(*Z*)-{1-[4-(Dimethylaminomethyl)-phenylamino]-1-(imidazo[1,2-a]pyridin-6-yl)-methylen}-6-fluor-2-indolinon
(423) 3-(*Z*)-{1-[4-(N-Methyl-N-{1-methylpiperazin-4-yl-methylcarbonyl}-amino)-phenylamino]-1-(imidazo[1,2-a]pyridin-6-yl)-methylen}-6-fluor-2-indolinon
(424) 3-(*Z*)-{1-[4-(N-Methansulfonyl-N-{2-dimethylamino-ethyl}-amino)-phenylamino]-1-(imidazo[1,2-a]pyridin-6-yl)-methylen}-6-fluor-2-indolinon
(425) 3-(*Z*)-{1-[4-(Dimethylaminomethyl)-phenylamino]-1-(2,3-dihydrobenzofuran-6-yl)-methylen}-6-chlor-2-indolinon
(426) 3-(*Z*)-{1-[4-(N-Methyl-N-{1-methylpiperazin-4-yl-methylcarbonyl}-amino)-phenylamino]-1-(2,3-dihydrobenzofuran-6-yl)-methylen}-6-chlor-2-indolinon
(427) 3-(*Z*)-{1-[4-(N-Methansulfonyl-N-{2-dimethylamino-ethyl}-amino)-phenylamino]-1-(2,3-dihydrobenzofuran-6-yl)-methylen}-6-chlor-2-indolinon
(428) 3-(*Z*)-{1-[4-(Dimethylaminomethyl)-phenylamino]-1-(2,3-dihydrobenzofuran-5-yl)-methylen}-6-chlor-2-indolinon
(429) 3-(*Z*)-{1-[4-(N-Methyl-N-{1-methylpiperazin-4-yl-methylcarbonyl}-amino)-phenylamino]-1-(2,3-dihydrobenzofuran-5-yl)-methylen}-6-chlor-2-indolinon
(430) 3-(*Z*)-{1-[4-(N-Methansulfonyl-N-{2-dimethylamino-ethyl}-amino)-phenylamino]-1-(2,3-dihydrobenzofuran-5-yl)-methylen}-6-chlor-2-indolinon
(431) 3-(*Z*)-{1-[4-(Dimethylaminomethyl)-phenylamino]-1-(imidazo[1,2-a]pyridin-7-yl)-methylen}-6-chlor-2-indolinon
(432) 3-(*Z*)-{1-[4-(N-Methyl-N-{1-methylpiperazin-4-yl-methylcarbonyl}-amino)-phenylamino]-1-(imidazo[1,2-a]pyridin-7-yl)-methylen}-6-chlor-2-indolinon
(433) 3-(*Z*)-{1-[4-(N-Methansulfonyl-N-{2-dimethylamino-ethyl}-amino)-phenylamino]-1-(imidazo[1,2-a]pyridin-7-yl)-methylen}-6-chlor-2-indolinon
(434) 3-(*Z*)-{1-[4-(Dimethylaminomethyl)-phenylamino]-1-(imidazo[1,2-a]pyridin-6-yl)-methylen}-6-chlor-2-indolinon
(435) 3-(*Z*)-{1-[4-(N-Methyl-N-{1-methylpiperazin-4-yl-methylcarbonyl}-amino)-phenylamino]-1-(imidazo[1,2-a]pyridin-6-yl)-methylen}-6-chlor-2-indolinon
(436) 3-(*Z*)-{1-[4-(N-Methansulfonyl-N-{2-dimethylamino-ethyl}-amino)-phenylamino]-1-(imidazo[1,2-a]pyridin-6-yl)-methylen}-6-chlor-2-indolinon
(437) 3-(*Z*)-{1-[4-(Dimethylaminomethyl)-phenylamino]-1-(2,3-dihydrobenzofuran-6-yl)-methylen}-6-cyano-2-indolinon
(438) 3-(*Z*)-{1-[4-(N-Methyl-N-{1-methylpiperazin-4-yl-methylcarbonyl}-amino)-phenylamino]-1-(2,3-dihydrobenzofuran-6-yl)-methylen}-6-cyano-2-indolinon
(439) 3-(*Z*)-{1-[4-(N-Methansulfonyl-N-{2-dimethylamino-ethyl}-amino)-phenylamino]-1-(2,3-dihydrobenzofuran-6-yl)-methylen}-6-cyano-2-indolinon
(440) 3-(*Z*)-{1-[4-(Dimethylaminomethyl)-phenylamino]-1-(2,3-dihydrobenzofuran-5-yl)-methylen}-6-cyano-2-indolinon
(441) 3-(*Z*)-{1-[4-(N-Methyl-N-{1-methylpiperazin-4-yl-methylcarbonyl}-amino)-phenylamino]-1-(2,3-dihydrobenzofuran-5-yl)-methylen}-6-cyano-2-indolinon
(442) 3-(*Z*)-{1-[4-(N-Methansulfonyl-N-{2-dimethylamino-ethyl}-amino)-phenylamino]-1-(2,3-dihydrobenzofuran-5-yl)-methylen}-6-cyano-2-indolinon
(443) 3-(*Z*)-{1-[4-(Dimethylaminomethyl)-phenylamino]-1-(imidazo[1,2-a]pyridin-7-yl)-methylen}-6-cyano-2-indolinon
(444) 3-(*Z*)-{1-[4-(N-Methyl-N-{1-methylpiperazin-4-yl-methylcarbonyl}-amino)-phenylamino]-1-(imidazo[1,2-a]pyridin-7-yl)-methylen}-6-cyano-2-indolinon
(445) 3-(*Z*)-{1-[4-(N-Methansulfonyl-N-{2-dimethylamino-ethyl}-amino)-phenylamino]-1-(imidazo[1,2-a]pyridin-7-yl)-methylen}-6-cyano-2-indolinon
(446) 3-(*Z*)-{1-[4-(Dimethylaminomethyl)-phenylamino]-1-(imidazo[1,2-a]pyridin-6-yl)-methylen}-6-cyano-2-indolinon
(447) 3-(*Z*)-{1-[4-(N-Methyl-N-{1-methylpiperazin-4-yl-methylcarbonyl}-amino)-phenylamino]-1-(imidazo[1,2-a]pyridin-6-yl)-methylen}-6-cyano-2-indolinon
(448) 3-(*Z*)-{1-[4-(N-Methansulfonyl-N-{2-dimethylamino-ethyl}-amino)-phenylamino]-1-(imidazo[1,2-a]pyridin-6-yl)-methylen}-6-cyano-2-indolinon
(449) 3-(*Z*)-{1-[4-(Dimethylaminomethyl)-phenylamino]-1-(2,3-dihydrobenzofuran-6-yl)-methylen}-6-methoxycarbonyl-2-indolinon
(450) 3-(*Z*)-{1-[4-(N-Methyl-N-{1-methylpiperazin-4-yl-methylcarbonyl}-amino)-phenylamino]-1-(2,3-dihydrobenzofuran-6-yl)-methylen}-6-methoxycarbonyl-2-indolinon
(451) 3-(*Z*)-{1-[4-(N-Methansulfonyl-N-{2-dimethylamino-ethyl}-amino)-phenylamino]-1-(2,3-dihydrobenzofuran-6-yl)-methylen}-6-methoxycarbonyl-2-indolinon
(452) 3-(*Z*)-{1-[4-(Dimethylaminomethyl)-phenylamino]-1-(2,3-dihydrobenzofuran-5-yl)-methylen}-6-methoxycarbonyl-2-indolinon
(453) 3-(*Z*)-{1-[4-(N-Methyl-N-{1-methylpiperazin-4-yl-methylcarbonyl}-amino)-phenylamino]-1-(2,3-dihydrobenzofuran-5-yl)-methylen}-6-methoxycarbonyl-2-indolinon
(454) 3-(*Z*)-{1-[4-(N-Methansulfonyl-N-{2-dimethylamino-ethyl}-amino)-phenylamino]-1-(2,3-dihydrobenzofuran-5-yl)-methylen}-6-methoxycarbonyl-2-indolinon
(455) 3-(*Z*)-{1-[4-(Dimethylaminomethyl)-phenylamino]-1-(imidazo[1,2-a]pyridin-7-yl)-methylen}-6-methoxycarbonyl-2-indolinon
(456) 3-(*Z*)-{1-[4-(N-Methyl-N-{1-methylpiperazin-4-yl-methylcarbonyl}-amino)-phenylamino]-1-(imidazo[1,2-a]pyridin-7-yl)-methylen}-6-methoxycarbonyl-2-indolinon
(457) 3-(*Z*)-{1-[4-(N-Methansulfonyl-N-{2-dimethylamino-ethyl}-amino)-phenylamino]-1-(imidazo[1,2-a]pyridin-7-yl)-methylen}-6-methoxycarbonyl-2-indolinon
(458) 3-(*Z*)-{1-[4-(Dimethylaminomethyl)-phenylamino]-1-(imidazo[1,2-a]pyridin-6-yl)-methylen}-6-methoxycarbonyl-2-indolinon
(459) 3-(*Z*)-{1-[4-(N-Methyl-N-{1-methylpiperazin-4-yl-methylcarbonyl}-amino)-phenylamino]-1-(imidazo[1,2-a]pyridin-6-yl)-methylen}-6-methoxycarbonyl-2-indolinon
(460) 3-(*Z*)-{1-[4-(N-Methansulfonyl-N-{2-dimethylamino-ethyl}-amino)-phenylamino]-1-(imidazo[1,2-a]pyridin-6-yl)-methylen}-6-methoxycarbonyl-2-indolinon

## Patentansprüche

1. Verbindungen der allgemeinen Formel I 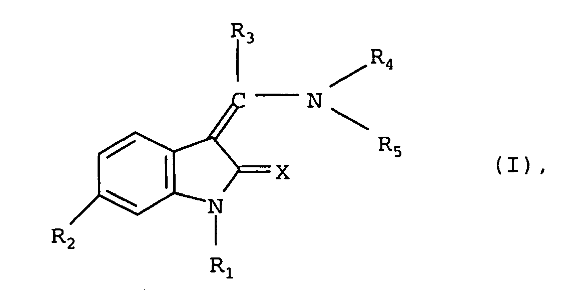 in denen
X ein Sauerstoffatom bedeutet,
R₁ und R₅ jeweils ein Wasserstoffatom bedeutet,
R₂ ein Fluor-, Brom- oder Chloratom bedeutet,
R₃ eine 3,4-Methylendioxy-1-phenyl-, 3,4-Ethylendioxy-1-phenyl-, Chinoxalin-6-yl-, Benzimidazol-5-yl-, 2-Methylbenzimidazol-5-yl- oder 1-Methyl-benzimidazol-5-yl-gruppe bedeutet, und
R₄ eine in 4-Position durch die Gruppe R₆ substituierte Phenylgruppe bedeutet, die zusätzlich in 3-Position durch ein Fluor- oder Chloratom oder eine Methoxygruppe substituiert sein kann, wobei
R₆ eine 1-(C₁₋₂-Alkyl)-imidazol-2-yl-gruppe,
eine 3,5-Dimethyl-pyrazol-1-yl-gruppe,
eine Pyrrolid-2-on-1-ylgruppe,
oder eine durch die Gruppe R₇ substituierte Methylgruppe bedeutet, wobei
R₇ eine Methylamino-, Ethylamino-, Isobutylamino-, Di-(C₁₋₂-alkyl)-amino-, N-(2-Hydroxyethyl)-methylamino-oder N-(2-Methoxyethyl)-methylaminogruppe,
eine Pyrrolidino-, 3-Hydroxypyrrolidino-, 2-Hydroxymethyl-pyrrolidino-, 4-Hydroxypiperidino-, Morpholino-, Piperazin-1-yl- oder 1-Methyl-piperazin-4-yl-gruppe, oder
eine 1,2,4-Triazol-1-yl-, 1,2,3-Triazol-1-yl- oder 1,2,3-Triazol-2-yl-gruppe bedeutet, oder
R₆ eine N-Acetyl-methylamino- oder N-Methoxyacetyl-methylaminogruppe,
eine Gruppe der Formel
-CO-R₈,
in der R₈ eine in 4-Stellung gegebenenfalls durch eine Methylgruppe substituierte Piperazino- oder Perhydro-1,4-diazepinogruppe bedeutet,
eine 4-Methyl-piperazin-1-yl-carbonyl-methyl-gruppe,
eine Gruppe der Formel
-CO-NR₉R₁₀,
in der
R₉ eine Methyl-, Cyanomethyl- oder Ethylgruppe, und
R₁₀ eine Methyl-, 1-Methylpiperidin-4-yl-, 2-Methylamino-ethyl-, 2-Dimethylamino-ethyl- oder 3-Dimethylamino-propylgruppe bedeuten,
eine Gruppe der Formel
-N(R₁₅)-CO-(CH₂)ₛ-NMe₂,
in der
s eine der Zahlen 1 oder 2, und
R₁₅ eine Methyl- oder Ethylgruppe oder, sofern n die Zahl 2 darstellt, auch eine 3-Pyridylgruppe bedeuten,
eine Gruppe der Formel
-N(R₁₅')-CO-(CH₂)ₛ-H,
in der
s eine der Zahlen 1 oder 2 und
R₁₅' eine 2-(Dimethylamino)-ethyl- oder 3-(Dimethylamino)-propylgruppe bedeuten,
eine Gruppe der Formel
-N(Me)-CO-(CH₂)ₛ-R₁₆',
in der
s eine der Zahlen 1 oder 2, und
R₁₆' eine Dimethylaminogruppe, oder, sofern s die Zahl 1 darstellt, auch eine 4-(C₁₋₂-Alkyl)-piperazin-1-yl-gruppe bedeuten,
eine Gruppe der Formel
-N(R₁₇)-SO₂-R₁₈,
in der a) R₁₇ eine Dimethylaminoethylgruppe und R₁₈ eine Methyl-, Ethyl- oder Propylgruppe bedeutet, oder
in der b) R₁₇ und R₁₈ jeweils eine Methylgruppe bedeuten,
eine Gruppe der Formel
-SO₂-N(R₂₀)-(CH₂)ᵤ-NMe₂,
in der
R₂₀ ein Wasserstoffatom oder eine Methylgruppe, und
u eine der Zahlen 2 oder 3 bedeuten,
eine Gruppe der Formel
-SO₂-R₂₆,
in der
R₂₆ eine Methylgruppe oder eine 2-Di-(C₁₋₂-alkyl)-amino-ethylgruppe bedeutet, oder
eine 2-Di-(C₁₋₂-alkyl)-amino-ethoxy-gruppe bedeutet,
wobei die in den vorstehend genannten Resten enthaltenen Dialkylaminogruppen zwei gleiche oder zwei unterschiedliche Alkylgruppen enthalten können,
deren Tautomere, deren Diastereomere, deren Enantiomere, deren Gemische und deren Salze.

2. Folgende Verbindungen der allgemeinen Formel I gemäß Anspruch 1:
(a) 3-(*Z*)-{1-[4-(Dimethylaminomethyl)-phenylamino]-1-(3,4-methylendioxyphenyl)-methylen}-6-chlor-2-indolinon
(b) 3-(*Z*)-{1-(4-[*N*-Methyl-*N*-(4-methylpiperazin-1-yl-methylcarbonyl)-amino]-phenylamino)-1-(chinoxalin-6-yl)-methylen}-6-chlor-2-indolinon
(c) 3-(*Z*)-{1-[4-(*N*-Ethyl-*N*-methyl-aminomethyl)-phenylamino]-1-(3,4-methylendioxyphenyl)-methylen}-6-chlor-2-indolinon
(d) 3-(*Z*)-{1-[4-(N-Methyl-N-{2-(dimethylamino)-ethyl-carbonyl}-amino)-phenylamino]-1-(3,4-ethylendioxyphenyl)-methylen}-6-fluor-2-indolinon
(e) 3-(*Z*)-{1-[4-(1,2,4-Triazol-1-yl-methyl)-phenylamino]-1-(1-methylbenzimidazol-5-yl)-methylen}-6-fluor-2-indolinon
(f) 3-(*Z*)-{1-[4-(Dimethylaminomethyl)-phenylamino]-1-(1-methyl-benzimidazol-5-yl)-methylen}-6-chlor-2-indolinon
(g) 3-(*Z*)-{1-[4-(Dimethylaminomethyl)-phenylamino]-1-(3,4-ethylendioxyphenyl)-methylen}-6-chlor-2-indolinon
(h) 3-(*Z*)-{1-(4-[*N*-Methansulfonyl-*N*-(2-dimethylaminoethyl)-amino]-phenylamino)-1-(3,4-ethylendioxyphenyl)-methylen}-6-chlor-2-indolinon
(k) 3-(*Z*)-{1-(4-[*N*-Acetyl-*N*-(2-dimethylaminoethyl)-amino]-phenylamino)-1-(3,4-ethylendioxyphenyl)-methylen}-6-fluor-2-indolinon
(l) 3-(*Z*)-{1-[4-(Ethylaminomethyl)-phenylamino]-1-(3,4-ethylendioxyphenyl)-methylen}-6-fluor-2-indolinon
(m) 3-(*Z*)-{1-(4-[*N*-Acetyl-*N*-(3-dimethylaminopropyl)-amino]-phenylamino)-1-(3,4-ethylendioxyphenyl)-methylen}-6-chlor-2-indolinon
(n) 3-(*Z*)-{1-(4-[*N*-Propionyl-*N*-(3-dimethylaminopropyl)-amino]-phenylamino)-1-(3,4-ethylendioxyphenyl)-methylen}-6-chlor-2-indolinon
(o) 3-(*Z*)-{1-(4-[*N*-Propionyl-*N*-(2-dimethylaminoethyl)-amino]-phenylamino)-1-(3,4-ethylendioxyphenyl)-methylen}-6-chlor-2-indolinon
(p) 3-(*Z*)-{1-(4-[*N*-Acetyl-*N*-(2-dimethylaminoethyl)-amino]-phenylamino)-1-(3,4-ethylendioxyphenyl)-methylen}-6-chlor-2-indolinon
(q) 3-(*Z*)-{1-(4-[4-Methylpiperazin-1-yl-methyl]-phenylamino)-1-(3,4-methylendioxyphenyl)-methylen}-6-chlor-2-indolinon
(y) 3-(*Z*)-{1-[4-(Dimethylaminocarbonyl)-phenylamino]-1-(1-methylbenzimidazol-5-yl)-methylen}-6-fluor-2-indolinon
(z) 3-(*Z*)-{1-(4-[*N*-Propionyl-*N*-(3-dimethylaminopropyl)-amino]-phenylamino)-1-(3,4-ethylendioxyphenyl)-methylen}-6-fluor-2-indolinon
(aa) 3-(*Z*)-{1-(4-[*N*-Propionyl-*N*-(2-dimethylaminoethyl)-amino]-phenylamino)-1-(3,4-ethylendioxyphenyl)-methylen}-6-fluor-2-indolinon
(ab) 3-(*Z*)-{1-(4-[*N*-Methansulfonyl-*N*-(2-dimethylaminoethyl)-amino]-phenylamino)-1-(3,4-ethylendioxyphenyl)-methylen}-6-fluor-2-indolinon
(az) 3-(*Z*)-{1-(4-[*N*-Methyl-*N*-(4-methylpiperazin-1-yl-methylcarbonyl)-amino]-phenylamino)-1-(3,4-methylendioxyphenyl)-methylen}-6-chlor-2-indolinon
(be) 3-(*Z*)-{1-(4-[*N*-Methyl-*N*-(4-methylpiperazin-1-yl-methylcarbonyl)-amino]-phenylamino)-1-(3,4-methylendioxyphenyl)-methylen}-6-fluor-2-indolinon und
(bf) 3-(*Z*)-{1-(4-[*N*-Methyl-*N*-(4-methylpiperazin-1-yl-methylcarbonyl)-amino]-phenylamino)-1-(3,4-methylendioxyphenyl)-methylen}-6-brom-2-indolinon,
deren Tautomere, deren Diastereomere, deren Enantiomere, deren Gemische, und deren Salze.

3. Physiologisch verträgliche Salze der Verbindungen gemäß den Ansprüchen 1 oder2.

4. Arzneimittel, enthaltend eine Verbindung nach einem der Ansprüche 1 oder 2 oder ein Salz gemäß Anspruch 3 neben gegebenenfalls einem oder mehreren inerten Trägerstoffen und/oder Verdünnungsmitteln.

5. Verwendung einer Verbindung nach einem der Ansprüche 1 oder 2 oder ein Salz gemäß Anspruch 3 zur Herstellung eines Arzneimittels, welches zur Behandlung von exzessiven oder anomalen Zellproliferationen geeignet ist.

6. Verfahren zur Herstellung eines Arzneimittels gemäß Anspruch 4 **dadurch gekennzeichnet, daß** auf nichtchemischem Wege eine Verbindung nach einem der Ansprüche 1 oder 2 oder ein Salz gemäß Anspruch 3 in einen oder mehrere inerte Trägerstoffe und/oder Verdünnungsmittel eingearbeitet wird.

7. Verfahren zur Herstellung der Verbindungen gemäß Anspruch 1 **dadurch gekennzeichnet, daß**
a. Eine Verbindung der allgemeinen Formel 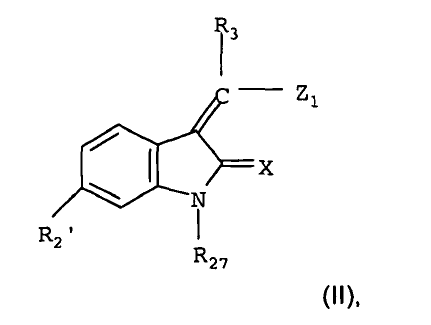 in der
X und R₃ wie in Anspruch 1 erwähnt definiert sind,
R₂' die für R₂ in Anspruch 1 erwähnten Bedeutungen besitzt,
R₂₇ ein Wasserstoffatom oder eine Schutzgruppe für das Stickstoffatom der Lactamgruppe, wobei einer der Reste R₂' und R₂₇ auch eine gegebenenenfalls über einen Spacer gebildete Bindung an eine Festphase darstellen kann und der andere der Reste R₂' und R₂₇ die vorstehend erwähnten Bedeutungen besitzt, und Z₁ ein Halogenatom, eine Hydroxy-, Alkoxy- oder Aryl-alkoxygruppe bedeuten,
mit einem Amin der allgemeinen Formel 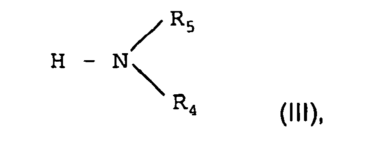 in der
R₄ und R₅ wie in Anspruch 1 erwähnt definiert sind, umgesetzt wird,
und erforderlichenfalls anschließend eine verwendete Schutzgruppe für das Stickstoffatom der Lactamgruppe oder eine so erhaltene Verbindung von einer Festphase abgespalten wird, oder
b. Zur Herstellung einer Verbindung der allgemeinen Formel 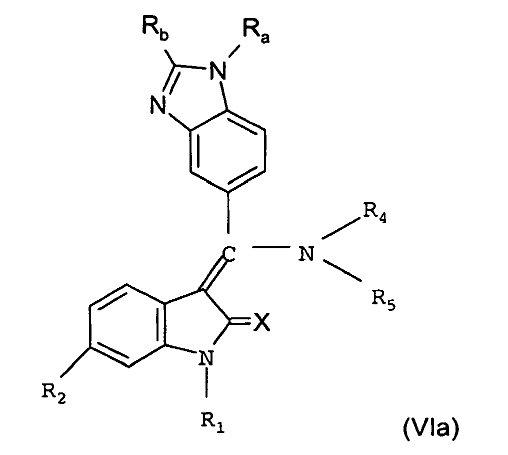 oder 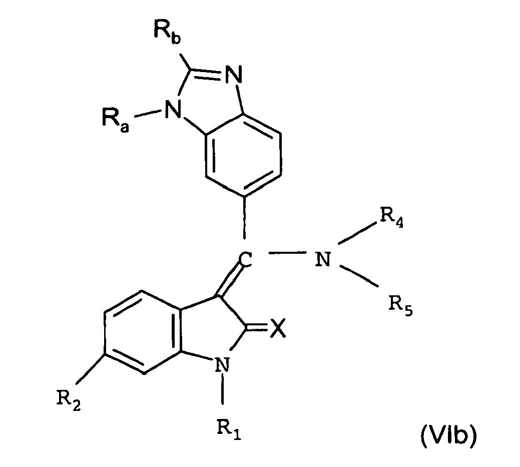 in der
X, R₁, R₂, R₄ und R₅ wie in Anspruch 1 erwähnt definiert sind und
Rₐ und R_{b} jeweils unabhängig voneinander ein Wasserstofftom, eine C₁₋₃-Alkylgruppe oder eine Carboxy-C₁₋₃-alkylgruppe sein können:
eine Verbindung der allgemeinen Formel 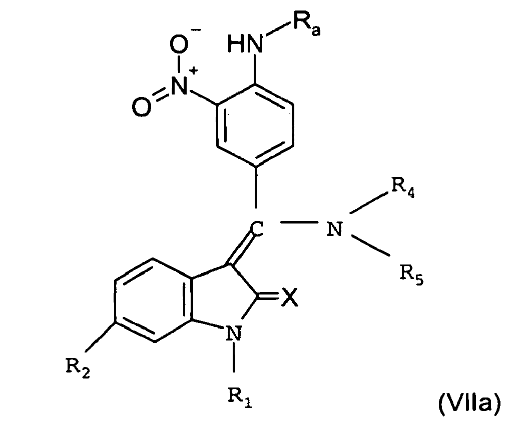 beziehungsweise 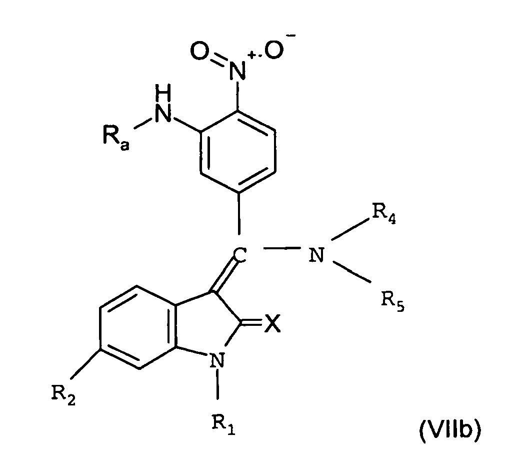 in der
X, R₁, R₂, R₄ und R₅ wie in Anspruch 1 erwähnt definiert sind und
Rₐ ein Wasserstoffatom, eine C₁₋₃-Alkylgruppe oder eine gegebenenfalls geschützte Carboxy-C₁₋₃-alkylgruppe sein kann, reduziert wird, oder
c. Zur Herstellung einer Verbindung der allgemeinen Formel I, in der R₄ eine durch die Gruppe R₆ in 3- oder 4-Position substituierte Phenylgruppe, die zusätzlich wie in Anspruch 1 beschrieben substituiert sein kann, darstellt, und
R₆ eine durch R₇ substituierte C₁₋₃-Alkylgruppe bedeutet, wobei
R₇ eine Heteroarylgruppe, welche über einen Iminostickstoff gebunden ist,
eine Hydroxy- oder C₁₋₃-Alkoxygruppe,
eine Amino-, C₁₋₇-Alkylamino-, Di-(C₁₋₇-alkyl)-amino-, N-(C₁₋₇-Alkyl)-allylamino-, Phenylamino-, N-Phenyl-C₁₋₃-alkyl-amino-, Phenyl-C₁₋₃-alkylamino-, N-(C₁₋₃-Alkyl)-phenyl-C₁₋₃-alkylamino- oder Di-(phenyl-C₁₋₃-alkyl)-aminogruppe,
eine Allylaminogruppe, in der ein oder zwei vinylische Wasserstoffatome jeweils durch eine Methylgruppe ersetzt sein können,
eine ω-Hydroxy-C₂₋₃-alkyl-amino-, N-(C₁₋₃-Alkyl)-ω-hydroxy-C₂₋₃-alkyl-amino-, Di-(ω-hydroxy-C₂₋₃-alkyl)-amino-, N-(C₁₋₃-Alkyl)-[ω-(C₁₋₃-alkoxy)-C₂₋₃-alkyl]-amino-, Di-(ω-(C₁₋₃-alkoxy)-C₂₋₃-alkyl)-amino- oder N-(Dioxolan-2-yl)-C₁₋₃-alkyl-aminogruppe,
eine C₁₋₃-Alkyl-carbonylamino-C₂₋₃-alkyl-amino- oder C₁₋₃-Alkyl-carbonyl-amino-C₂₋₃-alkyl-N-(C₁₋₃-alkyl)-aminogruppe,
eine Pyridylaminogruppe,
eine C₁₋₃-Alkylsulfonylamino-, N-(C₁₋₃-Alkyl)-C₁₋₃-alkylsulfonylamino-, C₁₋₃-Alkylsulfonylamino-C₂₋₃-alkyl-amino- oder C₁₋₃-Alkylsulfonylamino-C₂₋₃-alkyl-N-(C₁₋₃-alkyl)-aminogruppe,
eine Hydroxycarbonyl-C₁₋₃-alkylamino- oder N-(C₁₋₃-Alkyl)-hydroxycarbonyl-C₁₋₃-alkyl-aminogruppe,
eine Guanidinogruppe, in der ein oder zwei Wasserstoffatome jeweils durch eine C₁₋₃-Alkylgruppe ersetzt sein können,
eine 2-Pyrrolidon-1-yl-gruppe, in der die der Carbonylgruppe benachbarte Methylengruppe durch ein Sauerstoffatom oder durch eine -NH- oder -N(C₁₋₃-Alkyl)- Gruppe ersetzt sein kann,
eine Gruppe der Formel
-N(R₁₁)-CO-(CH₂)ₚ-R₁₂ ,
in der
R₁₁ ein Wasserstoffatom oder eine Allyl-, C₁₋₃-Alkyl-, C₁₋₃-Alkyl-amino-C₂₋₃-alkyl- oder Di-(C₁₋₃-alkyl)-amino-C₂₋₃-alkylgruppe,
p eine der Zahlen 0, 1, 2 oder 3 und
R₁₂ eine Amino-, C₁₋₄-Alkylamino-, Allylamino-, Di-allyl-amino-, Di-(C₁₋₄-alkyl)-amino-, Phenylamino-, N-(C₁₋₄-Alkyl)-phenylamino-, Benzylamino-, N-(C₁₋₄-Alkyl)-benzylamino-, C₁₋₄-Alkoxy- oder 2,5-Dihydropyrrol-1-yl-gruppe oder
eine 4- bis 7-gliedrige Cycloalkyleniminogruppe,
wobei jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Allyl)-, -N(Phenyl)-, -N(C₁₋₃-Alkyl-carbonyl)- oder -N(Benzoyl)- Gruppe ersetzt sein kann,
oder, sofern n eine der Zahlen 1, 2 oder 3 darstellt, auch ein Wasserstoffatom bedeuten,
eine Gruppe der Formel
-N(R₁₃)-(CH₂)_{q}-(CO)ᵣR₁₄ ,
in der
R₁₃ ein Wasserstoffatom oder eine C₁₋₃-Alkyl-, Allyl-, C₁₋₃-Alkylcarbonyl-, Arylcarbonyl-, Pyridylcarbonyl-, Phenyl-C₁₋₃-alkyl-carbonyl-,
C₁₋₃-Alkylsulfonyl-, Arylsulfonyl- oder Phenyl-C₁₋₃-alkylsulfonylgruppe,
q eine der Zahlen 1, 2, 3 oder 4,
r die Zahl 1 oder, sofern q eine der Zahlen 2, 3 oder 4 ist, auch die Zahl 0 und
R₁₄ eine Hydroxy-, Amino-, C₁₋₄-Alkylamino-, Di-(C₁₋₄-alkyl)-amino-, Phenylamino-, N-(C₁₋₄-Alkyl)-phenylamino-, Benzylamino-, N-(C₁₋₄-Alkyl)-benzylamino-, C₁₋₄-Alkoxy- oder C₁₋₃-Alkoxy-C₁₋₃-alkoxygruppe,
eine in 1-Position gegebenenfalls durch eine C₁₋₃-Alkylgruppe substituierte Di-(C₁₋₄-alkyl)-amino-C₁₋₃-alkylaminogruppe oder
eine 4- bis 7-gliedrige Cycloalkyleniminogruppe,
wobei der Cycloalkylenteil mit einem Phenylring kondensiert sein kann oder
jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Phenyl)-, -N(C₁₋₃-Alkyl-carbonyl)- oder -N(Benzoyl)- Gruppe ersetzt sein kann, bedeuten,
eine C₄₋₇-Cycloalkylamino-, C₄₋₇-Cycloalkyl-C₁₋₃-alkylamino- oder C₄₋₇-Cycloalkenylaminogruppe, in der die Position 1 des Rings nicht an der Doppelbindung beteiligt ist und wobei die vorstehend genannten Gruppen jeweils zusätzlich am Aminstickstoffatom durch eine C₅₋₇-Cycloalkyl-, C₂₋₄-Alkenyl- oder C₁₋₄-Alkylgruppe substituiert sein können,
eine 2,5-Dihydro-pyrrol-1-yl-gruppe oder
eine 4- bis 7-gliedrige Cycloalkyleniminogruppe, in der
der Cycloalkylenteil mit einer Phenylgruppe oder mit einer gegebenenfalls durch ein Fluor-, Chlor-, Brom- oder lodatom, durch eine Nitro-, C₁₋₃-Alkyl-, C₁₋₃-Alkoxy- oder Aminogruppe substituierten Oxazolo-, Imidazolo-, Thiazolo-, Pyridino-, Pyrazino- oder Pyrimidinogruppe kondensiert sein kann oder/und
ein oder zwei Wasserstoffatome jeweils durch eine C₁₋₃-Alkyl-, C₃₋₇-Cycloalkyl-, Hydroxy-, C₁₋₃-Alkoxy-, Hydroxy-C₁₋₃-alkyl- oder C₁₋₃-Alkoxy-C₁₋₃-alkyl- oder Phenylgruppe ersetzt sein können oder/und
die Methylengruppe in Position 3 einer 5-gliedrigen Cycloalkyleniminogruppe durch eine Hydroxy-, Hydroxy-C₁₋₃-alkyl-, C₁₋₃-Alkoxy- oder C₁₋₃-Alkoxy-C₁₋₃-alkylgruppe substituiert sein kann,
jeweils die Methylengruppe in Position 3 oder 4 einer 6- oder 7-gliedrigen Cycloalkyleniminogruppe durch eine Hydroxy-, Hydroxy-C₁₋₃-alkyl-, C₁₋₃-Alkoxy-, C₁₋₃-Alkoxy-C₁₋₃-alkyl-, Carboxy-, C₁₋₄-Alkoxycarbonyl-, Aminocarbonyl-, C₁₋₃-Alkylaminocarbonyl-, Di-(C₁₋₃-alkyl)-aminocarbonyl-, Phenyl-C₁₋₃-alkylamino- oder N-(C₁₋₃-Alkyl)-phenyl-C₁₋₃-alkylaminogruppe substituiert oder
die Methylengruppe in 4-Position einer 6- oder 7-gliedrigen Cycloalkyleniminogruppe durch ein Sauerstoff- oder Schwefelatom, durch eine Sulfinyl-, Sulfonyl-, -NH-, -N(C₁₋₃-Alkyl)-, -N(Allyl)-, -N(Phenyl)-, -N(Phenyl-C₁₋₃-alkyl)-, -N(C₁₋₃-Alkyl-carbonyl)-, -N(C₁₋₄-Hydroxy-carbonyl)-, -N(C₁₋₄-Alkoxy-carbonyl)-, - N(Benzoyl)- oder -N(Phenyl-C₁₋₃-alkyl-carbonyl)- Gruppe ersetzt sein kann,
wobei eine mit einem Imino-Stickstoffatom der Cycloalkyleniminogruppe verknüpfte Methylengruppe durch eine Carbonyl- oder Sulfonylgruppe ersetzt sein kann oder in einer 5- bis 7-gliedrigen monocyclischen oder mit einer Phenylgruppe kondensierten Cycloalkyleniminogruppe beide mit dem Imino-Stickstoffatom verknüpften Methylengruppen jeweils durch eine Carbonylgruppe ersetzt sein können,
bedeutet:
eine Verbindung der allgemeinen Formel 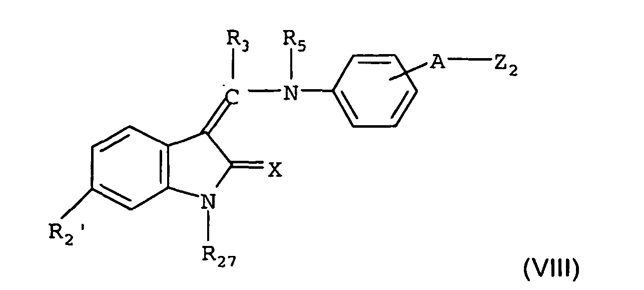 in der
R₃, R₅ und X wie in Anspruch 1 erwähnt definiert sind,
R₂' die für R₂ in Anspruch 1 erwähnten Bedeutungen besitzt,
R₂₇ ein Wasserstoffatom oder eine Schutzgruppe für das Stickstoffatom der Lactamgruppe, wobei einer der Reste R₂' und R₂₇ auch eine gegebenenenfalls über einen Spacer gebildete Bindung an eine Festphase darstellen kann und der andere der Reste R₂' und R₂₇ die vorstehend erwähnten Bedeutungen besitzt,
A eine C₁₋₃-Alkylgruppe und
Z₂ eine Austrittsgruppe darstellt, mit einem Amin der allgemeinen Formel
H-R₇. (IX),
in der
R_{7'} die vorstehend für R₇ genannten Bedeutungen besitzt, umgesetzt wird und erforderlichenfalls anschließend eine verwendete Schutzgruppe für das Stickstoffatom der Lactamgruppe oder eine so erhaltene Verbindung von einer Festphase abgespalten wird, und/oder
gewünschtenfalls anschließend eine so erhaltene Verbindung der allgemeinen Formel I, die eine Alkoxycarbonylgruppe enthält, mittels Hydrolyse in eine entsprechende Carboxyverbindung übergeführt wird oder
eine so erhaltene Verbindung der allgemeinen Formel I, die eine Amino- oder Alkylaminogruppe enthält, mittels reduktiver Alkylierung in eine entsprechende Alkylamino- oder Dialkylaminoverbindung übergeführt wird oder
eine so erhaltene Verbindung der allgemeinen Formel I, die eine Amino- oder Alkylaminogruppe enthält, mittels Acylierung oder Sulfonierung in eine entsprechende Acyl- oder Sulfonylverbindung übergeführt wird oder
eine so erhaltene Verbindung der allgemeinen Formel I, die eine Carboxygruppe enthält, mittels Veresterung oder Amidierung in eine entsprechende Ester- oder Aminocarbonylverbindung übergeführt wird oder
eine so erhaltene Verbindung der allgemeinen Formel I, die eine Cycloalkyleniminogruppe enthält, in der eine Methylengruppe durch ein Schwefelatom ersetzt ist, mittels Oxidation in eine entsprechende Sulfinyl- oder Sulfonylverbindung übergeführt wird, oder
eine so erhaltene Verbindung der allgemeinen Formel I, die eine Nitrogruppe enthält, mittels Reduktion in eine entsprechende Aminoverbindung übergeführt wird oder
eine so erhaltene Verbindung der allgemeinen Formel I, in der R₄ eine durch eine Amino-, Alkylamino-, Aminoalkyl- oder N-Alkyl-aminogruppe substituierte Phenylgruppe darstellt, mittels Umsetzung mit einem entsprechenden Cyanat, Isocyanat oder Carbamoylhalogenid in eine entsprechende Harnstoffverbindung der allgemeinen Formel I übergeführt wird oder
eine so erhaltene Verbindung der allgemeinen Formel I, in der R₄ eine durch eine Amino-, Alkylamino-, Aminoalkyl- oder N-Alkyl-aminogruppe substituierte Phenylgruppe darstellt, mittels Umsetzung mit einer entsprechenden die Amidinogruppe übertragenden Verbindung oder durch Umsetzung mit einem entsprechenden Nitril in eine entsprechende Guanidinoverbindung der allgemeinen Formel I übergeführt wird oder
erforderlichenfalls ein während den Umsetzungen zum Schutze von reaktiven Gruppen verwendeter Schutzrest abgespalten wird oder
gewünschtenfalls anschließend eine so erhaltene Verbindung der allgemeinen Formel I in ihre Stereoisomere aufgetrennt wird oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

## Claims

1. Compounds of general formula 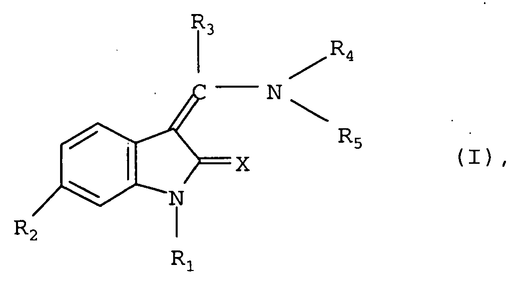 wherein
X denotes an oxygen atom,
R₁ and R₅ in each case denote a hydrogen atom,
R₂ denotes a fluorine, bromine or chlorine atom,
R₃ denotes a 3,4-methylenedioxy-1-phenyl, 3,4-ethylenedioxy-1-phenyl, quinoxalin-6-yl, benzimidazol-5-yl, 2-methylbenzimidazol-5-yl or 1-methyl-benzimidazol-5-yl group and
R₄ denotes a phenyl group substituted in the 4 position by the group R₆ which may additionally be substituted in the 3 position by a fluorine or chlorine atom or a methoxy group, wherein
R₆ denotes a 1-(C₁₋₂-alkyl)-imidazol-2-yl group,
a 3,5-dimethyl-pyrazol-1-yl group,
a pyrrolid-2-on-1-yl group,
or a methyl group substituted by the group R₇, wherein
R₇ denotes a methylamino, ethylamino, isobutylamino, di-(C₁₋₂-alkyl)-amino, N-(2-hydroxyethyl)-methylamino or N-(2-methoxyethyl)-methylamino group, a pyrrolidino, 3-hydroxypyrrolidino, 2-hydroxymethyl-pyrrolidino, 4-hydroxypiperidino, morpholino, piperazin-1-yl or 1-methyl-piperazin-4-yl group or
a 1,2,4-triazol-1-yl, 1,2,3-triazol-1-yl or 1,2,3-triazol-2-yl group, or
R₆ denotes an N-acetyl-methylamino or N-methoxyacetyl-methylamino group,
a group of formula
-CO-R₈ ,
wherein
R₈ denotes a piperazino or perhydro-1,4-diazepino group optionally substituted by a methyl group in the 4 position,
a 4-methyl-piperazin-1-yl-carbonyl-methyl group,
a group of formula
-CO-NR₉R₁₀
wherein
R₉ denotes a methyl, cyanomethyl or ethyl group and
R₁₀ denotes a methyl, 1-methylpiperidin-4-yl, 2-methylamino-ethyl, 2-dimethylamino-ethyl or 3-dimethylamino-propyl group,
a group of formula
-N(R₁₅)-CO-(CH₂)ₛ-NMe₂ ,
wherein
s denotes one of the numbers 1 or 2 and
R₁₅ denotes a methyl or ethyl group or, if n denotes the number 2, it may also represent a 3-pyridyl group,
a group of formula
-N(R₁₅')-CO-(CH₂)ₛ-H ,
wherein
s denotes one of the numbers 1 or 2 and
R₁₅' denotes a 2-(dimethylamino)-ethyl or 3-(dimethylamino)-propyl group,
or a group of formula
-N(Me)-CO-(CH₂)ₛ-R₁₆' ,
wherein
s denotes one of the numbers 1 or 2 and
R₁₆' denotes a dimethylamino group, or, if s denotes the number 1, it may also represent a 4-(C₁₋₂-alkyl)-piperazin-1-yl group,
a group of formula
-N(R₁₇)-SO₂-R₁₈ ,
wherein a) R₁₇ denotes a dimethylaminoethyl group and R₁₈ denotes a methyl, ethyl or propyl group or
wherein b) R₁₇ and R₁₈ each represent a methyl group,
a group of formula
-SO₂-N(R₂₀)-(CH₂)ᵤ-NMe₂ ,
wherein
R₂₀ denotes a hydrogen atom or a methyl group and
u denotes one of the numbers 2 or 3,
a group of formula
-SO₂-R₂₆ ,
wherein
R₂₆ denotes a methyl group or a 2-di-(C₁₋₂-alkyl)-amino-ethyl group,
or a 2-di-(C₁₋₂-alkyl)-amino-ethoxy group,
while the dialkylamino groups contained in the abovementioned groups may contain two identical or two different alkyl groups,
the tautomers, diastereomers, enantiomers, the mixtures thereof and the salts thereof.

2. The following compounds of general formula I according to claim 1:
(a) 3-(*Z*)-{1-[4-(dimethylaminomethyl)-phenylamino]-1-(3,4-methylenedioxyphenyl)-methylene}-6-chloro-2-indolinone
(b) 3-(*Z*)-{1-(4-[*N*-methyl-*N*-(4-methylpiperazin-l-yl-methylcarbonyl)-amino]-phenylamino)-1-(quinoxalin-6-yl)-methylene}-6-chloro-2-indolinone
(c) 3-(*Z*)-{1-[4-(*N*-ethyl-*N*-methyl-aminomethyl)-phenylamino]-1-(3,4-methylenedioxyphenyl)-methylene}-6-chloro-2-indolinone
(d) 3-(*Z*)-{1-[4-(N-methyl-N-{2-(dimethylamino)-ethyl-carbonyl}-amino)-phenylamino]-1-(3,4-ethylenedioxyphenyl)-methylene}-6-fluoro-2-indolinone
(e) 3-(*Z*)-{1-[4-(1,2,4-triazol-1-yl-methyl)-phenylamino]-1-(1-methyl-benzimidazol-5-yl )-methylene}-6-fluoro-2-indolinone
(f) 3-(*Z*)-{1-[4-(dimethylaminomethyl)-phenylamino]-1-(1-methyl-benzimidazol-5-yl)-methylene}-6-chloro-2-indolinone
(g) 3-(*Z*)-{1-[4-(dimethylaminomethyl)-phenylamino]-1-(3,4-ethylenedioxyphenyl)-methylene}-6-chloro-2-indolinone
(h) 3-(*Z*)-{1-(4-[*N*-methanesulphonyl-*N*-(2-dimethylaminoethyl)-amino]-phenylamino)-1-(3,4-ethylenedioxyphenyl)-methylene}-6-chloro-2-indolinone
(k) 3-(*Z*)-{1-(4-[*N*-acetyl-*N*-(2-dimethylaminoethyl)-amino]-phenylamino)-1-(3,4-ethylenedioxyphenyl)-methylene}-6-fluoro-2-indolinone
(l) 3-(*Z*)-{1-[4-(ethylaminomethyl)-phenylamino]-1-(3,4-ethylenedioxyphenyl)methylene}-6-fluoro-2-indolinone
(m) 3-(*Z*)-{1-(4-[*N*-acetyl-*N*-(3-dimethylaminopropyl)-amino]-phenylamino)-1-(3,4-ethylenedioxyphenyl)-methylene}-6-chloro-2-indolinone
(n) 3-(*Z*)-{1-(4-[*N*-propionyl-*N*-(3-dimethylaminopropyl)-amino]-phenylamino)-1-(3,4-ethylenedioxyphenyl)-methylene}-6-chloro-2-indolinone
(o) 3-(*Z*)-{1-(4-[*N*-propionyl-*N*-(2-dimethylaminoethyl)-amino]-phenylamino)-1-(3,4-ethylenedioxyphenyl)-methylene}-6-chloro-2-indolinone
(p) 3-(*Z*)-{1-(4-[*N*-acetyl-*N*-(2-dimethylaminoethyl)-amino]-phenylamino)-1-(3,4-ethylenedioxyphenyl)-methylene}-6-chloro-2-indolinone
(q) 3-(*Z*)-{1-(4-[4-methylpiperazin-1-yl-methyl]-phenylamino)-1-(3,4-methylenedioxyphenyl)-methylene}-6-chloro-2-indolinone
(y) 3-(Z)-{1-[4-(dimethylaminocarbonyl)-phenylamino]-1-(1-methyl-benzimidazol-5-yl)-methylene}-6-fluoro-2-indolinone
(z) 3-(*Z*)-{1-(4-[*N*-propionyl-*N*-(3-dimethylaminopropyl)-amino]-phenylamino)-1-(3,4-ethylenedioxyphenyl)-methylene}-6-fluoro-2-indolinone
(aa) 3-(*Z*)-{1-(4-[*N*-propionyl-*N*-(2-dimethylaminoethyl)-amino]-phenylamino)-1-(3,4-ethylenedioxyphenyl)-methylene}-6-fluoro-2-indolinone
(ab) 3-(*Z*)-{1-(4-[*N*-methanesulphonyl-*N*-(2-dimethylaminoethyl)-amino]-phenylamino)-1-(3,4-ethylenedioxyphenyl)-methylene}-6-fluoro-2-indolinone
(az) 3-(*Z*)-{1-(4-[*N*-methyl-*N*-(4-methylpiperazin-1-yl-methylcarbonyl)-amino]-phenylamino)-1-(3,4-methylenedioxyphenyl)-methylene}-6-chloro-2-indolinone
(be) 3-(*Z*)-{1-(4-[*N*-methyl-*N*-(4-methylpiperazin-1-yl-methylcarbonyl)-amino]-phenylamino)-1-(3,4-methylenedioxyphenyl)-methylene}-6-fluoro-2-indolinone and
(bf) 3-(*Z*)-{1-(4-[*N*-methyl-*N*-(4-methylpiperazin-1-yl-methylcarbonyl)-amino]-phenylamino)-1-(3,4-methylenedioxyphenyl)-methylene}-6-bromo-2-indolinone,
the tautomers, diastereomers, enantiomers, the mixtures thereof and the salts thereof.

3. Physiologically acceptable salts of the compounds according to claims 1 or 2.

4. Pharmaceutical compositions containing a compound according to at least one of claims 1 or 2 or a salt according to claim 3 optionally together with one or more inert carriers and/or diluents.

5. Use of a compound according to at least one of claims 1 or 2 or a salt according to claim 3 for preparing a pharmaceutical composition which is suitable for treating excessive or abnormal cell proliferation.

6. Process for preparing a pharmaceutical composition according to claim 4,
**characterised in that** a compound according to at least one of claims 1 or 2 or a salt according to claim 3 is incorporated by a non-chemical method in one or more inert carriers and/or diluents.

7. Process for preparing the compounds according to claim 1, **characterised in that**
a. a compound of general formula 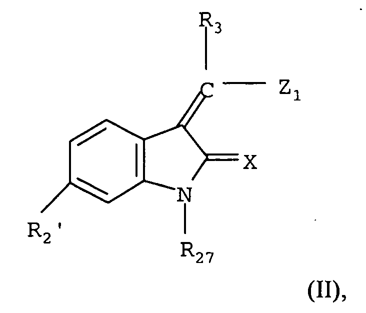 wherein
X and R₃ are defined as in claim 1,
R₂' has the meanings given for R₂ in claim 1,
R₂₇ denotes a hydrogen atom or a protective group for the nitrogen atom of the lactam group, while one of the groups R₂' and R₂₇ may also denote a bond to a solid phase optionally formed via a spacer and the other of the groups R₂' and R₂₇ is as hereinbefore defined, and Z₁ denotes a halogen atom, a hydroxy, alkoxy or aryl-alkoxy group,
is reacted with an amine of general formula 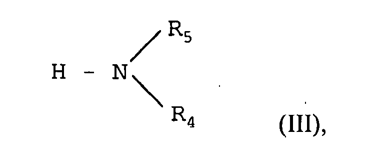 wherein
R₄ and R₅ are defined as in claim 1,
and if necessary any protective group used for the nitrogen atom of the lactam group or a compound thus obtained is subsequently cleaved from a solid phase, or
b. in order to prepare a compound of general formula 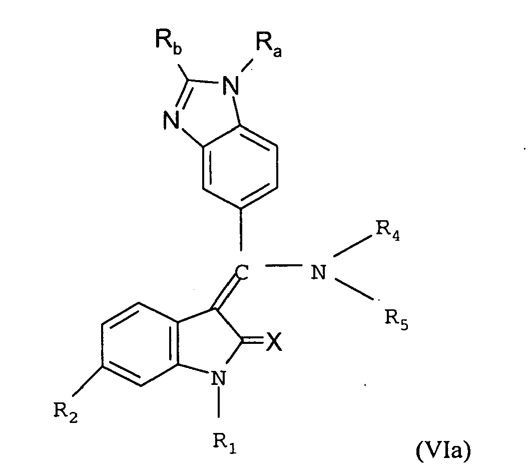 or 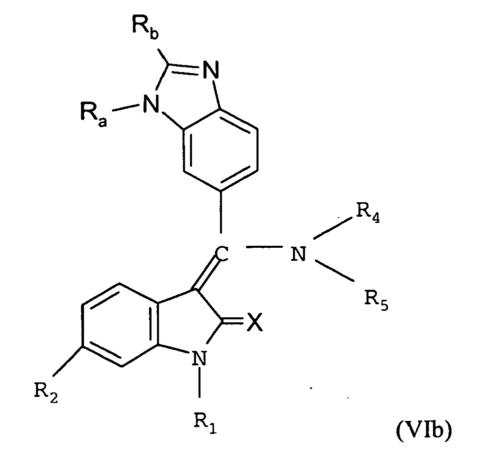 wherein
X, R₁, R₂, R₄ and R₅ are defined as in claim 1 and
Rₐ and R_{b} independently of one another may each be a hydrogen atom, a C₁₋₃-alkyl group or a carboxy-C₁₋₃-alkyl group:
a compound of general formula 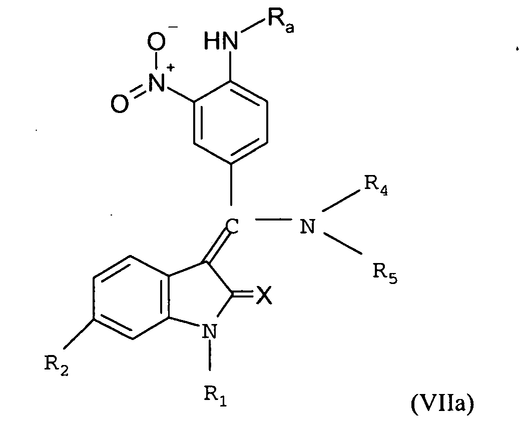
or 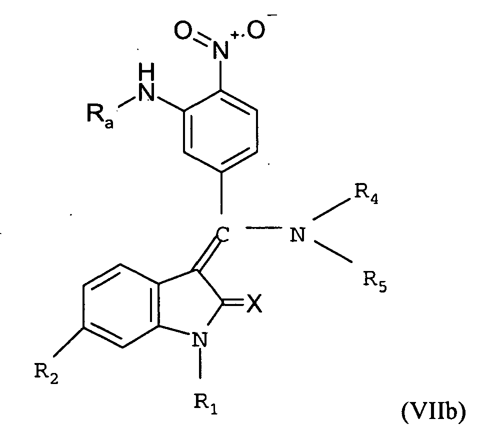
wherein
X, R₁, R₂, R₄ and R₅ are defined as in claim 1 and
Rₐ may be a hydrogen atom, a C₁₋₃-alkyl group or an optionally protected carboxy-C₁₋₃-alkyl group,
is reduced, or
c. in order to prepare a compound of general formula I wherein R₄ denotes a phenyl group substituted by the group R₆ in the 3- or 4-position which may additionally be substituted as described in claim 1, and R₆ denotes a C₁₋₃-alkyl group substituted by R₇, where
R₇ denotes a heteroaryl group which is bound via an imino-nitrogen,
a hydroxy or C₁₋₃-alkoxy group,
an amino, C₁₋₇-alkylamino, di-(C₁₋₇-alkyl)-amino, N-(C₁₋₇-alkyl)-allylamino, phenylamino, N-phenyl-C₁₋₃-alkyl-amino, phenyl-C₁₋₃-alkylamino, N-(C₁₋₃-alkyl)-phenyl-C₁₋₃-alkylamino or di-(phenyl-C₁₋₃-alkyl)-amino group,
an allylamino group wherein one or two vinylic hydrogen atoms may each be replaced by a methyl group,
a ω-hydroxy-C₂₋₃-alkyl-amino, N-(C₁₋₃-alkyl)-ω-hydroxy-C₂₋₃-alkyl-amino, di-(ω-hydroxy-C₂₋₃-alkyl)-amino, N-(C₁₋₃-alkyl)-[ω-(C₁₋₃-alkoxy)-C₂₋₃-alkyl]-amino, di-(ω-(C₁₋₃-alkoxy)-C₂₋₃-alkyl)-amino or N-(dioxolan-2-yl)-C₁₋₃-alkyl-amino group,
a C₁₋₃-alkyl-carbonylamino-C₂₋₃-alkyl-amino or C₁₋₃-alkyl-carbonylamino-C₂₋₃-alkyl-N-(C₁₋₃-alkyl)-amino group,
a pyridylamino group,
a C₁₋₃-alkylsulphonylamino, N-(C₁₋₃-alkyl)-C₁₋₃-alkylsulphonylamino, C₁₋₃-alkylsulphonylamino-C₂₋₃-alkyl-amino or C₁₋₃-alkylsulphonylamino-C₂₋₃-alkyl-N-(C₁₋₃-alkyl)-amino group,
a hydroxycarbonyl-C₁₋₃-alkylamino or N-(C₁₋₃-alkyl)-hydroxycarbonyl-C₁₋₃-alkyl-amino group,
a guanidino group wherein one or two hydrogen atoms may each be replaced by a C₁₋₃-alkyl group,
a 2-pyrrolidon-1-yl group wherein the methylene group adjacent to the carbonyl group may be replaced by an oxygen atom or by an -NH or -N(C₁₋₃-alkyl) group,
a group of formula
-N(R₁₁)-CO-(CH₂)ₚ-R₁₂ ,
wherein
R₁₁ denotes a hydrogen atom or an allyl, C₁₋₃-alkyl, C₁₋₃-alkyl-amino-C₂₋₃-alkyl or di-(C₁₋₃-alkyl)-amino-C₂₋₃-alkyl group,
p denotes one of the numbers 0, 1, 2 or 3 and
R₁₂ denotes an amino, C₁₋₄-alkylamino, allylamino, di-allyl-amino, di-(C₁₋₄-alkyl)-amino, phenylamino, N-(C₁₋₄-alkyl)-phenylamino, benzylamino, N-(C₁₋₄-alkyl)-benzylamino, C₁₋₄-alkoxy or 2,5-dihydropyrrol-1-yl group or
a 4- to 7-membered cycloalkyleneimino group,
while in each case the methylene group in the 4 position of a 6- or 7-membered cycloalkyleneimino group may be replaced by an oxygen or sulphur atom, by a sulphinyl, sulphonyl, -NH, -N(C₁₋₃-alkyl),
-N(allyl), -N(phenyl), -N(C₁₋₃-alkyl-carbonyl) or -N(benzoyl) group, or, if n denotes one of the numbers 1, 2 or 3, may also represent a hydrogen atom,
a group of formula
-N(R₁₃)-(CH₂)_{q}-(CO)ᵣ-R₁₄ ,
wherein
R₁₃ denotes a hydrogen atom or a C₁₋₃-alkyl, allyl, C₁₋₃-alkyl-carbonyl, arylcarbonyl, pyridylcarbonyl, phenyl-C₁₋₃-alkyl-carbonyl, C₁₋₃-alkylsulphonyl, arylsulphonyl or phenyl-C₁₋₃-alkylsulphonyl group,
q denotes one of the numbers 1, 2, 3 or 4,
r denotes the number 1 or, if q is one of the numbers 2, 3 or 4, may also represent the number 0 and
R₁₄ denotes a hydroxy, amino, C₁₋₄-alkylamino, di-(C₁₋₄-alkyl)-amino, phenylamino, N-(C₁₋₄-alkyl)-phenylamino, benzylamino,
N-(C₁₋₄-alkyl)-benzylamino, C₁₋₄-alkoxy or C₁₋₃-alkoxy-C₁₋₃-alkoxy group,
a di-(C₁₋₄-alkyl)-amino-C₁₋₃-alkylamino group optionally substituted in the 1 position by a C₁₋₃-alkyl group or
a 4- to 7-membered cycloalkyleneimino group,
while the cycloalkylene moiety may be fused to a phenyl ring or
in each case the methylene group in the 4 position of a 6- or 7-membered cycloalkyleneimino group may be replaced by an oxygen or sulphur atom or by a sulphinyl, sulphonyl, -NH, -N(C₁₋₃-alkyl), -N(phenyl), -N(C₁₋₃-alkyl-carbonyl) or -N(benzoyl) group,
a C₄₋₇-cycloalkylamino, C₄₋₇-cycloalkyl-C₁₋₃-alkylamino or C₄₋₇-cyclo-alkenylamino group wherein position 1 of the ring is not involved in the double bond and the abovementioned groups may each additionally be substituted at the amino-nitrogen atom by a C₅₋₇-cycloalkyl, C₂₋₄-alkenyl or C₁₋₄-alkyl group,
a 2,5-dihydro-pyrrol-1-yl group or
a 4- to 7-membered cycloalkyleneimino group wherein
the cycloalkylene moiety may be fused with a phenyl group or with an oxazolo, imidazolo, thiazolo, pyridino, pyrazino or pyrimidino group optionally substituted by a fluorine, chlorine, bromine or iodine atom or by a nitro, C₁₋₃-alkyl, C₁₋₃-alkoxy or amino group and/or
one or two hydrogen atoms may each be replaced by a C₁₋₃-alkyl, C₃₋₇-cycloalkyl, hydroxy, C₁₋₃-alkoxy, hydroxy-C₁₋₃-alkyl or C₁₋₃-alkoxy-C₁₋₃-alkyl or phenyl group and/or
the methylene group in position 3 of a 5-membered cycloalkyleneimino group may be substituted by a hydroxy, hydroxy-C₁₋₃-alkyl, C₁₋₃-alkoxy or C₁₋₃-alkoxy-C₁₋₃-alkyl group,
in each case the methylene group in the 3 or 4 position of a 6- or 7-membered cycloalkyleneimino group may be substituted by a hydroxy, hydroxy-C₁₋₃-alkyl, C₁₋₃-alkoxy, C₁₋₃-alkoxy-C₁₋₃-alkyl, carboxy, C₁₋₄-alkoxycarbonyl, aminocarbonyl, C₁₋₃-alkylaminocarbonyl,
di-(C₁₋₃-alkyl)-aminocarbonyl, phenyl-C₁₋₃-alkylamino or N-(C₁₋₃-alkyl)-phenyl-C₁₋₃-alkylamino group or
the methylene group in the 4 position of a 6- or 7-membered cycloalkyleneimino group may be replaced by an oxygen or sulphur atom, by a sulphinyl, sulphonyl, -NH, -N(C₁₋₃-alkyl), -N(allyl), -N(phenyl), -N(phenyl-C₁₋₃-alkyl), -N(C₁₋₃-alkyl-carbonyl), -N(C₁₋₄-hydroxy-carbonyl), -N(C₁₋₄-alkoxy-carbonyl), -N(benzoyl) or -N(phenyl-C₁₋₃-alkyl-carbonyl) group,
wherein a methylene group linked to an imino-nitrogen atom of the cycloalkyleneimino group may be replaced by a carbonyl or sulphonyl group or in a 5- to 7-membered monocyclic cycloalkyleneimino group or a cycloalkyleneimino group fused to a phenyl group the two methylene groups linked to the imino-nitrogen atom may each be replaced by a carbonyl group:
a compound of general formula 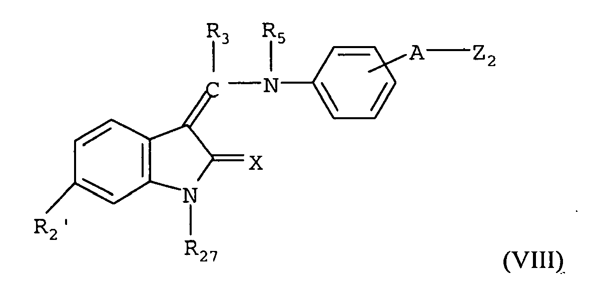
wherein
R₃, R₅ and X are defined as in claim 1,
R₂' has the meanings given for R₂ in claim 1,
R₂₇ denotes a hydrogen atom or a protective group for the nitrogen atom of the lactam group, while one of the groups R₂' and R₂₇ may also denote a bond to a solid phase optionally formed via a spacer and the other of the groups R₂' and R₂₇ is as hereinbefore defined,
A denotes a C₁₋₃-alkyl group and
Z₂ denotes a leaving group,
is reacted with an amine of general formula
H-R_{7'} (IX),
wherein
R_{7'} has the meanings given for R₇ hereinbefore, and
if necessary any protecting group used for the nitrogen atom of the lactam group is subsequently cleaved or a compound thus obtained is cleaved from a solid phase, and/or
subsequently, if desired, a compound of general formula I thus obtained which contains an alkoxycarbonyl group is converted by hydrolysis into a corresponding carboxy compound or
a compound of general formula I thus obtained which contains an amino or alkylamino group is converted by reductive alkylation into a corresponding alkylamino or dialkylamino compound, or
a compound of general formula I thus obtained which contains an amino or alkylamino group is converted by acylation or sulphonylation into a corresponding acyl or sulphonyl compound, or
a compound of general formula I thus obtained which contains a carboxy group is converted by esterification or amidation into a corresponding ester or aminocarbonyl compound, or
a compound of general formula I thus obtained which contains a cycloalkyleneimino group wherein a methylene group is replaced by a sulphur atom is converted by oxidation into a corresponding sulphinyl or sulphonyl compound, or
a compound of general formula I thus obtained which contains a nitro group is converted by reduction into a corresponding amino compound, or
a compound of general formula I thus obtained wherein R₄ denotes a phenyl group substituted by an amino, alkylamino, aminoalkyl or N-alkylamino group is converted by reacting with a corresponding cyanate, isocyanate or carbamoyl halide into a corresponding urea compound of general formula I or
a compound of general formula I thus obtained wherein R₄ denotes a phenyl group substituted by an amino, alkylamino, aminoalkyl or N-alkylamino group is converted by reacting with a corresponding compound which transfers the amidino group or by reacting with a corresponding nitrile into a corresponding guanidino compound of general formula I or
if necessary any protecting group used to protect reactive groups during the reactions is cleaved or
subsequently, if desired, a compound of general formula I thus obtained is resolved into the stereoisomers thereof or
a compound of general formula I thus obtained is converted into the salts thereof, more particularly, for pharmaceutical use, into the physiologically acceptable salts thereof with an inorganic or organic acid or base.

## Revendications

1. Composés de formule générale I 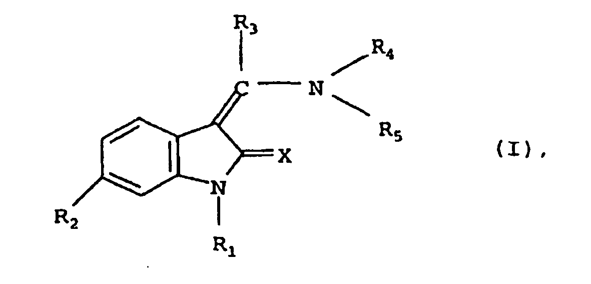 où
X représente un atome d'oxygène,
R₁ et R₅ représentent chacun un atome d'hydrogène,
R₂ représente un atome de fluor, de brome ou de chlore,
R₃ représente un groupe 3,4-méthylènedioxy-1-phényle, 3,4-éthylènedioxy-1-phényle, quinoxalin-6-yle, benzimidazol-5-yle, 2-méthylbenzimidazol-5-yle ou 1-méthyl-benzimidazol-5-yle et
R₄ représente un groupe phényle substitué en position 4 par le groupe R₆ qui peut être substitué en outre en position 3 par un atome de fluor ou de chlore ou un groupe méthoxy, où
R₆ représente un groupe 1-(C₁₋₂-alkyl)-imidazol-2-yle,
un groupe 3,5-diméthyl-pyrazol-1-yle,
un groupe pyrrolid-2-on-1-yle,
ou un groupe méthyle substitué par le groupe R₇, où
R₇ représente un groupe méthylamino, éthylamino, isobutylamino, di-(C₁₋₂-alkyl)-amino, N-(2-hydroxyéthyl)-méthylamino ou N-(2-méthoxyéthyl)-méthylamino,
un groupe pyrrolidino, 3-hydroxypyrrolidino, 2-hydroxyméthyl-pyrrolidino, 4-hydroxypipéridino, morpholino, pipérazin-1-yle ou 1-méthyl-pipérazin-4-yle, ou
un groupe 1,2,4-triazol-1-yle, 1,2,3-triazol-1-yle ou 1,2,3-triazol-2-yle, ou
R₆ représente un groupe N-acétyl-méthylamino ou N-méthoxyacétyl-méthylamino,
un groupe de formule
-CO-R₈,
où R₈ représente un groupe pipérazino ou perhydro-1,4-diazépino éventuellement substitué en position 4 par un groupe méthyle,
un groupe 4-méthyl-pipérazin-1-yl-carbonyl-méthyle,
un groupe de formule
-CO-NR₉R₁₀,
où
R₉ représente un groupe méthyle, cyanométhyle ou éthyle, et
R₁₀ représente un groupe méthyle, 1-méthylpipéridin-4-yle, 2-méthylaminoéthyle, 2-diméthylamino-éthyle ou 3-diméthylamino-propyle,
un groupe de formule
-N(R₁₅)-CO-(CH₂)ₛ-NMe₂,
où
s représente l'un des nombres 1 ou 2, et
R₁₅ représente un groupe méthyle ou éthyle, ou, dans la mesure où n représente le nombre 2, également un groupe 3-pyridyle,
un groupe de formule
-N(R₁₅')-CO-(CH₂)ₛ-H,
où
s représente l'un des nombres 1 ou 2 et
R₁₅' représente un groupe 2-(diméthylamino)-éthyle ou 3-(diméthylamino)-propyle,
un groupe de formule
-N(Me)-CO-(CH₂)ₛ-R₁₆',
où
s représente l'un des nombres 1 ou 2 et
R₁₆' représente un groupe diméthylamino ou, dans la mesure où s représente le nombre 1, également un groupe 4-(C₁₋₂-alkyl)-pipérazin-1-yle,
un groupe de formule
-N(R₁₇)-SO₂-R₁₈,
où a) R₁₇ représente un groupe diméthylaminoéthyle et R₁₈ représente un groupe méthyle, éthyle ou propyle, ou bien
où b) R₁₇ et R₁₈ représentent chacun un groupe méthyle, un groupe de formule
-SO₂-N(R₂₀)-(CH₂)ᵤ-NMe₂,
où
R₂₀ représente un atome d'hydrogène ou un groupe méthyle, et
u représente l'un des nombres 2 ou 3,
un groupe de formule
-SO₂-R₂₆,
où
R₂₆ représente un groupe méthyle ou un groupe 2-di-(C₁₋₂-alkyl)-aminoéthyle, ou
un groupe 2-di-(C₁₋₂-alkyl)-aminoéthoxy,
où les groupes dialkylamino contenus dans les groupements cités précédemment peuvent contenir deux groupes alkyle identiques ou deux groupes alkyle différents,
leurs tautomères, leurs diastéréoisomères, leurs énantiomères, leurs mélanges et leurs sels.

2. Composés de formule générale I selon la revendication 1 suivants :
(a) 3-(Z)-{1-[4-(diméthylaminométhyl)-phénylamino]-1-(3,4-méthylènedioxy-phényl)-méthylène}-6-chloro-2-indolinone
(b) 3-(Z)-{1-(4-[*N*-méthyl-*N*-(4-méthylpipérazin-1-yl-méthylcarbonyl)-amino]-phénylamino)-1-(quinoxalin-6-yl)-méthylène}-6-chloro-2-indolinone
(c) 3-(Z)-{1-[4-(*N*-éthyl-*N*-méthyl-aminométhyl)-phénylamino]-1-(3,4-méthylènedioxyphényl)-méthylène}-6-chloro-2-indolinone
(d) 3-(Z)-{1-[4-(*N*-méthyl-*N*-{2-(diméthylamino)-éthyl-carbonyl}-amino)-phénylamino]-1-(3,4-éthylènedioxyphényl)-méthylène}-6-fluoro-2-indolinone
(e) 3-(Z)-{1-[4-(1,2,4-triazol-1-yl-méthyl)-phénylamino]-1-(1-méthyl-benzimidazol-5-yl)-méthylène}-6-fluoro-2-indolinone
(f) 3-(Z)-{1-[4-(diméthylaminométhyl)-phénylamino]-1-(1-méthyl-benzimidazol-5yl)-méthylène}-6-chloro-2-indolinone
(g) 3-(Z)-{1-[4-(diméthylaminométhyl)-phénylamino]-1-(3,4-éthylènedioxy-phényl)méthylène}-6-chloro-2-indolinone
(h) 3-(Z)-{1-(4-[*N*-méthanesulfonyl-*N*-(2-diméthylaminoéthyl)-amino]-phénylamino)-1-(3,4-éthylènedioxyphényl)-méthylène}-6-chloro-2-indolinone
(k) 3-(Z)-{1-(4-[*N*-acétyl-*N*-(2-diméthylaminoéthyl)-amino]-phénylamino)-1-(3,4éthylènedioxyphényl)-méthylène}-6-fluoro-2-indolinone
(1) 3-(Z)-{1-[4-(éthylaminométhyl)-phénylamino]-1-(3,4-éthylènedioxyphényl)-méthylène}-6-fluoro-2-indolinone
(m) 3-(Z}-{1-(4-[*N*-acétyl-*N*-(3-diméthylaminopropyl)-amino]-phénylamino)-1-(3,4-éthylènedioxyphényl)-méthylène}-6-chloro-2-indolinone
(n) 3-(Z)-{1-(4-[*N*-propionyl-*N*-(3-diméthylaminopropyl)-amino]-phénylamino)-1-(3,4-éthylènedioxyphényl)-méthylène}-6-chloro-2-indolinone
(o) 3-(Z)-{1-(4-[*N*-propionyl-*N*-(2-diméthylaminoéthyl)-amino]-phénylamino}-1-(3,4-éthylènedioxyphényl)-méthylène}-6-chloro-2-indolinone
(p) 3-(Z)-{1-(4-[*N*-acétyl-*N*-(2-diméthylaminoéthyl)-amino]-phénylamino)-1-(3,4éthylènedioxyphényl)-méthylène}-6-chloro-2-indolinone
(q) 3-(Z)-{1-(4-[4-méthylpipérazin-1-yl-méthyl]-phénylamino)-1-(3,4-méthylène-dioxyphényl)-méthylène}-6-chloro-2-indolinone
(y) 3-(Z)-{1-[4-(diméthylaminocarbonyl)-phénylamino]-1-(1-méthyl-benzimidazol-5-yl)-méthylène}-6-fluoro-2-indolinone
(z) 3-(Z)-{1-(4-[*N*-propionyl-*N*-(3-diméthylaminopropyl)-amino]-phénylamino)-1(3,4-éthylènedioxyphényl)-méthylène}-6-fluoro-2-indolinone
(aa) 3-(Z)-{1-(4-[*N*-propionyl-*N*-(2-diméthylaminoéthyl)-amino]-phénylamino)-1-(3,4-éthylènedioxyphényl)-méthylène}-6-fluoro-2-indolinone
(ab) 3-(Z)-{1-(4-[*N*-méthanesulfonyl-*N*-(2-diméthylaminoéthyl)-amino]-phénylamino)-1-( 3,4-éthylènedioxyphényl)-méthylène}-6-fluoro-2-indolinone
(az) 3-(Z)-{1-(4-[*N*-méthyl-*N*-(4-méthylpipérazin-1-yl-méthylcarbonyl)-amino]-phénylamino)-1-(3,4-méthylènedioxyphényl)-méthylène}-6-chloro-2-indolinone
(be) 3-(Z)-{1-(4-[*N*-méthyl-*N*-(4-méthylpipérazin-1-yl-méthylcarbonyl)-amino]-phénylamino)-1-(3,4-méthylènedioxyphényl)-méthylène}-6-fluoro-2-indolinone et
(bf) 3-(Z)-{1-(4-[*N*-méthyl-*N*-(4-méthylpipérazin-1-yl-méthylcarbonyl)-amino]-phénylamino)-1-(3,4-méthylènedioxyphényl)-méthylène}-6-bromo-2-indolinone,
leurs tautomères, leurs diastéréoisomères, leurs énantiomères, leurs mélanges et leurs sels.

3. Sels physiologiquement acceptables des composés selon les revendications 1 ou 2.

4. Médicament contenant un composé selon l'une des revendications 1 ou 2 ou un sel selon la revendication 3 outre éventuellement un ou plusieurs vecteurs et/ou diluants inertes.

5. Utilisation d'un composé selon l'une des revendications 1 ou 2 ou d'un sel selon la revendication 3 pour la production d'un médicament qui convient pour le traitement de proliférations cellulaires excessives ou anormales.

6. Procédé de production d'un médicament selon la revendication 4 **caractérisé en ce que,** par voie non chimique, un composé selon l'une des revendications 1 ou 2 ou un sel selon la revendication 3 est incorporé dans un ou plusieurs vecteurs et/ou diluants inertes.

7. Procédé de production des composés selon la revendication 1 **caractérisé en ce que**
a. un composé de formule générale 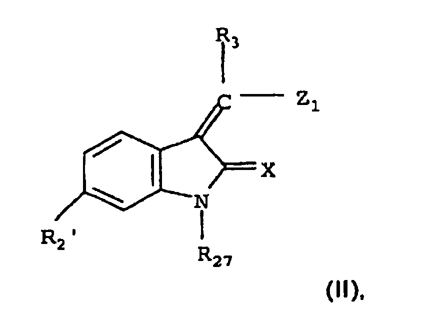 où
X et R₃ sont définis comme indiqué dans la revendication 1,
R₂' possède les significations indiquées pour R₂ dans la revendication 1,
R₂₇ représente un atome d'hydrogène ou un groupe protecteur pour l'atome d'azote du groupe lactame, où l'un des groupements R₂' et R₂₇ peut représenter aussi une liaison à une phase solide éventuellement formée via un espaceur et l'autre des groupements R₂' et R₂₇ possède les significations cités précédemment, et Z₁ représente un atome d'halogène, un groupe hydroxy, alcoxy ou aryl-alcoxy, est mis à réagir avec une amine de formule générale 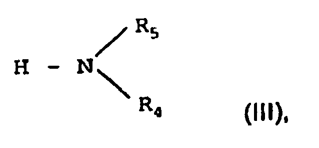 où
R₄ et R₅ sont définis comme indiqué dans la revendication 1, puis, si nécessaire, un groupe protecteur utilisé pour l'atome d'azote du groupe lactame est clivé ou un composé ainsi obtenu est clivé d'une phase solide, ou
b. pour la production d'un composé de formule générale 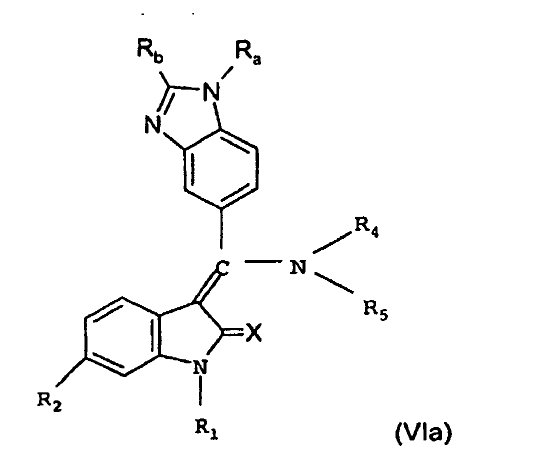 respectivement 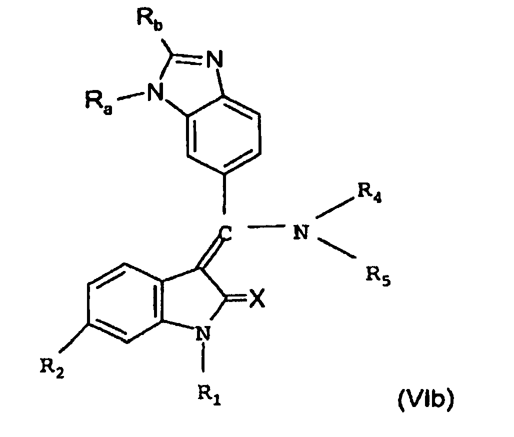 où
X, R₁, R₂, R₄ et R₅ sont définis comme indiqué dans la revendication 1 et
Rₐ et R_{b} peuvent être chacun indépendamment l'un de l'autre un atome d'hydrogène, un groupe C₁₋₃-alkyle ou un groupe carboxy-C₁₋₃-alkyle :
un composé de formule générale 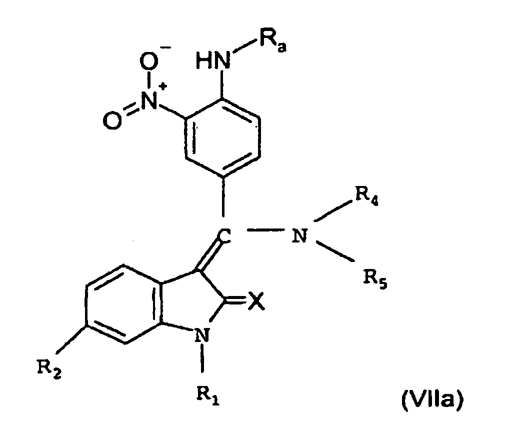
respectivement 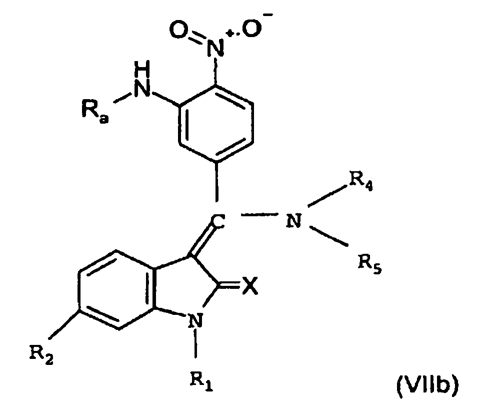
où
X, R₁, R₂, R₄ et R₅ sont définis comme indiqué dans la revendication 1 et
Rₐ peut être un atome d'hydrogène, un groupe C₁₋₃-alkyle ou un groupe carboxy-C₁₋₃-alkyle éventuellement protégé, est réduit, ou
c. pour la production d'un composé de formule générale I où R₄ représente un groupe phényle substitué par le groupe R₆ en position 3 ou 4, qui peut en outre être substitué comme décrit dans la revendication 1, et R₆ représente un groupe C₁₋₃-alkyle substitué par R₇, où
R₇ représente un groupe hétéroaryle qui est lié via un atome d'azote d'imino,
un groupe hydroxy ou C₁₋₃-alcoxy,
un groupe amino, C₁₋₇-alkylamino, di-(C₁₋₇-alkyl)-amino, N-(C₁₋₇-alkyl)-allylamino, phénylamino, N-phényl-C₁₋₃-alkyl-amino, phényl-C₁₋₃-alkylamino, N-(C₁₋₃-alkyl)-phényl-C₁₋₃-alkylamino ou di-(phényl-C₁₋₃-alkyl)-amino,
un groupe allylamino où un ou deux atomes d'hydrogène vinyliques peuvent être remplacés chacun par un groupe méthyle,
un groupe ω-hydroxy-C₂₋₃-alkyl-amino, N-(C₁₋₃-alkyl)-ω-hydroxy-C₂₋₃-alkyl-amino, di-(ω-hydroxy-C₂₋₃-alkyl)-amino, N-(C₁₋₃-alkyl)-[ω-(C₁₋₃-alcoxy)-C₂₋₃-alkyl]-amino, di-(ω-(C₁₋₃-alcoxy)-C₂₋₃-alkyl)-amino ou N-(dioxolan-2-yl)-C₁₋₃-alkyl-amino,
un groupe C₁₋₃-alkyl-carbonylamino-C₂₋₃-alkyl-amino ou C₁₋₃-alkyl-carbonyl-amino-C₂₋₃-alkyl-N-(C₁₋₃-alkyl)-amino,
un groupe pyridylamino,
un groupe C₁₋₃-alkyl-sulfonylamino, N-(C₁₋₃-alkyl)-C₂₋₃-alkylsulfonylamino, C₁₋₃-alkylsulfonylamino-C₂₋₃-alkyl-amino ou C₁₋₃-alkylsulfonylamino-C₂₋₃-alkyl-N-(C₁₋₃-alkyl)-amino,
un groupe hydroxycarbonyl-C₁₋₃-alkylamino ou N-(C₁₋₃-alkyl)hydroxy-carbonyl-C₁₋₃-alkyl-amino,
un groupe guanidino où un ou deux atomes d'hydrogène peuvent être remplacés chacun par un groupe C₁₋₃-alkyle,
un groupe 2-pyrrolidon-1-yle où le groupe méthylène voisin du groupe carbonyle peut être remplacé par un atome d'oxygène ou par un groupe -NH- ou -N(C₁₋₃-alkyl)-,
un groupe de formule
-N(R₁₁)-CO-(CH₂)ₚ-R₁₂,
où
R₁₁ représente un atome d'hydrogène ou un groupe allyle, C₁₋₃-alkyle, C₁₋₃-alkyl-amino-C₂₋₃-alkyle ou di-(C₁₋₃-alkyl)-amino-C₂₋₃-alkyle,
p représente l'un des nombres 0, 1, 2 ou 3 et
R₁₂ représente un groupe amino, C₁₋₄-alkylamino, allylamino, di-allyl-amino, di-(C₁₋₄-alkyl)-amino, phénylamino, N-(C₁₋₄-alkyl)-phénylamino, benzylamino, N-(C₁₋₄-alkyl)-benzylamino, C₁₋₄-alcoxy
ou 2,5-dihydropyrrol-1-yle ou
un groupe cycloalkylèneimino à 4 à 7 chaînons,
où dans chaque cas le groupe méthylène en position 4 d'un groupe cycloalkylèneimino à 6 ou 7 chaînons peut être remplacé par un atome d'oxygène ou de soufre, par un groupe sulfinyle, sulfonyle, -NH-, -N(C₁₋₃-alkyl)-, -N(allyl)-, -N(phényl)-, -N(C₁₋₃-alkyl-carbonyl)- ou -N(benzoyl)-,
ou bien, dans la mesure où n représente l'un des nombres 1, 2 ou 3, représente aussi un atome d'hydrogène,
un groupe de formule
-N(R₁₃)-(CH₂)_{q}-(CO)ᵣ-R₁₄,
où
R₁₃ représente un atome d'hydrogène ou un groupe C₁₋₃-alkyle, allyle, C₁₋₃-alkyl-carbonyle, arylcarbonyle, pyridylcarbonyle, phényl-C₁₋₃-alkyl-carbonyle, C₁₋₃-alkylsulfonyle, arylsulfonyle ou phényl-C₁₋₃-alkylsulfonyle,
q représente l'un des nombres 1, 2, 3 ou 4,
r représente le nombre 1 ou, dans la mesure où q est l'un des nombres 2, 3
ou 4, également le nombre 0 et
R₁₄ représente un groupe hydroxy, amino, C₁₋₄-alkylamino, di-(C₁₋₄-alkyl)-amino, phénylamino, N-(C₁₋₄-alkyl)-phénylamino, benzylamino, N-(C₁₋₄-alkyl)-benzylamino, C₁₋₄-alcoxy ou C₁₋₃-alcoxy- C₁₋₃-alcoxy,
un groupe di-(C₁₋₄-alkyl)-amino-C₁₋₃-alkylamino éventuellement substitué en position 1 par un groupe C₁₋₃-alkyle ou
un groupe cycloalkylèneimino à 4 à 7 chaînons,
où la partie cycloalkylène peut être condensée avec un cycle phényle ou dans chaque cas le groupe méthylène en position 4 d'un groupe cycloalkylèneimino à 6 ou 7 chaînons peut être remplacé par un atome d'oxygène ou de soufre, par un groupe sulfinyle, sulfonyle, -NH-, -N(C₁₋₃-alkyl)-, -N(phényl)-, -N(C₁₋₃-alkyl-carbonyl)- ou -N(benzoyl)-,
un groupe C₄₋₇-cycloalkylamino, C₄₋₇-cycloalkyl-C₁₋₃-alkylamino ou C₄₋₇-cycloalcénylamino où la position 1 du cycle ne participe pas à la double liaison et
où les groupes cités précédemment peuvent en outre être substitués chacun sur l'atome d'azote d'amine par un groupe C₅₋₇-cycloalkyle, C₂₋₄-alcényle ou C₁₋₄-alkyle,
un groupe 2,5-dihydro-pyrrol-1-yle ou
un groupe cycloalkylèneimino à 4 à 7 chaînons où
la partie cycloalkylène peut être condensée avec un groupe phényle ou avec un groupe oxazolo, imidazolo, thiazolo, pyridino, pyrazino ou pyrimidino éventuellement substitué par un atome de fluor, de chlore, de brome ou d'iode, par un groupe nitro, C₁₋₃-alkyle, C₁₋₃-alcoxy ou amino et/ou
un ou deux atomes d'hydrogène peuvent être remplacés chacun par un groupe C₁₋₃-alkyle, C₃₋₇-cycloalkyle, hydroxy, C₁₋₃-alcoxy, hydroxy-C₁₋₃-alkyle ou C₁₋₃-alcoxy-C₁₋₃-alkyle ou phényle et/ou
le groupe méthylène en position 3 d'un groupe cycloalkylèneimino à 3 à 5 chaînons peut être substitué par un groupe hydroxy, hydroxy-C₁₋₃-alkyle,
C₁₋₃-alcoxy ou C₁₋₃-alcoxy-C₁₋₃-alkyle,
dans chaque cas le groupe méthylène en position 3 ou 4 d'un groupe cycloalkylèneimino à 6 ou 7 chaînons peut être substitué par un groupe hydroxy, hydroxy-C₁₋₃-alkyle, C₁₋₃-alcoxy, C₁₋₃-alcoxy-C₁₋₃-alkyle, carboxy, C₁₋₄-alcoxycarbonyle, aminocarbonyle, C₁₋₃-alkylaminocarbonyle, di-(C₁₋₃-alkyl)-aminocarbonyle, phényl-C₁₋₃-alkylamino ou N-(C₁₋₃-alkyl)-phényl-C₁₋₃-alkylamino ou
le groupe méthylène en position 4 d'un groupe cycloalkylèneimino à 6 ou 7 chaînons peut être remplacé par un atome d'oxygène ou de soufre, par un groupe sulfinyle, sulfonyle, -NH-, -N(C₁₋₃-alkyl)-, -N(allyl)-, -N(phényl)-, - N(phényl-C₁₋₃-alkyl)-, -N(C₁₋₃-alkyl-carbonyl)-, N(C₁₋₄-hydroxy-carbonyl)-, -N(C₁₋₄-alcoxy-carbonyl)-, -N(benzoyl)- ou -N(phényl-C₁₋₃-alkyl-carbonyl)-,
où un groupe méthylène lié à un atome d'azote d'imino du groupe cycloalkylèneimino peut être remplacé par un groupe carbonyle ou sulfonyle, ou les deux groupes méthylène liés à l'atome d'azote d'imino dans un groupe cycloalkylèneimino monocyclique à 5 à 7 chaînons ou condensé avec un groupe phényle peuvent être remplacés chacun par un groupe carbonyle :
un composé de formule générale 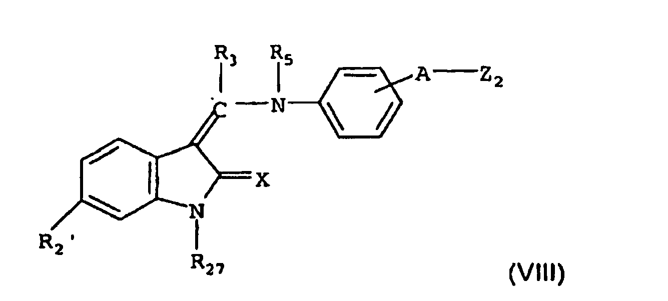
où
R₃, R₅ et X sont définis comme indiqué dans la revendication 1,
R₂' possède les significations indiquées pour R₂ dans la revendication 1,
R₂₇ représente un atome d'hydrogène ou un groupe protecteur pour l'atome d'azote du groupe lactame, où l'un des groupements R₂' et R₂₇ peut aussi représenter une liaison à une phase solide éventuellement formée via un espaceur et l'autre des groupements R₂' et R₂₇ possède les significations citées précédemment,
A représente un groupe C₁₋₃-alkyle et
Z₂ représente un groupe partant, est mis à réagir avec une amine de formule générale
H-R_{7'} (IX)
où
R_{7'} possède les significations citées précédemment pour R₇, puis, si nécessaire, un groupe protecteur utilisé pour l'atome d'azote du groupe lactame ou un composé ainsi obtenu est clivé d'une phase solide, et/ou
si on le souhaite, un composé de formule générale I ainsi obtenu, qui contient un groupe alcoxycarbonyle, est ensuite converti par hydrolyse en un composé carboxy correspondant ou
un composé de formule générale I ainsi obtenu, qui contient un groupe amino ou alkylamino, est converti par alkylation réductrice en un composé alkylamino ou dialkylamino correspondant ou
un composé de formule générale I ainsi obtenu, qui contient un groupe amino ou alkylamino, est converti par acylation ou sulfonation en un composé acyle ou sulfonyle correspondant ou
un composé de formule générale I ainsi obtenu, qui contient un groupe carboxy, est converti par estérification ou amidation en un composé ester ou aminocarbonyle correspondant ou
un composé de formule générale I ainsi obtenu, qui contient un groupe cycloalkylèneimino, où un groupe méthylène est remplacé par un atome de soufre, est converti par oxydation en un composé sulfinyle ou sulfonyle correspondant,
ou
un composé de formule générale I ainsi obtenu, qui contient un groupe nitro, est converti par réduction en un composé amino correspondant ou
un composé de formule générale I ainsi obtenu, où R₄ représente un groupe phényle substitué par un groupe amino, alkylamino, aminoalkyle ou N-alkylamino, est converti par réaction avec un cyanate, isocyanate ou halogénure de carbamoyle correspondant en un composé urée de formule générale I correspondant ou
un composé de formule générale I ainsi obtenu, où R₄ représente un groupe phényle substitué par un groupe amino, alkylamino, aminoalkyle ou N-alkylamino, est converti par réaction avec un composé transférant le groupe amidino correspondant ou par réaction avec un nitrile correspondant en un composé guanidino de formule générale I correspondant ou
si nécessaire un groupement protecteur utilisé pendant les réactions pour la protection de groupes réactifs est clivé ou
si on le souhaite, un composé de formule générale I ainsi obtenu est ensuite séparé en ses stéréoisomères ou
un composé de formule générale I ainsi obtenu est converti en ses sels, en particulier pour l'utilisation pharmaceutique en ses sels physiologiquement acceptables avec un acide ou base inorganique ou organique.
